# EUROPEAN PATENT SPECIFICATION

(11) **EP 3 807 274 B1**
(45) Date of publication and mention of the grant of the patent: **27.07.2022**
(21) Application number: 19769018.3
(22) Date of filing: 23.08.2019
(51) Int. Cl.: C07D 471/04, A61P 35/00, A61K 31/437, C07F 7/08

(54) **4-SUBSTITUTED PYRROLO[2,3-B]PYRIDINE AS ERBB MODULATORS USEFUL FOR TREATING CANCER**
4-SUBSTITUIERTE PYRROLO[2,3-B]PYRIDINE ALS ERBB-MODULATOREN ZUR BEHANDLUNG VON KREBS
4-SUBSTITUÉ PYRROLO[2,3-B] PYRIDINE EN TANT QUE MODULATEURS ERBB UTILES POUR LE TRAITEMENT DU CANCER

(30) Priority: 23.08.2018 EP 18190603
(43) Date of publication of application: 21.04.2021
(73) Proprietor: Lead Discovery Center GmbH, 44227 Dortmund (DE)
(72) Inventor: SCHULTZ-FADEMRECHT, Carsten, 44309 Dortmund (DE); KLEBL, Bert, 44227 Dortmund (DE); NUSSBAUMER, Peter, 44227 Dortmund (DE); DEGENHART, Carsten, 44225 Dortmund (DE); BAUMANN, Matthias, 44267 Dortmund (DE)
(74) Representative: Arth, Hans-Lothar
(86) International application number: PCT/EP2019/072564
(87) International publication number: WO 2020/039060

(56) References cited:
- WO-A1-2013/106792
- WO-A1-2016/133935
- WO-A1-2017/066193

### SEQUENCE LISTING

<110> Lead Discovery Center GmbH
<120> 4-Substituted pyrrolo[2,3-b]pyridine as ErbB modulators useful for treating cancer
<130> LDC-P03722WO
<160> 7
<170> PatentIn version 3.5
<210> 1
   <211> 345
   <212> DNA
   <213> Artificial Sequence
<220>
   <223> EGFR p.D770_N771insSVD mutation in BaF3
<400> 1
   ttcaaaaaga tcaaagtgct gggctccggt gcgttcggca cggtgtataa gggactctgg        60
   atcccagaag gtgagaaagt taaaattccc gtcgctatca aggaattaag agaagcaaca       120
   tctccgaaag ccaacaagga aatcctcgat gaagcctacg tgatggccag cgtggactca       180
   gtagacaacc cccacgtgtg ccgcctgctg ggcatctgcc tcacctccac cgtgcagctc       240
   atcacgcagc tcatgccctt cggctgcctc ctggactatg tccgggaaca caaagacaat       300
   attggctccc agtacctgct caactggtgt gtgcagatcg caaag                       345
<210> 2
   <211> 345
   <212> DNA
   <213> Artificial Sequence
<220>
   <223> EGFR p.H773_V774insNPH mutation in BaF3
<400> 2
   ttcaaaaaga tcaaagtgct gggctccggt gcgttcggca cggtgtataa gggactctgg        60
   atcccagaag gtgagaaagt taaaattccc gtcgctatca aggaattaag agaagcaaca       120
   tctccgaaag ccaacaagga aatcctcgat gaagcctacg tgatggccag cgtggacaac       180
   ccccacaatc cacatgtgtg ccgcctgctg ggcatctgcc tcacctccac cgtgcagctc       240
   atcacgcagc tcatgccctt cggctgcctc ctggactatg tccgggaaca caaagacaat       300
   attggctccc agtacctgct caactggtgt gtgcagatcg caaag                       345
<210> 3
   <211> 345
   <212> DNA
   <213> Artificial Sequence
<220>
   <223> EGFR p.V769_D770insASV mutation in BaF3
<400> 3
   ttcaaaaaga tcaaagtgct gggctccggt gcgttcggca cggtgtataa gggactctgg    60
   atcccagaag gtgagaaagt taaaattccc gtcgctatca aggaattaag agaagcaaca   120
   tctccgaaag ccaacaagga aatcctcgat gaagcctacg tgatggccag cgtggcctca   180
   gtcgacaacc cccacgtgtg ccgcctgctg ggcatctgcc tcacctccac cgtgcagctc   240
   atcacgcagc tcatgccctt cggctgcctc ctggactatg tccgggaaca caaagacaat   300
   attggctccc agtacctgct caactggtgt gtgcagatcg caaag                     345
<210> 4
   <211> 342
   <212> DNA
   <213> Artificial Sequence
<220>
   <223> EGFR p.P772_H773insPR mutation in BaF3
<400> 4
   ttcaaaaaga tcaaagtgct gggctccggt gcgttcggca cggtgtataa gggactctgg    60
   atcccagaag gtgagaaagt taaaattccc gtcgctatca aggaattaag agaagcaaca   120
   tctccgaaag ccaacaagga aatcctcgat gaagcctacg tgatggccag cgtggacaac   180
   cccccgcgtc acgtgtgccg cctgctgggc atctgcctca cctccaccgt gcagctcatc   240
   acgcagctca tgcccttcgg ctgcctcctg gactatgtcc gggaacacaa agacaatatt   300
   ggctcccagt acctgctcaa ctggtgtgtg cagatcgcaa ag                        342
<210> 5
   <211> 501
   <212> DNA
   <213> Artificial Sequence
<220>
   <223> HER2 INS8 INS YVMA mutation BaF3
<400> 5
   acacctagcg gagcgatgcc caaccaggcg cagatgcgga tcctgaaaga gacggagctg        60
   aggaaggtga aggtgcttgg atctggcgct tttggcacag tctacaaggg catctggatc       120
   cctgatgggg agaatgtgaa aattccagtg gccatcaaag tgttgaggga aaacacatcc       180
   cccaaagcca acaaagaaat cttagacgaa gcatacgtga tggcttacgt gatggctggt       240
   gtgggctccc catatgtctc ccgccttctg ggcatctgcc tgacatccac ggtgcagctg       300
   gtgacacagc ttatgcccta tggctgcctc ttagaccatg tccgggaaaa ccgcggacgc       360
   ctgggctccc aggacctgct gaactggtgt atgcagattg ccaaggggat gagctacctg       420
   gaggatgtgc ggctcgtaca cagggacttg gccgctcgga acgtgctggt caagagtccc       480
   aaccatgtca aaattacaga c                                                 501
<210> 6
   <211> 980
   <212> DNA
   <213> Artificial Sequence
<220>
   <223> EGFR T790M mutation in BaF3
<400> 6
   ggtgttccgg accccacaca gattcctaca ggccctcatg atattttaaa acacagcatc 60
   ctcaaccttg aggcggaggt cttcataaca aagatactat cagttcccaa actcagagat 120
   caggtgactc cgactcctcc tttatccaat gtgctcctca tggccactgt tgcctgggcc 180
   tctctgtcat ggggaatccc cagatgcacc caggaggggc cctctcccac tgcatctgtc 240
   acttcacagc cctgcgtaaa cgtccctgtg ctaggtcttt tgcaggcaca gcttttcctc 300
   catgagtacg tattttgaaa ctcaagatcg cattcatgcg tcttcacctg gaaggggtcc 360
   atgtgcccct ccttctggcc accatgcgaa gccacactga cgtgcctctc cctccctcca 420
   ggaagcctac gtgatggcca gcgtggacaa cccccacgtg tgccgcctgc tgggcatctg 480
   cctcacctcc accgtgcagc tcatcatgca gctcatgccc ttcggctgcc tcctggacta 540
   tgtccgggaa cacaaagaca atattggctc ccagtacctg ctcaactggt gtgtgcagat 600
   cgcaaaggta atcagggaag ggagatacgg ggaggggaga taaggagcca ggatcctcac 660
   atgcggtctg cgctcctggg atagcaagag tttgccatgg ggatatgtgt gtgcgtgcat 720
   gcagcacaca cacattcctt tattttggat tcaatcaagt tgatcttctt gtgcacaaat 780
   cagtgcctgt cccatctgca tgtggaaact ctcatcaatc agctaccttt gaagaatttt 840
   ctctttattg agtgctcagt gtggtctgat gtctctgttc ttatttctct ggaattcttt 900
   gtgaatactg tggtgatttg tagtggagaa ggaatattgc ttcccccatt caggacttga 960
   taacaaggta agcaagccag 980
<210> 7
   <211> 980
   <212> DNA
   <213> Artificial Sequence
<220>
   <223> EGFR T790ML858R mutantion in BaF3
<400> 7
   tccaaactgc acctacggat gcactgggcc aggtcttgaa ggctgtccaa cgaatgggcc 60
   taagatcccg tccatcgcca ctgggatggt gggggccctc ctcttgctgc tggtggtggc 120
   cctggggatc ggcctcttca tgcgaaggcg ccacatcgtt cggaagcgca cgctgcggag 180
   gctgctgcag gagagggagc ttgtggagcc tcttacaccc agtggagaag ctcccaacca 240
   agctctcttg aggatcttga aggaaactga attcaaaaag atcaaagtgc tgggctccgg 300
   tgcgttcggc acggtgtata agggactctg gatcccagaa ggtgagaaag ttaaaattcc 360
   cgtcgctatc aaggaattaa gagaagcaac atctccgaaa gccaacaagg aaatcctcga 420
   tgaagcctac gtgatggcca gcgtggacaa cccccacgtg tgccgcctgc tgggcatctg 480
   cctcacctcc accgtgcagc tcatcatgca gctcatgccc ttcggctgcc tcctggacta 540
   tgtccgggaa cacaaagaca atattggctc ccagtacctg ctcaactggt gtgtgcagat 600
   cgcaaagggc atgaactact tggaggaccg tcgcttggtg caccgcgacc tggcagccag 660
   gaacgtactg gtgaaaacac cgcagcatgt caagatcaca gattttgggc gggccaaact 720
   gctgggtgcg gaagagaaag aataccatgc agaaggaggc aaagtgccta tcaagtggat 780
   ggcattggaa tcaattttac acagaatcta tacccaccag agtgatgtct ggagctacgg 840
   ggtgaccgtt tgggagttga tgacctttgg atccaagcca tatgacggaa tccctgccag 900
   cgagatctcc tccatcctgg agaaaggaga acgcctccct cagccaccca tatgtaccat 960
   cgatgtctac atgatcatgg 980

## Description

The present invention relates to certain 4-substituted 1*H*-pyrrolo[2,3-b]pyridine compounds of the formula (I) and pharmaceutically acceptable salts thereof. These compounds is useful in the treatment or prevention of a disease or medical condition mediated through certain mutated forms of ErbB receptor, especially of Exon20 Her2 and EGFR mutations. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### Field of the Invention

The present invention relates to certain 4-substituted 1*H*-pyrrolo[2,3-b]pyridine compounds of the formula (I) and pharmaceutically acceptable salts thereof. These compounds is useful in the treatment or prevention of a disease or medical condition mediated through certain mutated forms of ErbB receptor, especially of Exon20 Her2 and EGFR mutations.

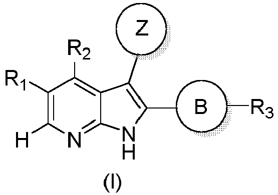

### Background of the Invention

Receptor tyrosine kinases (RTKs) are cell surface receptors that transmit signals from the extracellular environment to control growth, differentiation and survival of cells. Deregulated expression of protein kinases by gene deletion, -mutation or -amplification has been found to be important for tumor initiation and -progression, involving cancer cell proliferation, -survival, -motility and -invasivity as well tumor angiogenesis and chemotherapy resistance. Because of the advanced understanding of their critical role, protein kinases are important targets for novel therapies, especially for cancer (Hananhan et al. Cell 2000, 7, 100(1): 57-70; Blume-Jensen et al., Nature 2001, 17, 411(6835): 355-65).

In humans the receptor tyrosine kinase family ErbB comprises four members: EGFR (Her1), ErbB2 (Her2), ErbB3 (Her3) and ErbB4 (Her4). The binding of a ligand induces conformational change in receptors to form homo- and heterodimerization. The extracellular domain of Her2 is already fixed in a conformation without ligand binding that resembles the other ligand-activated ErbB members and hereby acts as a preferred dimerization partner for other ligand-bound ErbBs. The dimerization of receptors activates the intrinsic kinase activity and hereby yielding to the phosphorylation of its substrates, resulting in activation of multiple downstream pathways within the cell, including the anti-apoptotic/survival PI3K-AKT-mTOR and the mitogenic RAS-RAF-MEK-ERK-MAPK pathways (Chong et al. Nature Med. 2013; 19 (11):1389-1400).

There is strong precedent for the involvement of ErbB kinase family in human cancer as overexpression and/or mutations of ErbB is commonly found in cancers (for example breast, lung, head, neck and bladder). First generation small molecule EGFR inhibitors like Tarceva (Erlotinib) and Iressa (Gefitinib), both binding reversibly to EGFR, are currently first-line therapy for non-small cell lung cancer patients with tumors harbouring EGFR mutations in exon 19 and 21 (like L858R and deIE746-A750). Second and third generation small molecule EGFR inhibitors have been designed as irreversible EGFR inhibitors. These compounds (for example Afatinib, HKI-272, CI-1033, EKB-569, WZ-4002, AZ9291, CO-1686) bind irreversibly to EGFR, preferably to cysteine 797.

Approximately 10% of patients with NSCLC in the United States are reported to have tumor-associated EGFR mutations (Lynch et al. N Engl J Med. 2004, 350 (21): 2129-39). The EGFR mutations mostly occur within EGFR Exon 18-21 (Yasuda et al. Sci Transl Med. 2013, 18, 5(216): 216ra177; Leduc et al. Annals of Oncology 2017, 28, 11: 2715-2724; Arcila et al. Mol Cancer Ther. 2013, 12 (2): 220-229). These mutations increase the kinase activity of EGFR, leading to hyperactivation of downstream pro-survival signalling pathways (Pao et al. Nat Rev Cancer 2010, 10: 760-774). EGFR Exon 20 insertions reportedly comprise approximately 4-9.2 % of all EGFR mutant lung tumors (Arcila et al. Mol Cancer Ther. 2013, 12 (2): 220-9; Oxnard et al. J Thorac Oncol. 2013, 8(2): 179-184; Yasuda et al. Lancet Oncol. 2012, 13 (1): e23-31). Most EGFR Exon 20 insertions occur in the region encoding amino acids 767 through 774 of exon 20 within the loop that follows the C-helix of the kinase donmain of EGFR. Analysis of patients with tumors harbouring EGFR Exon 20 insertion mutations mostly displayed progressive disease in the course of treatment with Gefitinib, Erlotinib or Afatinib (Yasuda et al. Lancet Oncol. 2012, 13 (1): e23-31; Yasuda et al. Sci Transl Med. 2013, 18, 5(216): 216ra177).

Her2 mutations are reportedly present in ~2-4% of NSCLC (Buttitta et al. Int J Cancer. 2006, 119: 2586-2591). The most common mutation is an in-frame insertion within Exon 20. In 83% of patients having Her2 associated NSCLC, a four amino acid YVMA insertion mutation occurs at codon 775 in Exon 20 of Her2 (Arcila et al. Clin Cancer Res 2012, 18: 4910-4918). The Her2 Exon 20 insertion results in increased kinase activity and enhanced signalling through downstream pathways, resulting in increased survival, invasiveness, and tumorgenicity (Wang et al. Cancer Cell 2006; 10: 25-38). Tumors harbouring the Her2 YVMA mutation are largely resistant to known EGFR inhibitors (Arcila et al. Clin Cancer Res 2012, 18: 4910-4918). WO2017066193 discloses pyrrolopyridines that have biological activity to modulate mammalian protein kinase enzymes useful in the treatment of cancer.

It is object of the present invention to provide compounds as mutant-selective ErbB inhibitors, especially for the Exon 20 EGFR/Her2 mutations, and/or pharmaceutically acceptable salts thereof which can be used as pharmaceutically active agents, and use thereof especially for prophylaxis and/ or treatment of cell proliferative disease, especially cancer, a combination comprising the inventive compound and at least one anticancer drug; as well as compositions comprising at least one of those compounds and/or pharmaceutically acceptable salts thereof as pharmaceutically active ingredients

The present invention provides novel compound(s) being mutant-selective ErbB inhibitors, especially for the Exon 20 EGFR/Her2 mutations, and additionally being inhibitors for other mutants like EGFR T790ML858R mutation.

The objective of the present invention is solved by the teaching of the independent claims. Further advantageous features, aspects and details of the invention are evident from the dependent claims, the description, the figures, and the examples of the present application.

### Summary of this invention

The present invention provides a compound that exhibits inhibition of HER2 Exon20 A775_G776insYVMA (and similar mutations) and/or EGFR Exon20 H773_V774insNPH (and similar mutations). The present invention provides compound(s) being mutant-selective ErbB inhibitors, especially for the Exon20 EGFR/Her2 mutations, and additionally being inhibitors for other mutants like EGFR T790ML858R mutation. In certain embodiments the current invention is directed towards a compound that is mutant-specific for Exon20 of EGFR and Her2. In one aspect, the invention provides a compound comprising an irreversible kinase inhibitor. The compound covalently modifies cysteine 797/805 in EGFR/Her2.

The compound of the present invention and/or pharmaceutically acceptable salts thereof as pharmaceutical active ingredient can be used for treatment and/or prophylxis of a cell proliferative disease.

The cell proliferative disease is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, osteosarcoma, melanoma, glioblastoma and neuroblastoma. In an especially preferred embodiment, the disorders are selected from breast cancer, glioblastoma, renal cancer, non-small cell lung cancer (NSCLC), and melanoma. The compounds are also suitable for the prevention and/or treatment of other hyperproliferative disorders, particulary benign hyperproliferative disorders such as benign prostate hyperplasia.

Thus, the present invention is directed to a compound of the formula (I) 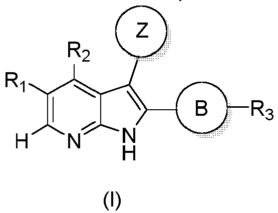 wherein
**Z** represents 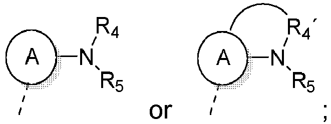
**A** represents 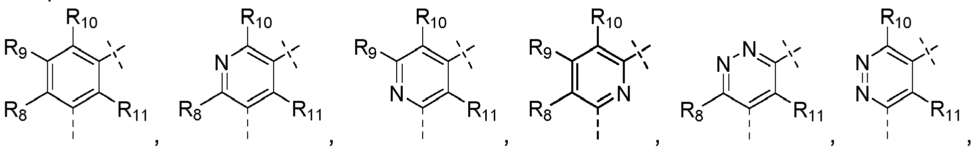 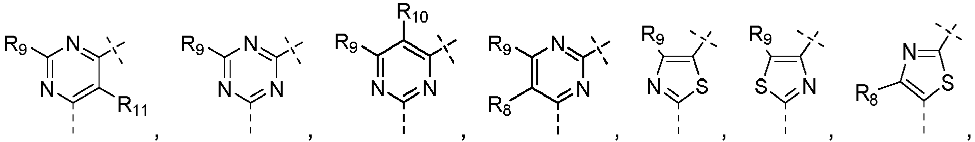 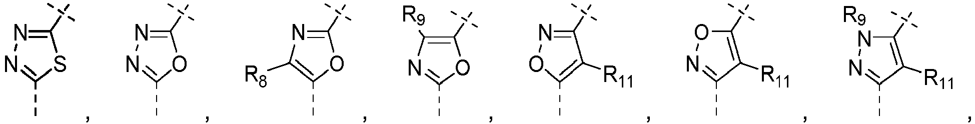 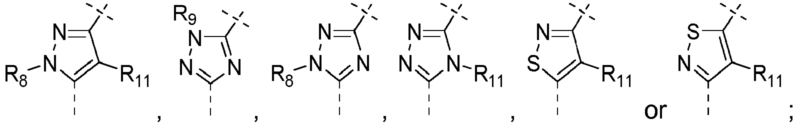 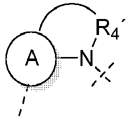 represents 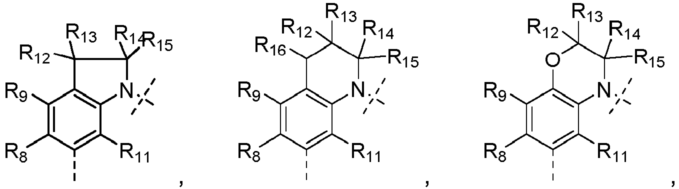 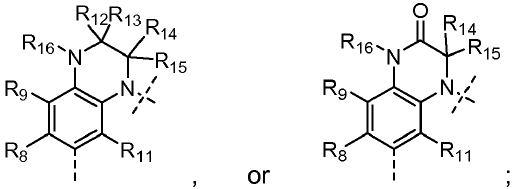
**B** represents 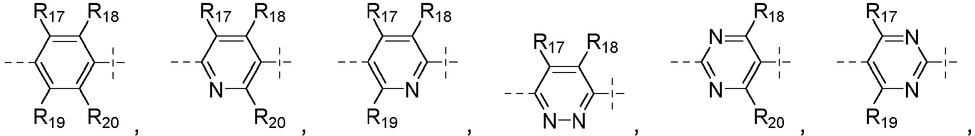 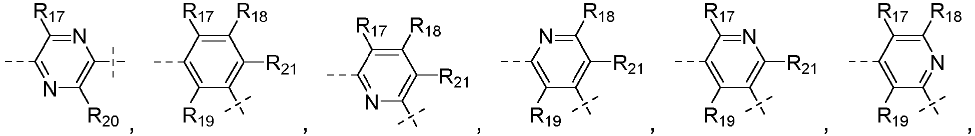 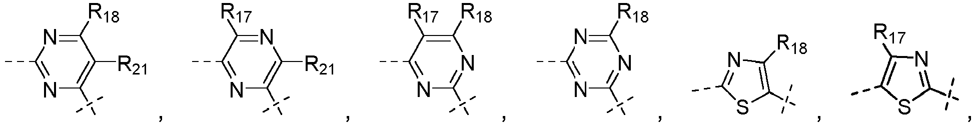 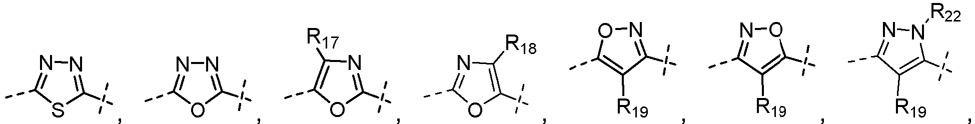 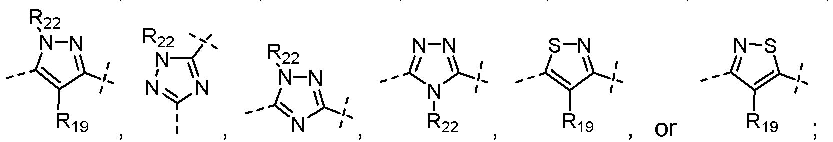
**R₁** represents -H, **-R*,** -CH₂-**R***, -CHR^{a}**-R***, -CR^{a}R^{b}**-R***, -CH₂CH₂-**R***, -CR^{a}R^{b}-CR^{c}R^{d}**-R***, -CH=CH-**R***, -CR^{a}=CR^{b}**-R***, -CH₂CH₂CH₂-**R*** or -CR^{a}R^{b}-CR^{c}R^{d}-CR^{e}R^{f}-**R***;
**R^{a}** - **R^{f}** represent independently of each other -H, -F, -CI, -CH₃, -OCH₃, -CH₂CH₃, -CH₂CH₂CH₃, -CH(CH₃)₂, -CH₂CH(CH₃)₂, or -cyclo-C₃H₅;
**R*** represents -H, -F, -CI, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHCOCH₃, -NHCOCH(CH₃)₂, -NHSO₂CH₃, -NHSO₂C₂H₅, -NHSO₂C₃H₇, -NHSO₂CH(CH₃)₂, -NHSO₂-cyclco-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclco-C₃H₅, -SO(NH)CH₃, -SO(NH)C₂H₅, -SO(NH)C₃H₇, -SO(NH)CH(CH₃)₂, -SO(NH)-cyclco-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclco-C₃H₅, 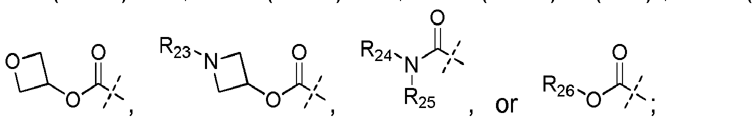
**R₂** represents -H, -F, -CI, -Br, -I, -OH, -CN, -CH₃, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅, or -O-C₂H₄-cyclo-C₃H₅;
**R₃** represents -H, -CH₃, -C₂H₄OH -OC₂H₄OH, -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇P₂₈, -OCH₂CH₂NR₂₇R₂₈, -OCH₂CH₂CH₂NR₂₇R₂₈, -NR₂₉CH₂NR₂₇R₂₈, -NR₂₉CH₂CH₂NR₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 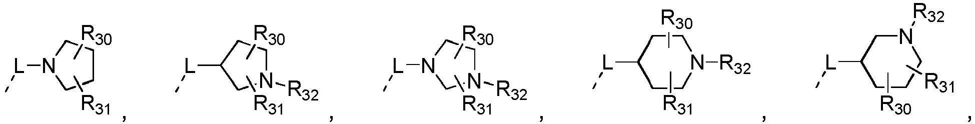 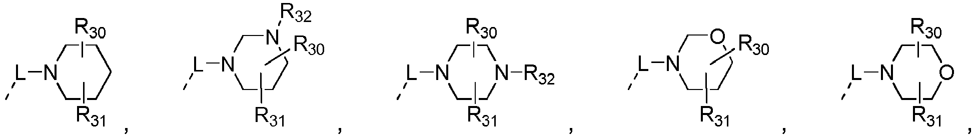 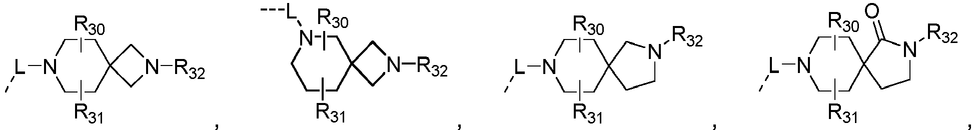 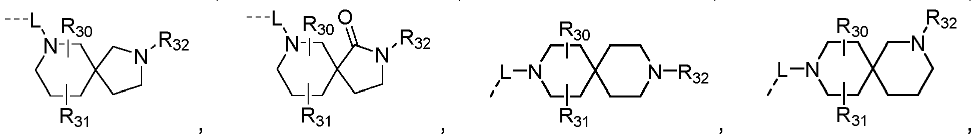 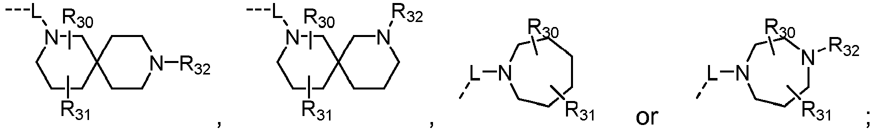 **L** is a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO--CH₂CO- -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO- or -CH₂CH₂OCO-;
**R₄** represents -H, -CH₃, or -C₂H₅;
**R₅** represents 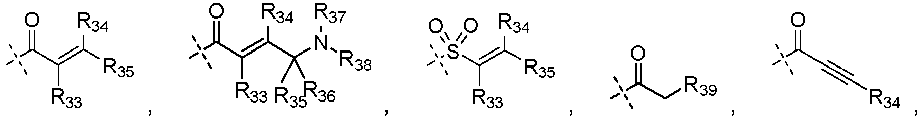 or 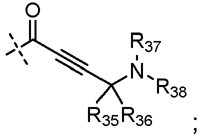
**R₈** - **R₁₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇, or **R₈** and **R₉** or **R₉** and **R₁₀** form together -CH₂-, -CH₂CH₂-, -CH₂CH₂CH₂-, -CH₂CH₂CH₂CH₂-, -CH₂OCH₂-, -CH₂OCH₂CH₂-, -OCH₂O-, or -OCH₂CH₂O-;
**R₁₂** - **R₁₆** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₁₇** - **R₂₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₂₂** represents -H, -CH₃, -CF₃, -C₂H₅, -C₃H₇, -COCH₃, -COC₂H₅, -SO₂CH₃, or -SO₂C₂H₅;
**R₂₃** represents -H, -CH₃, -CF₃, -C₂H₅, -C₃H₇, -CH₂CH₂OCH₃, or -C₄H₉;
**R₂₄** and **R₂₅** represent independently of each other -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 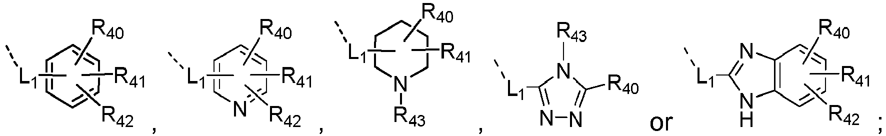 or **R²⁴** and **R²⁵** form together -CH₂-, -CH₂CH₂-, -CH₂CH₂CH₂-, -CH₂CH₂CH₂CH₂-, -CH₂OCH₂-, or -CH₂OCH₂CH₂-;
**L₁** represents a bond, -CH₂-, -CH₂CH₂-, or -CH₂CH₂CH₂-;
**R₂₆** represents -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph, or -CH₂Ph;
**R₂₇** - **R₃₁** represent independently of each other -H, -F, -CI, -OH, -CN, -NH₂, -OCH₃, -OC₂H₅, -OC₃H₇, -OCH(CH₃)₂, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, or -cyclo-C₆H₁₁;
**R₃₂, R₃₇**, **R₃₈**, and **R₄₃** represent independently of each other -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃, -SO₂C₂H₅, -SO₂CF₃, or -SO₂Ph;
**R₃₃**, **R₃₄, R₃₅,** and **R₃₆** represent independently of each other -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, or -COOC(CH₃)₃;
**R₃₉** represents -F, -Br, -CI, or -I;
**R₄₀, R₄₁,** and **R₄₂** represent independently of each other -H, -F, -CI, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂, -SO₂CH₃, -SO₂C₂H₅, -SO₂CF₃, -SO₂Ph, -SO₂NH₂, -SO₂NHCH₃, 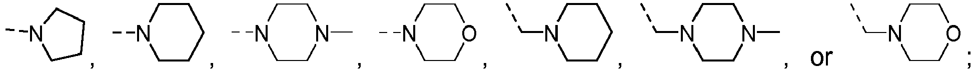
or an enantiomer, a mixture of enantiomers, a diastereomer, a mixture of diastereomers, a tautomer, a hydrate, a solvate, or pharmaceutically acceptable salts thereof.

In the definition of **A** ring, a bond " 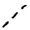 " of **A** ring is connected to C-3 of pyrrolo[2,3-b]pyridine backbone and a bond " 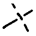 " of **A** ring is connected to nitrogen atom of the group NR₄R₅.

Similary, in the definition of the moiety 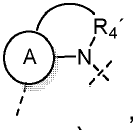 a bond " 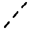 "is connected to C-3 of pyrrolo[2,3-b]pyridine backbone and a bond " 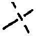 "is connected to the group **R₅.**

**R₄'** represents 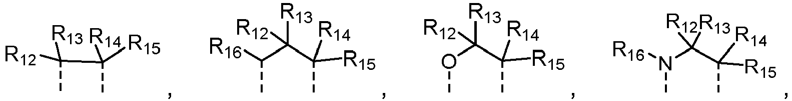 or

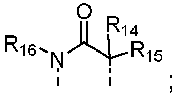

In the definition of **B** ring, a bond " 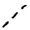 " of **B** ring is connected to C-2 of pyrrolo[2,3-b]pyridine backbone and a bond " 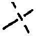 " of **A** ring is connected to the group **R₃.**

Preferred is the compound of the formula (I), wherein
**A** represents 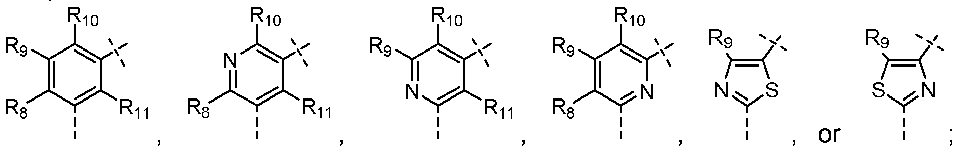 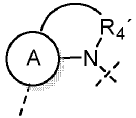 represents 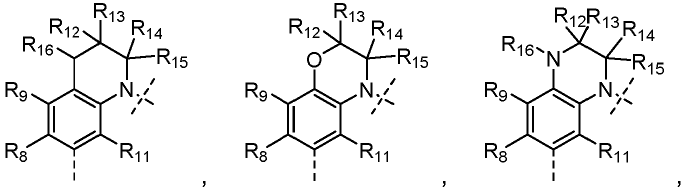 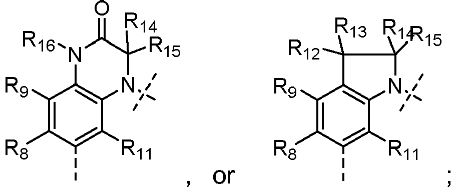
**B** represents 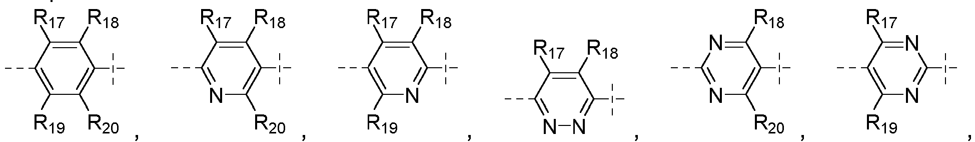 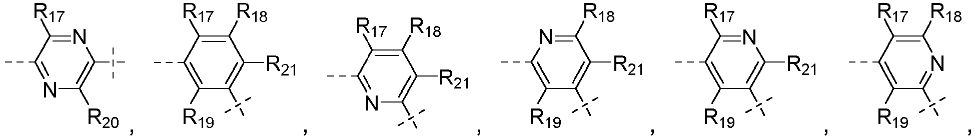 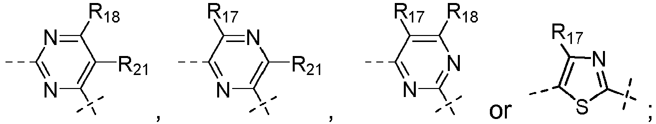 and **R₈** - **R₂₁** have the same meanings as defined herein.

Also preferred is the compound of the formula (I), wherein
**A** represents 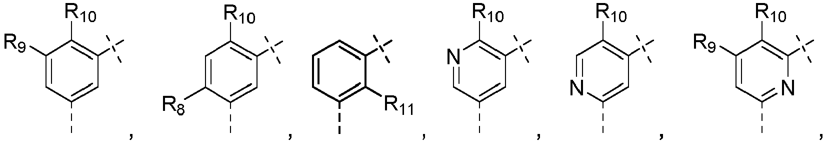 or 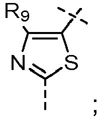 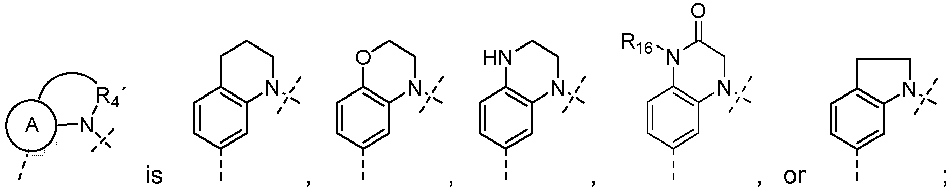
**B** represents 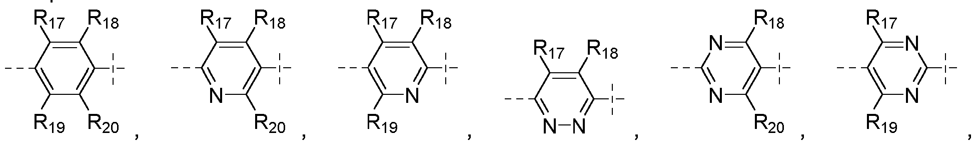 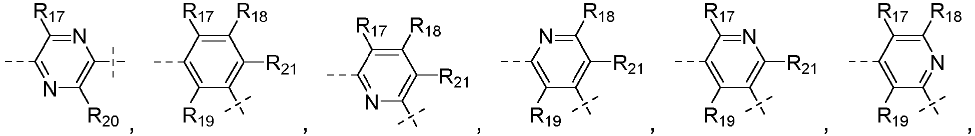 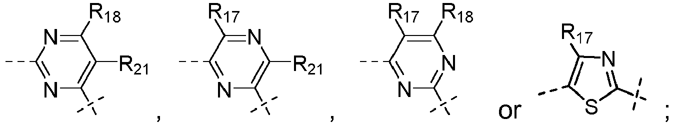
and **R₈- R₁₁,** and **R₁₆- R₂₁** have the meanings as defined herein.

More preferred is the compound of the formula (I), wherein
**A** represents 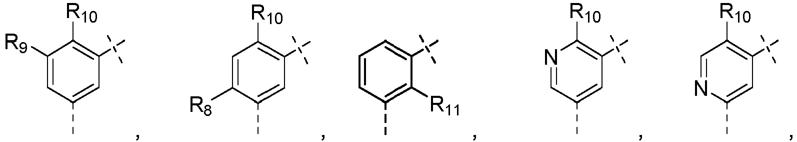 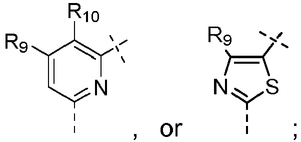 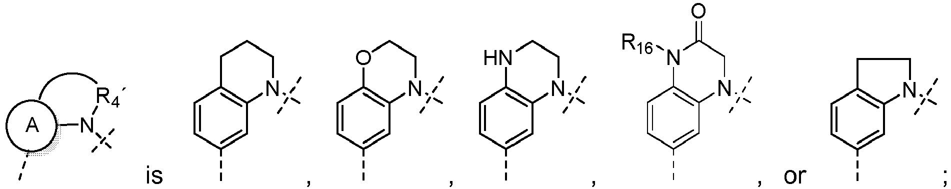
**B** represents 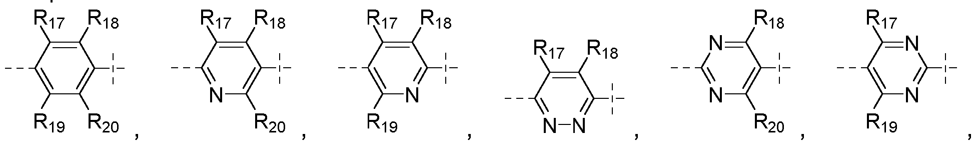 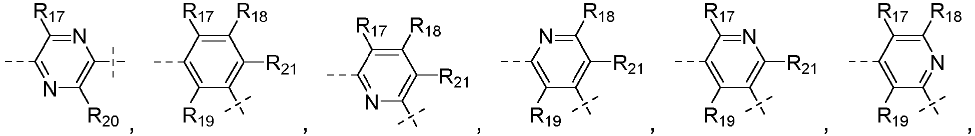 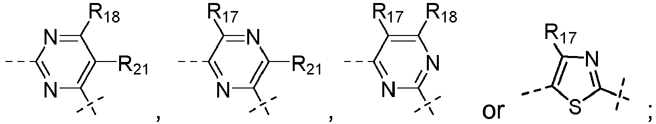
and **R₉** - **R₁₀,** and **R₁₆ - R₂₁** have the meanings as defined herein.

More preferred is the compound of the formula (I), wherein
**A** represents 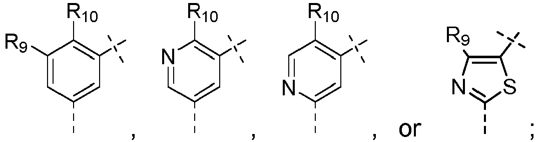 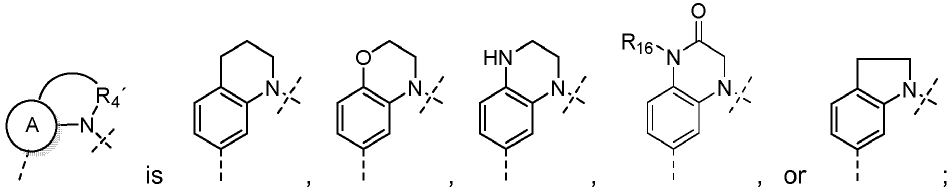
**B** represents 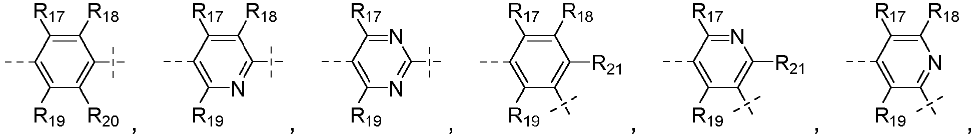 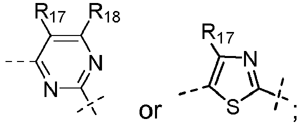
**R₈** - **R₁₁** and **R₁₆ - R₂₁** have the meanings as defined herein.

Still more preferred is the compound of the formula (I), wherein the compound has the formula **(Ia)** or **(Ib):** 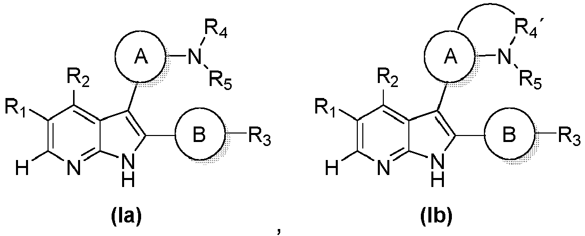 wherein **A** represents 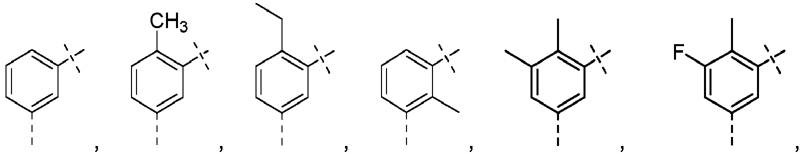 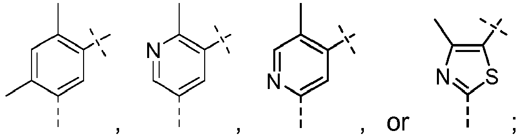 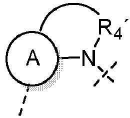 represents 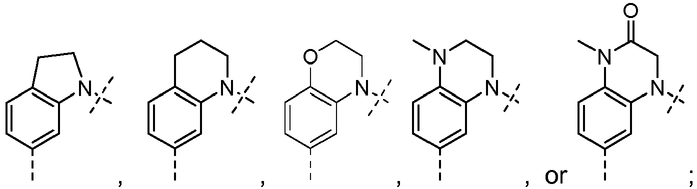 **B** represents 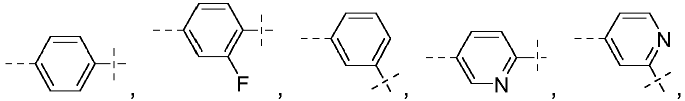 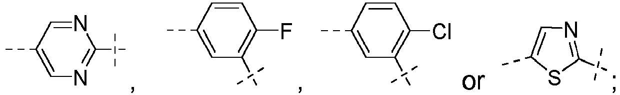 and **R₁, R₂, R₃, R₄,** and **R₅** have the same meanings as defined herein.

Still more preferred is the compound of any one of the formulae **(I), (Ia)** and **(Ib),** wherein
**Z** represents 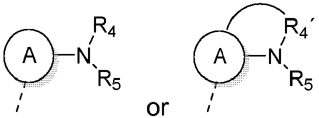 and is selected from 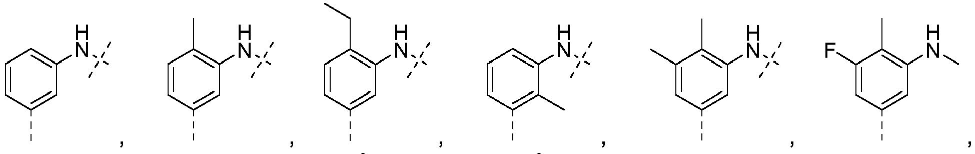 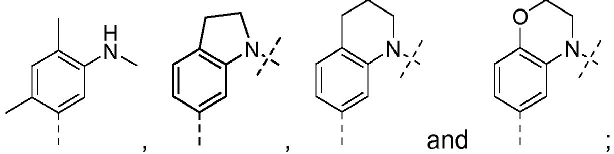
**R₁** represents -H, -CN, -C₂H₅, -OC₂H₅, -OCF₃, -CH₂OH, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, -CH₂COOH, -CH₂COOCH₃, -CH₂COOCH(CH₃)₂, -CH₂CONH(CH₃), -CH₂CON(CH₃)₂, -NHCOCH₃, -NHCOCH(CH₃)₂, 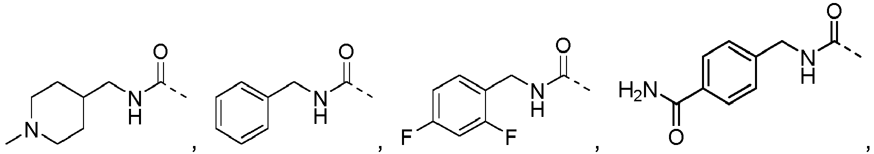 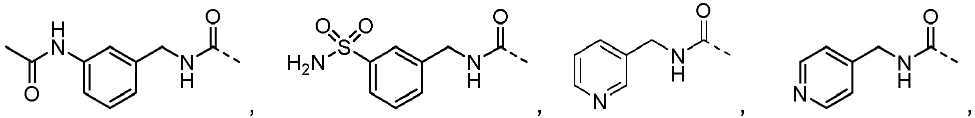 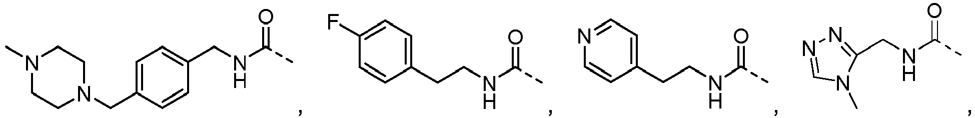 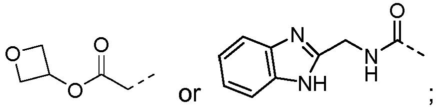
**R₂** represents -H, -CI, -CH₃, -OCH₃, -OC₂H₅, or -OCH(CH₃)₂;
**B** represents 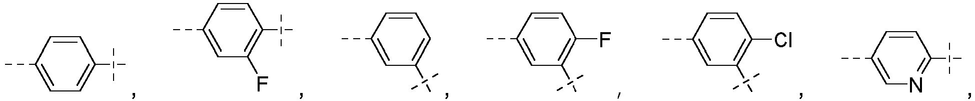 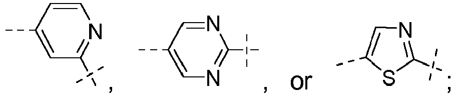
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, -CH₂CH₂OH, -OCH₂CH₂OH, 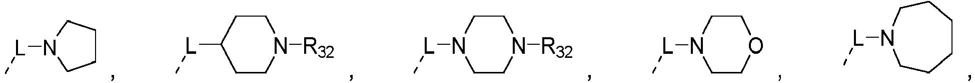 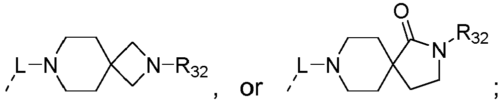
**R₅** represents 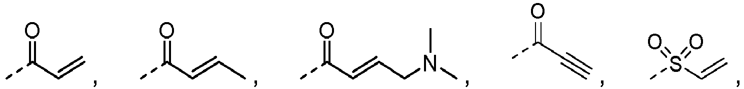 or 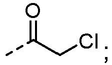
**L** is a bond, -CH₂-, -OCH₂CH₂-, -CO-, -CONH-, -SO₂-, or -CONHCH₂CH₂CH₂-; and
**R₃₂** represents -H, -CH₃, -C₂H₅, -CH(CH₃)₂, -CH₂Ph, -COCH₃, or -SO₂C₂H₅.

In certain aspects, the compound described above is a compound of formula **(II)** and **(III)** : 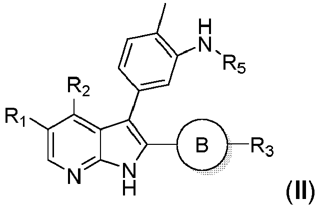 wherein **B, R₁, R₂, R₃** and **R₅** have the same meanings as defined herein.

Preferred is the compound of the formula **(II),** wherein
**B** represents 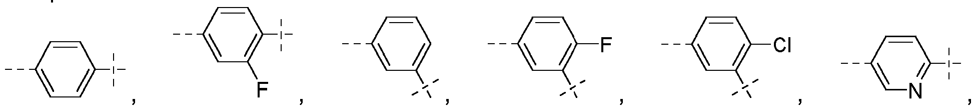 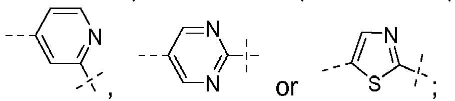
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, -CH₂CH₂OH, -OCH₂CH₂OH, 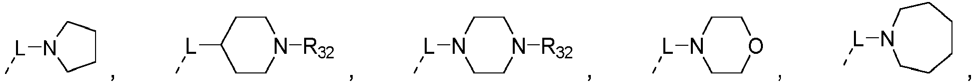 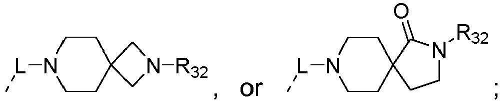
**R₅** represents 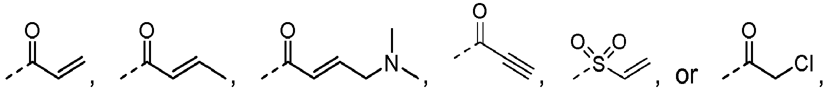 **L** is a bond, -CH₂-, -OCH₂CH₂-, -CO-, -CONH-, -SO₂-, or -CONHCH₂CH₂CH₂-; and
**R₃₂** represents -H, -CH₃, -C₂H₅, -CH(CH₃)₂, -CH₂Ph, -COCH₃, or -SO₂C₂H₅.

Preferred are also compounds of formula (III): 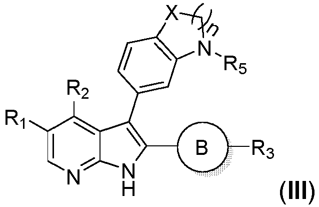 wherein
**R₁, R₂, R₃** and **R₅** have the same meanings as defined above;
**X** represents CH₂, when n is 1, or 2; or
**X** represents O, when n is 2;

Preferred is the compound of the formula **(III),** wherein
**B** represents 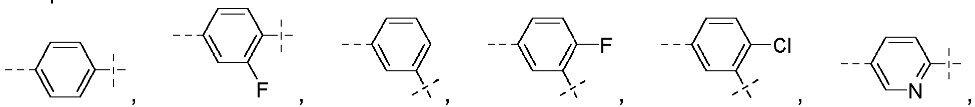 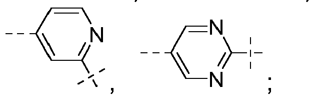
**R₅** represents

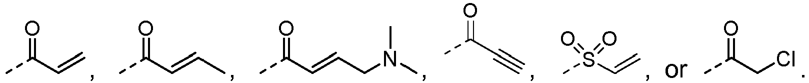

Also preferred is the compound of any one of the formulae **(IIIa), (IIIb)** and **(IIIc**): 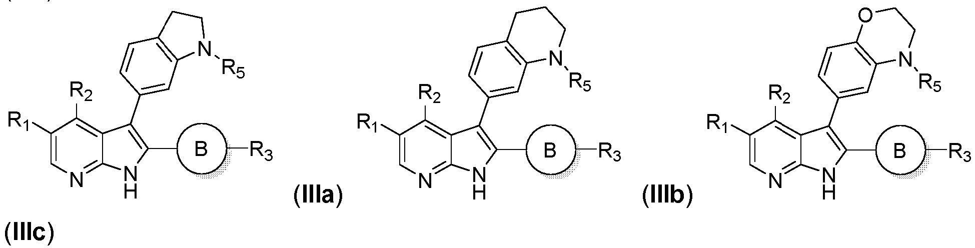 wherein, B, **R₁, R₂, R₃,** and **R₅,** have the same meanings as defined in the formula (I).

Still more preferred are the compounds of any one of the formulae **(II-1)** - **(II-6), (IIa), (IIb), (IIc), (IId), (IIe), (IIf-1)-(IIf-3), (IIIa-1)-(IIIa-5), (IIIb-1)-(IIIb-5)** and **(IIIc-1)** - **(IIIc-5).** 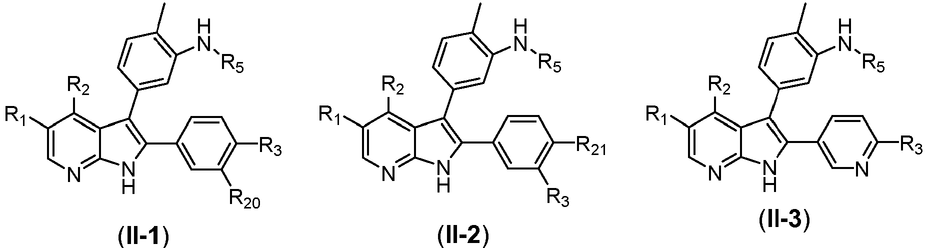 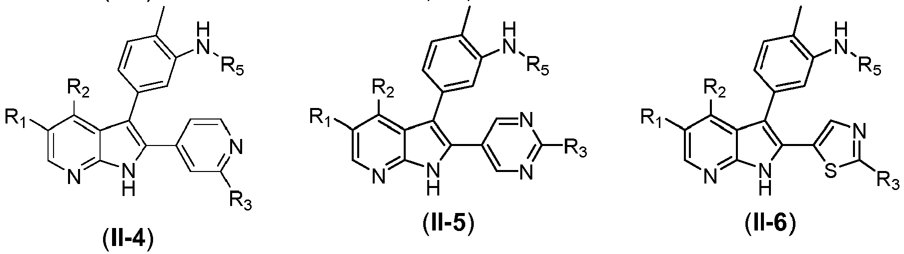 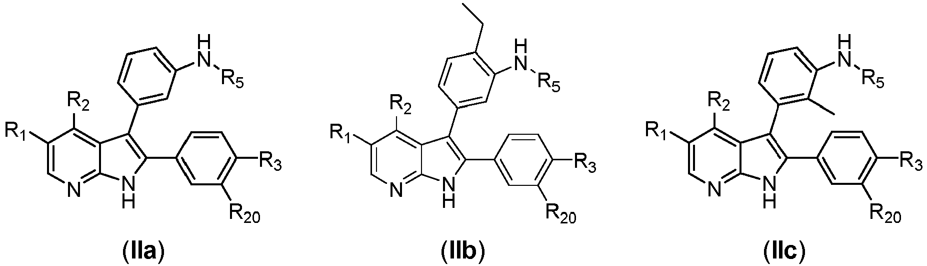 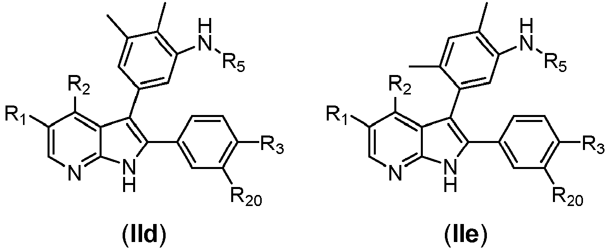 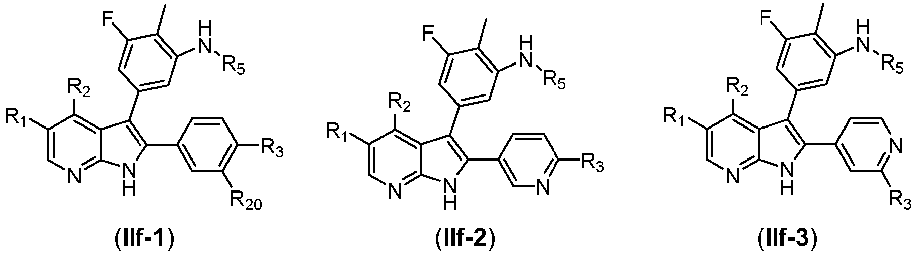 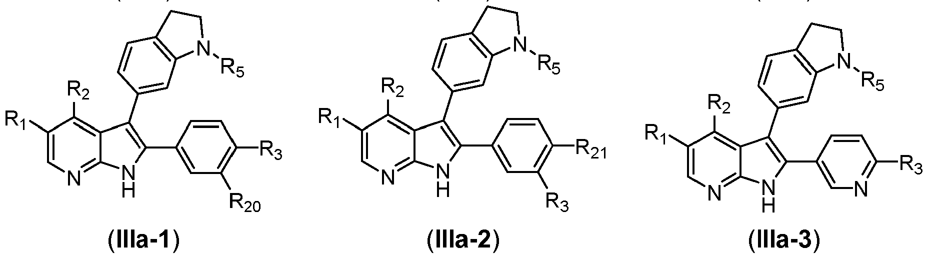 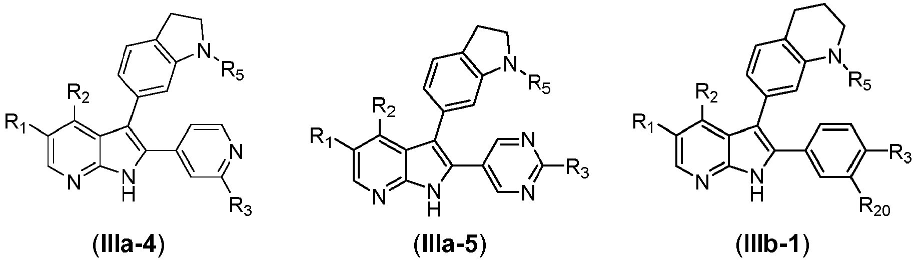 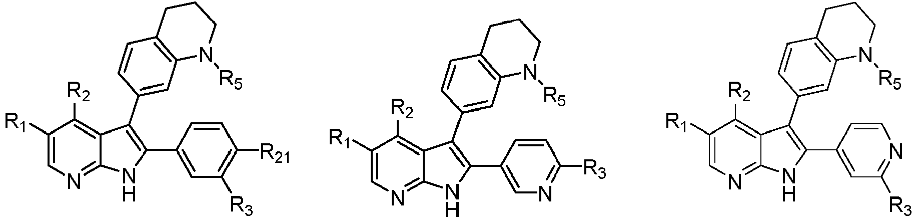 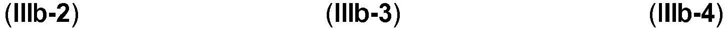 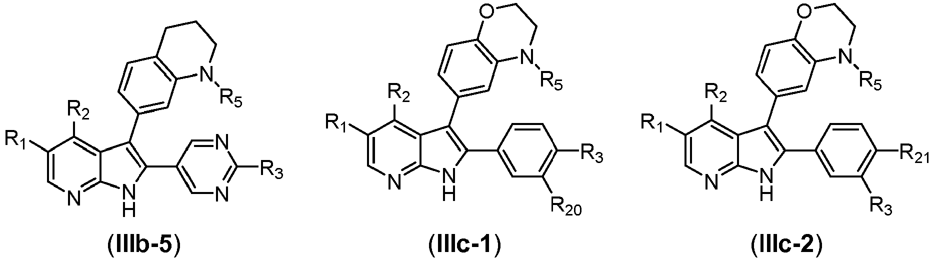 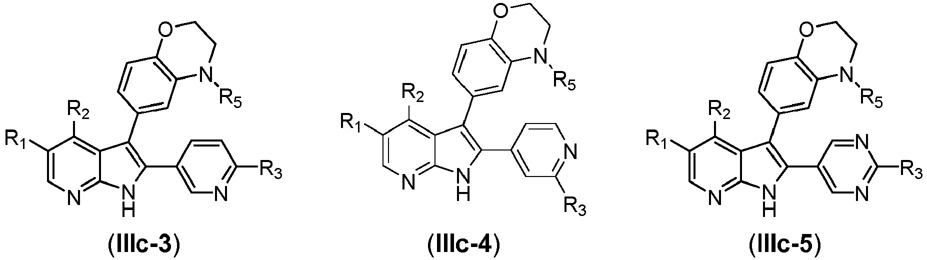 wherein, **R₁, R₂, R₃, R₅**, **R₂₀** and **R₂₁** have the meanings as defined in the formula (I).

In any one of the formulae **(II-1)** - **(II-6), (IIa), (IIb), (IIc), (IId), (IIe), (IIf-1)** - **(IIf-3), (IIIa-1)** - **(IIIa-5), (IIIb-1)** - **(IIIb-5), (IIIc-1)** - **(IIIc-5),** preferred, **R₁** represents -H, -CN,-C₂H₅, -OC₂H₅, -OCF₃, -CH₂OH, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, -CH₂COOH, -CH₂COOCH₃, -CH₂COOCH(CH₃)₂, -CH₂CONH(CH₃), -CH₂CON(CH₃)₂, -NHCOCH₃, -NHCOCH(CH₃)₂, 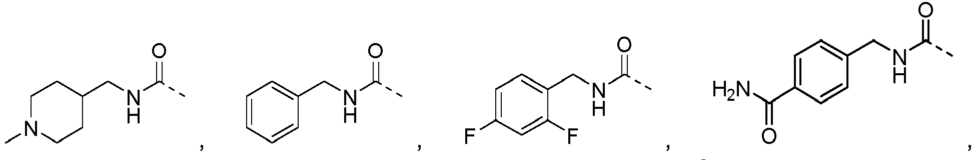 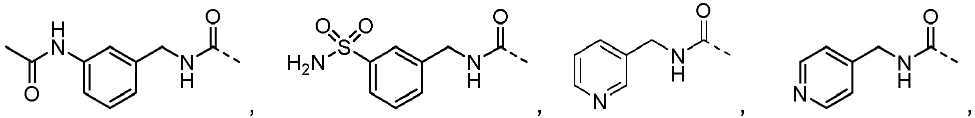 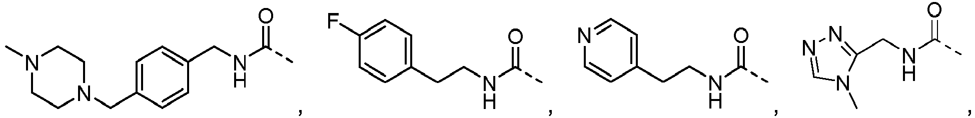 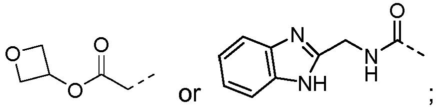 **R₂** represents -H, -CI, -CH₃, -OCH₃, -OC₂H₅, or -OCH(CH₃)₂;
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, -CH₂CH₂OH, -OCH₂CH₂OH, 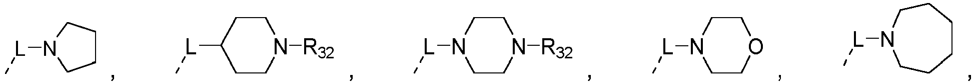 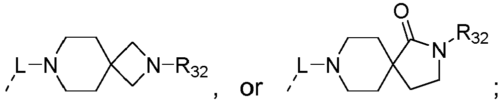 **R₅** represents 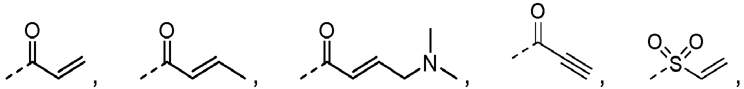 or 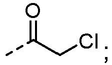 **L** is a bond, -CH₂-, -OCH₂CH₂-, -CO-, -CONH-, -SO₂-, or -CONHCH₂CH₂CH₂-; and
**R₃₂** represents -H, -CH₃, -C₂H₅, -CH(CH₃)₂, -CH₂Ph, -COCH₃, or -SO₂C₂H₅.

Especially preferred compounds according to the present invention include compounds presented by **Table 1.**

**Table 1**

| **Example** | **Structure** | **Nomenclature** |
|---|---|---|
| **1** | 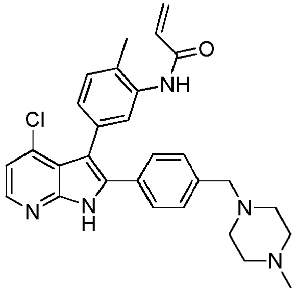 | N-(5-(4-chloro-2-(4-((4-methylpiperazin-1-yl)methyl)phenyl)-1H-pyrrolo[2,3-b]pyridin-3-yl)-2-methylphenyl)acrylamide |
| **2** | 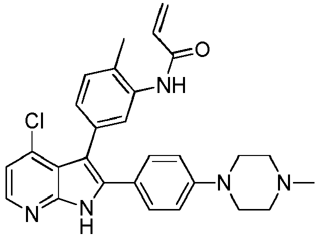 | N-(5-(4-chloro-2-(4-(4-methylpiperazin-1-yl)phenyl)-1H-pyrrolo[2,3-b]pyrid in-3-yl)-2-methylphenyl)acrylamide |
| **3** | 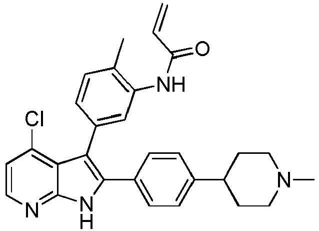 | N-(5-(4-chloro-2-(4-(1-methylpiperidin-4-yl)phenyl)-1H-pyrrolo[2,3-b]pyridin-3-yl)-2-methylphenyl)acrylamide |
| **4** | 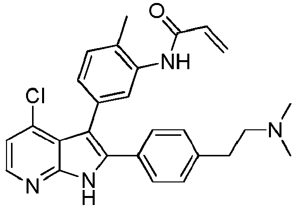 | N-(5-(4-chloro-2-(4-(2-(dimethylamino)ethyl)phenyl)-1H-pyrrolo[2,3-b]pyridin-3-yl)-2-methylphenyl)acrylamide |
| **5** | 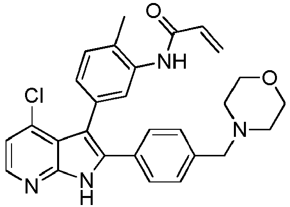 | N-(5-(4-chloro-2-(4-(morpholinomethyl)phenyl)-1H-pyrrolo[2,3-b]pyridin-3-yl)-2-methylphenyl)acrylamide |
| **6** | 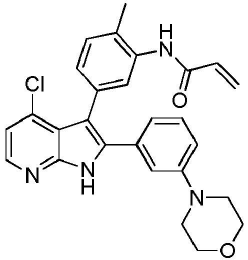 | N-(5-(4-chloro-2-(3-morpholinophenyl)-1H-pyrrolo[2,3-b]pyridin-3-yl)-2-methylphenyl)acrylamide |
| **7** | 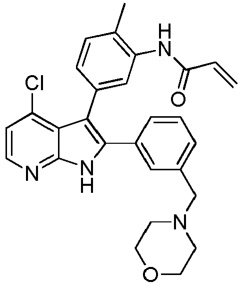 | N-(5-(4-chloro-2-(3-(morpholinomethyl)phenyl)-1H-pyrrolo[2,3-b]pyridin-3-yl)-2-methylphenyl)acrylamide |
| **8** | 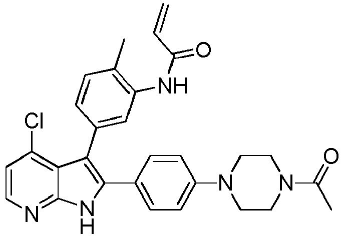 | N-(5-(2-(4-(4-acetylpiperazin-1-yl)phenyl)-4-chloro-1H-pyrrolo[2,3-b]pyridin-3-yl)-2-methylphenyl)acrylamide |
| **9** | 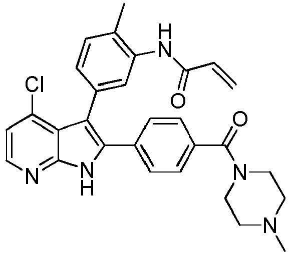 | N-(5-(4-chloro-2-(4-(4-methylpiperazine-1-carbonyl)phenyl)-1H-pyrrolo[2,3-b]pyridin-3-yl)-2-methylphenyl)acrylamide |
| **10** | 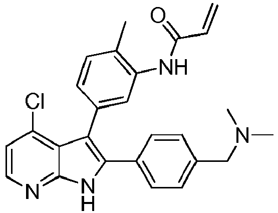 | N-(5-(4-chloro-2-(4-((dimethylamino)methyl)phenyl)-1H-pyrrolo[2,3-b]pyridin-3-yl)-2-methylphenyl)acrylamide |
| **11** | 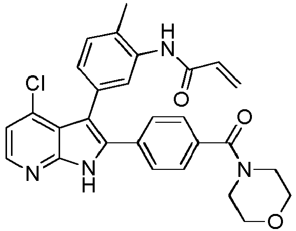 | N-(5-(4-chloro-2-(4-(morpholine-4-carbonyl)phenyl)-1H-pyrrolo[2,3-b]pyridin-3-yl)-2-methylphenyl)acrylamide |
| **12** | 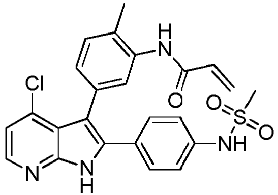 | N-(5-(4-chloro-2-(4-(methylsulfonamido)phenyl)-1H-pyrrolo[2,3-b]pyridin-3-yl)-2-methylphenyl)acrylamide |
| **13** | 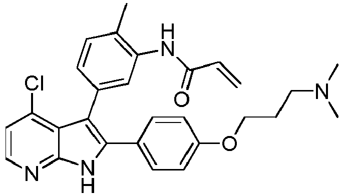 | N-(5-(4-chloro-2-(4-(3-(dimethylamino)propoxy)phenyl)-1H-pyrrolo[2,3-b]pyridin-3-yl)-2-methylphenyl)acrylamide |
| **14** | 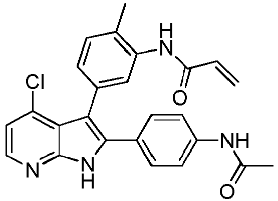 | N-(5-(2-(4-acetamidophenyl)-4-chloro-1H-pyrrolo[2,3-b]pyridin-3-yl)-2-methylphenyl)acrylamide |
| **15** | 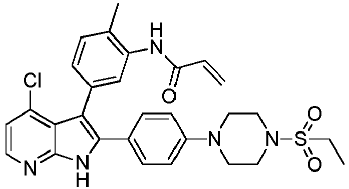 | N-(5-(4-chloro-2-(4-(4-(ethylsulfonyl)piperazin-1-yl)phenyl)-1H-pyrrolo[2,3-b]pyridin-3-yl)-2-methylphenyl)acrylamide |
| **16** | 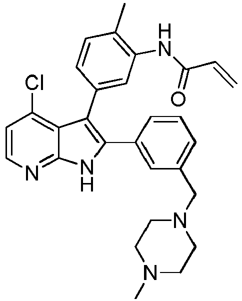 | N-(5-(4-chloro-2-(3-((4-methylpiperazin-1-yl)methyl)phenyl)-1H-pyrrolo[2,3-b]pyridin-3-yl)-2-methylphenyl)acrylamide |
| **17** | 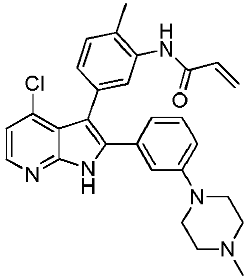 | N-(5-(4-chloro-2-(3-(4-methylpiperazin-1-yl)phenyl)-1H-pyrrolo[2,3-b]pyridin-3-yl)-2-methylphenyl)acrylamide |
| **18** | 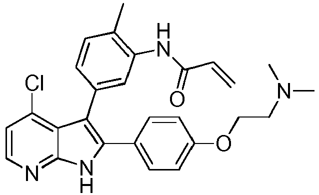 | N-(5-(4-chloro-2-(4-(2-(dimethylamino)ethoxy)phenyl)-1H-pyrrolo[2,3-b]pyridin-3-yl)-2-methylphenyl)acrylamide |
| **19** | 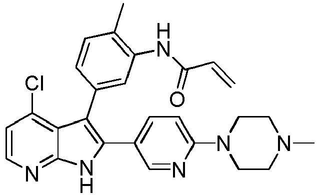 | N-(5-(4-chloro-2-(6-(4-methylpiperazin-1-yl)pyridin-3-yl)-1H-pyrrolo[2,3-b]pyridin-3-yl)-2-methylphenyl)acrylamide |
| **20** | 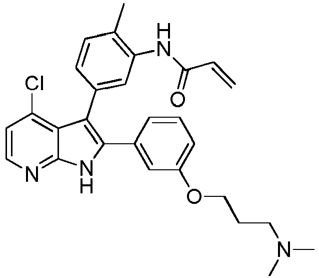 | N-(5-(4-chloro-2-(3-(3-(dimethylamino)propoxy)phenyl)-1H-pyrrolo[2,3-b]pyridin-3-yl)-2-methylphenyl)acrylamide |
| **21** | 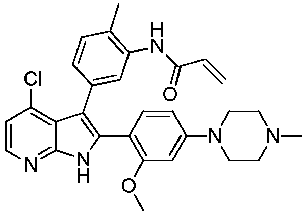 | N-(5-(4-chloro-2-(2-methoxy-4-(4-methylpiperazin-1-yl)phenyl)-1H-pyrrolo[2,3-b]pyridin-3-yl)-2-methyl phenyl)acrylamide |
| **22** | 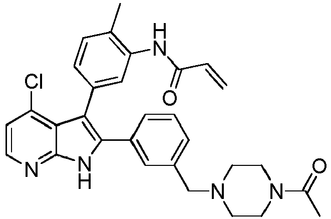 | N-(5-(2-(3-((4-acetylpiperazin-1 - yl)methyl)phenyl)-4-chloro-1H-pyrrolo[2,3-b]pyridin-3-yl)-2-methylphenyl)acrylamide |
| **23** | 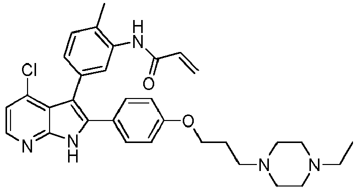 | N-(5-(4-chloro-2-(4-(3-(4-ethylpiperazin-1-yl)propoxy)phenyl)-1H-pyrrolo[2,3-b]pyridin-3-yl)-2-methyl phenyl)acrylamide |
| **24** | 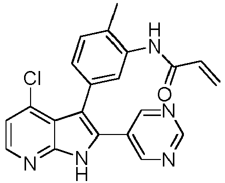 | N-(5-(4-chloro-2-(pyrimidin-5-yl)-1H-pyrrolo[2,3-b]pyridin-3-yl)-2-methylphenyl)acrylamide |
| **25** | 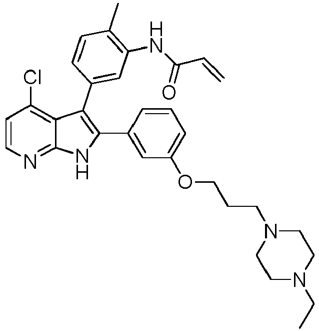 | N-(5-(4-chloro-2-(3-(3-(4-ethylpiperazin-1-yl)propoxy)phenyl)-1H-pyrrolo[2,3-b]pyridin-3-yl)-2-methylphenyl)acrylamide |
| **26** | 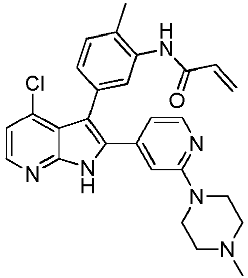 | N-(5-(4-chloro-2-(2-(4-methylpiperazin-1-yl)pyridin-4-yl)-1H-pyrrolo[2,3-b]pyridin-3-yl)-2-methylphenyl)acrylamide |
| **27** | 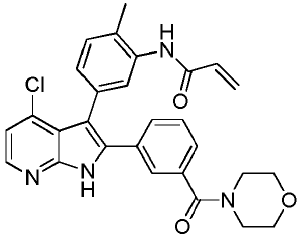 | N-(5-(4-chloro-2-(3-(morpholine-4-carbonyl)phenyl)-1H-pyrrolo[2,3-b]pyridin-3-yl)-2-methylphenyl)acrylamide |
| **28** | 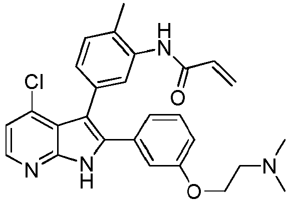 | N-(5-(4-chloro-2-(3-(2-(dimethylamino)ethoxy)phenyl)-1H-pyrrolo[2,3-b]pyridin-3-yl)-2-methylphenyl)acrylamide |
| **29** | 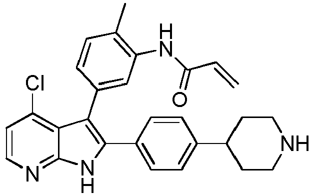 | N-(5-(4-chloro-2-(4-(piperidin-4-yl)phenyl)-1H-pyrrolo[2,3-b]pyridin-3-yl)-2-methylphenyl)acrylamide |
| **30** | 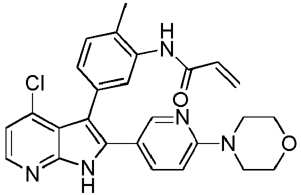 | N-(5-(4-chloro-2-(6-morpholinopyridin-3-yl)-1H-pyrrolo[2,3-b]pyridin-3-yl)-2-methylphenyl)acrylamide |
| **31** | 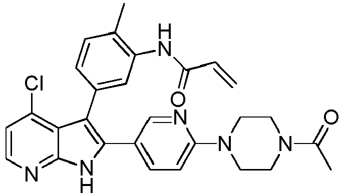 | N-(5-(2-(6-(4-acetylpiperazin-1-yl)pyridin-3-yl)-4-chloro-1H-pyrrolo[2,3-b]pyrid in-3-yl)-2-methylphenyl)acrylamide |
| **32** | 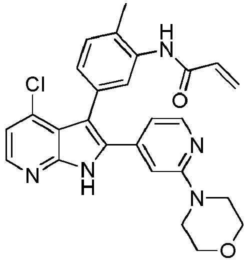 | N-(5-(4-chloro-2-(2-morpholinopyridin-4-yl)-1H-pyrrolo[2,3-b]pyridin-3-yl)-2-methylphenyl)acrylamide |
| **33** | 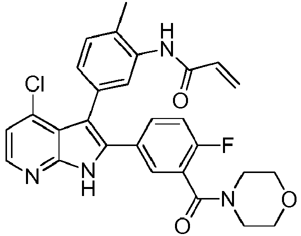 | N-(5-(4-chloro-2-(4-fluoro-3-(morpholine-4-carbonyl)phenyl)-1H-pyrrolo[2,3-b]pyridin-3-yl)-2-methylphenyl)acrylamide |
| **34** | 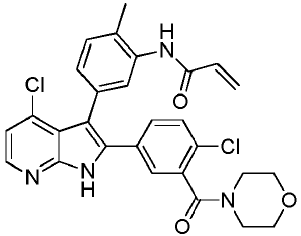 | N-(5-(4-chloro-2-(4-chloro-3-(morpholine-4-carbonyl)phenyl)-1H-pyrrolo[2,3-b]pyridin-3-yl)-2-methylphenyl)acrylamide |
| **35** | 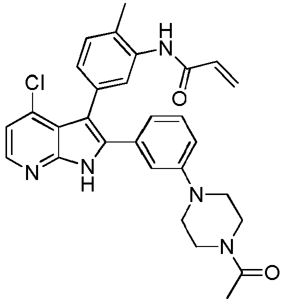 | N-(5-(2-(3-(4-acetylpiperazin-1-yl)phenyl)-4-chloro-1H-pyrrolo[2,3-b]pyridin-3-yl)-2-methylphenyl)acrylamide |
| **36** | 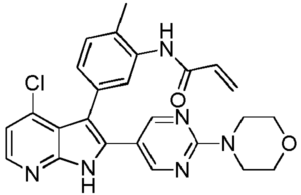 | N-(5-(4-chloro-2-(2-morpholinopyrimidin-5-yl)-1H-pyrrolo[2,3-b]pyridin-3-yl)-2-methylphenyl)acrylamide |
| **37** | 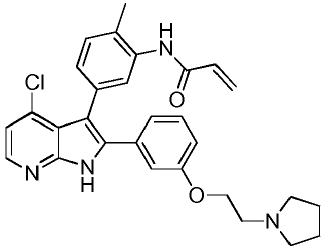 | N-(5-(4-chloro-2-(3-(2-(pyrrolidin-1-yl)ethoxy)phenyl)-1H-pyrrolo[2,3-b]pyridin-3-yl)-2-methylphenyl)acrylamide |
| **38** | 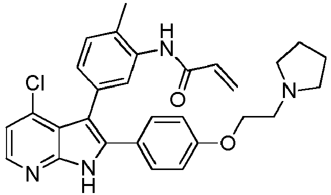 | N-(5-(4-chloro-2-(4-(2-(pyrrolidin-1-yl)ethoxy)phenyl)-1H-pyrrolo[2,3-b]pyridin-3-yl)-2-methylphenyl)acrylamide |
| **39** | 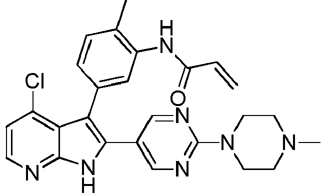 | N-(5-(4-chloro-2-(2-(4-methylpiperazin-1-yl)pyrimidin-5-yl)-1H-pyrrolo[2,3-b]pyridin-3-yl)-2-methylphenyl)acrylamide |
| **40** | 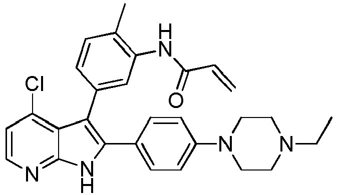 | N-(5-(4-chloro-2-(4-(4-ethylpiperazin-1-yl)phenyl)-1H-pyrrolo[2,3-b]pyridin-3-yl)-2-methylphenyl)acrylamide |
| **41** | 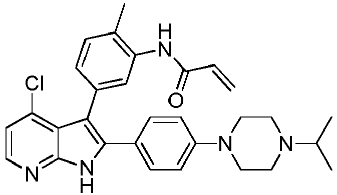 | N-(5-(4-chloro-2-(4-(4-isopropylpiperazin-1-yl)phenyl)-1H-pyrrolo[2,3-b]pyridin-3-yl)-2-methylphenyl)acrylamide |
| **42** | 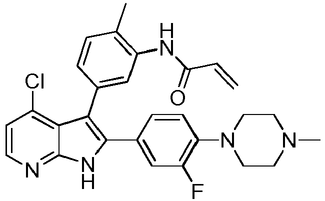 | N-(5-(4-chloro-2-(3-fluoro-4-(4-methylpiperazin-1-yl)phenyl)-1H-pyrrolo[2,3-b]pyridin-3-yl)-2-methylphenyl)acrylamide |
| **43** | 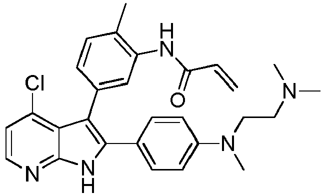 | N-(5-(4-chloro-2-(4-((2-(dimethylamino)ethyl)(methyl)ami no)phenyl)-1H-pyrrolo[2,3-b]pyridin-3-yl)-2-methylphenyl)acrylamide |
| **44** | 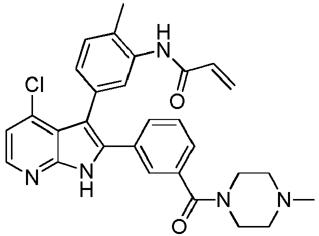 | N-(5-(4-chloro-2-(3-(4-methylpiperazine-1-carbonyl)phenyl)-1H-pyrrolo[2,3-b]pyridin-3-yl)-2-methylphenyl)acrylamide |
| **45** | 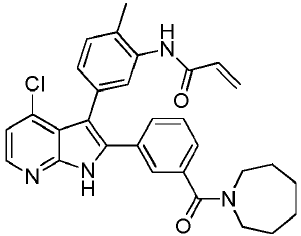 | N-(5-(2-(3-(azepane-1-carbonyl)phenyl)-4-chloro-1H-pyrrolo[2,3-b]pyridin-3-yl)-2-methylphenyl)acrylamide |
| **46** | 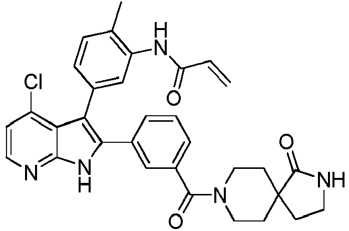 | N-(5-(4-chloro-2-(3-(1-oxo-2,8-diazaspiro[4.5]decane-8-carbonyl)phenyl)-1H-pyrrolo[2,3-b]pyridin-3-yl)-2-methylphenyl)acrylamide |
| **47** | 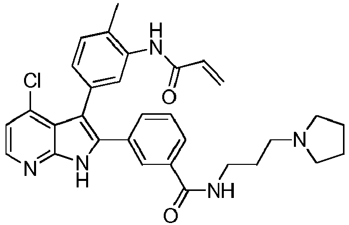 | 3-(3-(3-acrylamido-4-methylphenyl)-4-chloro-1H-pyrrolo[2,3-b]pyridin-2-yl)-N-(3-(pyrrolidin-1-yl)propyl)benzamide |
| **48** | 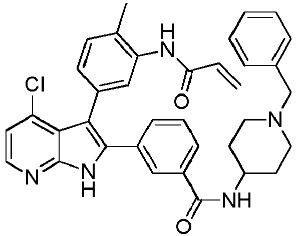 | 3-(3-(3-acrylamido-4-methylphenyl)-4-chloro-1H-pyrrolo[2,3-b]pyridin-2-yl)-N-(1-benzylpiperidin-4-yl)benzamide |
| **49** | 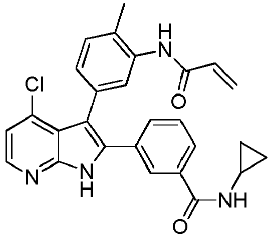 | 3-(3-(3-acrylamido-4-methylphenyl)-4-chloro-1H-pyrrolo[2,3-b]pyridin-2-yl)-N-cyclopropylbenzamide |
| **50** | 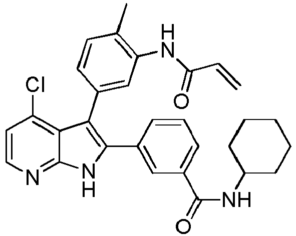 | 3-(3-(3-acrylamido-4-methylphenyl)-4-chloro-1H-pyrrolo[2,3-b]pyridin-2-yl)-N-cyclohexylbenzamide |
| **51** | 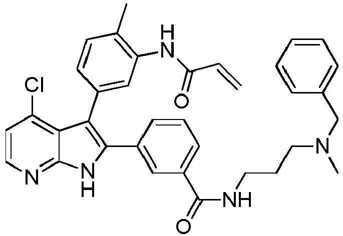 | 3-(3-(3-acrylamido-4-methylphenyl)-4-chloro-1H-pyrrolo[2,3-b]pyridin-2-yl)-N-(3-(benzyl(methyl)amino)propyl)ben zamide |
| **52** | 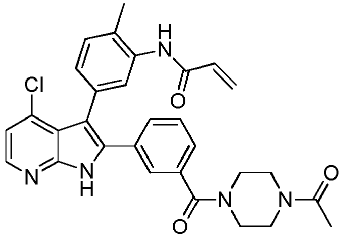 | N-(5-(2-(3-(4-acetylpiperazine-1-carbonyl)phenyl)-4-chloro-1H-pyrrolo[2,3-b]pyridin-3-yl)-2-methylphenyl)acrylamide |
| **53** | 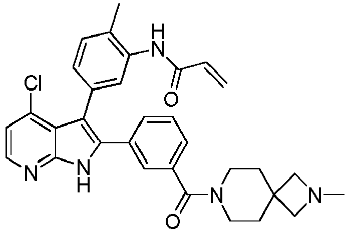 | N-(5-(4-chloro-2-(3-(2-methyl-2,7-diazaspiro[3.5]nonane-7-carbonyl)phenyl)-1H-pyrrolo[2,3-b]pyridin-3-yl)-2-methylphenyl)acrylamide |
| **54** | 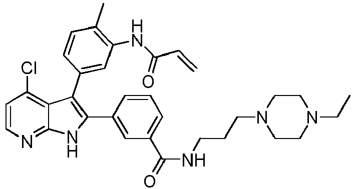 | 3-(3-(3-acrylamido-4-methylphenyl)-4-chloro-1H-pyrrolo[2,3-b]pyridin-2-yl)-N-(3-(4-ethylpiperazin-1-yl)propyl)benzamide |
| **55** | 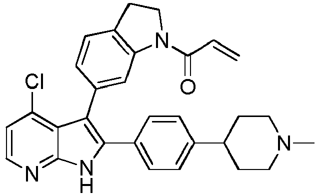 | 1-(6-(4-chloro-2-(4-(1-methylpiperidin-4-yl)phenyl)-1H-pyrrolo[2,3-b]pyridin-3-yl)indolin-1-yl)prop-2-en-1-one |
| **56** | 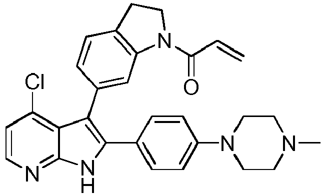 | 1-(6-(4-chloro-2-(4-(4-methylpiperazin-1-yl)phenyl)-1H-pyrrolo[2,3-b]pyridin-3-yl)indolin-1-yl)prop-2-en-1-one |
| **57** | 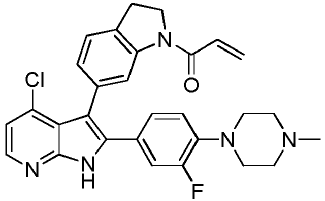 | 1-(6-(4-chloro-2-(3-fluoro-4-(4-methylpiperazin-1-yl)phenyl)-1H-pyrrolo[2,3-b]pyridin-3-yl)indolin-1-yl)prop-2-en-1-one |
| **58** | 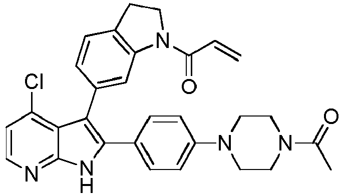 | 1-(6-(2-(4-(4-acetylpiperazin-1-yl)phenyl)-4-chloro-1H-pyrrolo[2,3-b]pyridin-3-yl)indolin-1-yl)prop-2-en-1-one |
| **59** | 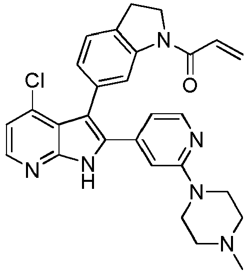 | 1-(6-(4-chloro-2-(2-(4-methylpiperazin-1-yl)pyridin-4-yl)-1H-pyrrolo[2,3-b]pyridin-3-yl)indolin-1-yl)prop-2-en-1-one |
| **60** | 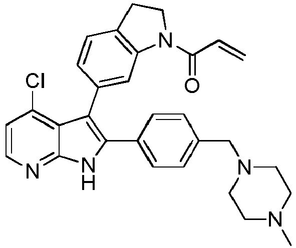 | 1-(6-(4-chloro-2-(4-((4-methylpiperazin-1-yl)methyl)phenyl)-1H-pyrrolo[2,3-b]pyridin-3-yl)indolin-1-yl)prop-2-en-1-one |
| **61** | 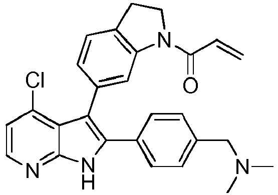 | 1-(6-(4-chloro-2-(4-((dimethylamino)methyl)phenyl)-1H-pyrrolo[2,3-b]pyridin-3-yl)indolin-1-yl)prop-2-en-1-one |
| **62** | 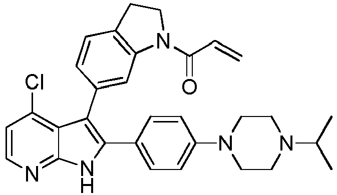 | 1-(6-(4-chloro-2-(4-(4-isopropylpiperazin-1-yl)phenyl)-1H-pyrrolo[2,3-b]pyridin-3-yl)indolin-1-yl)prop-2-en-1-one |
| **63** | 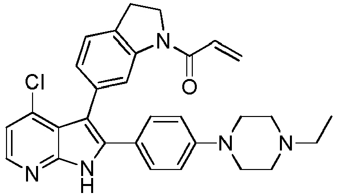 | 1-(6-(4-chloro-2-(4-(4-ethylpiperazin-1-yl)phenyl)-1H-pyrrolo[2,3-b]pyridin-3-yl)indolin-1-yl)prop-2-en-1-one |
| **64** | 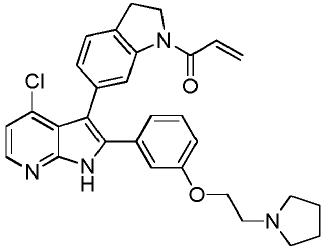 | 1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one |
| **65** | 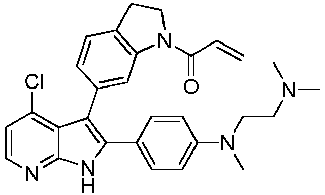 | 1-(6-(4-chloro-2-(4-((2-(dimethylamino)ethyl)(methyl)ami no)phenyl)-1H-pyrrolo[2,3-b]pyridin-3-yl)indolin-1-yl)prop-2-en-1-one |
| **66** | 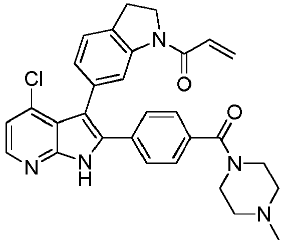 | 1-(6-(4-chloro-2-(4-(4-methylpiperazine-1-carbonyl)phenyl)-1H-pyrrolo[2,3-b]pyridin-3-yl)indolin-1-yl)prop-2-en-1-one |
| **67** | 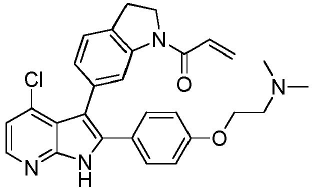 | 1-(6-(4-chloro-2-(4-(2-(dimethylamino)ethoxy)phenyl)-1H-pyrrolo[2,3-b]pyridin-3-yl)indolin-1-yl)prop-2-en-1-one |
| **68** | 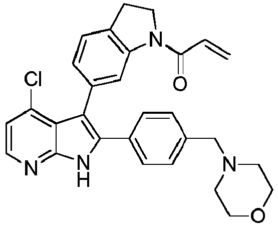 | 1-(6-(4-chloro-2-(4-(morpholinomethyl)phenyl)-1H-pyrrolo[2,3-b]pyridin-3-yl)indolin-1-yl)prop-2-en-1-one |
| **69** | 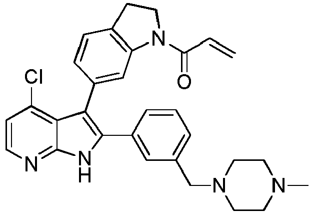 | 1-(6-(4-chloro-2-(3-((4-methylpiperazin-1-yl)methyl)phenyl)-1H-pyrrolo[2,3-b]pyridin-3-yl)indolin-1-yl)prop-2-en-1-one |
| **70** | 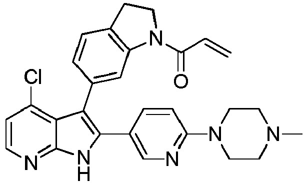 | 1-(6-(4-chloro-2-(6-(4-methylpiperazin-1-yl)pyridin-3-yl)-1H-pyrrolo[2,3-b]pyridin-3-yl)indolin-1-yl)prop-2-en-1-one |
| **71** | 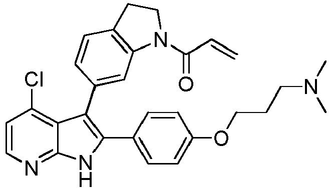 | 1-(6-(4-chloro-2-(4-(3-(dimethylamino)propoxy)phenyl)-1H-pyrrolo[2,3-b]pyridin-3-yl)indolin-1-yl)prop-2-en-1-one |
| **72** | 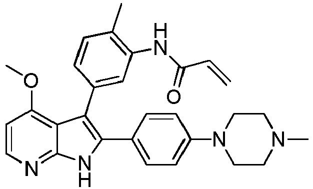 | N-(5-(4-methoxy-2-(4-(4-methylpiperazin-1-yl)phenyl)-1H-pyrrolo[2,3-b]pyridin-3-yl)-2-methylphenyl)acrylamide |
| **73** | 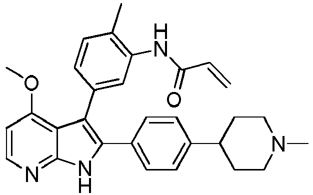 | N-(5-(4-methoxy-2-(4-(1-methylpiperidin-4-yl)phenyl)-1H-pyrrolo[2,3-b]pyridin-3-yl)-2-methylphenyl)acrylamide |
| **74** | 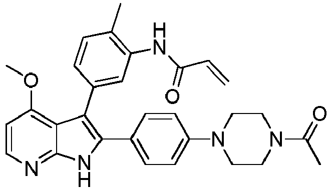 | N-(5-(2-(4-(4-acetylpiperazin-1-yl)phenyl)-4-methoxy-1H-pyrrolo[2,3-b]pyridin-3-yl)-2-methylphenyl)acrylamide |
| **75** | 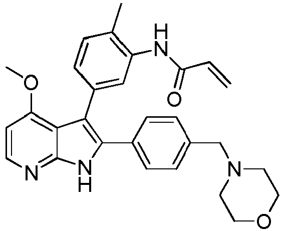 | N-(5-(4-methoxy-2-(4-(morpholinomethyl)phenyl)-1H-pyrrolo[2,3-b]pyridin-3-yl)-2-methylphenyl)acrylamide |
| **76** | 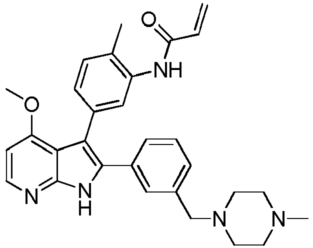 | N-(5-(4-methoxy-2-(3-((4-methylpiperazin-1-yl)methyl)phenyl)-1H-pyrrolo[2,3-b]pyridin-3-yl)-2-methylphenyl)acrylamide |
| **77** | 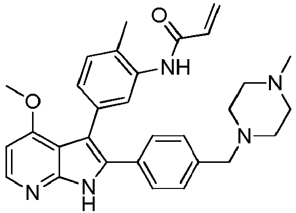 | N-(5-(4-methoxy-2-(4-((4-methylpiperazin-1-yl)methyl)phenyl)-1H-pyrrolo[2,3-b]pyridin-3-yl)-2-methylphenyl)acrylamide |
| **78** | 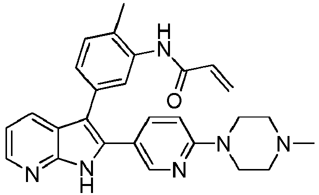 | N-(2-methyl-5-(2-(6-(4-methylpiperazin-1-yl)pyridin-3-yl)-1H-pyrrolo[2,3-b]pyridin-3-yl)phenyl)acrylamide |
| **79** | 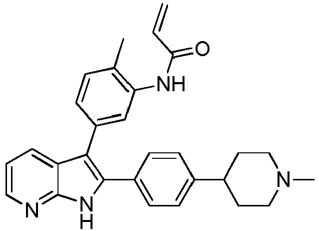 | N-(2-methyl-5-(2-(4-(1-methylpiperidin-4-yl)phenyl)-1H-pyrrolo[2,3-b]pyridin-3-yl)phenyl)acrylamide |
| **80** | 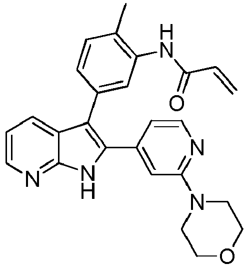 | N-(2-methyl-5-(2-(2-morpholinopyridin-4-yl)-1H-pyrrolo[2,3-b]pyrid in-3-yl)phenyl)acrylamide |
| **81** | 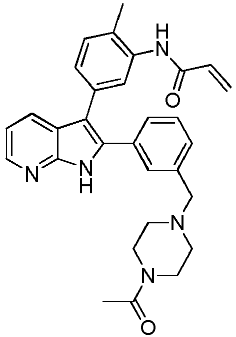 | N-(5-(2-(3-((4-acetylpiperazin-1 - yl)methyl)phenyl)-1H-pyrrolo[2,3-b]pyridin-3-yl)-2-methylphenyl)acrylamide |
| **82** | 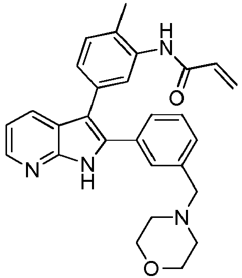 | N-(2-methyl-5-(2-(3-(morpholinomethyl)phenyl)-1H-pyrrolo[2,3-b]pyridin-3-yl)phenyl)acrylamide |
| **83** | 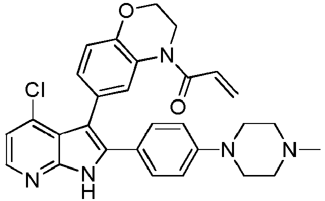 | 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one |
| **84** | 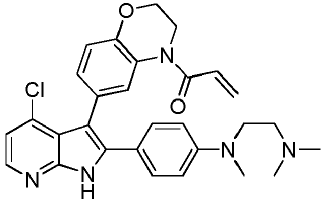 | 1-(6-(4-chloro-2-(4-((2-(dimethylamino)ethyl)(methyl)ami no)phenyl)-1H-pyrrolo[2,3-b]pyridin-3-yl)-2H-benzo[b][1,4]oxazin-4(3H)-yl)prop-2-en-1-one |
| **85** | 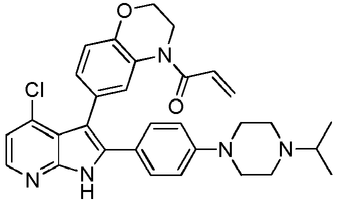 | 1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one |
| **86** | 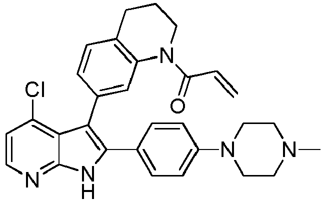 | 1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one |
| **87** | 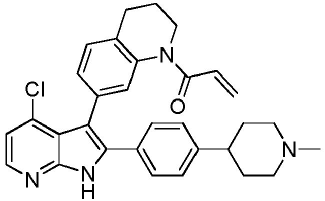 | 1-(7-(4-chloro-2-(4-(1-methylpiperidin-4-yl)phenyl)-1H-pyrrolo[2,3-b]pyridin-3-yl)-3,4-dihydroquinolin-1(2H)-yl)prop-2-en-1-one |
| **88** | 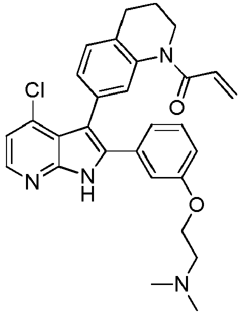 | 1-(7-(4-chloro-2-(3-(2-(dimethylamino)ethoxy)phenyl)-1H-pyrrolo[2,3-b]pyridin-3-yl)-3,4-dihydroquinolin-1(2H)-yl)prop-2-en-1-one |
| **89** | 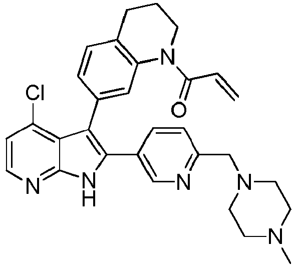 | 1-(7-(4-chloro-2-(6-((4-methylpiperazin-1-yl)methyl)pyridin-3-yl)-1H-pyrrolo[2,3-b]pyridin-3-yl)-3,4-dihydroquinolin-1(2H)-yl)prop-2-en-1-one |
| **90** | 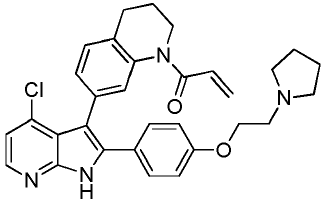 | 1-(7-(4-chloro-2-(4-(2-(pyrrolidin-1-yl)ethoxy)phenyl)-1H-pyrrolo[2,3-b]pyridin-3-yl)-3,4-dihydroquinolin-1(2H)-yl)prop-2-en-1-one |
| **91** | 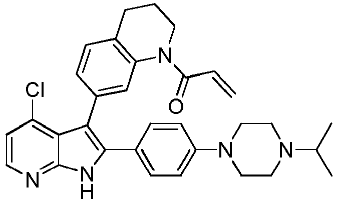 | 1-(7-(4-chloro-2-(4-(4-isopropylpiperazin-1-yl)phenyl)-1H-pyrrolo[2,3-b]pyridin-3-yl)-3,4-dihydroquinolin-1(2H)-yl)prop-2-en-1-one |
| **92** | 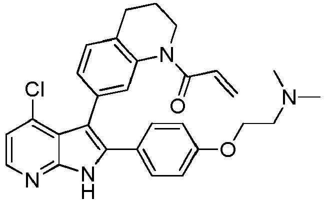 | 1-(7-(4-chloro-2-(4-(2-(dimethylamino)ethoxy)phenyl)-1H-pyrrolo[2,3-b]pyridin-3-yl)-3,4-dihydroquinolin-1(2H)-yl)prop-2-en-1-one |
| **93** | 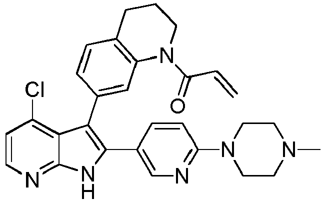 | 1-(7-(4-chloro-2-(6-(4-methylpiperazin-1-yl)pyridin-3-yl)-1H-pyrrolo[2,3-b]pyridin-3-yl)-3,4-dihydroquinolin-1(2H)-yl)prop-2-en-1-one |
| **94** | 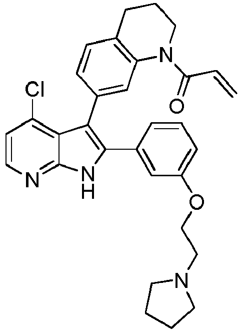 | 1-(7-(4-chloro-2-(3-(2-(pyrrolidin-1-yl)ethoxy)phenyl)-1H-pyrrolo[2,3-b]pyridin-3-yl)-3,4-dihydroquinolin-1(2H)-yl)prop-2-en-1-one |
| **95** | 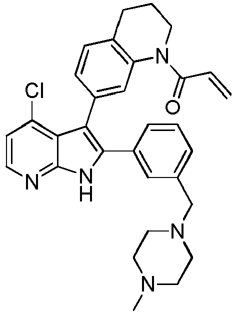 | 1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one |
| **96** | 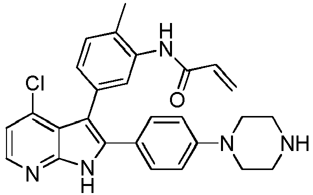 | N-(5-(4-chloro-2-(4-(piperazin-1-yl)phenyl)-1H-pyrrolo[2,3-b]pyridin-3-yl)-2-methylphenyl)acrylamide |
| **97** | 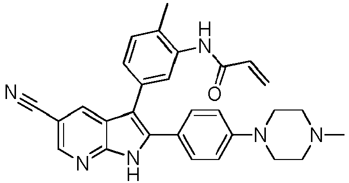 | N-(5-(5-cyano-2-(4-(4-methylpiperazin-1-yl)phenyl)-1H-pyrrolo[2,3-b]pyridin-3-yl)-2-methylphenyl)acrylamide |
| **98** | 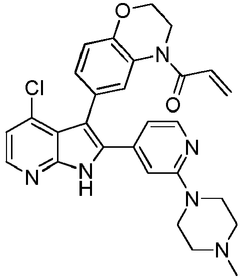 | 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one |
| **99** | 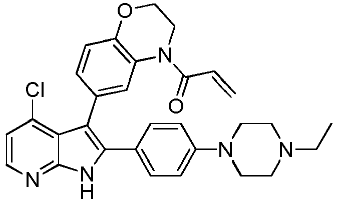 | 1-(6-(4-chloro-2-(4-(4-ethylpiperazin-1-yl)phenyl)-1H-pyrrolo[2,3-b]pyridin-3-yl)-2H-benzo[b][1,4]oxazin-4(3H)-yl)prop-2-en-1-one |
| **100** | 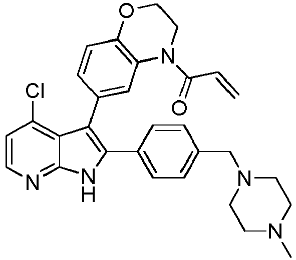 | 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one |
| **101** | 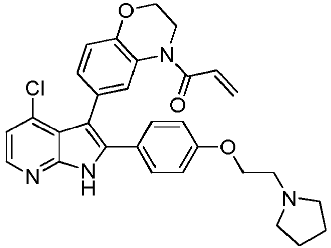 | 1-(6-(4-chloro-2-(4-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one |
| **102** | 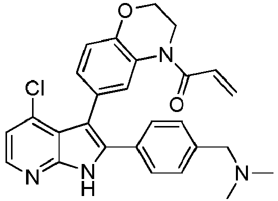 | 1-(6-(4-chloro-2-(4-((dimethylamino)methyl)phenyl)-1H-pyrrolo[2,3-b]pyridin-3-yl)-2H-benzo[b][1,4]oxazin-4(3H)-yl)prop-2-en-1-one |
| **103** | 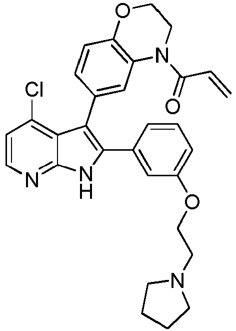 | 1-(6-(4-chloro-2-(3-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one |
| **104** | 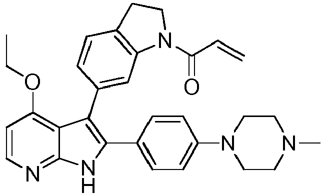 | 1-(6-(4-ethoxy-2-(4-(4-methylpiperazin-1-yl)phenyl)-1H-pyrrolo[2,3-b]pyridin-3-yl)indolin-1-yl)prop-2-en-1-one |
| **105** | 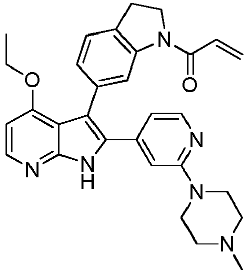 | 1-(6-(4-ethoxy-2-(4-(4-methylpiperazin-1-yl)phenyl)-1H-pyrrolo[2,3-b]pyridin-3-yl)indolin-1-yl)prop-2-en-1-one |
| **106** | 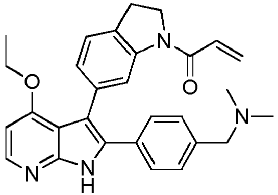 | 1-(6-(2-(4-((dimethylamino)methyl)phenyl)-4-ethoxy-1H-pyrrolo[2,3-b]pyridin-3-yl)indolin-1-yl)prop-2-en-1-one |
| **107** | 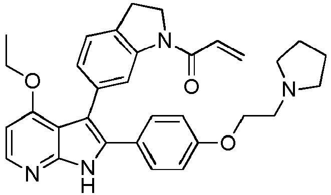 | 1-(6-(4-ethoxy-2-(4-(2-(pyrrolidin-1-yl)ethoxy)phenyl)-1H-pyrrolo[2,3-b]pyridin-3-yl)indolin-1-yl)prop-2-en-1-one |
| **108** | 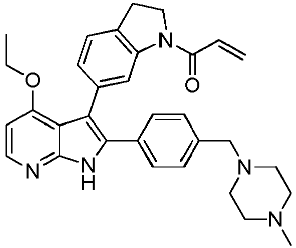 | 1-(6-(4-ethoxy-2-(4-((4-methylpiperazin-1-yl)methyl)phenyl)-1H-pyrrolo[2,3-b]pyridin-3-yl)indolin-1-yl)prop-2-en-1-one |
| **109** | 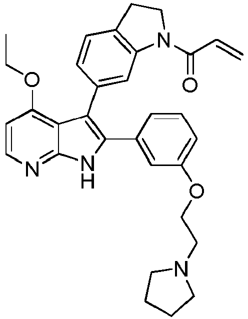 | 1-(6-(4-ethoxy-2-(3-(2-(pyrrolidin-1 -yl)ethoxy)phenyl)-1H-pyrrolo[2,3-b]pyridin-3-yl)indolin-1-yl)prop-2-en-1-one |
| **110** | 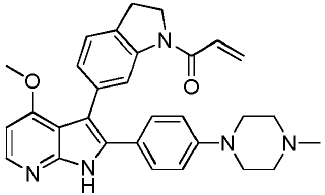 | 1-(6-(4-methoxy-2-(4-(4-methylpiperazin-1-yl)phenyl)-1H-pyrrolo[2,3-b]pyridin-3-yl)indolin-1-yl)prop-2-en-1 -one |
| **111** | 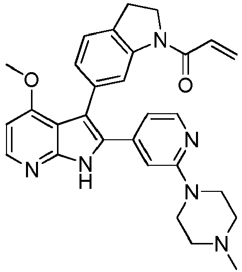 | 1-(6-(4-methoxy-2-(2-(4-methylpiperazin-1-yl)pyridin-4-yl)-1H-pyrrolo[2,3-b]pyridin-3-yl)indolin-1-yl)prop-2-en-1 -one |
| **112** | 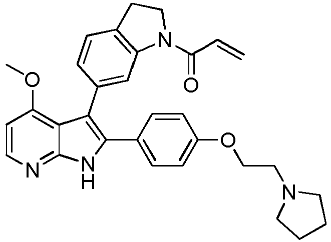 | 1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1 -one |
| **113** | 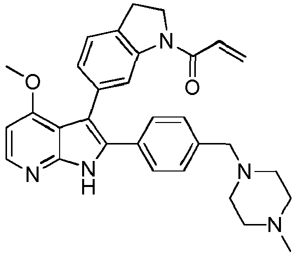 | 1-(6-(4-methoxy-2-(4-((4-methylpiperazin-1-yl)methyl)phenyl)-1H-pyrrolo[2,3-b]pyridin-3-yl)indolin-1-yl)prop-2-en-1-one |
| **114** | 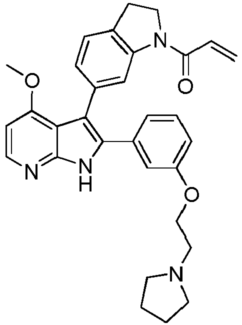 | 1-(6-(4-methoxy-2-(3-(2-(pyrrol idin-1-yl)ethoxy)phenyl)-1H-pyrrolo[2,3-b]pyridin-3-yl)indolin-1-yl)prop-2-en-1 -one |
| **115** | 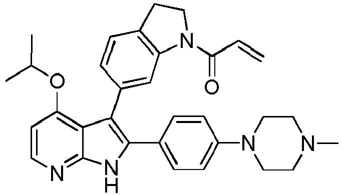 | 1-(6-(4-isopropoxy-2-(4-(4-methylpiperazin-1-yl)phenyl)-1H-pyrrolo[2,3-b]pyridin-3-yl)indolin-1-yl)prop-2-en-1-one |
| **116** | 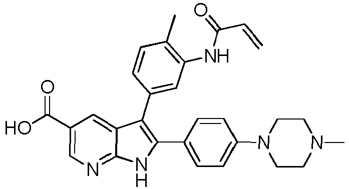 | 3-(3-acrylamido-4-methylphenyl)-2-(4-(4-methylpiperazin-1-yl)phenyl)-1H-pyrrolo[2,3-b]pyridine-5-carboxylic acid |
| **117** | 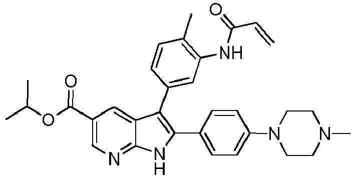 | isopropyl 3-(3-acrylamido-4-methylphenyl)-2-(4-(4-methylpiperazin-1-yl)phenyl)-1H-pyrrolo[2,3-b]pyridine-5-carboxylate |
| **118** | 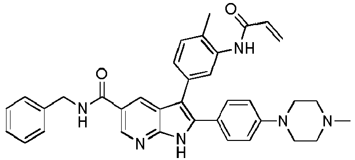 | 3-(3-acrylamido-4-methylphenyl)-N-benzyl-2-(4-(4-methylpiperazin-1-yl)phenyl)-1H-pyrrolo[2,3-b]pyrid in e-5-carboxamide |
| **119** | 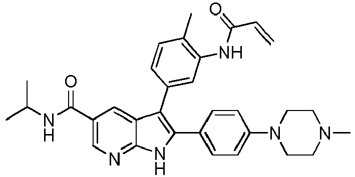 | 3-(3-acrylamido-4-methylphenyl)-N-isopropyl-2-(4-(4-methylpiperazin-1-yl)phenyl)-1H-pyrrolo[2,3-b]pyridine-5-carboxamide |
| **120** | 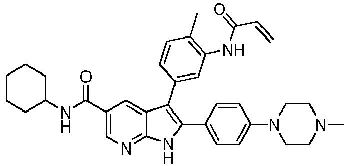 | 3-(3-acrylamido-4-methylphenyl)-N-cyclohexyl-2-(4-(4-methylpiperazin-1-yl)phenyl)-1H-pyrrolo[2,3-b]pyridine-5-carboxamide |
| **121** | 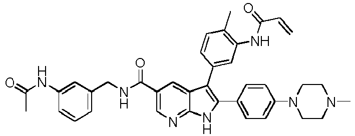 | N-(3-acetamidobenzyl)-3-(3-acryl amido-4-methylphenyl)-2-(4-(4-methylpiperazin-1-yl)phenyl)-1H-pyrrolo[2,3-b]pyridine-5-carboxamide |
| **122** | 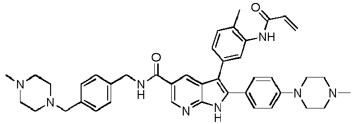 | 3-(3-acrylamido-4-methylphenyl)-N-(4-((4-methylpiperazin-1-yl)methyl)benzyl)-2-(4-(4-methylpiperazin-1-yl)phenyl)-1H-pyrrolo[2,3-b]pyridine-5-carboxamide |
| **123** | 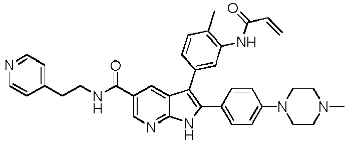 | 3-(3-acrylamido-4-methylphenyl)-2-(4-(4-methylpiperazin-1-yl)phenyl)-N-(2-(pyridin-4-yl)ethyl)-1H-pyrrolo[2,3-blpyridine-5-carboxamide |
| **124** | 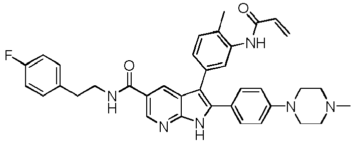 | 3-(3-acrylamido-4-methylphenyl)-N-(4-fluorophenethyl)-2-(4-(4-methylpiperazin-1-yl)phenyl)-1H-pyrrolo[2,3-b]pyridine-5-carboxamide |
| **125** | 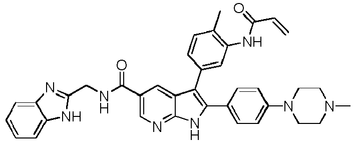 | N-((1H-benzo[d]imidazol-2-yl)methyl)-3-(3-acrylamido-4-methylphenyl)-2-(4-(4-methylpiperazin-1-yl)phenyl)-1H-pyrrolo[2,3-b]pyridine-5-carboxamide |
| **126** | 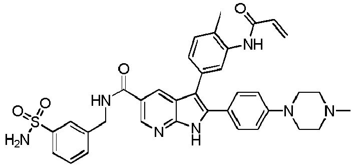 | 3-(3-acrylamido-4-methylphenyl)-2-(4-(4-methylpiperazin-1-yl)phenyl)-N-(3-sulfamoylbenzyl)-1H-pyrrolo[2,3-b]pyridine-5-carboxamide |
| **127** | 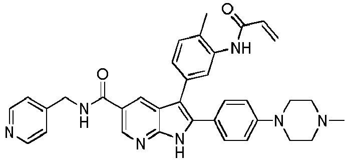 | 3-(3-acrylamido-4-methylphenyl)-2-(4-(4-methylpiperazin-1-yl)phenyl)-N-(pyridin-4-ylmethyl)-1H-pyrrolo[2,3-b]pyridine-5-carboxamide |
| **128** | 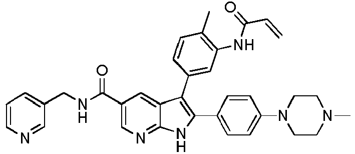 | 3-(3-acrylamido-4-methylphenyl)-2-(4-(4-methylpiperazin-1-yl)phenyl)-N-(pyridin-3-ylmethyl)-1H-pyrrolo[2,3-b]pyridine-5-carboxamide |
| **129** | 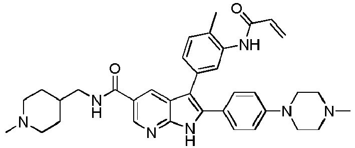 | 3-(3-acrylamido-4-methylphenyl)-2-(4-(4-methylpiperazin-1-yl)phenyl)-N-((1-methylpiperidin-4-yl)methyl)-1H-pyrrolo[2,3-b]pyridine-5-carboxamide |
| **130** | 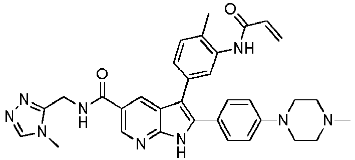 | 3-(3-acrylamido-4-methylphenyl)-N-((4-methyl-4H-1,2,4-triazol-3-yl)methyl)-2-(4-(4-methylpiperazin-1-yl)phenyl)-1H-pyrrolo[2,3-b]pyridine-5-carboxamide |
| **131** | 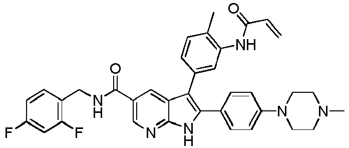 | 3-(3-acrylamido-4-methylphenyl)-N-(2,4-difluorobenzyl)-2-(4-(4-methylpiperazin-1-yl)phenyl)-1H-pyrrolo[2,3-b]pyridine-5-carboxamide |
| **132** | 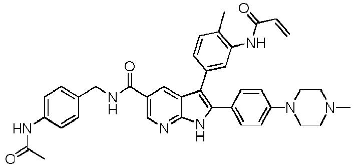 | N-(4-acetamidobenzyl)-3-(3-acrylamido-4-methylphenyl)-2-(4-(4-methylpiperazin-1-yl)phenyl)-1H-pyrrolo[2,3-b]pyridine-5-carboxamide |
| **133** | 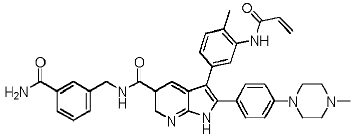 | 3-(3-acrylamido-4-methylphenyl)-N-(3-carbamoylbenzyl)-2-(4-(4-methylpiperazin-1-yl)phenyl)-1H-pyrrolo[2,3-b]pyridine-5-carboxamide |
| **134** | 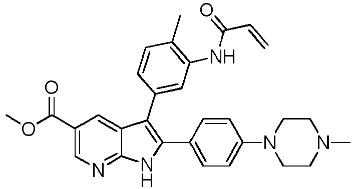 | methyl 3-(3-acrylamido-4-methylphenyl)-2-(4-(4-methylpiperazin-1-yl)phenyl)-1H-pyrrolo[2,3-b]pyridine-5-carboxylate |
| **135** | 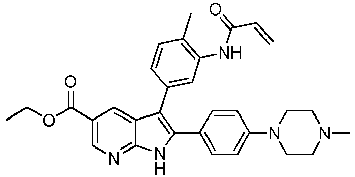 | ethyl 3-(3-acrylamido-4-methylphenyl)-2-(4-(4-methylpiperazin-1-yl)phenyl)-1H-pyrrolo[2,3-b]pyridine-5-carboxylate |
| **136** | 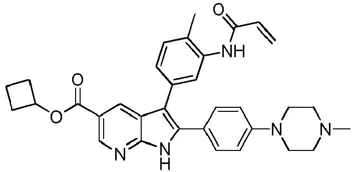 | cyclobutyl 3-(3-acrylamido-4-methylphenyl)-2-(4-(4-methylpiperazin-1-yl)phenyl)-1H-pyrrolo[2,3-b]pyridine-5-carboxylate |
| **137** | 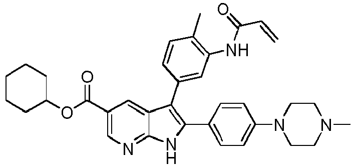 | cyclohexyl 3-(3-acrylamido-4-methylphenyl)-2-(4-(4-methylpiperazin-1-yl)phenyl)-1H-pyrrolo[2,3-b]pyridine-5-carboxylate |
| **138** | 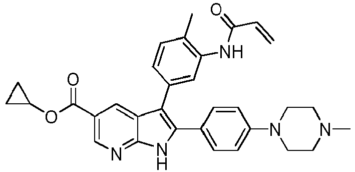 | cyclopropyl 3-(3-acrylamido-4-methylphenyl)-2-(4-(4-methylpiperazin-1-yl)phenyl)-1H-pyrrolo[2,3-b]pyridine-5-carboxylate |
| **139** | 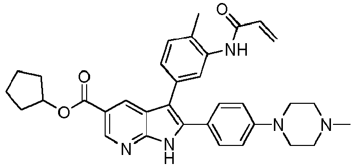 | cyclopentyl 3-(3-acrylamido-4-methylphenyl)-2-(4-(4-methylpiperazin-1-yl)phenyl)-1H-pyrrolo[2,3-b]pyridine-5-carboxylate |
| **140** | 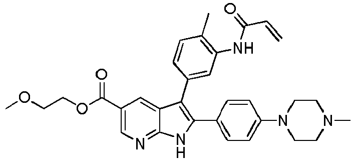 | 2-methoxyethyl 3-(3-acrylamido-4-methylphenyl)-2-(4-(4-methylpiperazin-1-yl)phenyl)-1H-pyrrolo[2,3-b]pyridine-5-carboxylate |
| **141** | 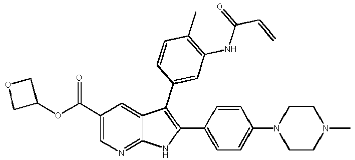 | oxetan-3-yl 3-(3-acrylamido-4-methylphenyl)-2-(4-(4-methylpiperazin-1-yl)phenyl)-1H-pyrrolo[2,3-b]pyridine-5-carboxylate |
| **142** | 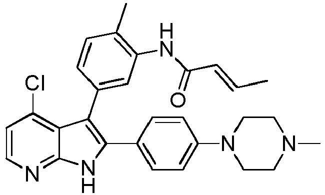 | (E)-N-(5-(4-chloro-2-(4-(4-methylpiperazin-1-yl)phenyl)-1H-pyrrolo[2,3-b]pyridin-3-yl)-2-methylphenyl)but-2-enamide |
| **143** | 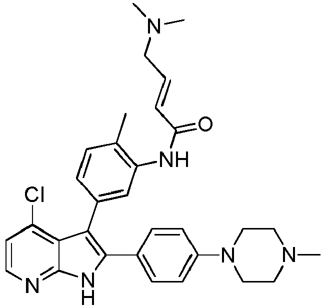 | (E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide |
| **144** | 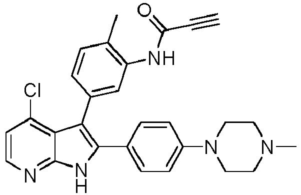 | N-(5-(4-chloro-2-(4-(4-methylpiperazin-1-yl)phenyl)-1H-pyrrolo[2,3-b]pyridin-3-yl)-2-methyl phenyl)propiolamide |
| **145** | 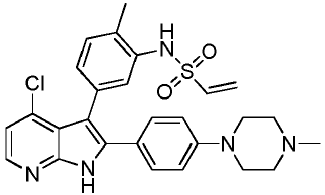 | N-(5-(4-chloro-2-(4-(4-methylpiperazin-1-yl)phenyl)-1H-pyrrolo[2,3-b]pyridin-3-yl)-2-methylphenyl)-ethenesulfonamide |
| **146** | 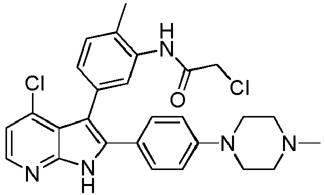 | 2-chloro-N-(5-(4-chloro-2-(4-(4-methylpiperazin-1-yl)phenyl)-1H-pyrrolo[2,3-b]pyridin-3-yl)-2-methyl phenyl)acetamide |
| **147** | 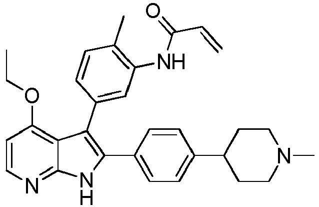 | N-(5-(4-ethoxy-2-(4-(1-methylpiperidin-4-yl)phenyl)-1H-pyrrolo[2,3-b]pyridin-3-yl)-2-methylphenyl)acrylamide |
| **148** | 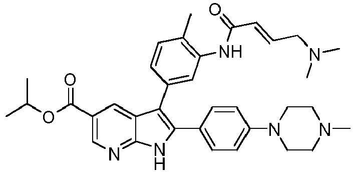 | (E)-isopropyl 3-(3-(4-(dimethylamino)but-2-enamido)-4-methylphenyl)-2-(4-(4-methylpiperazin-1-yl)phenyl)-1H-pyrrolo[2,3-b]pyridine-5-carboxylate |
| **149** | 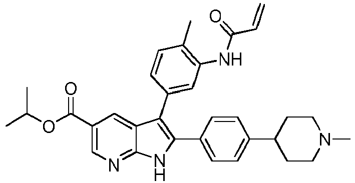 | isopropyl 3-(3-acrylamido-4-methylphenyl)-2-(4-(1-methylpiperidin-4-yl)phenyl)-1H-pyrrolo[2,3-b]pyridine-5-carboxylate |
| **150** | 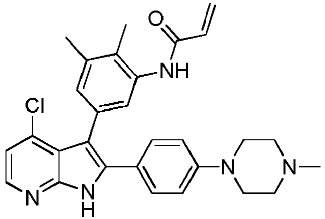 | N-(5-(4-chloro-2-(4-(4-methylpiperazin-1-yl)phenyl)-1H-pyrrolo[2,3-b]pyridin-3-yl)-2,3-dimethylphenyl)acrylamide |
| **151** | 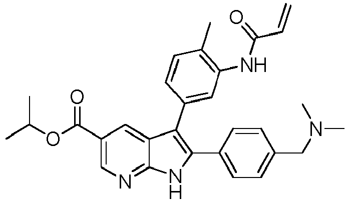 | isopropyl 3-(3-acrylamido-4-methylphenyl)-2-(4-((dimethylamino)methyl)phenyl)-1H-pyrrolo[2,3-b]pyridine-5-carboxylate |
| **152** | 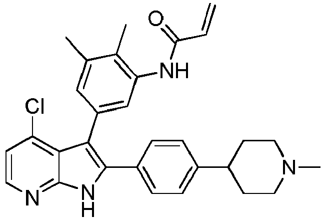 | N-(5-(4-chloro-2-(4-(1-methylpiperidin-4-yl)phenyl)-1H-pyrrolo[2,3-b]pyridin-3-yl)-2,3-dimethylphenyl)acrylamide |
| **153** | 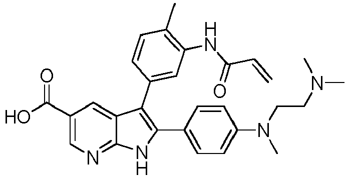 | 3-(3-acrylamido-4-methylphenyl)-2-(4-((2-(dimethylamino)ethyl)(methyl)ami no)phenyl)-1H-pyrrolo[2,3-b]pyridine-5-carboxylic acid |
| **154** | 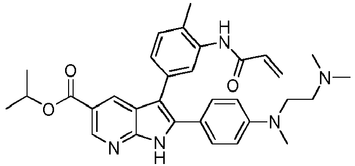 | isopropyl 3-(3-acrylamido-4-methylphenyl)-2-(4-((2-(dimethylamino)ethyl)(methyl)ami no)phenyl)-1H-pyrrolo[2,3-b]pyridine-5-carboxylate |
| **155** | 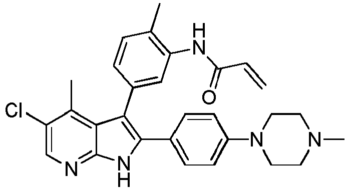 | N-(5-(5-chloro-4-methyl-2-(4-(4-methylpiperazin-1-yl)phenyl)-1H-pyrrolo[2,3-b]pyridin-3-yl)-2-methylphenyl)acrylamide |
| **156** | 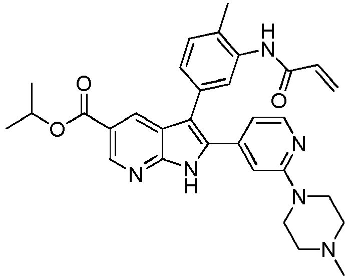 | isopropyl 3-(3-acrylamido-4-methylphenyl)-2-(2-(4-methylpiperazin-1-yl)pyridin-4-yl)-1H-pyrrolo[2,3-b]pyridine-5-carboxylate |
| **157** | 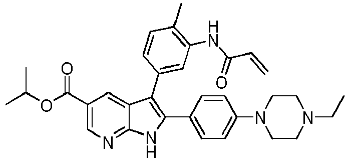 | isopropyl 3-(3-acrylamido-4-methylphenyl)-2-(4-(4-ethylpiperazin-1-yl)phenyl)-1H-pyrrolo[2,3-b]pyridine-5-carboxylate |
| **158** | 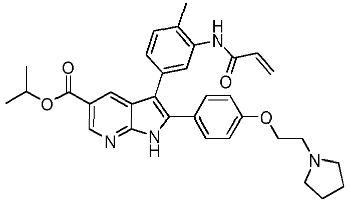 | isopropyl 3-(3-acrylamido-4-methylphenyl)-2-(4-(2-(pyrrolidin-1 -yl)ethoxy)phenyl)-1H-pyrrolo[2,3-b]pyridine-5-carboxylate |
| **159** | 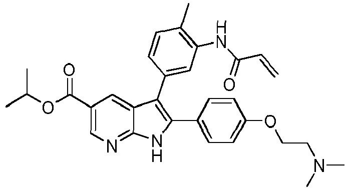 | isopropyl 3-(3-acrylamido-4-methylphenyl)-2-(4-(2-(dimethylamino)ethoxy)phenyl)-1H-pyrrolo[2,3-b]pyridine-5-carboxylate |
| **160** | 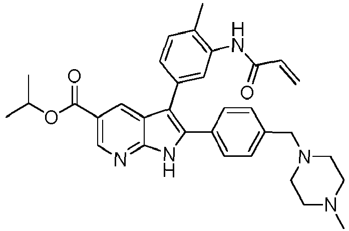 | isopropyl 3-(3-acrylamido-4-methylphenyl)-2-(4-((4-methylpiperazin-1-yl)methyl)phenyl)-1H-pyrrolo[2,3-b]pyridine-5-carboxylate |
| **161** | 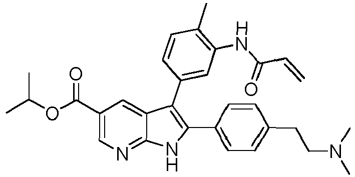 | isopropyl 3-(3-acrylamido-4-methylphenyl)-2-(4-(2-(dimethylamino)ethyl)phenyl)-1 H-pyrrolo[2,3-b]pyridine-5-carboxylate |
| **162** | 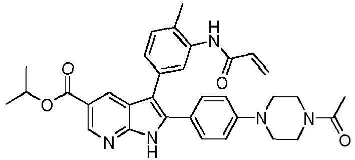 | isopropyl 2-(4-(4-acetylpiperazin-1-yl)phenyl)-3-(3-acrylamido-4-methylphenyl)-1H-pyrrolo[2,3-b]pyridine-5-carboxylate |
| **163** | 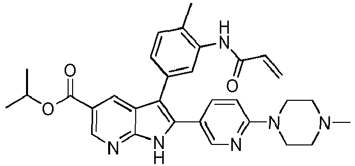 | isopropyl 3-(3-acrylamido-4-methylphenyl)-2-(6-(4-methylpiperazin-1-yl)pyridin-3-yl)-1H-pyrrolo[2,3-b]pyridine-5-carboxylate |
| **164** | 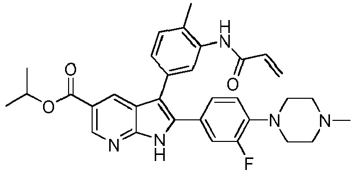 | isopropyl 3-(3-acrylamido-4-methylphenyl)-2-(3-fluoro-4-(4-methylpiperazin-1-yl)phenyl)-1H-pyrrolo[2,3-b]pyridine-5-carboxylate |
| **165** | 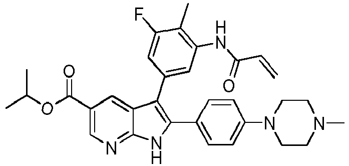 | isopropyl 3-(3-acrylamido-5-fluoro-4-methylphenyl)-2-(4-(4-methylpiperazin-1-yl)phenyl)-1H-pyrrolo[2,3-b]pyridine-5-carboxylate |
| **166** | 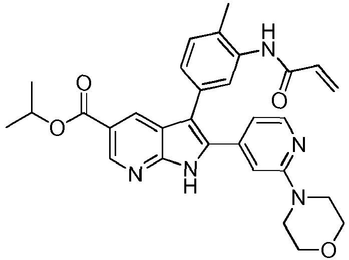 | isopropyl 3-(3-acrylamid0-4-methylphenyl)-2-(2-morpholinopyridin-4-yl)-1H-pyrrolo[2,3-b]pyridine-5-carboxylate |
| **167** | 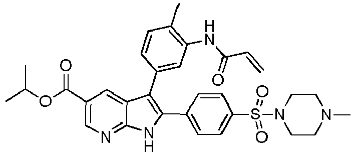 | isopropyl 3-(3-acrylamido-4-methylphenyl)-2-(4-((4-methylpiperazin-1-yl)sulfonyl)phenyl)-1H-pyrrolo[2,3-b]pyridine-5-carboxylate |
| **168** | 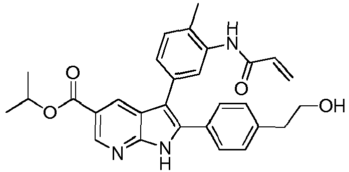 | isopropyl 3-(3-acrylamido-4-methylphenyl)-2-(4-(2-hydroxyethyl)phenyl)-1H-pyrrolo[2,3-b]pyridine-5-carboxylate |
| **169** | 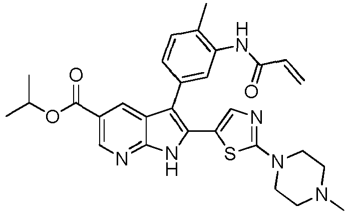 | isopropyl 3-(3-acrylamido-4-methylphenyl)-2-(2-(4-methylpiperazin-1-yl)thiazol-5-yl)-1H-pyrrolo[2,3-b]pyridine-5-carboxylate |
| **170** | 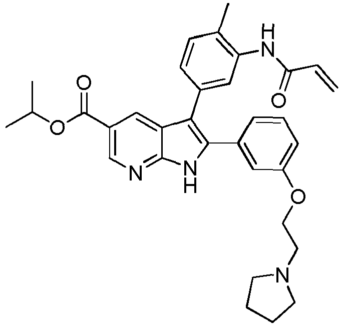 | isopropyl 3-(3-acrylamido-4-methylphenyl)-2-(3-(2-(pyrrolidin-1 -yl)ethoxy)phenyl)-1H-pyrrolo[2,3-b]pyridine-5-carboxylate |
| **171** | 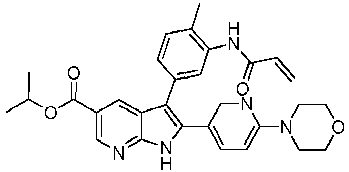 | isopropyl 3-(3-acrylamido-4-methylphenyl)-2-(6-morpholinopyridin-3-yl)-1H-pyrrolo[2,3-b]pyridine-5-carboxylate |
| **172** | 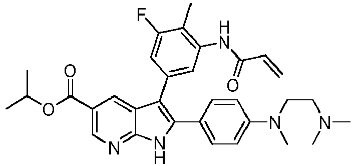 | isopropyl 3-(3-acrylamido-5-fluoro-4-methylphenyl)-2-(4-((2-(dimethylamino)ethyl)(methyl)ami no)phenyl)-1H-pyrrolo[2,3-b]pyridine-5-carboxylate |
| **173** | 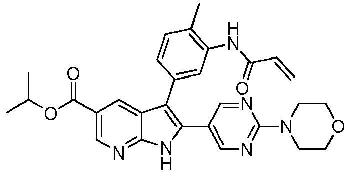 | isopropyl 3-(3-acrylamido-4-methylphenyl)-2-(2-morpholinopyrimidin-5-yl)-1H-pyrrolo[2,3-b]pyridine-5-carboxylate |
| **174** | 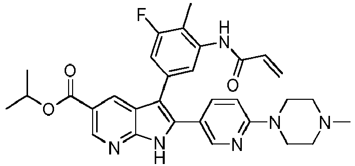 | isopropyl 3-(3-acrylamido-5-fluoro-4-methylphenyl)-2-(6-(4-methylpiperazin-1-yl)pyridin-3-yl)-1H-pyrrolo[2,3-b]pyridine-5-carboxylate |
| **175** | 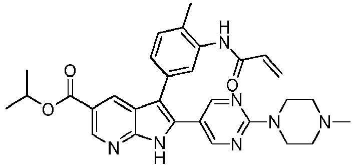 | isopropyl 3-(3-acrylamido-4-methylphenyl)-2-(2-(4-methylpiperazin-1-yl)pyrimidin-5-yl)-1H-pyrrolo[2,3-b]pyridine-5-carboxylate |
| **176** | 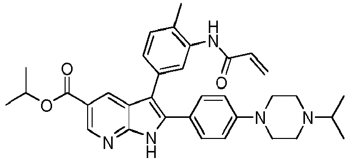 | isopropyl 3-(3-acrylamido-4-methylphenyl)-2-(4-(4-isopropylpiperazin-1-yl)phenyl)-1H-pyrrolo[2,3-b]pyridine-5-carboxylate |
| **177** | 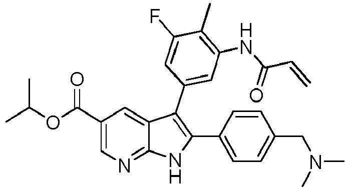 | isopropyl 3-(3-acrylamido-5-fluoro-4-methylphenyl)-2-(4-((dimethylamino)methyl)phenyl)-1H-pyrrolo[2,3-b]pyridine-5-carboxylate |
| **178** | 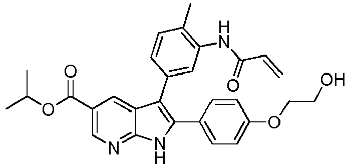 | isopropyl 3-(3-acrylamido-4-methylphenyl)-2-(4-(2-hydroxyethoxy)phenyl)-1 H-pyrrolo[2,3-b]pyridine-5-carboxylate |
| **179** | 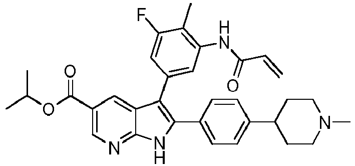 | isopropyl 3-(3-acrylamido-5-fluoro-4-methylphenyl)-2-(4-(1 - methylpiperidin-4-yl)phenyl)-1H-pyrrolo[2,3-b]pyridine-5-carboxylate |
| **180** | 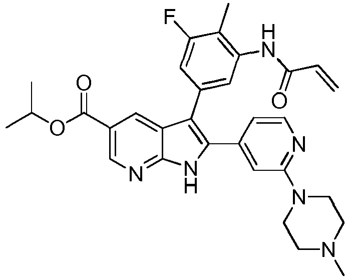 | isopropyl 3-(3-acrylamido-5-fluoro-4-methylphenyl)-2-(2-(4-methylpiperazin-1-yl)pyridin-4-yl)-1H-pyrrolo[2,3-b]pyridine-5-carboxylate |
| **181** | 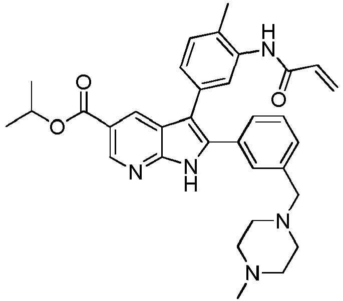 | isopropyl 3-(3-acrylamido-4-methylphenyl)-2-(3-((4-methylpiperazin-1-yl)methyl)phenyl)-1H-pyrrolo[2,3-b]pyrid ine-5-carboxylate |
| **182** | 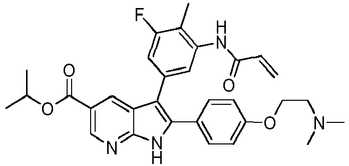 | isopropyl 3-(3-acrylamido-5-fluoro-4-methylphenyl)-2-(4-(2-(dimethylamino)ethoxy)phenyl)-1H-pyrrolo[2,3-b]pyridine-5-carboxylate |
| **183** | 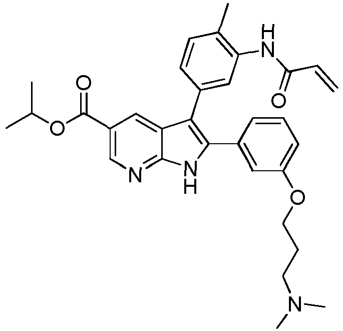 | isopropyl 3-(3-acrylamido-4-methyl phenyl)-2-(3-(3-(dimethylamino)propoxy)phenyl)-1H-pyrrolo[2,3-b]pyridine-5-carboxylate |
| **184** | 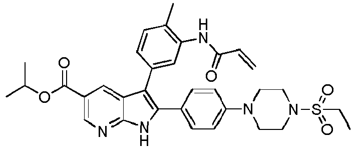 | isopropyl 3-(3-acrylamido-4-methylphenyl)-2-(4-(4-(ethylsulfonyl)piperazin-1-yl)phenyl)-1H-pyrrolo[2,3-b]pyridine-5-carboxylate |
| **185** | 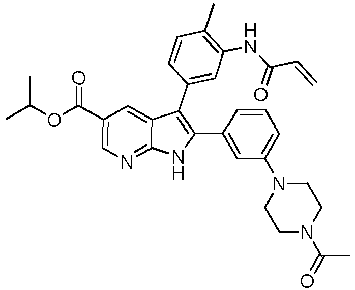 | isopropyl 2-(3-(4-acetylpiperazin-1-yl)phenyl)-3-(3-acrylamido-4-methylphenyl)-1H-pyrrolo[2,3-b]pyridine-5-carboxylate |
| **186** | 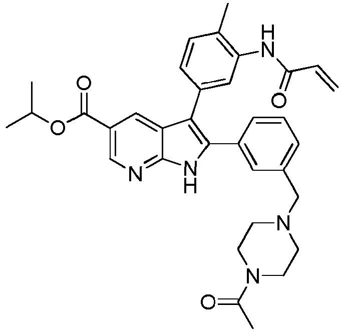 | isopropyl 2-(3-((4-acetylpiperazin-1-yl)methyl)phenyl)-3-(3-acrylamido-4-methylphenyl)-1H-pyrrolo[2,3-b]pyridine-5-carboxylate |
| **187** | 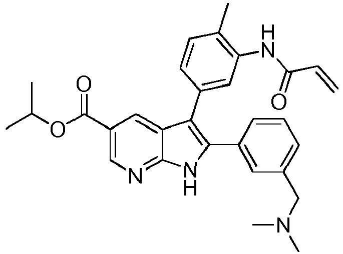 | isopropyl 3-(3-acrylamido-4-methylphenyl)-2-(3-((dimethylamino)methyl)phenyl)-1H-pyrrolo[2,3-b]pyridine-5-carboxylate |
| **188** | 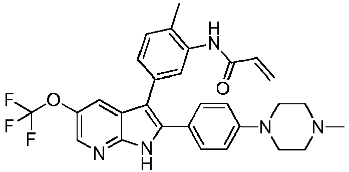 | N-(2-methyl-5-(2-(4-(4-methylpiperazin-1-yl)phenyl)-5-(trifluoromethoxy)-1H-pyrrolo[2,3-b]pyridin-3-yl)phenyl)acrylamide |
| **189** | 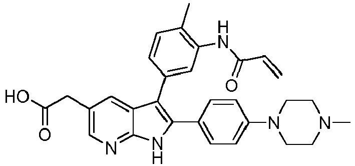 | 2-(3-(3-acrylamido-4-methylphenyl)-2-(4-(4-methylpiperazin-1-yl)phenyl)-1H-pyrrolo[2,3-b]pyridin-5-yl)acetic acid |
| **190** | 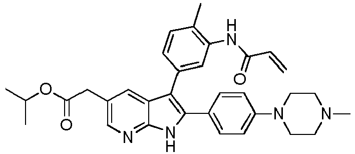 | isopropyl 2-(3-(3-acrylamido-4-methylphenyl)-2-(4-(4-methylpiperazin-1-yl)phenyl)-1H-pyrrolo[2,3-b]pyridin-5-yl)acetate |
| **191** | 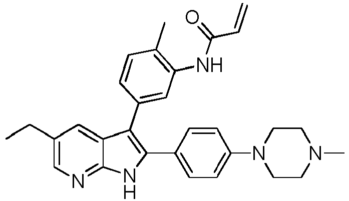 | N-(5-(5-ethyl-2-(4-(4-methylpiperazin-1-yl)phenyl)-1H-pyrrolo[2,3-b]pyridin-3-yl)-2-methyl phenyl)acrylamide |
| **192** | 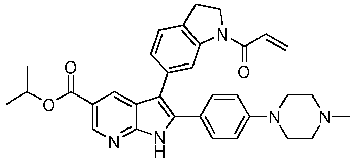 | isopropyl 3-(1-acryloylindolin-6-yl)-2-(4-(4-methylpiperazin-1-yl)phenyl)-1H-pyrrolo[2,3-b]pyridine-5-carboxylate |
| **193** | 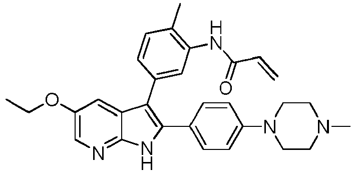 | N-(5-(5-ethoxy-2-(4-(4-methylpiperazin-1-yl)phenyl)-1H-pyrrolo[2,3-b]pyridin-3-yl)-2-methylphenyl)acrylamide |
| **194** | 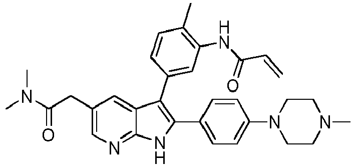 | N-(5-(5-(2-(dimethylamino)-2-oxoethyl)-2-(4-(4-methylpiperazin-1-yl)phenyl)-1H-pyrrolo[2,3-b]pyridin-3-yl)-2-methylphenyl)acrylamide |
| **195** | 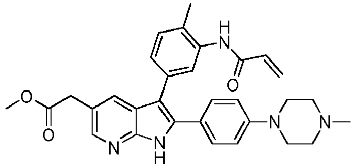 | methyl 2-(3-(3-acrylamido-4-methylphenyl)-2-(4-(4-methylpiperazin-1-yl)phenyl)-1H-pyrrolo[2,3-b]pyridin-5-yl)acetate |
| **196** | 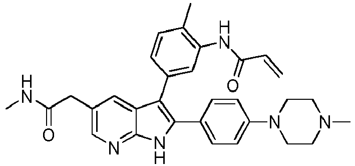 | N-(2-methyl-5-(5-(2-(methylamino)-2-oxoethyl)-2-(4-(4-methylpiperazin-1-yl)phenyl)-1H-pyrrolo[2,3-b]pyridin-3-yl)phenyl)acrylamide |
| **197** | 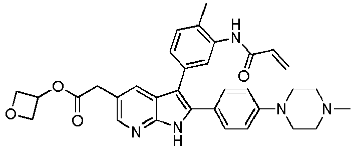 | oxeta n -3-yl 2-(3-(3-acrylamido-4-methylphenyl)-2-(4-(4-methylpiperazin-1-yl)phenyl)-1H-pyrrolo[2,3-b]pyridin-5-yl)acetate |
| **198** | 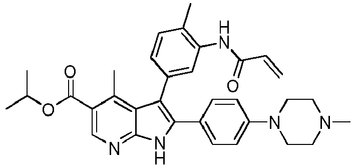 | isopropyl 3-(3-acrylamido-4-methylphenyl)-4-methyl-2-(4-(4-methylpiperazin-1-yl)phenyl)-1H-pyrrolo[2,3-b]pyridine-5-carboxylate |
| **199** | 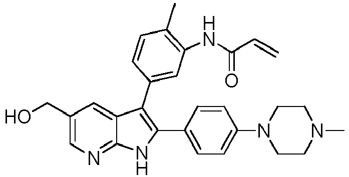 | N-(5-(5-(hydroxymethyl)-2-(4-(4-methylpiperazin-1-yl)phenyl)-1H-pyrrolo[2,3-b]pyridin-3-yl)-2-methyl phenyl)acrylamide |
| **200** | 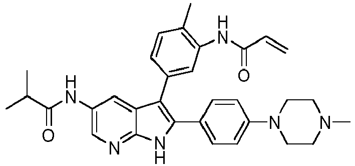 | N-(5-(5-isobutyramido-2-(4-(4-methylpiperazin-1-yl)phenyl)-1H-pyrrolo[2,3-b]pyridin-3-yl)-2-methyl phenyl)acrylamide |
| **201** | 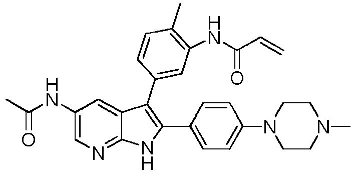 | N-(5-(5-acetamido-2-(4-(4-methylpiperazin-1-yl)phenyl)-1H-pyrrolo[2,3-b]pyridin-3-yl)-2-methylphenyl)acrylamide |
| **202** | 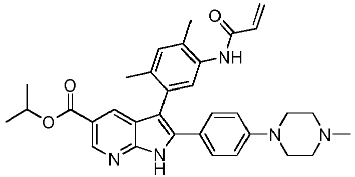 | isopropyl 3-(5-acrylamido-2,4-dimethylphenyl)-2-(4-(4-methylpiperazin-1-yl)phenyl)-1H-pyrrolo[2,3-b]pyridine-5-carboxylate |
| **203** | 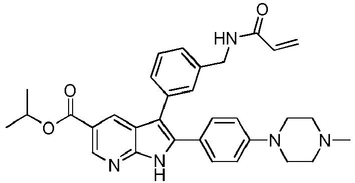 | isopropyl 3-(3-(acrylamidomethyl)phenyl)-2-(4-(4-methylpiperazin-1-yl)phenyl)-1H-pyrrolo[2,3-b]pyridine-5-carboxylate |
| **204** | 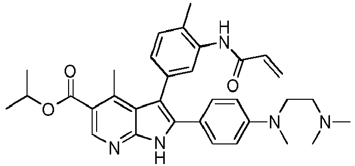 | isopropyl 3-(3-acrylamido-4-methylphenyl)-2-(4-((2-(dimethylamino)ethyl)(methyl)ami no)phenyl)-4-methyl-1H-pyrrolo[2,3-b]pyridine-5-carboxylate |
| **205** | 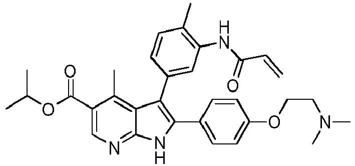 | isopropyl 3-(3-acrylamido-4-methylphenyl)-2-(4-(2-(dimethylamino)ethoxy)phenyl)-4-methyl-1H-pyrrolo[2,3-b]pyridine-5-carboxylate |
| **206** | 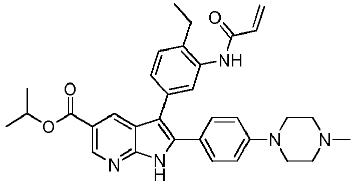 | isopropyl 3-(3-acrylamido-4-ethyl phenyl)-2-(4-(4-methylpiperazin-1-yl)phenyl)-1H-pyrrolo[2,3-b]pyridine-5-carboxylate |
| **207** | 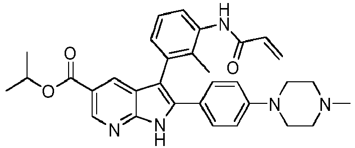 | isopropyl 3-(3-acrylamido-2-methylphenyl)-2-(4-(4-methylpiperazin-1-yl)phenyl)-1H-pyrrolo[2,3-b]pyridine-5-carboxylate |
| **208** | 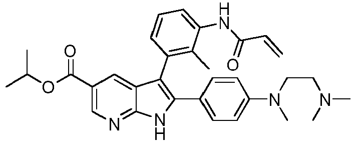 | isopropyl 3-(3-acrylamido-2-methylphenyl)-2-(4-((2-(dimethylamino)ethyl)(methyl)ami no)phenyl)-1H-pyrrolo[2,3-blpyridine-5-carboxylate |
| **209** | 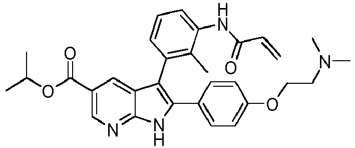 | isopropyl 3-(3-acrylamido-2-methylphenyl)-2-(4-(2-(dimethylamino)ethoxy)phenyl)-1H-pyrrolo[2,3-b]pyridine-5-carboxylate |

or an enantiomer, a mixture of enantiomers, a diastereomer, a mixture of diastereomers, a tautomer, a hydrate, a solvate of the above mentioned compounds, or pharmaceutically acceptable salts thereof.

The expression prodrug is defined as a substance, which is applied in an inactive or significantly less active form. Once applied and incorporated, the prodrug is metabolized in the body *in vivo* into the active compound. Conventional procedures for the selection and preparation of suitable prodrug derivatives are described, for example in "Design of Prodrugs", ed. H. B. Bundgaard, Elsevier, 1985.

The present invention also includes within its scope N-oxides of the compounds of formula (I) above. In general, such N-oxides may be formed by conventional means, such as reacting the compound of formula (I) with oxone in the presence of wet alumina.

The expression tautomer is defined as an organic compound that is interconvertible by a chemical reaction called tautomerization. Tautomerization can be catalyzed preferably by bases or acids or other suitable compounds.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

The inventive compounds may exist in a number of different polymorphic forms.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

The inventors found that in order to obtain inhibitors which exhibit metabolic stability against liver microsomes the 2-position at the phenyl ring next to the warhead must be substituted. The warhead can be a but-2-enamide, 4-(dimethylamino)but-2-enamide, propiolamide, ethenesulfonamide, acrylamide, or 2-chloro-acetamide residue. The position 2 on the phenyl ring next to the warhead must be different from hydrogen and is preferably an alkyl or alkylenyl residue such as a methyl group (like in compounds No. 1 - 54, 72 - 82, 116 - 148) or can be part of a ring system preferably containing the nitrogen atom of the warhead (like in compounds No. 55 - 71, 83 - 95, 98 - 115). Without this substituent and especially without this -CH₃ or -CH₂- substituent, no metabolic stability against liver microsomes can be achieved and consequently no pharmaceutical activity can be obtained in vivo.

### Syntheses of compounds

The compound of formula (I) is prepared by reference to the methods illustrated in the following schemes 1-4. The compound of formula (I) is produced by Schemes 1-3 by the suitable selection of reagents with appropriate substitution. Solvents, temperatures, pressures, and other reaction conditions may readily be selected by one of ordinary skill in the art. Starting materials are commercially available or readily prepared by one of ordinary skill in the art.

As shown in **Scheme** 1, the present invention is directed to a method for producing the compound of formula **(Ia-1)** comprising:
**Step A1:** perfoming a first cross coupling reaction of pyridine compound 1* with alkyne comopund **2a*** 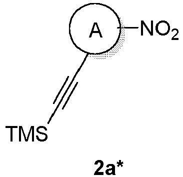 to obtain a compound **3*** 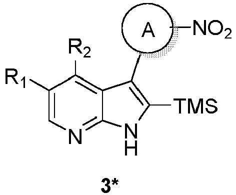 in the presence of a first palladium catalyst, and a first base;
Step B1: converting a trimethylsilyl group of the compound 3* to a halide like an iodide to obtain a compound **4*** 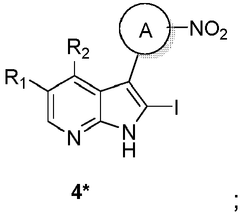
**Step C1:** perfoming a second cross coupling reaction **of 4*** with a compound **5*** 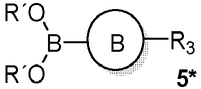 in the presence of a second palladium catalyst, and a second base to obtain a compound **6*** 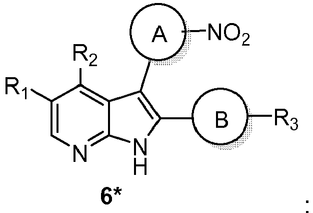
**Step D1:** reducing nitro (NO₂) group of the compound 6* to a primary amine (NH₂) group to obtain a compound 7*; and
**Step E1:** perfoming a coupling reaction of the compound 7* 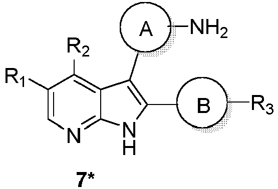 with a compound HO**-R₅** or AG**-R₅** to obtain a product compound of the formula **(Ia-1)**

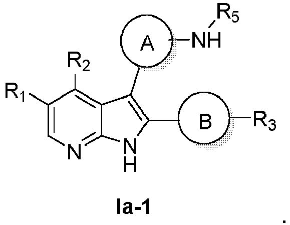

The compound **1*** has following formula 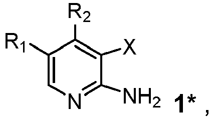 wherein R₁, R₂ have the same meanings as definced in the formula (I);
X is a leaving group and represents Cl, Br, I, or OTf.

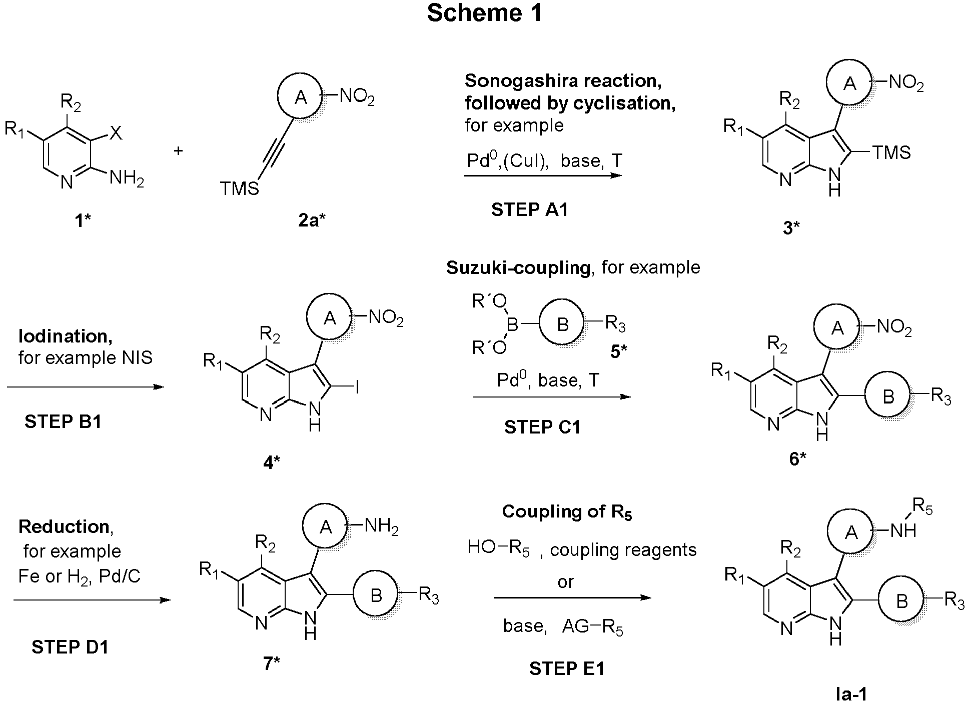

Alternatively, the sequence of the second cross coupling reaction, reduction of nitro group and further coupling reaction may be changed and therefore the product compound **Ia-1** is obtained by the following method.

A method for producing a compound of the formula **(Ia-1)** comprising **(Scheme 2):**
**Step A1:** perfoming a first cross coupling reaction of pyridine compound 1* with alkyne comopund **2a*** 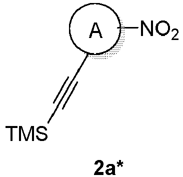 to obtain a compound 3* 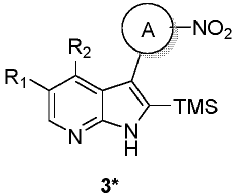 in the presence of a first palladium catalyst, and a first base;
**Step D2:** reducing nitro (NO₂) group of the compound **3*** to a primary amine (NH₂) group to obtain a compound **10*** 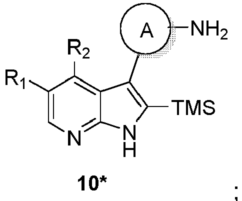
**Step E2:** perfoming a coupling reaction of the compound **10*** with a compound HO-**R₅** or AG-**R₅** to obtain a compound **11*** 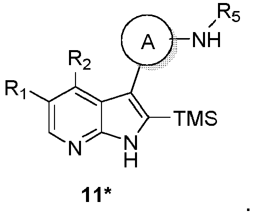
**Step B2:** converting a trimethylsilyl group of the compound **11*** to a halide like an iodide to obtain a compound **12*** 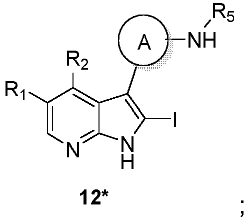 and
**Step C2:** perfoming a second cross coupling reaction of the compound **12*** with a compound **5*** in the presence of a second palladium catalyst, and a second base to obtain a product compound of the formula **(Ia-1)**

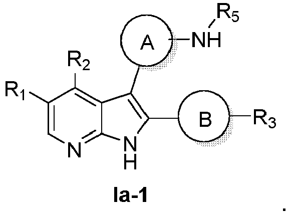

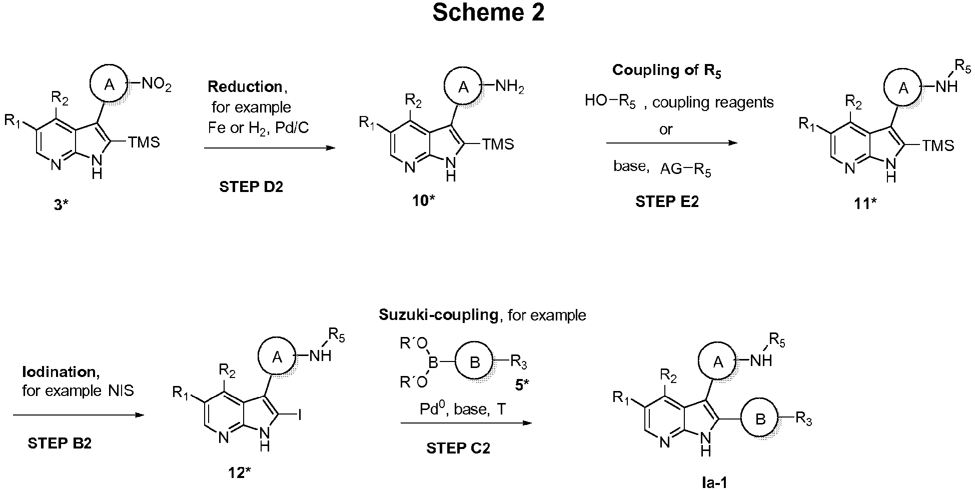

Another aspect of the present invention is a method for producing the compound of formula (Ib) is described in Scheme 3.

A method for producing the compound of formula (Ib) comprising:
**Step A3:**
   i) perfoming a first cross coupling reaction of pyridine compound **1*** 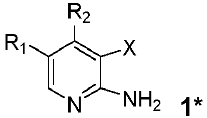 with alkyne comopund **2b*** 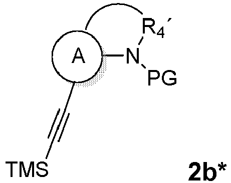 in the presence of a first palladium catalyst, and a first base; and
   ii) removing a protecting group PG of a resulting comopund after the step i) to obtain a compound **3b*** 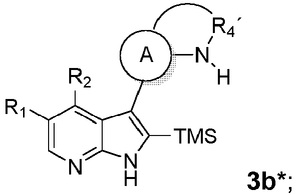
**Step E3:** perfoming a coupling reaction of the compound **3b*** with a compound HO-**R₅** or AG-**R₅** to obtain a compound **11b*** 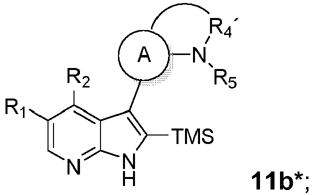
**Step B3:** converting a trimethylsilyl group of the compound **11b*** to a halide like an iodide to obtain a compound **12b*** 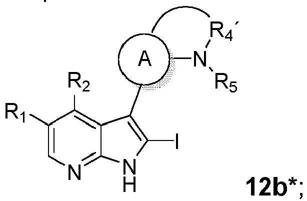 and
**Step C3:** perfoming a second cross coupling reaction of the compound **12b*** with a compound **5*** 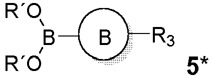 in the presence of a second palladium catalyst, and a second base to obtain a product compound of the formula **(Ib)**

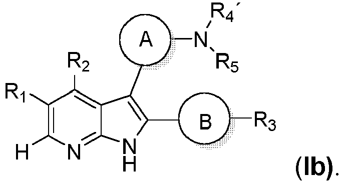

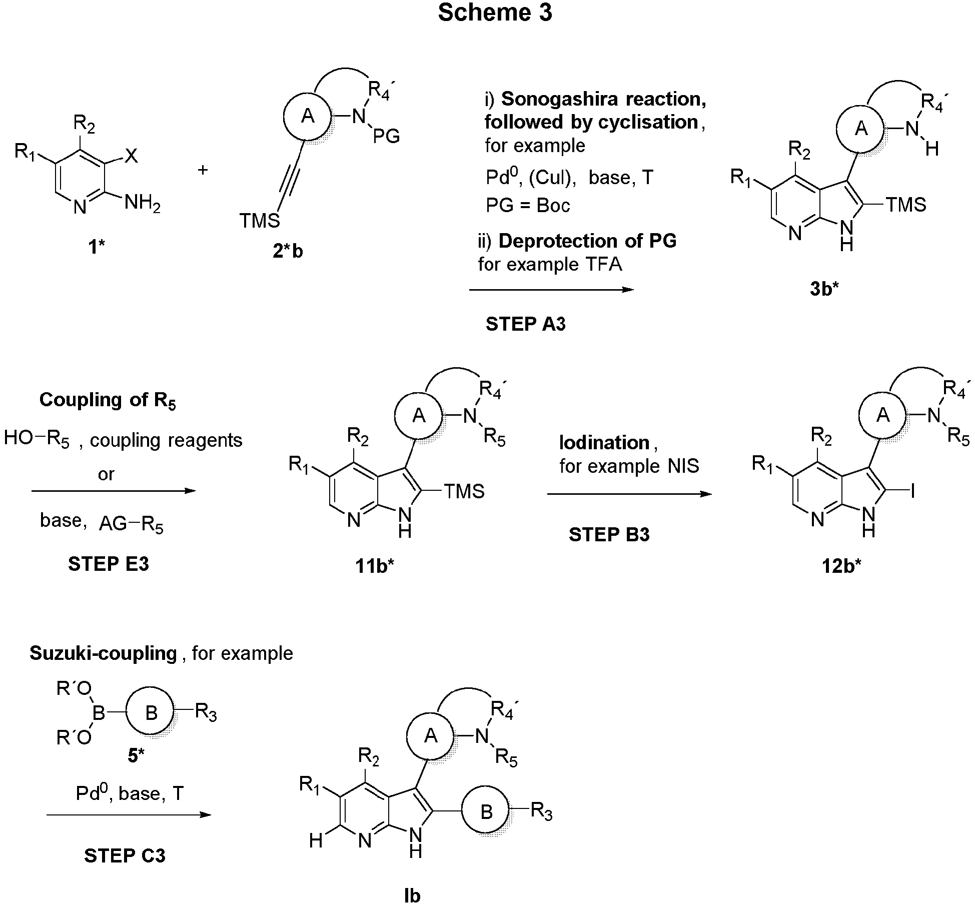

A further method for producing the compound of formula (la-1) is described in **Scheme 4.**

A further method for producing the compound of formula (la-1) compring:
**Step A4:** perfoming a first cross coupling reaction of pyridine compound **1*** 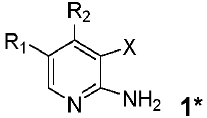 with alkyne comopund **2b*** 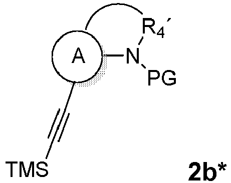 to obtain a compound **13*** 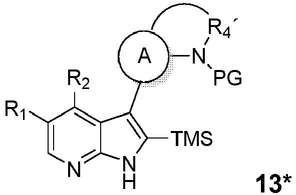 in the presence of a first palladium catalyst, and a first base; and
**Step B4:** converting a trimethylsilyl group of the compound 13* to a halide like an iodide to obtain a compound **14*** 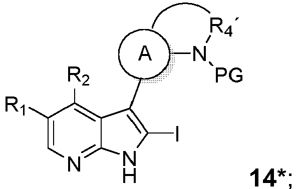
**Step C4:** perfoming a second cross coupling reaction of the compound **14*** with a compound **5*** 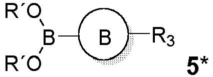 in the presence of a second palladium catalyst, and a second base to obtain a compound **15*** 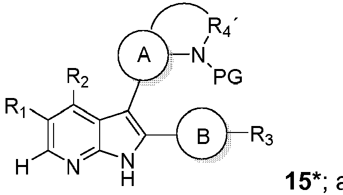 and
**Step E4:**
   i) removing a protecting group PG of the compound **15*;**
   ii) perfoming a coupling reaction of the resulting compound after step i) with a compound HO-**R₅** or AG-**R₅** to obtain product compound of the formula **(Ib).**

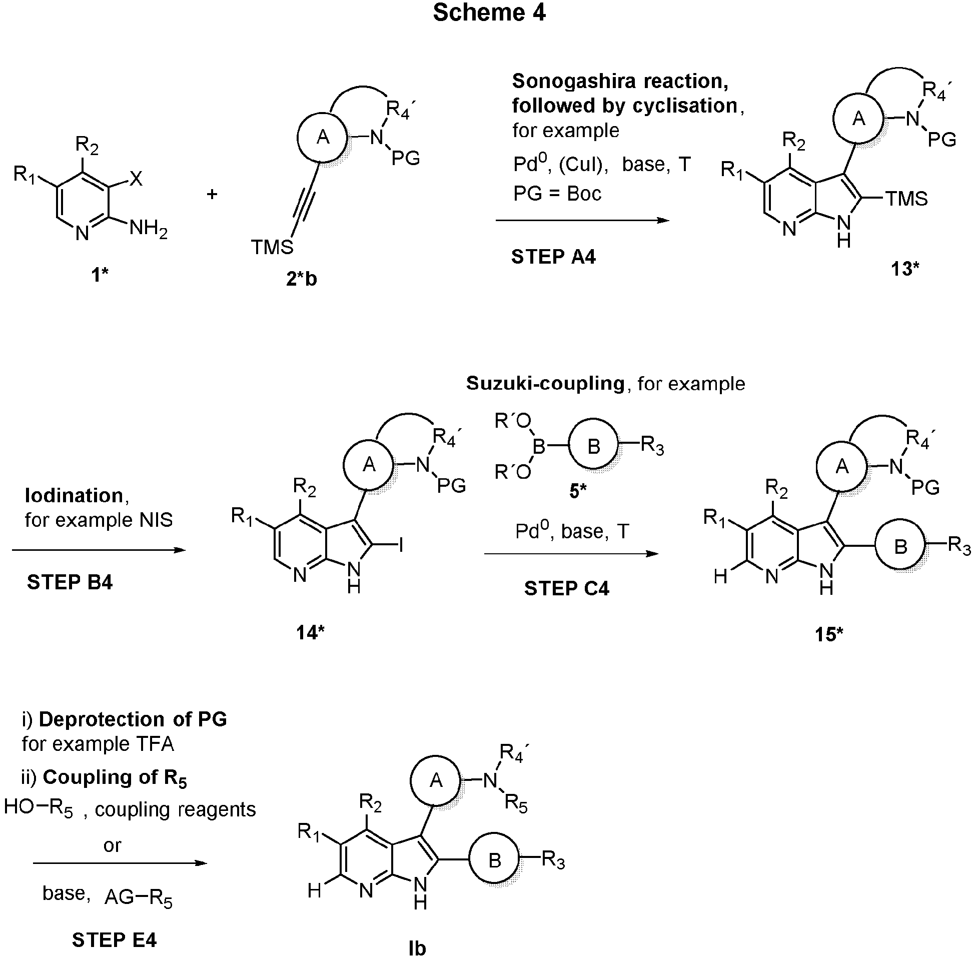

In the above-mentioned method for producing the compound of formula **(Ia-1)** or **(Ib),** and in the schemes 1 to 4, A, B, R₁, R₂, R₃, R₄, R₄', R₅ and 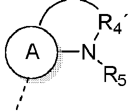 have the same meanings as defined in the formula (I);
X is a leaving group and represents Cl, Br, I, or OTf;
AG is an activating group of carboxylic acid;
PG is an amino protecting group;
TMS is a trimethylsilyl group; and
R' is H or an alkyl chain with 1-10 carbon atoms or a cycloalkyl chain with 3 to 12 carbon atoms or both residues R' represent together a residue derived from pinacol.,

In each of the steps **A1, A3,** and **A4,** Sonogashia coupling reaction of compound **1*** and domino cyclization of the resulting coupling compound is performed in the present of the first palladium catalyst, and the base. Optionally, copper catalyst may be used as cocatalyst and is preferred Cu(I) and more preferred CuI.

The first palladium catalyst for C-C coupling reaction is Pd(0) or Pd(II) catalyst, preferred PdCl₂, Pd(PPh₃)₄, Pd(acac)₂, PdCl₂(CH₃CN)₂, PdCl₂(PCy₃)₂, PdCl₂(PPh₃)₂, Pd(dppf)Cl₂, [(π-ally)PdCl]₂, (SIPr)PdCl₂(TEA). Optionally, further phosphine ligand may be used together with the first palladium catalyst.

A ratio of the palladium catalyst to the starting meterial is in the range of 0.01 to 20 mol-%, preferred 0.01-10 mol-%, more preferred 0.01-5 mol-%, most preferred 0.01-1 mol-%.

The first base may be an organic base or inorganic bases. The organic base may be tertiary amine such as Et₃N, and DIPEA, DABCO, DBU, pyrrolidine or piperidine. The inorganic base may be K₂CO₃, Cs₂CO₃ or K₃PO₄. A ratio of the first base to the starting meterial is in the range of 1.0 to 5.0 equivalents, preferred 1.0 to 3.0 equivalents, more preferred 1.0 to 3.0 equivalents, most preferred 1.0 to 1.5 equivalents.

Preferred, this reaction is performed in a polar aprotic solvent such as DMF or DMSO under N₂ atmosphere at a temperature in a range of 80 to 200°C, preferred 100 to 180°C, more preferred 100 to 150°C, most preferred 120 to 150°C.

In each of the steps **B1, B2, B3** and **B4,** conversion of trimethylsilyl (TMS) group to iodide group is performed by treating with N-iodosuccinimide (NIS) as an idonation reagent for 15 h at a temperature in a range of 10 to 35°C, preferred, 15 to 30 °C, more preferred 20 to 30 °C. A polar aprotic solvent such as dichlorormethane or chlororform is used.

In each of the steps **C1, C2, C3,** and **C4,** Suzuki coupling reaction of iodinated compound **4*, 12*, 12b*** or **14*** with a compound **5*** as a boronic acid derivative is performed in the present in the second palladium catalyst and the second base 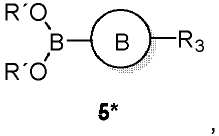 wherein **R'** is H or an alkyl chain with 1-10 carbon atoms or a cycloalkyl chain with 3 to 12 carbon atoms or both residues R' represent together a residue derived from pinacol.

Preferred, the boronic acid derivative **5*** may be a boronic acid (R' = -H) or an ester of the boronic acid, e.g. its isopropyl ester (R' = -CH(CH₃)₂), an ester derived from pinacol (R'-R' = -C(CH₃)₂-C(CH₃)₂-).

The second palladium catalyst is Pd(0) or Pd(II) catalyst. The Pd(0) catalyst may be tetrakis(triphenylphosphine)palladium(0) [Pd(PPh₃)_{4]}, tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or more preferred [1,1'-bis(diphenylphosphino)ferrocene]palladium dichloride. The reaction is preferably carried out in a mixture of a solvent like dioxane, DMF, DME, THF, or isopropanol with water and in the presence of the second base like aqueous sodium bicarbonate or K₃PO₄.

In each of the Steps **D1** and **D2,** a nitro (NO₂) group is reduced to a primary amine (NH₂) group with the treatment of a reducing agent. Preferred, Fe, or Pd/H₂ is used as a reducing agent.

In each of the Steps **E1, E2, E3** and **E4, R₅** group is introduced by a coupling reaction with HO-**R₅** or AG-**R₅**.

For perfoming the coupling reaction, firstly carboxylic acid or sulfonic acid group of HO-R₅ is activated *in situ* to promote the coupling reaction with amino group of intermediate compound. If HO-R₅ is a carboxylic acid, the activating group (AG) of carboxylic acid may be introduced *in situ* reaction. Preferably, the activating group (AG) may be selected from the group consisting of or comprising: halides such as -F, -Br, -CI, -I, anhydride group such as -OCOCH₃, N-oxy-benzotriazol group and N-oxy-succinimide. Preferably,

HO-**R₅** represents 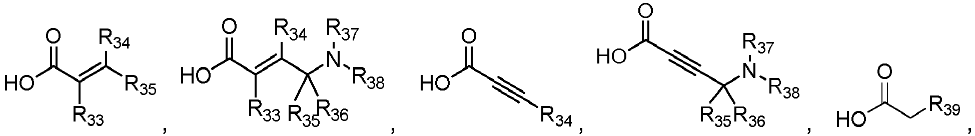 or

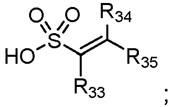

Further, a carboxylic acid of HO-**R₅** is coupled with the amine group of intermediate compound by a well-known amide coupling reaction. Any of the following coupling reagent can be used for amide coupling reaction: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP.

For perfoming the coupling reaction, also an activated compound AG-R₅ having activated carboxyl sulfony group can be used.

AG-**R₅** represents

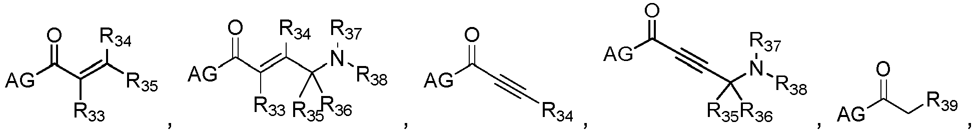

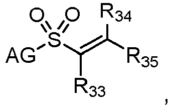

As above described, the activating group (AG) may be selected from the group consisting of or comprising: halides such as -F, -Br, -CI, -I, anhydride group such as - OCOCH₃, N-oxy-benzotriazol group and N-oxy-succinimide, preferred AG is Cl.

Preferred, AG-R₅ is

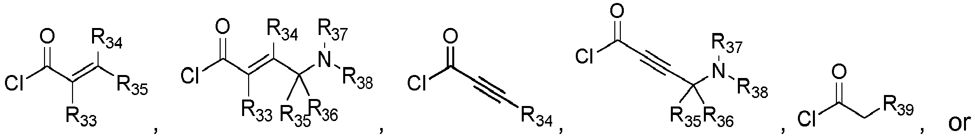

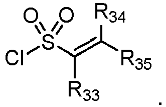

In the Step **A3** and **E4,** a protecting group (PG) is an amino protecting group. The amine protecting group may be t-butyloxycarbonyl (Boc) or benzyloxycarbonyl (Cbz) and removed under acidic condition, for example, treating with HCl, or TFA.

In an embodiment, the present invention is directed to an intermediate compound selected from the group consisting of the compounds **3*, 3b*, 4*, 7*, 10*, 11*, 11b*, 12***, **12b*, 13*, 14*, and 15***: 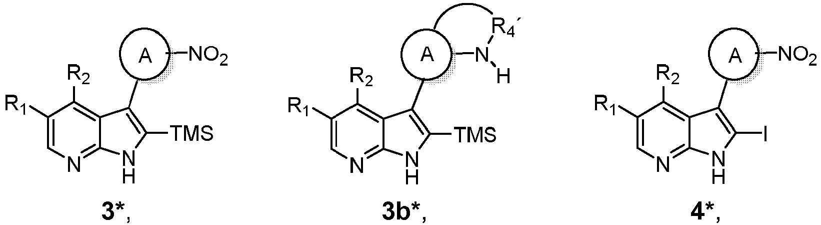 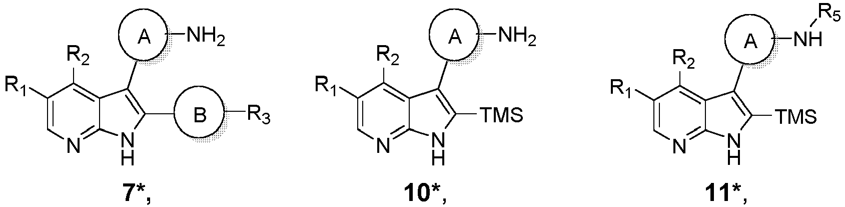 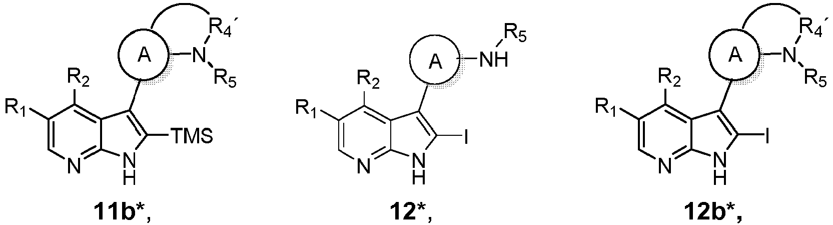 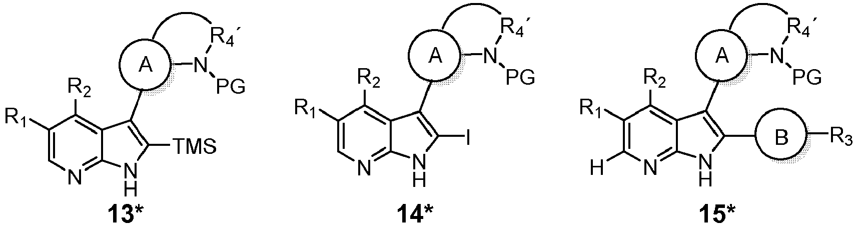 wherein A, B, R₁, R₂, R₃, R₄, R₄, R₅, PG and 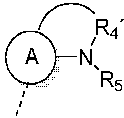 have the same meanings as defined above; and
TMS is trimethyl silyl group.

The structure activity relationship (SAR) of the compounds of the present invention as represented by the following formula (IV) shows covalent inhibitors as cellular potent mutant-selective ErbB inhibitors. The covalent binding mode, obtained for example through the introduction of an acrylic moiety, is crucial for cellular activity. The direct comparison of examples from this invention having a acryl moiety for the covalent binding with corresponding molecules having a propionic moiety are showing the improved cellular activities for the covalent binder.

Table 6 shows comparison of covalent inhibitors (Example 2 and 148) with the corresponding reversible analogues (References 3 and 4). In both cases the covalent inhibitor shows (Example 2 and 148) improved cellular activities compared to the reversible analogues (References 3 and 4).

It is found that the compound of the present invention is useful for the prophylaxis and/or the treatment of cancer having activating mutation of a receptor belonging to the ErbB family of receptor, preferred, the mutation is an insertion within exon 20 of EGFR or within exon 20 of HER2.

Further aspect of the present invention relates to the use of the compound of general formula (I) for the preparation of a pharmaceutical composition useful for prophylaxis and/or treatment of a cancer having activating mutation of a receptor belonging to the ErbB family of receptor, preferred, the mutation is an insertion within exon 20 of EGFR or within exon 20 of HER2.

Another aspect of the present invention relates to a method of treatment. This method comprises administering a therapeutically effective amount of at least one compound of general formula (I) to a patient suffering from a cancer having activating mutation of a receptor belonging to the ErbB family of receptor, preferred, the mutation is an insertion within exon 20 of EGFR or within exon 20 of HER2

The compound of the present invention is a selective inhibitor of mutants of EGFR and Her2, preferred Exon 20 mutations as shown in Table 3.

Thus, the compound of the present invention is useful as a medicament. The compound of the present invention is useful for the prophylaxis and/or the treatment of cell proliferative disease, especially cancer.

Said cancer is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, lymphoma, osteosarcoma, mamma carcinoma, melanoma, glioblastoma and neuroblastoma.

In specific embodiment, the compound is useful for in the the prophylaxis and/or the treatment of non small cell lung cancer (NSCLC) or mamma carcinoma.

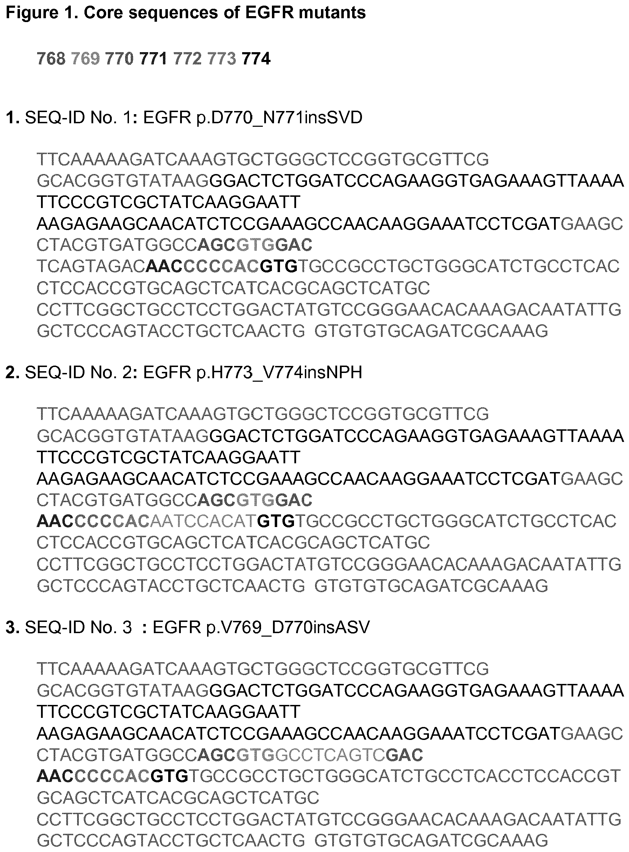

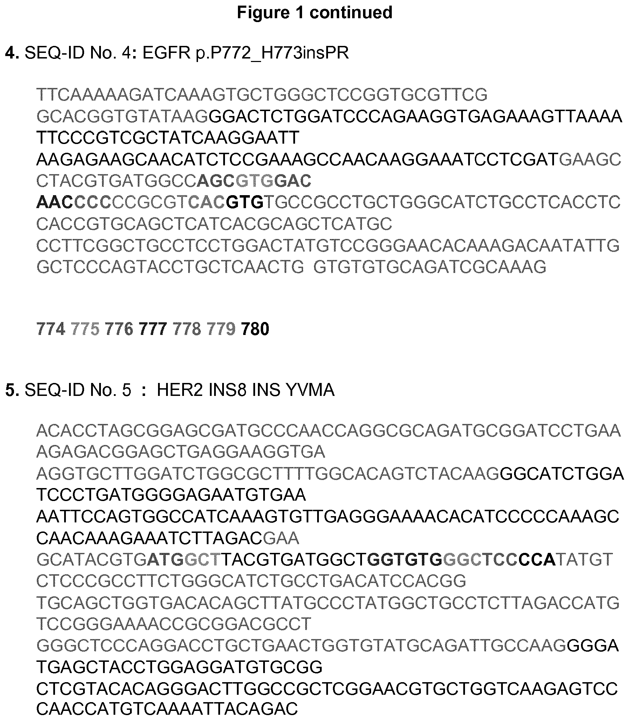

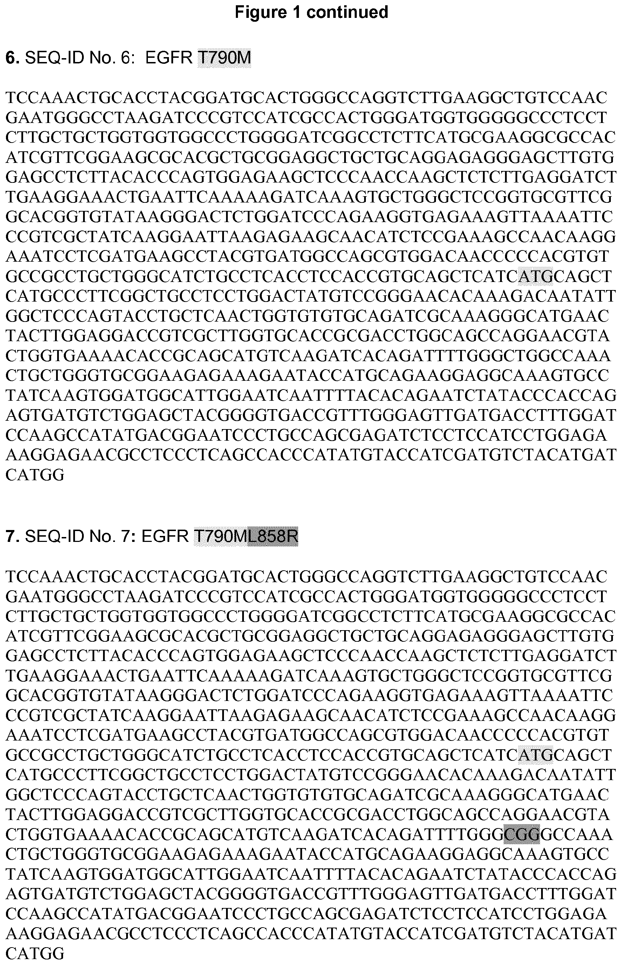

Preferred, the mutation is an insertion within exon 20 of EGFR or within exon 20 of HER2. One aspect herein are the compounds of the present invention for use in the prophylaxis and/or the treatment of cancer caused by or associated with mutations associated with EGFR TKI resistance and in particular caused by or associated with inframe insertions and/or duplications of 3 to 21 base pairs (bp) between codons 763 and 775 of the Her2 gene or the EGFR (Her1) gene. More preferred, the mutation is selected from the group consisting of Her2 INS8 INS YVMA, EGFR D770_N771 insSVD, EGFR H773_V774insNPH, EGFR V769_D770insASV, EGFR P772_H773insPR, EGFR T790M and EGFR T790ML858R. The important sequences are shown in SEQ-ID No. 1 to SEQ:NO 7 in Figure 1

The compounds of the present invention have improved stability towards hydrolysis of the acrylamide moiety in liver microsomes as summairzed in Table 5.

Preferably, the following compounds have improved stability:
N-(5-(4-chloro-2-(4-((4-methylpiperazin-1-yl)methyl)phenyl)-1H-pyrrolo[2,3-b]pyridin-3-yl)-2-methylphenyl)acrylamide, N-(5-(4-chloro-2-(4-(4-methylpiperazin-1-yl)phenyl)-1H-pyrrolo[2,3-b]pyridin-3-yl)-2-methylphenyl)acrylamide, N-(5-(4-chloro-2-(4-(1-methylpiperidin-4-yl)phenyl)-1H-pyrrolo[2,3-b]pyridin-3-yl)-2-methylphenyl)acrylamide, N-(5-(4-chloro-2-(4-(2-(dimethylamino)ethyl)phenyl)-1H-pyrrolo[2,3-b]pyridin-3-yl)-2-methylphenyl)acrylamide, N-(5-(4-chloro-2-(4-(morpholinomethyl)phenyl)-1H-pyrrolo[2,3-b]pyridin-3-yl)-2-methylphenyl)acrylamide, N-(5-(4-chloro-2-(3-morpholinophenyl)-1H-pyrrolo[2,3-b]pyridin-3-yl)-2-methylphenyl)acrylamide, N-(5-(2-(4-(4-acetylpiperazin-1-yl)phenyl)-4-chloro-1H-pyrrolo[2,3-b]pyridin-3-yl)-2-methylphenyl)acrylamide, N-(5-(4-chloro-2-(4-(4-methylpiperazine-1-carbonyl)phenyl)-1H-pyrrolo[2,3-b]pyridin-3-yl)-2-methylphenyl)acrylamide, N-(5-(4-chloro-2-(4-((dimethylamino)methyl)phenyl)-1H-pyrrolo[2,3-b]pyridin-3-yl)-2-methylphenyl)acrylamide, N-(5-(4-chloro-2-(4-(morpholine-4-carbonyl)phenyl)-1H-pyrrolo[2,3-b]pyridin-3-yl)-2-methylphenyl)acrylamide, N-(5-(4-chloro-2-(4-(3-(dimethylamino)propoxy)phenyl)-1H-pyrrolo[2,3-b]pyridin-3-yl)-2-methylphenyl)acrylamide, N-(5-(4-chloro-2-(4-(4-(ethylsulfonyl)piperazin-1-yl)phenyl)-1H-pyrrolo[2,3-b]pyridin-3-yl)-2-methylphenyl)acrylamide, N-(5-(4-chloro-2-(3-((4-methylpiperazin-1-yl)methyl)phenyl)-1H-pyrrolo[2,3-b]pyridin-3-yl)-2-methylphenyl)acrylamide, N-(5-(4-chloro-2-(3-(4-methylpiperazin-1-yl)phenyl)-1H-pyrrolo[2,3-b]pyridin-3-yl)-2-methylphenyl)acrylamide, N-(5-(4-chloro-2-(4-(2-(dimethylamino)ethoxy)phenyl)-1H-pyrrolo[2,3-b]pyridin-3-yl)-2-methylphenyl)acrylamide, N-(5-(4-chloro-2-(6-(4-methylpiperazin-1-yl)pyridin-3-yl)-1H-pyrrolo[2,3-b]pyridin-3-yl)-2-methylphenyl)acrylamide, N-(5-(4-chloro-2-(3-(3-(dimethylamino)propoxy)phenyl)-1H-pyrrolo[2,3-b]pyridin-3-yl)-2-methylphenyl)acrylamide, N-(5-(4-chloro-2-(2-methoxy-4-(4-methylpiperazin-1-yl)phenyl)-1H-pyrrolo[2,3-b]pyridin-3-yl)-2-methylphenyl)acrylamide, N-(5-(2-(3-((4-acetylpiperazin-1-yl)methyl)phenyl)-4-chloro-1H-pyrrolo[2,3-b]pyridin-3-yl)-2-methylphenyl)acrylamide, N-(5-(4-chloro-2-(4-(3-(4-ethylpiperazin-1-yl)propoxy)phenyl)-1H-pyrrolo[2,3-b]pyridin-3-yl)-2-methylphenyl)acrylamide, N-(5-(4-chloro-2-(pyrimidin-5-yl)-1H-pyrrolo[2,3-b]pyridin-3-yl)-2-methylphenyl)acrylamide, N-(5-(4-chloro-2-(3-(3-(4-ethylpiperazin-1-yl)propoxy)phenyl)-1H-pyrrolo[2,3-b]pyridin-3-yl)-2-methylphenyl)acrylamide, N-(5-(4-chloro-2-(2-(4-methylpiperazin-1-yl)pyridin-4-yl)-1H-pyrrolo[2,3-b]pyridin-3-yl)-2-methylphenyl)acrylamide, N-(5-(4-chloro-2-(3-(morpholine-4-carbonyl)phenyl)-1H-pyrrolo[2,3-b]pyridin-3-yl)-2-methylphenyl)acrylamide, N-(5-(4-chloro-2-(3-(2-(dimethylamino)ethoxy)phenyl)-1H-pyrrolo[2,3-b]pyridin-3-yl)-2-methylphenyl)acrylamide, N-(5-(4-chloro-2-(4-(piperidin-4-yl)phenyl)-1H-pyrrolo[2,3-b]pyridin-3-yl)-2-methylphenyl)acrylamide, N-(5-(4-chloro-2-(6-morpholinopyridin-3-yl)-1H-pyrrolo[2,3-b]pyridin-3-yl)-2-methylphenyl)acrylamide, N-(5-(2-(6-(4-acetylpiperazin-1-yl)pyridin-3-yl)-4-chloro-1H-pyrrolo[2,3-b]pyridin-3-yl)-2-methylphenyl)acrylamide, N-(5-(4-chloro-2-(2-morpholinopyridin-4-yl)-1H-pyrrolo[2,3-b]pyridin-3-yl)-2-methylphenyl)acrylamide, N-(5-(4-chloro-2-(2-morpholinopyrimidin-5-yl)-1H-pyrrolo[2,3-b]pyridin-3-yl)-2-methylphenyl)acrylamide, N-(5-(4-chloro-2-(3-(2-(pyrrolidin-1-yl)ethoxy)phenyl)-1H-pyrrolo[2,3-b]pyridin-3-yl)-2-methylphenyl)acrylamide, N-(5-(4-chloro-2-(4-(2-(pyrrolidin-1-yl)ethoxy)phenyl)-1H-pyrrolo[2,3-b]pyridin-3-yl)-2-methylphenyl)acrylamide, N-(5-(4-chloro-2-(2-(4-methylpiperazin-1-yl)pyrimidin-5-yl)-1H-pyrrolo[2,3-b]pyridin-3-yl)-2-methylphenyl)acrylamide, N-(5-(4-chloro-2-(4-(4-ethylpiperazin-1-yl)phenyl)-1H-pyrrolo[2,3-b]pyridin-3-yl)-2-methylphenyl)acrylamide, N-(5-(4-chloro-2-(4-(4-isopropylpiperazin-1-yl)phenyl)-1H-pyrrolo[2,3-b]pyridin-3-yl)-2-methylphenyl)acrylamide, N-(5-(4-chloro-2-(3-fluoro-4-(4-methylpiperazin-1-yl)phenyl)-1H-pyrrolo[2,3-b]pyridin-3-yl)-2-methylphenyl)acrylamide, N-(5-(4-chloro-2-(4-((2-(dimethylamino)ethyl)(methyl)amino)phenyl)-1H-pyrrolo[2,3-b]pyridin-3-yl)-2-methylphenyl)acrylamide, 1-(6-(4-chloro-2-(4-(1-methylpiperidin-4-yl)phenyl)-1H-pyrrolo[2,3-b]pyridin-3-yl)indolin-1-yl)prop-2-en-1-one, 1-(6-(4-chloro-2-(4-(4-methylpiperazin-1-yl)phenyl)-1H-pyrrolo[2,3-b]pyridin-3-yl)indolin-1-yl)prop-2-en-1 -one, 1-(6-(4-chloro-2-(3-fluoro-4-(4-methylpiperazin-1-yl)phenyl)-1H-pyrrolo[2,3-b]pyridin-3-yl)indolin-1-yl)prop-2-en-1 -one, 1-(6-(2-(4-(4-acetylpiperazin-1-yl)phenyl)-4-chloro-1H-pyrrolo[2,3-b]pyridin-3-yl)indolin-1-yl)prop-2-en-1-one, 1-(6-(4-chloro-2-(2-(4-methylpiperazin-1-yl)pyridin-4-yl)-1H-pyrrolo[2,3-b]pyridin-3-yl)indolin-1-yl)prop-2-en-1-one, 1-(6-(4-chloro-2-(4-((4-methylpiperazin-1-yl)methyl)phenyl)-1H-pyrrolo[2,3-b]pyridin-3-yl)indolin-1-yl)prop-2-en-1-one, 1-(6-(4-chloro-2-(4-((dimethylamino)methyl)phenyl)-1H-pyrrolo[2,3-b]pyridin-3-yl)indolin-1-yl)prop-2-en-1-one, 1-(6-(4-chloro-2-(4-(4-isopropylpiperazin-1-yl)phenyl)-1H-pyrrolo[2,3-b]pyridin-3-yl)indolin-1-yl)prop-2-en-1 -one, 1-(6-(4-chloro-2-(4-(4-ethylpiperazin-1-yl)phenyl)-1H-pyrrolo[2,3-b]pyridin-3-yl)indolin-1-yl)prop-2-en-1 -one, 1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one, 1-(6-(4-chloro-2-(4-((2-(dimethylamino)ethyl)(methyl)amino)phenyl)-1H-pyrrolo[2,3-b]pyridin-3-yl)indolin-1-yl)prop-2-en-1-one, 1-(6-(4-chloro-2-(4-(4-methylpiperazine-1-carbonyl)phenyl)-1H-pyrrolo[2,3-b]pyridin-3-yl)indolin-1-yl)prop-2-en-1 -one, 1-(6-(4-chloro-2-(4-(2-(dimethylamino)ethoxy)phenyl)-1H-pyrrolo[2,3-b]pyridin-3-yl)indolin-1-yl)prop-2-en-1-one, 1-(6-(4-chloro-2-(4-(morpholinomethyl)phenyl)-1H-pyrrolo[2,3-b]pyridin-3-yl)indolin-1-yl)prop-2-en-1-one, 1-(6-(4-chloro-2-(3-((4-methylpiperazin-1-yl)methyl)phenyl)-1H-pyrrolo[2,3-b]pyridin-3-yl)indolin-1-yl)prop-2-en-1-one, 1-(6-(4-chloro-2-(6-(4-methylpiperazin-1-yl)pyridin-3-yl)-1H-pyrrolo[2,3-b]pyridin-3-yl)indolin-1-yl)prop-2-en-1-one, 1-(6-(4-chloro-2-(4-(3-(dimethylamino)propoxy)phenyl)-1H-pyrrolo[2,3-b]pyridin-3-yl)indolin-1-yl)prop-2-en-1 -one, N-(5-(4-methoxy-2-(4-(4-methylpiperazin-1-yl)phenyl)-1H-pyrrolo[2,3-b]pyridin-3-yl)-2-methylphenyl)acrylamide, N-(5-(4-methoxy-2-(4-(1-methylpiperidin-4-yl)phenyl)-1H-pyrrolo[2,3-b]pyridin-3-yl)-2-methylphenyl)acrylamide, N-(5-(2-(4-(4-acetylpiperazin-1-yl)phenyl)-4-methoxy-1H-pyrrolo[2,3-b]pyridin-3-yl)-2-methylphenyl)acrylamide, N-(5-(4-methoxy-2-(4-(morpholinomethyl)phenyl)-1H-pyrrolo[2,3-b]pyridin-3-yl)-2-methylphenyl)acrylamide, N-(5-(4-methoxy-2-(3-((4-methylpiperazin-1-yl)methyl)phenyl)-1H-pyrrolo[2,3-b]pyridin-3-yl)-2-methylphenyl)acrylamide, N-(5-(4-methoxy-2-(4-((4-methylpiperazin-1-yl)methyl)phenyl)-1H-pyrrolo[2,3-b]pyridin-3-yl)-2-methylphenyl)acrylamide, N-(2-methyl-5-(2-(6-(4-methylpiperazin-1-yl)pyridin-3-yl)-1H-pyrrolo[2,3-b]pyridin-3-yl)phenyl)acrylamide, N-(2-methyl-5-(2-(4-(1-methylpiperidin-4-yl)phenyl)-1H-pyrrolo[2,3-b]pyridin-3-yl)phenyl)acrylamide, N-(2-methyl-5-(2-(2-morpholinopyridin-4-yl)-1H-pyrrolo[2,3-b]pyridin-3-yl)phenyl)acrylamide, N-(5-(2-(3-((4-acetylpiperazin-1 - yl)methyl)phenyl)-1H-pyrrolo[2,3-b]pyridin-3-yl)-2-methylphenyl)acrylamide, N-(2-methyl-5-(2-(3-(morpholinomethyl)phenyl)-1H-pyrrolo[2,3-b]pyridin-3-yl)phenyl)acrylamide, 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-((2-(dimethylamino)ethyl)(methyl)amino)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one, 1-(7-(4-chloro-2-(4-(4-methylpiperazin-1-yl)phenyl)-1H-pyrrolo[2,3-b]pyridin-3-yl)-3,4-dihydroquinolin-1 (2H)-yl)prop-2-en-1-one, 1-(7-(4-chloro-2-(3-(2-(dimethylamino)ethoxy)phenyl)-1H-pyrrolo[2,3-b]pyridin-3-yl)-3,4-dihydroquinolin-1(2H)-yl)prop-2-en-1-one, 1-(7-(4-chloro-2-(4-(4-isopropylpiperazin-1-yl)phenyl)-1H-pyrrolo[2,3-b]pyridin-3-yl)-3,4-dihydroquinolin-1(2H)-yl)prop-2-en-1-one, 1-(7-(4-chloro-2-(4-(2-(dimethylamino)ethoxy)phenyl)-1H-pyrrolo[2,3-b]pyridin-3-yl)-3,4-dihydroquinolin-1 (2H)-yl)prop-2-en-1-one, 1-(7-(4-chloro-2-(6-(4-methylpiperazin-1 - yl)pyridin-3-yl)-1H-pyrrolo[2,3-b]pyridin-3-yl)-3,4-dihydroquinolin-1(2H)-yl)prop-2-en-1-one, 1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one, N-(5-(4-chloro-2-(4-(piperazin-1-yl)phenyl)-1H-pyrrolo[2,3-b]pyridin-3-yl)-2-methylphenyl)acrylamide, N-(5-(5-cyano-2-(4-(4-methylpiperazin-1-yl)phenyl)-1H-pyrrolo[2,3-b]pyridin-3-yl)-2-methylphenyl)acrylamide, 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one, 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one, 1-(6-(4-ethoxy-2-(4-(4-methylpiperazin-1-yl)phenyl)-1H-pyrrolo[2,3-b]pyridin-3-yl)indolin-1 -yl)prop-2-en-1 -one, 1 -(6-(4-ethoxy-2-(4-((4-methylpiperazin-1-yl)methyl)phenyl)-1H-pyrrolo[2,3-b]pyridin-3-yl)indolin-1-yl)prop-2-en-1-one, 1-(6-(4-ethoxy-2-(3-(2-(pyrrolidin-1-yl)ethoxy)phenyl)-1H-pyrrolo[2,3-b]pyridin-3-yl)indolin-1-yl)prop-2-en-1-one, 1-(6-(4-methoxy-2-(4-(4-methylpiperazin-1-yl)phenyl)-1H-pyrrolo[2,3-b]pyridin-3-yl)indolin-1-yl)prop-2-en-1 -one, 1-(6-(4-methoxy-2-(2-(4-methylpiperazin-1-yl)pyridin-4-yl)-1H-pyrrolo[2,3-b]pyridin-3-yl)indolin-1-yl)prop-2-en-1 - one, 1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1-one, 1-(6-(4-methoxy-2-(4-((4-methylpiperazin-1-yl)methyl)phenyl)-1H-pyrrolo[2,3-b]pyridin-3-yl)indolin-1-yl)prop-2-en-1-one, 1-(6-(4-isopropoxy-2-(4-(4-methylpiperazin-1-yl)phenyl)-1H-pyrrolo[2,3-b]pyridin-3-yl)indolin-1-yl)prop-2-en-1-one, 3-(3-acrylamido-4-methylphenyl)-2-(4-(4-methylpiperazin-1-yl)phenyl)-1H-pyrrolo[2,3-b]pyridine-5-carboxylic acid, isopropyl 3-(3-acrylamido-4-methylphenyl)-2-(4-(4-methylpiperazin-1-yl)phenyl)-1H-pyrrolo[2,3-b]pyridine-5-carboxylate, 3-(3-acrylamido-4-methylphenyl)-N-isopropyl-2-(4-(4-methylpiperazin-1-yl)phenyl)-1H-pyrrolo[2,3-b]pyridine-5-carboxamide, 3-(3-acrylamido-4-methylphenyl)-N-cyclohexyl-2-(4-(4-methylpiperazin-1-yl)phenyl)-1H-pyrrolo[2,3-b]pyridine-5-carboxamide, methyl 3-(3-acrylamido-4-methylphenyl)-2-(4-(4-methylpiperazin-1-yl)phenyl)-1H-pyrrolo[2,3-b]pyridine-5-carboxylate, ethyl 3-(3-acrylamido-4-methylphenyl)-2-(4-(4-methylpiperazin-1-yl)phenyl)-1H-pyrrolo[2,3-b]pyridine-5-carboxylate, cyclopropyl 3-(3-acrylamido-4-methylphenyl)-2-(4-(4-methylpiperazin-1-yl)phenyl)-1H-pyrrolo[2,3-b]pyridine-5-carboxylate, N-(5-(4-ethoxy-2-(4-(1-methylpiperidin-4-yl)phenyl)-1H-pyrrolo[2,3-b]pyridin-3-yl)-2-methylphenyl)acrylamide, isopropyl 3-(3-acrylamido-4-methylphenyl)-2-(4-((2-(dimethylamino)ethyl)(methyl)amino)phenyl)-1H-pyrrolo[2,3-b]pyridine-5-carboxylate, N-(5-(5-chloro-4-methyl-2-(4-(4-methylpiperazin-1-yl)phenyl)-1H-pyrrolo[2,3-b]pyridin-3-yl)-2-methylphenyl)acrylamide, isopropyl 3-(3-acrylamido-4-methylphenyl)-2-(4-(4-ethylpiperazin-1-yl)phenyl)-1H-pyrrolo[2,3-b]pyridine-5-carboxylate, isopropyl 3-(3-acrylamido-4-methylphenyl)-2-(4-(2-(pyrrolidin-1-yl)ethoxy)phenyl)-1H-pyrrolo[2,3-b]pyridine-5-carboxylate, isopropyl 3-(3-acrylamido-4-methylphenyl)-2-(4-(2-(dimethylamino)ethoxy)phenyl)-1H-pyrrolo[2,3-b]pyridine-5-carboxylate, isopropyl 3-(3-acrylamido-4-methylphenyl)-2-(4-((4-methylpiperazin-1-yl)methyl)phenyl)-1H-pyrrolo[2,3-b]pyridine-5-carboxylate, isopropyl 3-(3-acrylamido-4-methylphenyl)-2-(4-(2-(dimethylamino)ethyl)phenyl)-1H-pyrrolo[2,3-b]pyridine-5-carboxylate, isopropyl 3-(3-acrylamido-4-methylphenyl)-2-(3-fluoro-4-(4-methylpiperazin-1-yl)phenyl)-1H-pyrrolo[2,3-b]pyridine-5-carboxylate, isopropyl 3-(3-acrylamido-5-fluoro-4-methylphenyl)-2-(4-(4-methylpiperazin-1-yl)phenyl)-1H-pyrrolo[2,3-b]pyridine-5-carboxylate, isopropyl 3-(3-acrylamido-4-methylphenyl)-2-(3-(2-(pyrrolidin-1-yl)ethoxy)phenyl)-1H-pyrrolo[2,3-b]pyridine-5-carboxylate, isopropyl 3-(3-acrylamido-5-fluoro-4-methylphenyl)-2-(4-((2-(dimethylamino)ethyl)(methyl)amino)phenyl)-1H-pyrrolo[2,3-b]pyridine-5-carboxylate, isopropyl 3-(3-acrylamido-4-methylphenyl)-2-(4-(4-isopropylpiperazin-1-yl)phenyl)-1H-pyrrolo[2,3-b]pyridine-5-carboxylate, isopropyl 3-(3-acrylamido-5-fluoro-4-methylphenyl)-2-(4-((dimethylamino)methyl)phenyl)-1H-pyrrolo[2,3-b]pyridine-5-carboxylate, isopropyl 3-(3-acrylamido-5-fluoro-4-methylphenyl)-2-(4-(1-methylpiperidin-4-yl)phenyl)-1H-pyrrolo[2,3-b]pyridine-5-carboxylate, isopropyl 3-(3-acrylamido-5-fluoro-4-methylphenyl)-2-(4-(2-(dimethylamino)ethoxy)phenyl)-1H-pyrrolo[2,3-b]pyridine-5-carboxylate, isopropyl 3-(3-acrylamido-4-methylphenyl)-2-(3-((dimethylamino)methyl)phenyl)-1H-pyrrolo[2,3-b]pyridine-5-carboxylate.

In an embodiment, the present invention is directed to the compound of in combination with at least one anticancer drug for use in treatment of cancer. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway.

The anticancer drug inhibting EGFR/HER2 tyrosine kinases is selected from the group consisting of A928605, ABT414, ABT806, AC480, Adgef (gefitinib), AEE788, AF802 (alectinib hydrochloride), Afatinib, Afinitor (everolimus), AFM21, AG1478, AGT2000, AL6802, ALL3, AMG595, Anti-Cripto-1 monoclonal antibodies PRIMA BIOMED, AP23464, AP26113, ARQ197 (tivantinib), ARRY380, ARRY543 (varlitinib tosylate), ASP7487 (linsitinib), ASP8273, AV203, AVL291, AZD4769, AZD9291, B-Peptimetics ANTYRA, BGB324, BIBW2948BS, Bispecific Anti-Her2 Zybodies ZYNGENIA (trastuzumab), Bispecific Anti-Her3 Zybodies ZYNGENIA (cetuximab), Bispecific Antibody Drug Conjugate Program AVIDBIOLOGICS, BL7030, BMS536924, BMS582664 (brivanib alaninate), BMS690514, BMSATI2, BT2111 (trastuzumab), CAB051, Canertinib, Caprelsa (vandetanib), CCX140, CDX110, CetuGEX, Cetuximab AMGEN, Cetuximab BIOXPRESS THERAPEUTICS, Cetuximab HARVEST MOON, Cetuximab ONCOBIOLOGICS, Cetuximab PANACEA BIOTECH, Cetuximab PLANTFORM, Cetuximab ZENOTECH, CGP59326A, Chemofit (gefitinib), CI1033 (canertinib dihydrochloride), CIMAher (nimotuzumab), Citoprot-P, CMAB009 (cetuximab), cMet-EGFR Dual Inhibitor CRYSTALGENOMICS, CO-1686, Cometriq (cabozantinib), Conmana (icotinib hydrochloride), CTP15 (cetuximab), CTX023, CUDC101, CYC202 (seliciclib), DC101, DXL1218, EDP13, EGF816, EGFR Antibody Drug Conjugate AVIDBIOLOGICS, EGFR BiTE antibody MICROMET, EGFR Monoclonal Antibody REVO, EGFR Probody Drug Conjugate CYTOMX, EGFR-Antibody-Targeted Endostatin Payload, EGFR501, EKB569 (pelitinib), EM1-mAb GENMAB, EMD121974 (cilengitide), EMD72000 (matuzumab), Emfib (gefitinib), Epidermal Growth Factor Receptor (EGFR) humanized antibody CHINA PHARMAHUB, Erbitux (cetuximab), Erlobenz (erlotinib), Erlocip (erlotinib), Erlonat NATCO (erlotinib), Erlonat RADIANCE (erlotinib), Erlons (erlotinib hydrochloride), Erlostar (erlotinib), Erloswift (erlotinib), Erlotad (erlotinib), Erlotaz (erlotinib), Erlotinib CYNO PHARMACEUTICALS (erlotinib hydrochloride), Erlotinib hydrochloride HETERO, Erlotinib Hydrochloride MYLAN, Erlotinib hydrochloride TEVA, Erlotinib LABORATORIOS INDUQUIMICA, Erlotinib NAPROD, Erlotinib P2D BIOSCEINCE, Erlotinib SALIUS, Erlotinib SRS PHARMA, Erlotinib UNITED BIOTECH, Etk/Bmx Kinase Inhibitor ASTEX, EZN3920, Fderceptin SHANGHAI FUDAN-ZHANGJIANG, FV225, Geffy (gefitinib), Geficad (gefitinib), Gefitinib CELOGEN, Gefitinib CYNO PHARMACEUTICALS, Gefitinib HETERO, Gefitinib LABORATORIOS INDUQUIMICA, Gefitinib MARKSANS, Gefitinib MISSION VIVACARE, Gefitinib NAPROD, Gefitinib SALIUS, Gefitinib SAVA, Gefitinib SRS PHARMA, Gefitinib UNITED BIOTECH, Gefitoz (gefitinib), GefiTrust (gefitinib), Gefnib (gefitinib), Geftex (gefitinib), Geftib (gefitinib), Gefticip (gefitinib), Geftifos (gefitinib), Geftiget (gefitinib), Geftin (gefitinib), Geftinat NATCO (gefitinib), Geftinat RADIANCE (gefitinib), Geftistar (gefitinib), Genasense (oblimersen sodium), GI3000, Gilotrif (aftinib), GSK2136773, HC15, HD201 (trastuzumab), HE39, HER-1 therapeutic cancer vaccine BIOVEN, HER1 Cancer Vaccine, HER2 BiTE Antibody AMGEN, Herzuma (trastuzumab), HKI357, HM60390, HM61713, HM78136B (poziotinib), HMPL309 (theliatinib), HMPL504 (volitinib), HMPL813 (epitinib), HuMax-EGFr (zalutumumab), HyERB (cetuximab), ICS283, Imatinib Ecker hydrochloride, Imbruvica (ibrutinib), IMC11F8 (necitumumab), IMCA12 (cixutumumab), IMGN289, INC280, INCB7839 (aderbasib), Inlyta (axitinib), Iressa (gefitinib), ISU101 (cetuximab), JNJ26483327, JNJ28871063, JX929, Kabigef (gefitinib), KD019, KD020, KHK2866, KRN633, KRN951 (tivozanib), KSB102, KT6587, Lapatinib AQVIDA, Lapatinib ditosylate, Lapatinib SRS PHARMA, Lefibenz (gefitinib), Lortinib (erlotinib), LY2875358 (emibetuzumab), MabionEGFR (cetuximab), MDP01, MDX214 (CD89 monoclonal antibody), MDX447, Meftinib (gefitinib), MEHD7945A, Menadione TALON, MGCD265, mir-7 mimetic SILENCE, MM121, MM151, MP412, MP470 (amuvatinib), MT062, Novel Tyrosine kinase Inhibitor MEBIOPHARM, NT004, NT113, ORIL003, ORIL007, OSI632, Panitumumab BIOXPRESS THERAPEUTICS (panitumumab), Panitumumab PANPHARMACEUTICALS, PB272 (neratinib), PBI1737, PBI4050, Perjeta (pertuzumab), PF00299804 (dacomitinib), PKI166, PM92102 (kahalalide F), RadioTheraCIM, RAF and HER inhibitors DECIPHERA, RBLX200, RC3095, Reglycosylated Cetuximab FOUNTAIN BIOPHARM, RG3638 (onartuzumab), RG7160 (imgatuzumab), RX1792, S222611, SAB-Y1 SYNTAB, Sai Pu Ting, SAI-EGFR-ECD MICROMET, SAR103168, SC100, Selatinib Ditosilate QILU, Selective EGFR Inhibitors VICHEM, SL175, SL176, SL433, SL461, SL634, SRXMs MAXOCARE, STIA020X, STIA050X, Stivarga (regorafenib), STP523, STP801, Stridessa (gefitinib), Surrobody Drug Conjugate SEA LANE, Sym004, Sym013, TaiXinSheng (nimotuzumab), TAK285, Tarceva (erlotinib hydrochloride), Targretin (bexarotene), TAS2913, TG03 (apricoxib), TGF therapeutic cancer vaccine BIOVEN, TGF-alpha Cancer Vaccine MICROMET, Trastuzumab ZENOTECH, Trisilensa, Trispecific Anti-Her1/Her3 Zybodies ZYNGENIA (cetuximab), Tykerb (lapatinib ditosylate), Tyrogef, Tyrokinin 100 (erlotinib hydrochloride), U31287 (partitumab), Ultragef (gefitinib), VCC395122, VCC868449, Vectibix (panitumumab), VI14442, Xefta (gefitinib), YMB1005, Zefotib (gefitinib), and Zufinib (gefitinib).

The anticancer drug targeting the RAS/RAF/MEK/ERK signal pathway is selected from the group consisting of: Dabrafenib, GSK2118436, LGX818, Vemurafenib, RAF265, RO5126766, Sorafenib, XL281 (Raf inhibitor); ARRY-300, , AZD8330, E6201, PD-0325901, RO4987655, Bimetinib (ARRY-162/ MEK162), Cobimetinib (GDC-0973/XL518), Refametinib (BAY86-9766/RDEA119), Pisasertib (AS703026), Selumetinib (AZD6244/ARRY-142886), TAK-733, Trametinib (GSK1120212) (MEK inhibitor), BVD-523, and MK8553 (ERK inhibitor) .

The anticancer drug targeting PI3K/AKT/mTOR signal pathway is selected from the group consisting of : BGT226, BEZ235, GDC-0980, GSK2126458, PF-04691502, PF-05212384/PKI-587, SF1126, XL765/SAR245409, BKM120, BYL719, CAL-101, GDC-0032 , GDC-0941, GSK2636771, IPI-145, NVP-BEZ235, PX866, XL147 (PI3K inhibitor); Erucylphosphocholine, GSK2141795, GSK690693, Miltefosine, MK2206 , PBI-05204 , Perifosine, RX-0201, SR13668, and XL-418 (AKT inhibitor); AZD2014, AZD8055, CC-223, Everolimus(RAD001), Ridaforolimus (MK-8669), OSI-027, Sirolimus (Rapamycin), Temsirolmus (Torisel^{®}, CCI-779) (mTOR inhibitor).

The anticancer drug targeting JAK/STAT signal pathway is JAK kinase inhibitor or STAT inhibitor. JAK kinase inhibitor has inhibitory activity against JAK1, JAK2, and/or JAK3 and is selected from the group consisting of ABT-494, AT9283, atiprimod dihydrochloride, AZD1480, Baricitinib, BMS-911543, CP 690550, Cucurbitacin I, Decernotinib, Filgotinib, Gandotinib, GSK2586184, Itacitinib (INCB039110), INCB018424, INCB047986, INCB052793, Lestaurtinib, Momelotinib (CYT387), NS-018, NSC 33994, Pacritinib, Peficitinib, Ruxolitinib (Jakafi), PF-04965842, SD 1008, Tofacitinib, Upadacitinib, XL019, and WP1066.

STAT inhibitor has inhibitory activity against STATs 1, 2, 3, 4, 5a, 5b, and 6, in particular STAT3 and STAT5b and is selected from the group consisting of AZD9150, Capsaicin, CPA-1, CPA-7, FLLL11, FLLL12, FLLL32, FLLL62, IS3295, JQ1, OPB-111077, OPB-31121, OPB-51602 and pimozide.

### Pharmaceutical Composition

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically made adjuvant at suitable dosage level in a known way. The preferred preparations are adapted for oral application. These administration forms include, for example, pills, tablets, film tablets, coated tablets, capsules, powders and deposits.

Optionally, the pharmaceutical compositions according to the present invention comprise further at least one anticancer drug. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway as defined above.

Furthermore, the present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anticancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to 1 weight %.

The compounds of the present invention are suitable for use in medicine, particulary in human medicine, but also in veterinary medicine. The dosage of the compounds may be determined by a skilled practitioner according to the type and severity of the disorder to be treated.

The compounds of the present invention may be admistered as a monotherapy or together with further active agents, particulary chemotherapeutic agents or antitumor antibodies. Furthermore they may be used in combination with surgery and/or irradiation.

### Description of Figures

**Figure 1****:** core sequences of EGFR mutants EGFR D770_N771 insSVD, EGFR H773_V774insNPH, EGFR V769_D770insASV, EGFR P772_H773insPR, EGFR T790M and EGFR T790ML858R and HER mutant Her2 INS8 INS YVMA.
**Figure 2** shows the hydrolysis of the acrylamide moiety of reference 1 in the presence of liver microsomes after 50 minutes.

### Preparation of compounds:

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

### Abbreviations used in the description of the chemistry and in the Examples that follow are:

ACN (acetonitrile); br (broad); CDCl₃ (deuterated chloroform); cHex (cyclohexane); DCM (dichloromethane); DIPEA (di-iso-propylethylamine); DMF (dimethylformamide); DMSO (dimethyl sulfoxide); eq. (equivalent); ES (electrospray); EtOAc (ethyl acetate); EtOH (ethanol); HATU (O-(7-azabenzotriazol-1-yl)-*N*,*N*,*N*',*N*'-tetramethyluronium hexafluorophosphate); HCl (hydrochloric acid); HOAc (acetic acid); H₂O (water); K₂CO₃ (potassium carbonate); KOH (potassium hydroxide); MeOH (methanol); MS (mass spectrometry); Mwt (molecular weight); NaHCO₃ (sodium hydrogencarbonate); NH₃ (ammonia); NH₄Cl (ammonium chloride); NIS (N-Iodosuccinimide); NMP (N-methyl-2-pyrrolidon); NMR (nuclear magnetic resonance); Pd(dppf)Cl₂ ([1,1'-bis(diphenylphosphino)ferrocene]dichloro palladium(II) complex with dichloromethane); iPrOH (iso-propanol); PyBroP (Bromotripyrrolidinophosphonium hexafluorophosphate); RP (reversed-phase); RT (room temperature); sat. aq. (saturated aqueous); SiO₂ (silica gel); TFA (trifluoroacetic acid); THF (tetrahydrofurane).

### Preparative Examples

### Example 1:

### N-(5-(4-chloro-2-(4-((4-methylpiperazin-1-yl)methyl)phenyl)-1H-pyrrolo[2,3-b]pyridin-3-yl)-2-methylphenyl)acrylamide 2,2,2-trifluoroacetate (A1)

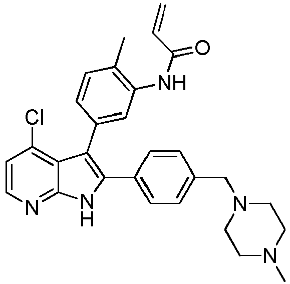

### Step 1: 4-Chloro-3-(4-methyl-3-nitrophenyl)-2-(trimethylsilyl)-1H-pyrrolo[2,3-b]pyridine (A1)

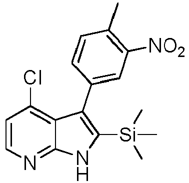

4-Chloro-3-iodopyridine-2-amine (5.0g, 19.0mmol, 1.0eq.), trimethyl((4-methyl-3-nitrophenyl)ethynyl)silane (5.7g, 25.0mmol, 1.25eq.), 1,4-diazabicyclo[2.2.2]octane (3.6g, 32.3mmol, 1.7eq.) and dichlorobis(triphenylphosphine)palladium(II) (1.4g, 2.0mmol, 0.1eq.) in dry DMF (40mL) under N₂ atmosphere was splitted in three microwave vials. Each vial was heated in the microwave at 145°C for 2 h. EtOAc (250mL) was added and the organic phase was washed three times with aq. sat. NaHCO₃-solution. The organic phase was dried over MgSO₄ and solvents were removed in vacuo. The crude product was purified by flash chromatography on silica gel (cHex/EtOAc = 100:0 to 1:1) to yield the desired product **A1** (3.9g, 10.8mmol, 57%) as a beige solid. ¹H NMR (400MHz, d₆-DMSO, 300K) δ 0.11 (s, 9H), 2.58 (s, 3H), 7.12 (d, J = 5.0 Hz, 1H), 7.52 (d, J = 7.9 Hz, 1H), 7.63 (d, J = 7.9 Hz, 1H), 7.91 (s, 1H), 8.21 (d, J = 5.0 Hz, 1H), 12.04 (s, 1H). MS (ES) C₁₇H₁₈ClN₃O₂Si requires: 359, found: 360 (M+H)⁺.

### Step 2: 5-(4-Chloro-2-(trimethylsilyl)-1H-pyrrolo[2,3-b]pyridin-3-yl)-2-methylaniline (A2)

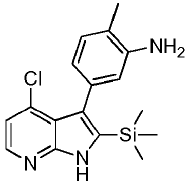

A solution of 4-chloro-3-(4-methyl-3-nitrophenyl)-2-(trimethylsilyl)-1H-pyrrolo[2,3-b]pyridine **A1** (5.6g, 15.56mmol, 1.0eq.) and iron (4.3g, 77.8mmol, 5 eq.) in EtOH (100mL) and aq. sat. NH₄Cl-solution (10mL) was stirred for 5h at 80°C. The solution was filtered through a pad of Celite^{®}. Solvents were removed in vacuo. The crude was solved in EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 0:100) to yield the desired product **A2** (5.0g, 15.2mmol, 97%) as a beige solid. ¹H NMR (400MHz, d₆-DMSO, 300K) δ 0.10 (s, 9H), 2.10 (s, 3H), 4.76 (br s, 2H), 6.44 (d, J = 7.4 Hz, 1H), 6.60 (s, 1H), 6.89 (d, J = 7.4 Hz, 1H), 7.04 (d, J = 5.1 Hz, 1H), 8.15 (d, J = 5.1 Hz, 1H), 11.71 (s, 1H). MS (ES) C₁₇H₂₀ClN₃Si requires: 329, found: 330 (M+H)⁺.

### Step 3: N-(5-(4-chloro-2-(trimethylsilyl)-1H-pyrrolo[2,3-b]pyridin-3-yl)-2-methylphenyl)acrylamide (A3)

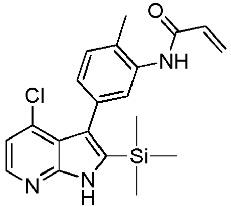

**A3** was prepared either by Procedure A or Procedure B:

### Procedure A:

To a solution of **A2** (2760mg, 8.4mmol, 1.0eq.) and DIPEA (14.6mL, 84.0mmol, 10.0eq.) in dry DCM (100mL) at 0°C was added slowly acrylolyl chloride (757mg, 8.4mmol, 1.0eq.) in dry DCM (5mL). The mixture was stirred for 5min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product **A3** (3070mg, 8.0mmol, 95%) as a white solid. ¹H NMR (400MHz, d₆-DMSO, 300K) δ 0.11 (s, 9H), 2.28 (s, 3H), 5.72 (dd, J = 2.1 Hz, J = 10.2 Hz, 1H), 6.22 (dd, J = 2.1 Hz, J = 16.9 Hz, 1H), 6.55 (dd, J = 10.2 Hz, J = 16.9 Hz, 1H), 7.07 (m, 2H), 7.22 (d, J = 7.7 Hz, 1H), 7.56 (s, 1H), 8.17 (d, J = 5.0 Hz, 1H), 9.42 (s, 1H), 11.83 (s, 1H). MS (ES) C₂₀H₂₂ClN₃OSi requires: 383, found: 384 (M+H)⁺.

### Procedure B:

To a solution of HATU (3460mg, 9.1mmol, 1.5eq.) in DMF (10mL) at 0°C was added acrylic acid (655mg, 9.1mmol, 1.5eq.) and DIPEA (1548mg, 9.1mmol, 2.0eq.). The mixture was stirred for 15min. Then **A2** (2000mg, 6.0mmol, 1.0eq.) was added and the mixture was stirred for 4h at 0°C. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product **A3** (890mg, 2.3mmol, 39%) as a white solid. MS (ES) C₂₀H₂₂ClN₃OSi requires: 383, found: 384 (M+H)⁺.

### Step 4: N-(5-(4-chloro-2-iodo-1H-pyrrolo[2,3-b]pyridin-3-yl)-2-methylphenyl)acrylamide (A4)

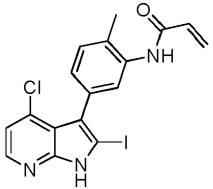

N-(5-(4-chloro-2-(trimethylsilyl)-1H-pyrrolo[2,3-b]pyridin-3-yl)-2-methylphenyl)acrylamide **A3** (890mg, 2.32mmol, 1.0eq.) and N-iodosuccinimide (937mg, 4.18mmol, 1.8eq.) were solved in dry dichloromethane (300mL) and stirred for 15h at RT. The organic phase was washed once with aq. sat. Na₂S₂O₃-sol. and three times with aq. sat. NaHCO₃-solution. The organic phase was dried over Na₂SO₄ and solvents were removed in vacuo yielding the desired product **A4** (970mg, 2.21mmol, 95%) as a yellow solid. The crude was used without purification in the next step. MS (ES) C₁₇H₁₃ClIN₃O requires: 437, found: 438 (M+H)⁺.

### Step 5: N-(5-(4-chloro-2-(4-((4-methylpiperazin-1-yl)methylphenyl-1H-pyrrolo[2,3-b]pyridin-3-yl)-2-methylphenyl)acrylamide 2,2,2-trifluoroacetate (A5)

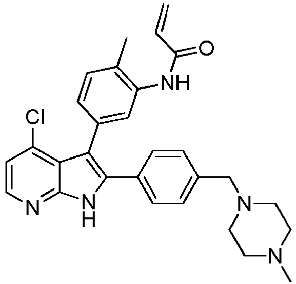

A mixture of N-(5-(4-chloro-2-iodo-1H-pyrrolo[2,3-b]pyridin-3-yl)-2-methylphenyl)acrylamide **A4** (40mg, 0.09mmol, 1.0eq.), {4-[(4-methylpiperazin-1-yl)methyl]phenyl}boronic acid dihydrochloride (32mg, 0.14mmol, 1.5eq.), and K₃PO₄ (38mg, 0.18mmol, 2.0 eq) in dioxane/H₂O (3mL/0.6mL) was degassed with a stream of N₂ for 5min. [1,1'-Bis(diphenylphosphino)ferrocene]palladium dichloride dichloromethane adduct (8mg, 0.01mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **A5** (5mg, 0.01mmol, 8%) as a yellow powder. ¹H NMR (400MHz, CDCl₃, 300K) δ 12.45 (s, 1H), 9.44 (s, 1H), 8.16 (dd, J = 1.3 Hz, J = 5.2 Hz, 1H), 7.52 (s, 1H), 7.44 (d, J = 7.9 Hz, 2H), 7.25 (d, J = 7.9 Hz, 2H), 7.20 (d, J = 7.8 Hz, 1H), 7.10 (dd, J = 1.3 Hz, J = 5.2 Hz, 1H), 7.04 (d, J = 7.8 Hz, 1H), 6.51 (dd, J = 10.1 Hz, J = 17.1 Hz, 1H), 6.17 (d, J = 17.1 Hz, 1H), 5.70 (d, J = 10.1 Hz, 1H), 3.58 (s, 2H), 3.01 - 2.89 (m, 4H), 2.74 (s, 3H), 2.65 (m, 2H), 2.31 (m, 2H), 2.25 (s, 3H). MS (ES) C₂₉H₃₀ClN₅O requires: 500, found: 501 (M+H)⁺.

The Examples in the following table were prepared according to the procedure described for **A5** (Example 1).

| **Example** | **Name** | **Mwt** | **[M+H]⁺** |
|---|---|---|---|
| 2 | 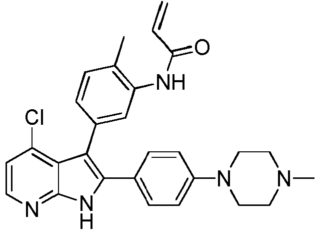 | 485 | 486 |
| 3 | 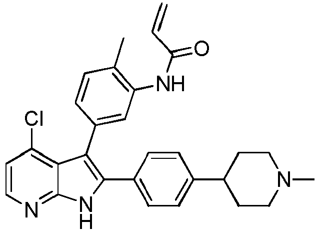 | 484 | 485 |
| 4 | 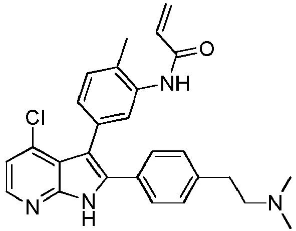 | 458 | 459 |
| 5 | 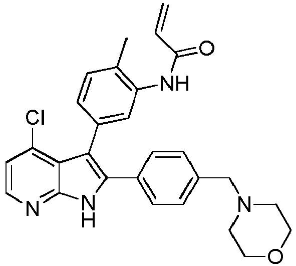 | 486 | 487 |
| 6 | 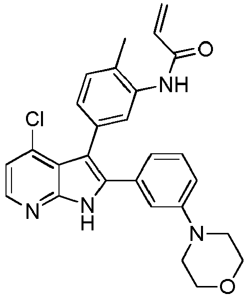 | 472 | 473 |
| 7 | 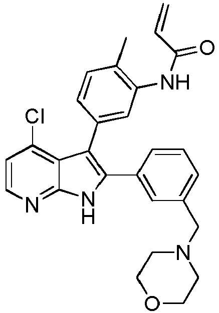 | 486 | 487 |
| 8 | 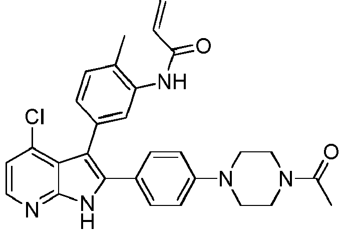 | 513 | 514 |
| 9 | 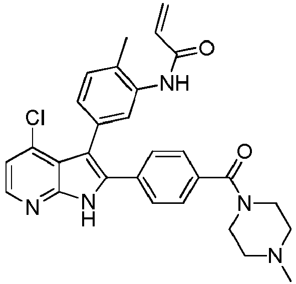 | 513 | 514 |
| 10 | 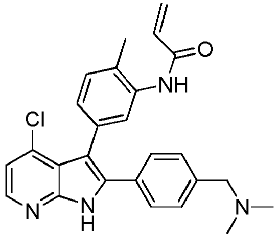 | 444 | 445 |
| 11 | 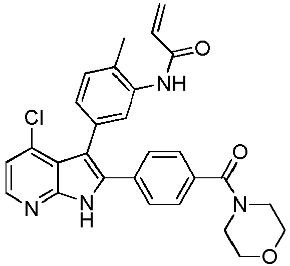 | 500 | 501 |
| 12 | 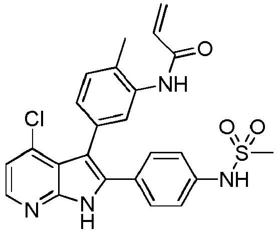 | 480 | 481 |
| 13 | 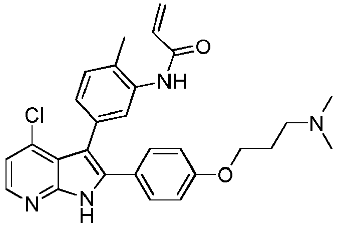 | 488 | 489 |
| 14 | 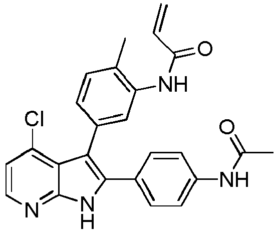 | 444 | 445 |
| 15 | 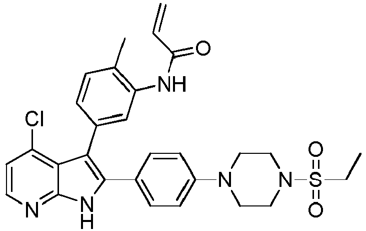 | 563 | 564 |
| 16 | 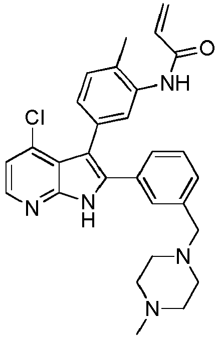 | 499 | 500 |
| 17 | 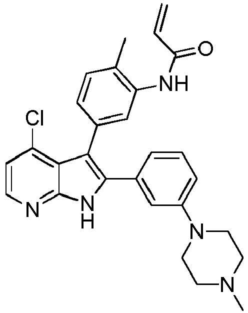 | 485 | 486 |
| 18 | 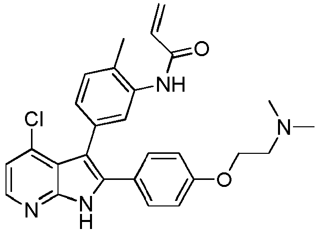 | 474 | 475 |
| 19 | 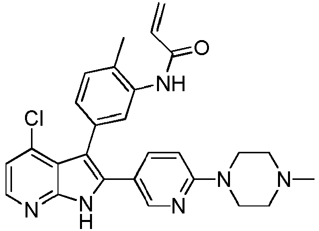 | 486 | 487 |
| 20 | 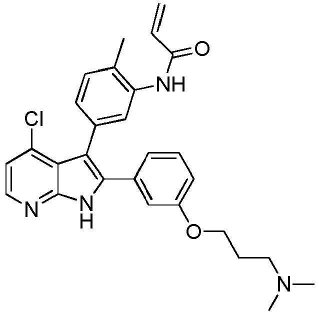 | 488 | 489 |
| 21 | 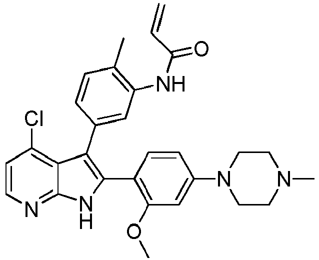 | 515 | 516 |
| 22 | 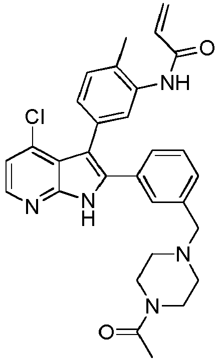 | 527 | 528 |
| 23 | 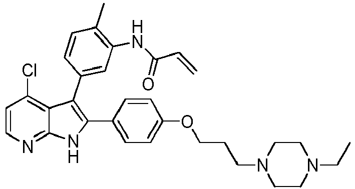 | 557 | 558 |
| 24 | 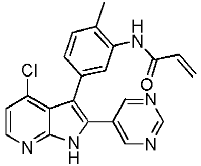 | 389 | 390 |
| 25 | 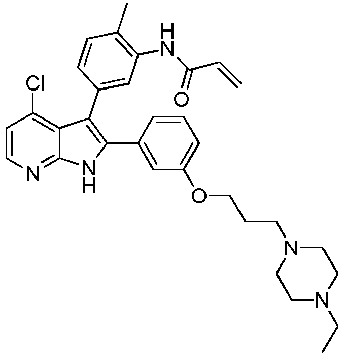 | 557 | 558 |
| 26 | 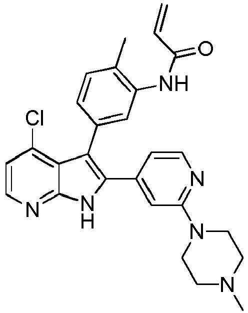 | 486 | 487 |
| 27 | 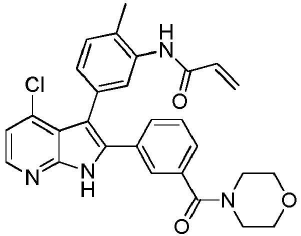 | 500 | 501 |
| 28 | 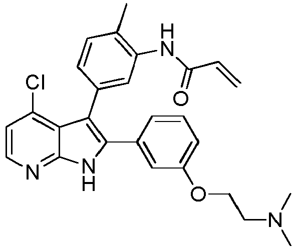 | 474 | 475 |
| 29 | 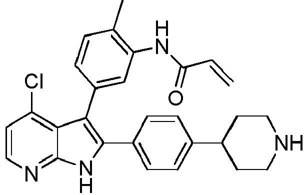 | 470 | 471 |
| 30 | 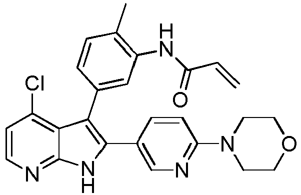 | 473 | 474 |
| 31 | 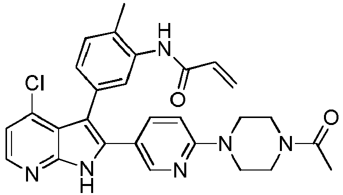 | 514 | 515 |
| 32 | 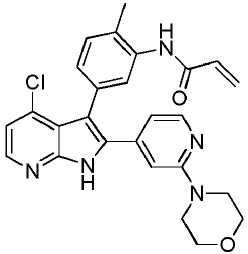 | 473 | 474 |
| 33 | 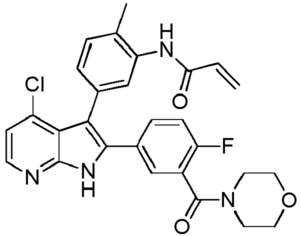 | 518 | 519 |
| 34 | 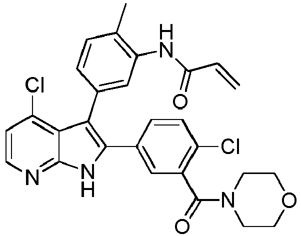 | 534 | 535 |
| 35 | 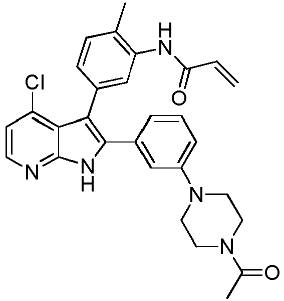 | 513 | 514 |
| 36 | 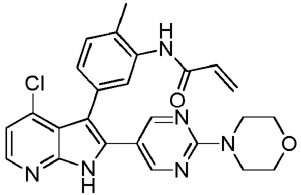 | 474 | 475 |
| 37 | 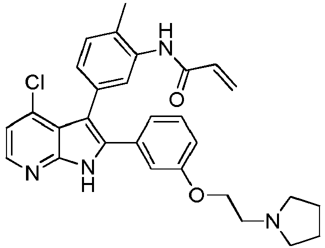 | 500 | 501 |
| 38 | 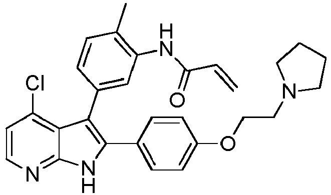 | 500 | 501 |
| 38 | 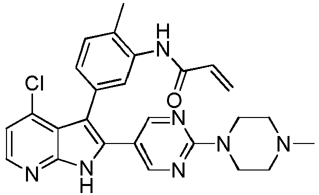 | 487 | 488 |
| 40 | 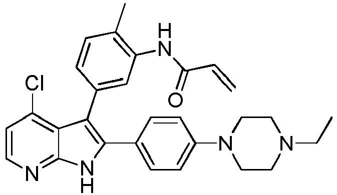 | 499 | 500 |
| 41 | 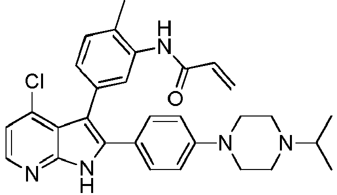 | 513 | 514 |
| 42 | 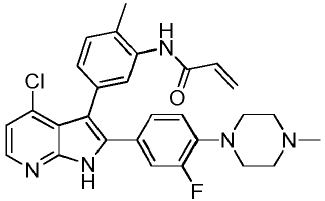 | 503 | 504 |
| 43 | 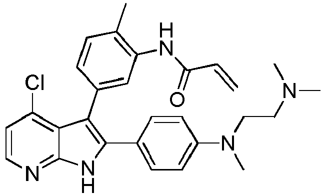 | 487 | 488 |

### Example 44:

### N-(5-(4-chloro-2-(3-(4-methylpiperazine-1-carbonyl)phenyl)-1H-pyrrolor2,3-b]pyridin-3-yl)-2-methylphenyl)acrylamide 2,2,2-trifluoroacetate (B2)

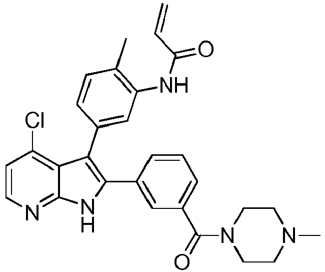

### Step 1: 3-(3-(3-acrylamido-4-methylphenyl)-4-chloro-1H-pyrrolo[2,3-b]pyridin-2-yl)benzoic acid (B1)

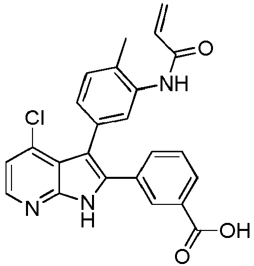

A mixture of N-(5-(4-chloro-2-iodo-1H-pyrrolo[2,3-b]pyridin-3-yl)-2-methylphenyl)acrylamide **A4** (500mg, 1.14mmol, 1.0eq.), 3-carboxyphenylboronic acid (284mg, 1.71mmol, 1.5eq.), and K₃PO₄ (483mg, 2.28mmol, 2.0 eq) in dioxane/H₂O (15mL/3mL) was degassed with a stream of N₂ for 5min. [1,1'-Bis(diphenylphosphino)ferrocene]palladium dichloride dichloromethane adduct (93mg, 0.11mmol, 0.1 eq) was added and the reaction mixture heated to 130°C for 2 h in the microwave oven. Solvents were removed and the crude was purified by flash chromatography on silica gel (DCM/MeOH = 100:0 to 0:100) to yield the desired product **B1** (200mg, 0.46mmol, 41%) as a beige solid. MS (ES) C₂₄H₁₈ClN₃O₃ requires: 431, found: 432 (M+H)⁺.

### Step 2: N-(5-(4-chloro-2-(3-(4-methylpiperazine-1-carbonyl)phenyl)-1H-pyrrolo[2,3-b]pyridin-3-yl)-2-methylphenyl)acrylamide 2,2,2-trifluoroacetate (B2)

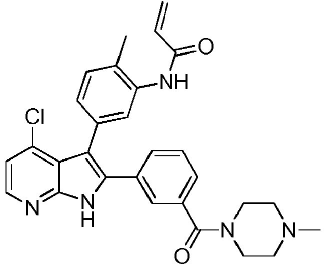

To a solution of **B1** (10mg, 0.02mmol, 1.0eq.) and HATU (18mg, 0.05mmol, 2.0eq.) in DMF (2mL) at 0⁰C was added 1-methylpiperazine (4mg, 0.03mmol, 1.5 eq.) and DIPEA (20uL, 0.11mmol, 5.0eq.). The mixture was stirred for 1h. The reaction mixture was diluted with EtOAc, washed three times with aq. sat. NaHCO₃-solution. The organic phase was dried over MgSO₄ and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **B2** (3mg, 0.003mmol, 14%) as a yellow powder. ¹H NMR (400MHz, MeOD, 300K) δ 8.17 (d, J = 5.3 Hz, 1H), 7.77 (d, J = 7.9 Hz, 1H), 7.57 (t, J = 7.7 Hz, 1H), 7.46 (d, J = 7.7 Hz, 1H), 7.40 (m, 2H), 7.25 (d, J = 7.7 Hz, 1H), 7.12 (m, 2H), 6.50 (dd, J = 10.2 Hz, J = 17.0 Hz, 1H), 6.35 (d, J = 17.0 Hz, 1H), 5.80 (d, J = 10.2 Hz, 1H), 3.54 - 2.80 (m, 8H), 2.95 (s, 3H), 2.32 (s, 3H). MS (ES) C₂₉H₂₈CIN₅O₂ requires: 513, found: 514 (M+H)⁺.

The Examples in the following table were prepared according to the procedure described for **B2** (Example 44).

| **Example** | **Name** | **Mwt** | **[M+H^{t}** |
|---|---|---|---|
| 45 | 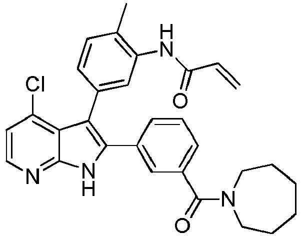 | 512 | 513 |
| 46 | 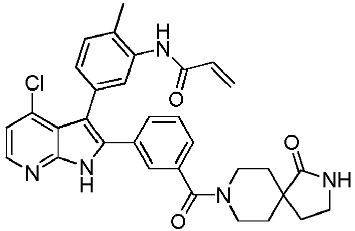 | 567 | 568 |
| 47 | 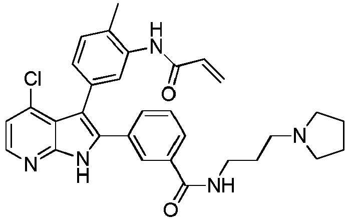 | 541 | 542 |
| 48 | 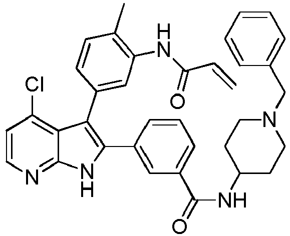 | 603 | 604 |
| 49 | 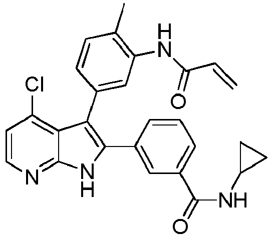 | 470 | 471 |
| 50 | 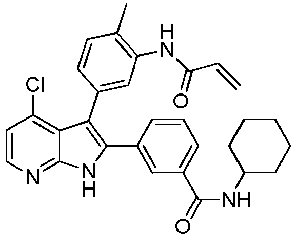 | 512 | 513 |
| 51 | 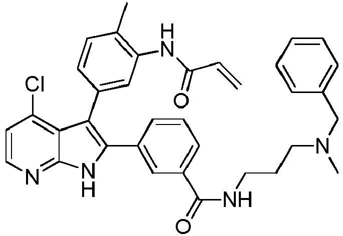 | 591 | 592 |
| 52 | 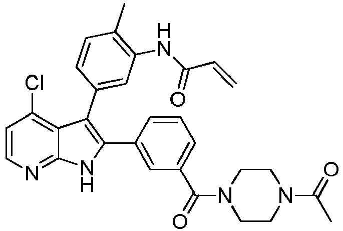 | 541 | 542 |
| 53 | 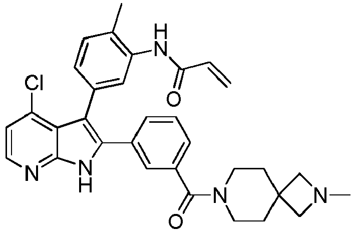 | 553 | 554 |
| 54 | 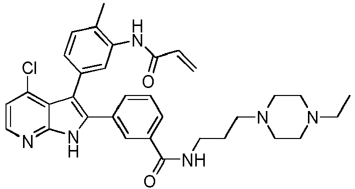 | 584 | 585 |

### Example 55:

### 1-(6-(4-Chloro-2-(4-(1-methylpiperidin-4-yl)phenyl)-1H-pyrrolo[2,3-b]pyridin-3-yl)indolin-1-yl)prop-2-en-1-one 2,2,2-trifluoroacetate (C5)

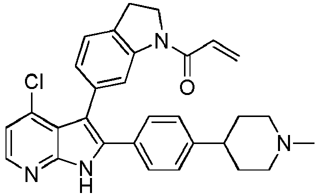

### Step 1: Tert-butvl 6-(4-chloro-2-(trimethylsilyl)-1H-pyrrolo[2,3-b]pyridin-3-yl)indoline-1-carboxylate (C1)

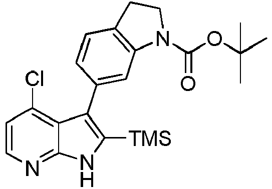

**C1** (1.0g, 2.26mmol, 76%, beige solid) was prepared from 4-chloro-3-iodopyridine-2-amine and tert-butyl 6-((trimethylsilyl)ethynyl)indoline-1-carboxylate following the general procedure reported in Preparative Example 1 Step 1. MS (ES) C₂₃H₂₈CIN₃O₂Si requires: 441, found: 442 (M+H)⁺.

### Step 2: 4-Chloro-3-(indolin-6-yl)-2-(trimethylsilyl)-1H-pyrrolo[2,3-b]pyridine (C2)

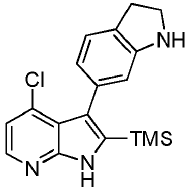

Tert-butyl 6-(4-chloro-2-(trimethylsilyl)-1H-pyrrolo[2,3-b]pyridin-3-yl)indoline-1-carboxylate **C1** (1.0g, 2.26mmol) in 30% TFA in DCM (60mL) at RT was stirred for 2h. Evaporation of the solvents yielded the desired product C2 as beige solid, which was used in the next step without purification. MS (ES) C₁₈H₂₀CIN₃Si requires: 341, found: 342 (M+H)⁺.

### Step 3: 1-(6-(4-Chloro-2-(trimethylsilyl)-1H-pyrrolo[2,3-b]pyridin-3-yl)indolin-1-vl)prop-2-en-1-one (C3)

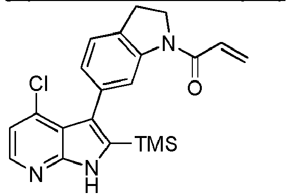

**C3** (50mg, 0.13mmol, 6%, beige solid) was prepared from **C2** following the general procedure reported in Preparative Example 1 Step 3 Procedure A. MS (ES) C₂₁H₂₂CIN₃OSi requires: 395, found: 396 (M+H)⁺.

### Step 4: 1-(6-(4-Chloro-2-iodo-1H-pyrrolo[2,3-b]pyridin-3-yl)indolin-1-yl)prop-2-en-1-one (C4)

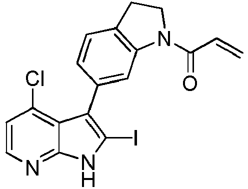

**C4** (51mg, 0.11mmol, 84%, yellow solid) was prepared from **C3** following the general procedure reported in Preparative Example 1 Step 4. MS (ES) C₁₈H₁₃ClIN₃O requires: 448, found: 449 (M+H)⁺.

### Step 5: 1-(6-(4-chloro-2-(4-(1-methylpiperidin-4-yl)phenyl)-1H-pyrrolo[2.3-b]pyridin-3-yl)indolin-1-yl)prop-2-en-1-one 2,2,2-trifluoroacetate (C5)

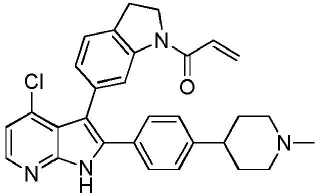

**C5** was prepared from **C4** following the general procedure reported in Preparative Example 1 Step 5. The crude was purified by reverse phase RP-HPLC (column: C18), using H₂O (0.1%TFA) and ACN (0.1%TFA) as eluents. The desired fractions were lyophilized to yield the title compound **C5** (10mg, 0.01 mmol, 12%) as a yellow solid. ¹H NMR (400MHz, d₄-MeOD, 300K) δ 1.92 (m, 3H), 2.10 (d, J = 13.6 Hz, 2H), 2.85 (m, 1H), 2.91 (s, 3H), 3.15 (m, 3H), 3.59 (d, J = 10.3 Hz, 2H), 4.31 (t, J = 8.5 Hz, 2H), 5.82 (d, J = 10.6 Hz, 1H), 6.33 (d, J = 16.8 Hz, 1H), 6.76 (dd, J = 10.6 Hz, J = 16.8 Hz, 1H), 7.05 (d, J = 7.7 Hz, 1H), 7.10 (d, J = 5.2 Hz, 1H), 7.30 - 7.19 (m, 3H), 7.46 (d, J = 8.1 Hz, 2H), 7.71 (d, J = 8.1 Hz, 1H), 8.12 (d, J = 5.2 Hz, 1H), 8.25 (s, 1H). MS (ES) C₃₀H₂₉CIN₄O requires: 496, found: 497 (M+H)⁺.

The Examples in the following table were prepared according to the procedure described for **C5** (Example 55).

| **Example** | **Name** | **Mwt** | **[M+H]⁺** |
|---|---|---|---|
| 56 | 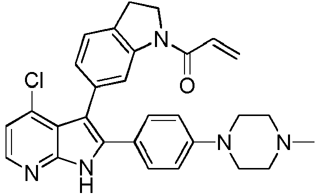 | 497 | 498 |
| 57 | 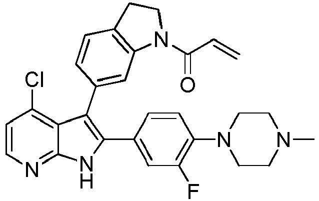 | 515 | 516 |
| 58 | 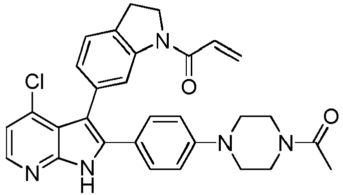 | 525 | 526 |
| 59 | 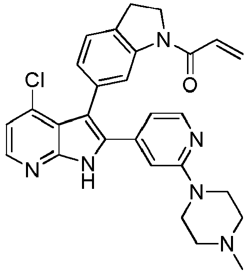 | 498 | 499 |
| 60 | 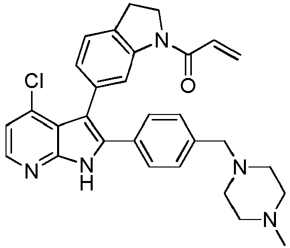 | 511 | 512 |
| 61 | 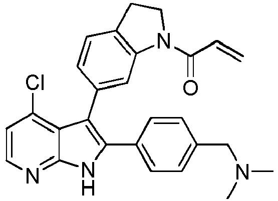 | 456 | 457 |
| 62 | 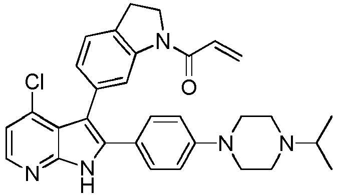 | 525 | 526 |
| 63 | 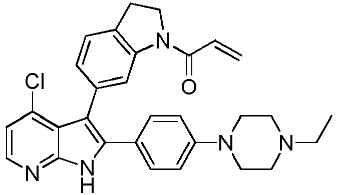 | 511 | 512 |
| 64 | 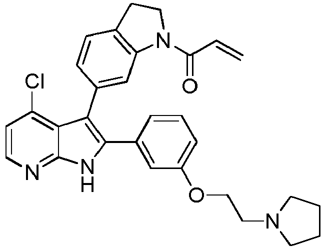 | 512 | 513 |
| 65 | 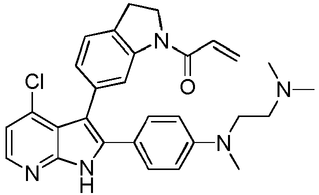 | 499 | 500 |
| 66 | 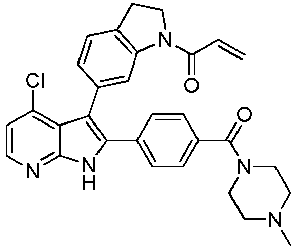 | 525 | 526 |
| 67 | 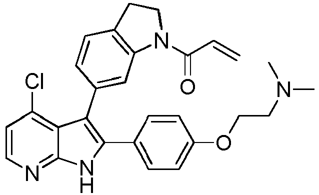 | 486 | 487 |
| 68 | 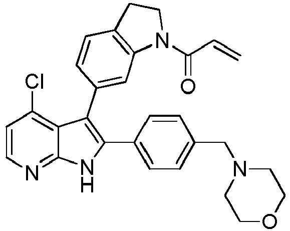 | 498 | 499 |
| 69 | 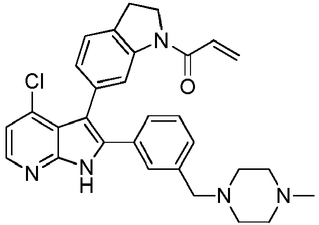 | 511 | 512 |
| 70 | 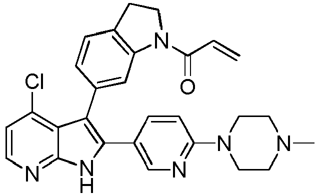 | 498 | 499 |
| 71 | 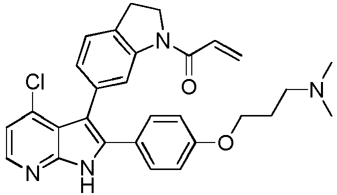 | 500 | 501 |

### Example 72

### N-(5-(4-methoxy-2-(4-(4-methylpiperazin-1-yl)phenyl)-1H-pyrrolo[2,3-b]pyridin-3 yl)-2-methylphenyl)acrylamide 2,2,2-trifluoroacetate (D5)

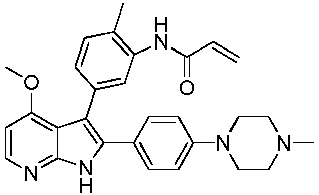

### Step 1: 4-Methoxy-3-(4-methyl-3-nitrophenyl)-2-(trimethylsilyl)-1H-pyrrolor2,3-b]pyridine (D1)

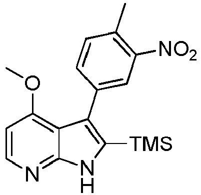

**D1** (3.50g, 9.86mmol, 38%, beige solid) was prepared from 3-iodo-4-methoxypyridin-2-amine and trimethyl((4-methyl-3-nitrophenyl)ethynyl)silane following the general procedure reported in Preparative Example 1 Step 1. MS (ES) C₁₈H₂₁N₃O₃Si requires: 355, found: 356 (M+H)⁺.

### Step 2: 5-(4-Methoxy-2-(trimethylsilyl)-1H-pyrrolo[2,3-b]pyridin-3-yl)-2-methylaniline (D2)

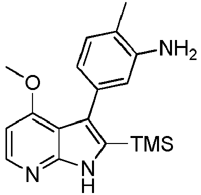

A solution of **D1** (2.5g, 7mmol, 1eq.) and iron (1.9g, 35mmol, 5eq.) in EtOH (100mL) and aq. sat. NH₄CI-solution (10mL) was stirred for 5h at 80⁰C. The solution was filtered through a pad of Celite^{®}. Solvents were removed in vacuo. The crude was solved in EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 0:100) to yield the desired product **D2** (1.0g, 3.1mmol, 44%) as a beige solid. MS (ES) C₁₈H₂₃N₃OSi requires: 325, found: 326 (M+H)⁺.

### Step 3: N-(5-(4-methoxy-2-(trimethylsilyl)-1H-pyrrolo[2,3-b]pyridin-3-yl)-2-methylphenyl)acrylamide (D3)

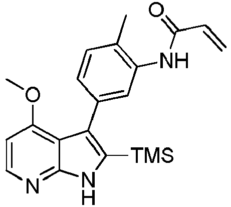

**D3** (712mg, 1.87mmol, 40%, white solid) was prepared from **D2** following the general procedure reported in Preparative Example 1 Step 3 Procedure A. MS (ES) C₂₁H₂₅N₃O₂Si requires: 379, found: 380 (M+H)⁺.

### Step 4: N-(5-(2-iodo-4-methoxy-1H-pyrrolo[2,3-b]pyridin-3-yl)-2-methylphenyl)acrylamide (D4)

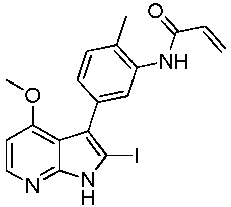

**D4** (512mg, 1.18mmol, 66%, yellow solid) was prepared from **D3** following the general procedure reported in Preparative Example 1 Step 4. MS (ES) C₁₈H₁₆IN₃O₂ requires: 433, found: 434 (M+H)⁺.

### Step 5: N-(5-(4-methoxy-2-(4-(4-methylpiperazin-1-yl)phenyl)-1H-pyrrolo[2,3-b]pyridin-3-yl)-2-methylphenyl)acrylamide 2,2,2-trifluoroacetate (D5)

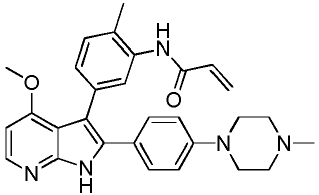

**D5** was prepared from **D4** following the general procedure reported in Preparative Example 1 Step 5. The crude was purified by reverse phase RP-HPLC (column: C18), using H₂O (0.1%TFA) and ACN (0.1%TFA) as eluents. The desired fractions were lyophilized to yield the title compound **D5** (2mg, 0.01mmol, 3%) as a yellow solid. ¹H NMR (400MHz, d₄-MeOD, 300K) δ 2.30 (s, 3H), 2.96 (s, 3H), 3.08 (m, 2H), 3.26 (m, 2H), 3.57 (m, 2H), 3.91 (m, 2H), 3.98 (s, 3H), 5.78 (dd, J = 10.2 Hz, J = 1.5 Hz, 1H), 6.34 (dd, J = 17.0 Hz, J = 1.5 Hz, 1H), 6.51 (dd, J = 10.2 Hz, J = 17.0 Hz, 1H), 6.99 (d, J = 8.8 Hz, 2H), 7.03 (d, J = 6.7 Hz, 1H), 7.08 (d, J = 8.1 Hz, 1H), 7.19 (d, J = 7.8 Hz, 1H), 7.38 (d, J = 8.8 Hz, 2H), 7.46 (s, 1H), 8.22 (d, J = 7.6 Hz, 1H). MS (ES) C₂₉H₃₁N₅O₂ requires: 481, found: 482 (M+H)⁺.

The Examples in the following table were prepared according to the procedure described for D5 (Example 72).

| **Example** | **Name** | **Mwt** | **[M+H]⁺** |
|---|---|---|---|
| 73 | 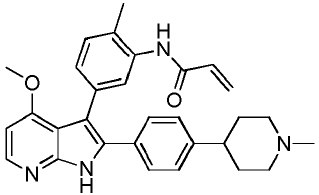 | 480 | 481 |
| 74 | 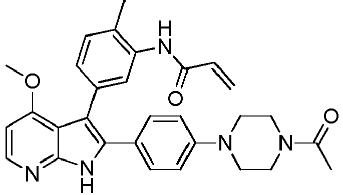 | 509 | 510 |
| 75 | 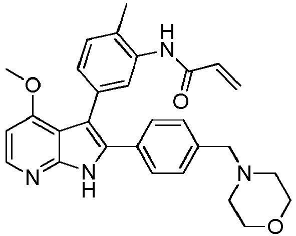 | 482 | 483 |
| 76 | 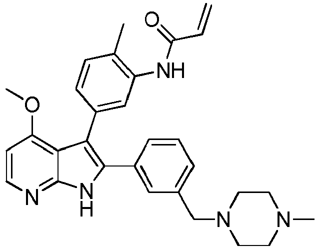 | 495 | 496 |
| 77 | 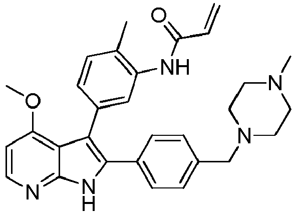 | 495 | 496 |

### Example 78:

### N-(2-methyl-5-(2-(6-(4-methylpiperazin-1-yl)pyridin-3-yl)-1H-pyrrolo[2,3-b]pyridin-3-yl)phenyl)acrylamide 2,2,2-trifluoroacetate (E1)

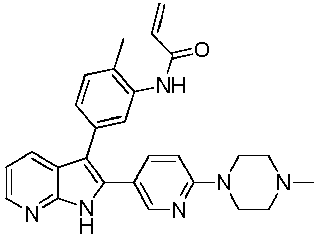

### Step 1: 3-(4-methyl-3-nitrophenyl)-2-(trimethylsilyl)-1H-pyrrolor2,3-blpyridine (E1)

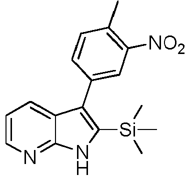

2-Amino-3-iodopyridine (5.65g, 25.71mmol, 1.2eq.), trimethyl((4-methyl-3-nitrophenyl)ethynyl)silane (5.00g, 21.43mmol, 1.0eq.), 1,4-diazabicyclo[2.2.2]octane (4.08g, 32.3mmol, 1.7eq.) and dichlorobis(triphenylphosphine)palladium(II) (1.51g, 2.14mmol, 0.1eq.) in dry DMF (40mL) under N₂ atmosphere was splitted in three microwave vials. Each vial was heated in the microwave at 145⁰C for 2 h. EtOAc (250mL) was added and the organic phase was washed three times with aq. sat. NaHCO₃-solution. The organic phase was dried over MgSO₄ and solvents were removed in vacuo. The crude product was purified by flash chromatography on silica gel (DCM/MeOH = 100:0 to 10:1) to yield the desired product **E1** (4.96g, 10.25mmol, 71%) as an orange solid. MS (ES) C₁₇H₁₉N₃O₂Si requires: 325, found: 326 (M+H)⁺.

### Step 2: 2-Methyl-5-(2-(trimethylsilyl)-1H-pyrrolo[2,3-b]pyridin-3-yl)aniline (E2)

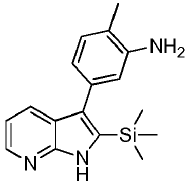

A solution of 3-(4-methyl-3-nitrophenyl)-2-(trimethylsilyl)-1H-pyrrolo[2,3-b]pyridine **E1** (4.96g, 15.24mmol, 1.0eq.) and iron (8.51g, 152.4mmol, 10 eq.) in EtOH (100mL) and aq. sat. NH₄CI-solution (20mL) was stirred for 15h at 80⁰C. The solution was filtered through a pad of Celite^{®}. Solvents were removed in vacuo. The crude was solved in EtOAc and washed twice with aq. sat. NaHCO₃-solution. The organic phase was dried over MgSO₄ and solvent was removed in vacuo. The crude product (3.04g, 10.3mmol, 68%, orange solid) was used without further purification in the next step. MS (ES) C₁₇H₂₁N₃Si requires: 295, found: 296 (M+H)⁺.

### Step 3: N-(2-methyl-5-(2-(trimethylsilyl)-1H-pyrrolo[2,3-b]pyridin-3-yl)phenyl)acrylamide (E3)

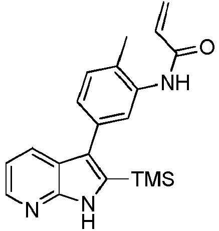

To a solution of **E2** (1000mg, 3.38mmol, 1.0eq.) and DIPEA (2.96mL, 16.92mmol, 5.0eq.) in dry DCM (100mL) at 0⁰C was added slowly acrylolyl chloride (306mg, 3.38mmol, 1.0eq.) in dry DCM (5mL). The mixture was stirred for 30min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:2) to yield the desired product **E3** (498mg, 1.43mmol, 42%) as a white solid. MS (ES) C₂₀H₂₃N₃OSi requires: 349, found: 350 (M+H)⁺.

### Step 4: N-(5-(2-iodo-1H-pyrrolo[2,3-b]pyridin-3-yl)-2-methylphenyl)acrylamide (E4)

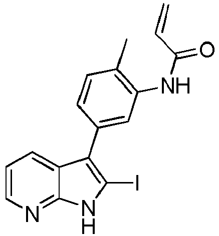

N-(2-methyl-5-(2-(trimethylsilyl)-1H-pyrrolo[2,3-b]pyridin-3-yl)phenyl)acrylamide **E3** (497mg, 1.42mmol, 1.0eq.) and N-iodosuccinimide (576mg, 2.56mmol, 1.8eq.) were solved in dry dichloromethane (100mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **E4** (337mg, 0.84mmol, 59%) as a yellow solid. The crude was used without purification in the next step. MS (ES) C₁₇H₁₄IN₃O requires: 403, found: 404 (M+H)⁺.

### Step 5: N-(2-methyl-5-(2-(6-(4-methylpiperazin-1-yl)pyridin-3-yl)-1H-pyrrolo[2,3-b]pyridin-3-yl)phenyl)acrylamide 2,2,2-trifluoroacetate (E5)

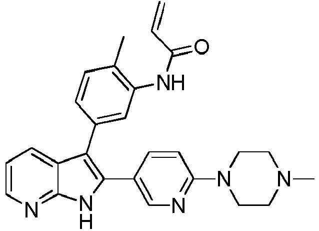

A mixture of N-(5-(2-iodo-1H-pyrrolo[2,3-b]pyridin-3-yl)-2-methylphenyl)acrylamide **E4** (50mg, 0.12mmol, 1.0eq.), 2-(4-methylpiperazino)pyridine-5-boronic acid pinacol ester (49mg, 0.16mmol, 1.3eq.), and K₃PO₄ (53mg, 0.24mmol, 2.0 eq) in dioxane/H₂O (3mL/0.6mL) was degassed with a stream of N₂ for 5min. [1,1'-Bis(diphenylphosphino)-ferrocene]palladium dichloride dichloromethane adduct (10mg, 0.01 mmol, 0.1 eq) was added and the reaction mixture heated to 110°C for 1 h in the microwave oven. The crude solution was directly purified by reverse phase RP-HPLC (column: C18), using H₂O (0.1%TFA) and ACN (0.1%TFA) as eluents. The desired fractions were lyophilized to yield the title compound **E5** (22mg, 0.03mmol, 23%) as a yellow powder. ¹H NMR (400MHz, d₆-DMSO, 300K) δ 2.27 (s, 3H), 2.85 (s, 3H), 3.12 (m, 4H), 3.51 (d, J = 11.3 Hz, 2H), 4.46 (d, J = 12.3 Hz, 2H), 5.76 (d, J = 10.3 Hz, 1H), 6.24 (d, J = 17.0 Hz, 1H), 6.57 (dd, J = 10.3 Hz, J = 17.0 Hz, 1H), 6.99 (m, 2H), 7.12 (dd, J = 4.7 Hz, J = 7.9 Hz, 1H), 7.24 (d, J = 7.9 Hz, 1H), 7.67 (s, 1H), 7.72 (dd, J = 2.4 Hz, J = 8.7 Hz, 1H), 7.89 (d, J = 7.9 Hz, 1H), 8.26 (d, J = 4.7 Hz, 1H), 8.33 (d, J = 2.4 Hz, 1H), 9.51 (s, 1H), 12.14 (s, 1H). MS (ES) C₂₇H₂₈N₆O requires: 452, found: 453 (M+H)⁺.

The Examples in the following table were prepared according to the procedure described for **E5** (Example 78).

| **Example** | **Name** | **Mwt** | **[M+H]⁺** |
|---|---|---|---|
| 79 | 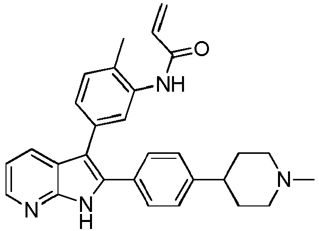 | 450 | 451 |
| 80 | 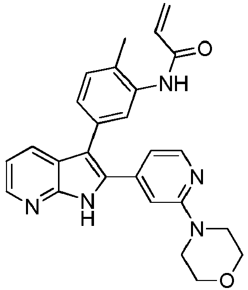 | 439 | 440 |
| 81 | 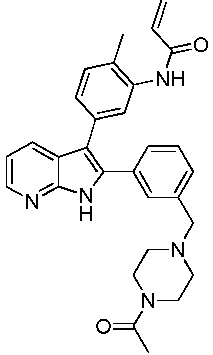 | 493 | 494 |
| 82 | 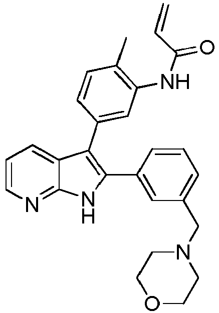 | 452 | 453 |

### Example 83:

### 1 -(6-(4-chloro-2-(4-(4-methylpiperazin-1-yl)phenyl)-1H-pyrolo[2,3-b]pyridin-3-yl)-2H-benzo[b][1,4]oxazin-4(3H)-yl)prop-2-en-1-one 2,2,2-trifluoroacetate (F4)

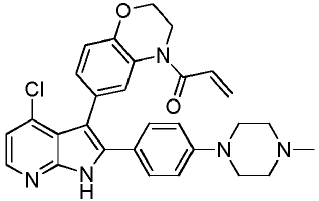

### Step 1: tert-butvl 6-(4-chloro-2-(trimethylsilyl)-1H-pyrrolor2,3-blpyridin-3-yl)-2H-benzo[b][1,4]oxazine-4(3H)-carboxylate (F1)

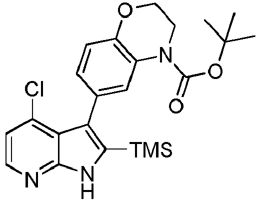

**F1** (632mg, 1.37mmol, 40%, beige solid) was prepared from 4-chloro-3-iodopyridine-2-amine (867mg, 3.41 mmol, 1.0 eq.) and tert-butyl 6-((trimethylsilyl)ethynyl)-2H-benzo[b][1,4]oxazine-4(3H)-carboxylate (1.131mg, 3.41mmol, 1.0eq.) following the general procedure reported in Preparative Example 1 Step 1. MS (ES) C₂₃H₂₈CIN₃O₃Si requires: 458, found: 459 (M+H)⁺.

### Step 2: tert-butvl 6-(4-chloro-2-iodo-1H-pyrrolo[2,3-b]pyridin-3-yl)-2H-benzo[b][1,4]oxazine-4(3H)-carboxylate (F2)

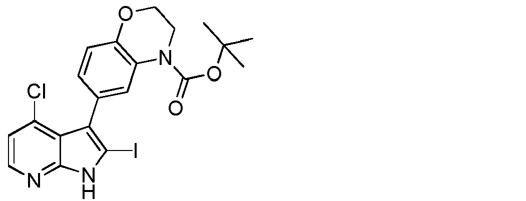

Tert-butyl 6-(4-chloro-2-(trimethylsilyl)-1H-pyrrolo[2,3-b]pyridin-3-yl)-2H-benzo[b][1,4]oxazine-4(3H)-carboxylate **F1** (300mg, 0.65mmol) and N-iodosuccinimide (264mg, 1.17mmol, 1.8eq.) were solved in dry dichloromethane (100mL) and stirred for 5h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **F2** (341mg, 0.65mmol, 100%) as a brown solid. The crude was used without purification in the next step. MS (ES) C₂₀H₁₉CIIN₃O₃ requires: 511, found: 512 (M+H)⁺.

### Step 3: 6-(4-chloro-2-(4-(4-methylpiperazin-1 -yl)phenyl)-1H-pyrrolo[2,3-b]pyridin-3-yl)-3,4-dihydro-2H-benzo[b][1,4]oxazine (F3)

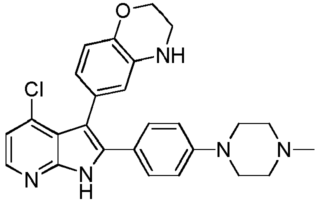

A mixture of tert-butyl 6-(4-chloro-2-iodo-1H-pyrrolo[2,3-b]pyridin-3-yl)-2H-benzo[b][1,4]oxazine-4(3H)-carboxylate **F2** (341mg, 0.65mmol, 1.0eq.), 4-(4-methylpiperazin-1-yl)phenylboronic acid (186mg, 0.85mmol, 1.3eq.), and K₃PO₄ (275mg, 1.30mmol, 2.0 eq) in dioxane/H₂O (10mL/1mL) was degassed with a stream of N₂ for 5min. [1,1'-Bis(diphenylphosphino)ferrocene]palladium dichloride dichloromethane adduct (53mg, 0.06mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude was solved in 30% TFA in DCM (60mL) and stirred for 2h at RT. Solvents were removed in vacuo. The crude product was purified by flash chromatography on silica gel (DCM/MeOH with 0.1% NEt₃ = 100:0 to 5:1) to yield the desired product **F3** (145mg, 0.31 mmol, 49%) as a white solid. MS (ES) C₂₆H₂₆ClN₅O requires: 459, found: 460 (M+H)⁺.

### Step 4: 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one 2,2,2-trifluoroacetate (F4)

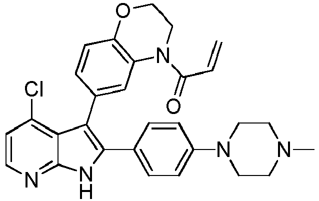

To a solution of **F3** (29mg, 0.063mmol, 1.0eq.) and DIPEA (110uL, 0.63mmol, 10.0eq.) in dry DCM (4mL) at 0⁰C was added slowly acrylolyl chloride (5.1mg, 0.057mmol, 0.9eq.) in dry DCM (5mL). The mixture was stirred for 5min. The solution was diluted with EtOAc and washed twice with aq. sat. NaHCO₃-solution. The organic phase was dried over MgSO₄ and solvent was removed in vacuo. The crude solution was purified by reverse phase RP-HPLC (column: C18), using H₂O (0.1%TFA) and ACN (0.1%TFA) as eluents. The desired fractions were lyophilized to yield the title compound **F4** (24mg, 0.03mmol, 51%) as a yellow powder. ¹H NMR (400MHz, d₄-MeOH, 300K) δ 2.96 (s, 3H), 3.06 (m, 2H), 3.25 (m, 4H), 3.59 (m, 2H), 3.92 (m, 2H), 4.34 (m, 2H), 5.38 (d, J = 10.4 Hz, 1H), 6.16 (dd, J = 2.0 Hz, J = 16.8 Hz, 1H), 6.36 ( m, 1H), 6.98 (d, J = 8.3 Hz, 1H), 7.02 (d, J = 8.9 Hz, 2H), 7.12 (d, J = 5.4 Hz, 1H), 7.22 (d, J = 8.3 Hz, 1H), 7.37 (d, J = 8.9 Hz, 2H), 8.10 (d, J = 5.4 Hz, 1H). MS (ES) C₂₃H₂₈CIN₃O₃Si requires: 513, found: 514 (M+H)⁺.

The Examples in the following table were prepared according to the procedure described for **F4** (Example 83).

| **Example** | **Name** | **Mwt** | **[M+H]⁺** |
|---|---|---|---|
| 84 | 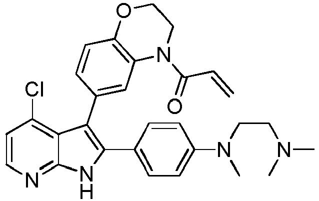 | 515 | 516 |
| 85 | 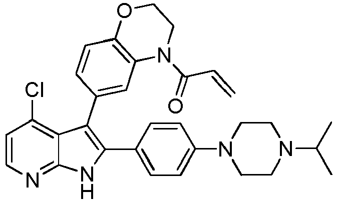 | 541 | 542 |

### Example 86:

### 1 -(7-(4-chloro-2-(4-(4-methylpiperazin-1 -yl)phenyl)-1H-pyrrolo[2,3-b]pyridin-3-yl)-3,4-dihydroquinolin-1(2H)-yl)prop-2-en-1-one 2,2,2-trifluoroacetate (G5)

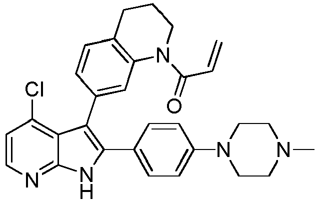

### Step 1: tert-butvl 7-(4-chloro-2-(trimethylsilyl)-1H-pyrrolor2,3-blpyridin-3-yl)-3,4-dihydroquinoline-1(2H)-carboxylate (G1)

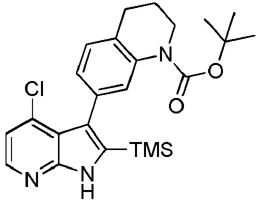

4-Chloro-3-iodopyridine-2-amine (1175mg, 4.6mmol, 1.0 eq.), tert-butyl 7-((trimethylsilyl)ethynyl)-3,4-dihydroquinoline-1(2H)-carboxylate (1522mg, 4.6mmol, 1.0 eq.), 1,4-diazabicyclo[2.2.2]octane (876mg, 7.8mmol, 1.7eq.) and dichlorobis(triphenylphosphine)palladium(ll) (351mg, 0.4mmol, 0.1eq.) in dry DMF (30mL) under N2 atmosphere was splitted in three microwave vials. Each vial was heated in the microwave at 145⁰C for 2 h. The solvent was removed in vacuo. The crude product was purified by flash chromatography on silica gel (cHex/EtOAc = 100:0 to 1:1) to yield the desired product **G1** (1010mg, 2.2mmol, 48%) as a beige solid. ¹H NMR (400MHz, d₆-DMSO, 300K) δ 0.09 (s, 9H), 1.39 (s, 9H), 1.87 (m, 2H), 2.79 (7, J = 6.5 Hz, 2H), 3.65 (m, 2H), 6.94 (dd, J = 1.7 Hz, J = 7.7 Hz, 1H), 7.06 (d, J = 5.1 Hz, 1H), 7.09 (d, J = 7.7 Hz, 1H), 7.61 (d, J = 1.7 Hz, 1H), 8.17 (d, J = 5.1 Hz, 1H), 11.82 (s, 1H). MS (ES) C₂₄H₃₀CIN₃O₂Si requires: 455, found: 456 (M+H)⁺.

### Step 2: 7-(4-chloro-2-(trimethylsilyl)-1H-pyrrolor[2,3-b]pyridin-3-yl)-1,2,3,4-tetrahydroquinoline (G2)

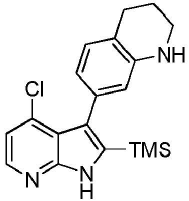

Tert-butyl 7-(4-chloro-2-(trimethylsilyl)-1H-pyrrolo[2,3-b]pyridin-3-yl)-3,4-dihydroquinoline-1(2H)-carboxylate **G1** (1.0g, 2.19mmol) in 50% TFA in DCM (20mL) at RT was stirred for 2h. Evaporation of the solvents yielded the desired product **G2** as brown solid, which was used in the next step without purification. MS (ES) C₁₉H₂₂ClN₃Si requires: 355, found: 356 (M+H)⁺.

### Step 3: 1-(7-(4-chloro-2-(trimethylsilyl)-1H-pyrrolo[2,3-b]pyridin-3-yl)-3,4-dihydroquinolin-1(2H)-yl)prop-2-en-1-one (G3)

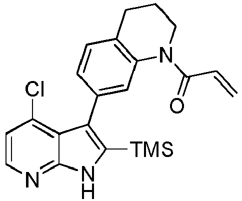

To a solution of **G2** (2.19mmol, 1.0eq.) and DIPEA (2.5mL, 14.5mmol, 5.0eq.) in dry DCM (40mL) at 0⁰C was added slowly acrylolyl chloride (235mg, 2.6mmol, 1.0eq.) in dry DCM (5mL). The mixture was stirred for 10min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product G3 (581mg, 1.42mmol, 65%) as a white solid. MS (ES) C₂₂H₂₄CIN₃OSi requires: 409, found: 410 (M+H)⁺.

### Step 4: 1-(7-(4-chloro-2-iodo-1H-pyrrolo[2,3-b]pyridin-3-yl)-3,4-dihydroquinolin-1(2H)-vl)prop-2-en-1-one (G4)

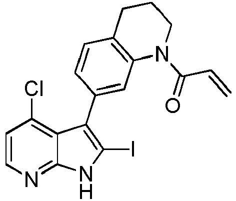

1 -(7-(4-chloro-2-(trimethylsilyl)-1H-pyrrolo[2,3-b]pyridin-3-yl)-3,4-dihydroquinolin-1 (2H)-yl)prop-2-en-1-one **G3** (371mg, 0.91mmol, 1.0eq.) and N-iodosuccinimide (366mg, 1.63mmol, 1.8eq.) were solved in dry dichloromethane (300mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **G4** as a yellow solid. The crude was used without purification in the next step. MS (ES) C₁₉H₁₅CIIN₃O requires: 462, found: 463 (M+H)⁺.

### Step 5: 1-(7-(4-chloro-2-(4-(4-methylpipeazin-1-yl)phenyl)-1H-pyrrolor2,3-b]pyridin-3-yl)-3,4-dihydroquinolin-1(2H)-yl)prop-2-en-1-one 2,2,2-trifluoroacetate (G5)

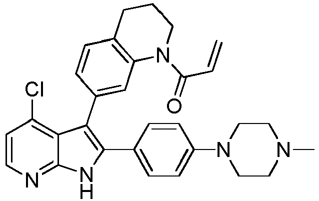

A mixture of 1-(7-(4-chloro-2-iodo-1H-pyrrolo[2,3-b]pyridin-3-yl)-3,4-dihydroquinolin-1(2H)-yl)prop-2-en-1-one **G4** (50mg, 0.10mmol, 1.0eq.), 4-(4-methylpiperazin-1-yl)phenylboronic acid (31mg, 0.14mmol, 1.5eq.), and K₃PO₄ (38mg, 0.18mmol, 2.0 eq) in dioxane/H₂O (3mL/0.6mL) was degassed with a stream of N₂ for 5min. [1,1'-Bis(diphenylphosphino)ferrocene]palladium dichloride dichloromethane adduct (8mg, 0.01 mmol, 0.1 eq) was added and the reaction mixture heated to 130°C for 2 h in the microwave oven. The crude reaction mixture was purified by reverse phase RP-HPLC (column: C18), using H₂O (0.1%TFA) and ACN (0.1%TFA) as eluents. The desired fractions were lyophilized to yield the title compound **G5** (22mg, 0.03mmol, 30%) as a yellow powder. ¹H NMR (400MHz, d₆-DMSO, 300K) δ 1.92 (q, J = 6.5 Hz, 2H), 2.52 (m, 2H), 2.79 (t, J = 6.5 Hz, 2H), 2.85 (s, 3H), 2.96 (t, J = 12.2 Hz, 2 H), 3.12 (m, 2H), 3.51 (d, J = 12.2 Hz, 2H), 3.75 (m, 2H), 5.22 (dd, J = 2.4 Hz, J = 10.2 Hz, 1H), 6.03 (dd, J = 2.4 Hz, J = 16.7 Hz, 1H), 6.25 (dd, J = 10.2 Hz, J = 16.7 Hz, 1H), 6.88 (s, 1H), 6.99 (d, J = 9.0 Hz, 2H), 7.12 (d, J = 5.2 Hz, 1H), 7.21 (dd, J = 1.6 Hz, J = 7.6 Hz, 1H), 7.28 (d, J = 7.6 Hz, 1H), 7.31 (d, J = 9.0 Hz, 2H), 8.15 (d, J = 5.2 Hz, 1H), 12.40 (s, 1H). MS (ES) C₃₀H₃₀CIN₅O requires: 511, found: 512 (M+H)⁺.

The Examples in the following table were prepared according to the procedure described for **G5** (Example 86).

| **Example** | **Name** | **Mwt** | **[M+H]⁺** |
|---|---|---|---|
| 87 | 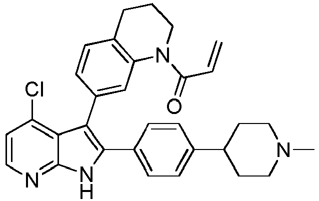 | 510 | 511 |
| 88 | 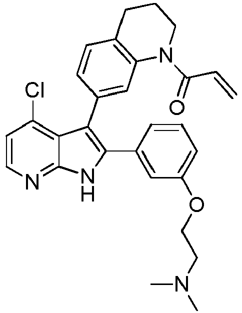 | 500 | 501 |
| 89 | 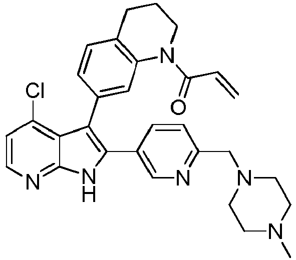 | 526 | 527 |
| 90 | 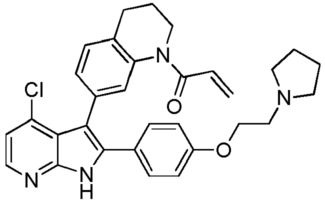 | 526 | 527 |
| 91 | 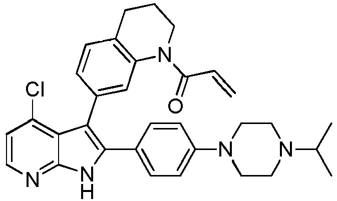 | 539 | 540 |
| 92 | 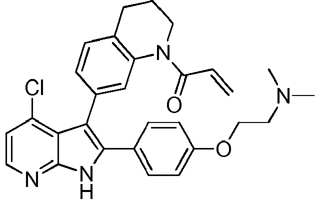 | 500 | 501 |
| 93 | 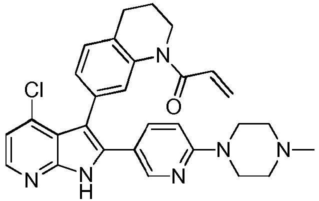 | 512 | 513 |
| 94 | 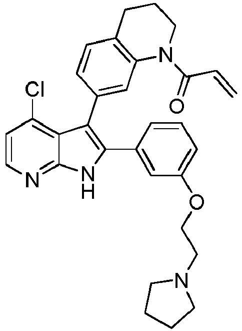 | 526 | 527 |
| 95 | 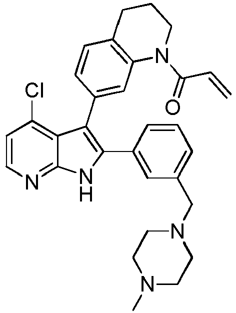 | 525 | 526 |

### Example 96:

### N-(5-(4-chloro-2-(4-(piperazin-1-yl)phenyl)-1H-pyrrolo2[2,3-b]pyridin-3-yl)-2-methylphenyl)acrylamide 2,2,2-trifluoroacetate (H2)

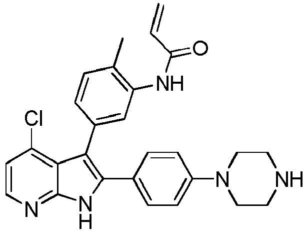

### Step 1: tert-butvl 4-(4-(3-(3-acrylamido-4-methylphenyl)-4-chloro-1H-pyrrolor2,3-b]pyridin-2-yl)phenyl)piperazine-1-carboxylate (H1)

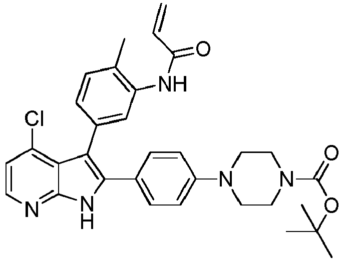

A mixture of N-(5-(4-chloro-2-iodo-1H-pyrrolo[2,3-b]pyridin-3-yl)-2-methylphenyl)acrylamide **A4** (100mg, 0.22mmol, 1.0eq.), 4-(4-bocpiperazino)phenylboronic acid (105mg, 0.34mmol, 1.5eq.), and K₃PO₄ (97mg, 0.44mmol, 2.0 eq) in dioxane/H₂O (3mL/0.6mL) was degassed with a stream of N₂ for 5min. [1,1'-bis(diphenylphosphino)ferrocene]palladium dichloride dichloromethane adduct (19mg, 0.02mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude product was purified by flash chromatography on silica gel (cHex/EtOAc = 100:0 to 0:100) to yield the desired product **H1** (41mg, 0.07mmol, 32%) as a beige solid. MS (ES) C₃₂H₃₄CIN₅O₃ requires: 571, found: 572 (M+H)⁺.

### Step 2: N-(5-(4-chloro-2-(4-(piperazin-1-yl)phenyl)-1H-pyrrolo[2,3-b]pyridin-3-yl)-2 methylphenyl)acrylamide 2,2,2-trifluoroacetate (H2)

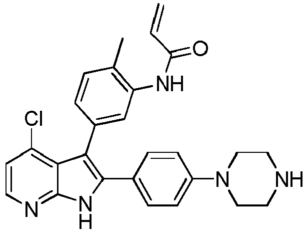

Tert-butyl 4-(4-(3-(3-acrylamido-4-methylphenyl)-4-chloro-1H-pyrrolo[2,3-b]pyridin-2-yl)phenyl)piperazine-1-carboxylate **H1** (41mg, 0.07mmol) in 30% TFA in DCM (5mL) at RT was stirred for 2h. After evaporation of the solvents, the crude was purified by reverse phase RP-HPLC (column: C18), using H₂O (0.1%TFA) and ACN (0.1%TFA) as eluents. The desired fractions were lyophilized to yield the title compound **H2** (18mg, 0.02mmol, 37%) as a yellow powder. ¹H NMR (400MHz, d₄-MeOH, 300K) δ 2.11 (s, 3H), 3.14 (m, 4H), 3.22 (m, 4H), 5.57 (dd, J = 1.3 Hz, J = 10.2 Hz, 1H), 6.13 (dd, J = 1.3 Hz, J = 16.9 Hz, 1H), 6.30 (dd, J = 10.2 Hz, J = 16.9 Hz, 1H), 6.75 (d, J = 8.8 Hz, 2H), 6.90 (m, 2H), 7.03 (d, J = 7.8 Hz, 1H), 7.23 (m, 3H), 7.89 (d, J = 5.4 Hz, 1H). MS (ES) C₂₇H₂₆CIN₅O requires: 471, found: 472 (M+H)⁺.

### Example 97:

### N-(5-(5-cyano-2-(4-(4-methylpiperazin-1-yl)phenyl)-1H-pyrrolo[2,3-b]pyridin-3-yl)-2-methylphenyl)acrylamide 2,2,2-trifluoroacetate (I5)

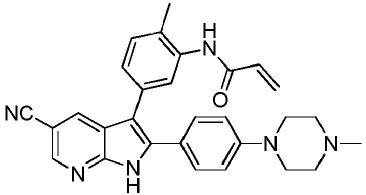

### Step 1: 3-(4-methyl-3-nitrophenyl)-2-(trimethylsilyl)-1H-pyrrolo[2,3-b]pyridine-5-carbonitrile (I1)

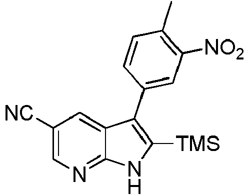

**I1** (2.6g, 7.43mmol, 40%, yellow solid) was prepared from 6-amino-5-bromonicotinonitrile (3.6g, 18.2mmol, 1.00eq.) and trimethyl((4-methyl-3-nitrophenyl)ethynyl)silane (4.5g, 19.0mmol, 1.05eq.) following the general procedure reported in Preparative Example 1 Step 1. MS (ES) C₁₈H₁₈N₄O₂Si requires: 350, found: 351 (M+H)⁺.

### Step 2: 3-(4-methyl-3-nitrophenyl)-2-(trimethylsilyl)-1H-pyrrolor2,3-blpyridine-5-carbonitrile (I2)

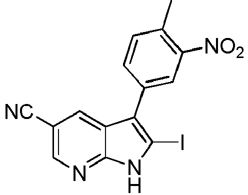

3-(4-Methyl-3-nitrophenyl)-2-(trimethylsilyl)-1H-pyrrolo[2,3-b]pyridine-5-carbonitrile **I1** (270mg, 0.8mmol, 1.0eq.) and N-iodosuccinimide (208mg, 0.96mmol, 1.2eq.) were solved in dry dichloromethane (40mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **I2** (123mg, 0.30mmol, 38%) as a brown solid. The crude was used without purification in the next step. MS (ES) C₁₅H₉IN₄O₂ requires: 403, found: 404 (M+H)⁺.

### Step 3: 3-(4-methyl-3-nitrophenyl)-2-(4-(4-methylpiperazin-1-yl)phenyl)-1H-pyrrolo[2,3-b]pyridine-5-carbonitrile (I3)

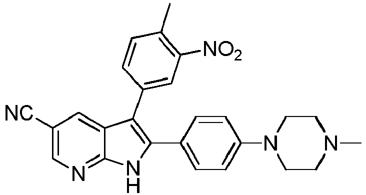

A mixture of 3-(4-methyl-3-nitrophenyl)-2-(trimethylsilyl)-1H-pyrrolo[2,3-b]pyridine-5-carbonitrile **I2** (1.41g, 3.5mmol, 1.0eq.), 4-(4-methylpiperazin-1-yl)phenylboronic acid (1.14mg, 5.2mmol, 1.5eq.), and K₃PO₄ (1.47g, 7.0mmol, 2.0 eq) in dioxane/H₂O (40mL/4mL) was degassed with a stream of N₂ for 5min. [1,1'-Bis(diphenylphosphino)ferrocene]palladium dichloride dichloromethane adduct (284mg, 0.35mmol, 0.1eq.) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude product was purified by flash chromatography on silica gel (DCM/MeOH with 0.1% NEt₃ = 100:0 to 1:1) to yield the desired product I3 (500mg, 1.10mmol, 32%) as a yellow solid. MS (ES) C₂₆H₂₄N₆O₂ requires: 452, found: 453 (M+H)⁺.

### Step 4: 3-(3-amino-4-methylphenyl)-2-(4-(4-methylpiperazin-1-yl)phenyl)-1H-pyrrolo[2,3-b]pyridine-5-carbonitrile (I4)

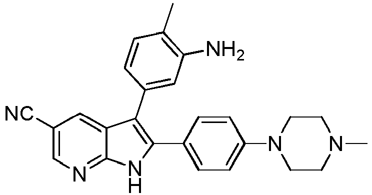

**I4** (93mg, 0.22mmol) was prepared from **I3** following the general procedure reported in Preparative Example 1 Step 2. MS (ES) C₁₈H₁₈N₄O₂Si requires: 350, found: 351 (M+H)⁺.

### Step 5: N-(5-(5-cyano-2-(4-(4-methylpiperazin-1-yl)phenyl)-1H-pyrrolor2,3-b]pyridin-3-yl)-2-methylphenyl)acrylamide 2,2,2-trifluoroacetate (I5)

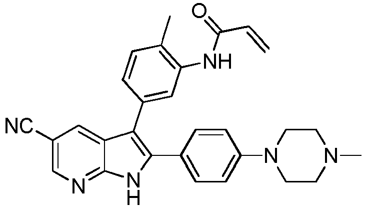

To a solution of **I4** (30mg, 0.071mmol, 1.0eq.) and DIPEA (92mg, 0.71mmol, 10.0eq.) in dry DCM (2mL) at 0⁰C was added slowly acrylolyl chloride (32mg, 0.355mmol, 5.0eq.) in dry DCM (1mL). The mixture was stirred for 5min. The crude solution was directly purified by reverse phase RP-HPLC (column: C18), using H₂O (0.1%TFA) and ACN (0.1%TFA) as eluents. The desired fractions were lyophilized to yield the title compound **I6** (3mg, 0.004mmol, 5%) as a yellow powder. ¹H NMR (400MHz, d₄-MeOH, 300K) δ 2.31 (s, 3H), 2.95 (s, 3H), 3.30 - 3.48 (m, 8H), 5.79 (dd, J = 1.6 Hz, J = 10.2 Hz, 1H), 6.36 (dd, J = 1.6 Hz, J = 17.0 Hz, 1H), 6.52 (dd, J = 10.2 Hz, J = 17.0 Hz, 1H), 7.02 (d, J = 9.0 Hz, 2H), 7.09 (d, J = 8.1 Hz, 1H), 7.27 (d, J = 7.8 Hz, 1H), 7.50 (m, 3H), 8.23 (d, J = 1.9 Hz, 1H), 8.51 (d, J = 1.9 Hz, 1H). MS (ES) C₂₉H₂₈N₆O requires: 476, found: 477 (M+H)⁺.

### Example 98:

### 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one 2,2,2-trifluoroacetate (J4)

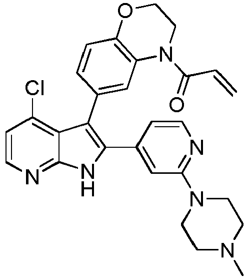

### Step 1: 6-(4-chloro-2-(trimethylsilyl)-1H-pyrrolo[2,3-b]pyridin-3-yl)-3,4-dihydro-2H benzo[b][1,4]oxazine (J1)

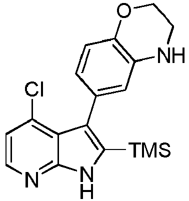

Tert-butyl 6-(4-chloro-2-(trimethylsilyl)-1H-pyrrolo[2,3-b]pyridin-3-yl)-2H-benzo[b][1,4]oxazine-4(3H)-carboxylate **F1** (3489mg, 7.62mmol) in 30% TFA in DCM (20mL) at RT was stirred for 2h. Evaporation of the solvents yielded a brown solid, which was purified by flash chromatography on silica gel (DCM/MeOH = 100:0 to 5:1) to yield the desired product **J1** (2726mg, 7.62mmol, 100%) as a yellow solid. MS (ES) C₁₈H₂₀ClN₃OSi requires: 357, found: 358 (M+H)⁺.

### Step 2: 1-(6-(4-chloro-2-(trimethylsilyl)-1H-pyrrolo[2,3-b]pyridin-3-yl)-2H-benzo[b][1.4]oxazin-4(3H)-yl)prop-2-en-1-one (J2)

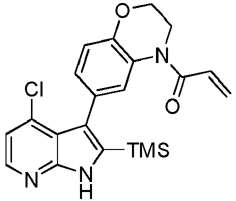

**J2** (635mg, 1.54mmol, 55%, white solid) was prepared from **J1** following the general procedure reported in Preparative Example 1 Step 3 Procedure A. MS (ES) C₂₁H₂₂ClN₃O₂Si requires: 411, found: 412 (M+H)⁺.

### Step 3: 1-(6-(4-chloro-2-iodo-1H-pyrrolo[2,3-b]pyridin-3-yl)-2H-benzo[b][1,4]oxazin-4(3H)-yl)prop-2-en-1-one (J3)

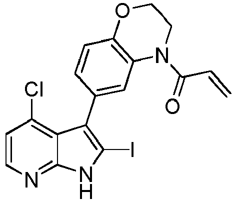

**J3** (465mg, 1.00mmol, 100%, yellow solid) was prepared from **J2** following the general procedure reported in Preparative Example 1 Step 4. MS (ES) C₁₈H₁₃CIN₃O₂ requires: 465, found: 466 (M+H)⁺.

### Step 4: 1-(6-(4-chloro-2-(2-(4-methylpiperazin-1-yl)pyridin-4-yl)-1H-pyrrolor2,3-b]pyridin-3-yl)-2H-benzo[b][1,4]oxazin-4(3H)-yl)prop-2-en-1-one 2,2,2-trifluoroacetate (J4)

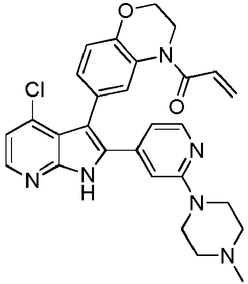

**J4** (7.4mg, 0.01 mmol, 9%, yellow solid) was prepared from **J3** and 2-(4-methylpiperazino)pyridine-4-boronic acid pinacol ester following the general procedure reported in Preparative Example 1 Step 5. ¹H NMR (400MHz, d₆-DMSO, 300K) δ 2.86 (s, 3H), 3.06 (m, 4H), 3.50 (d, J = 10.3 Hz, 2H), 4.27 - 4.40 (m, 6H), 5.52 (d, J = 10.6 Hz, 1H), 6.15 (dd, J = 1.9 Hz, J = 16.9 Hz, 1H), 6.59 (m, 1H), 6.66 (d, J = 5.3 Hz, 1H), 7.00 (d, J = 8.3 Hz, 1H), 7.03 (s, 1H), 7.12 (d, J = 7.6 Hz, 1H), 7.20 (d, J = 5.1 Hz, 1H), 8.10 (d, J = 5.3 Hz, 1H), 8.26 ( d, J = 5.1 Hz, 1H), 9.73 (s, 1H), 12.66 (s, 1H). MS (ES) C₂₈H₂₇CIN₆O₂ requires: 514, found: 515 (M+H)⁺.

The Examples in the following table were prepared according to the procedure described for **J4** (Example 98).

| **Example** | **Name** | **Mwt** | **[M+H]⁺** |
|---|---|---|---|
| 99 | 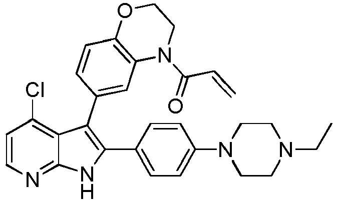 | 527 | 528 |
| 100 | 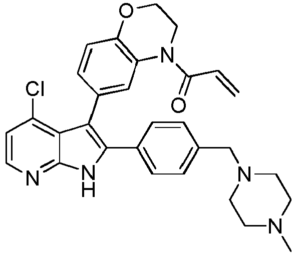 | 527 | 528 |
| 101 | 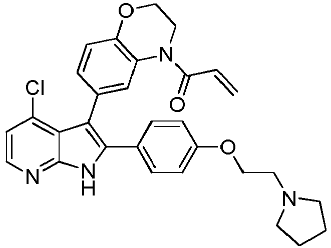 | 528 | 529 |
| 102 | 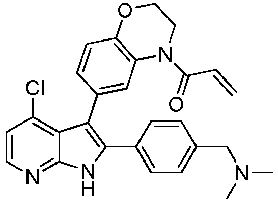 | 472 | 473 |
| 103 | 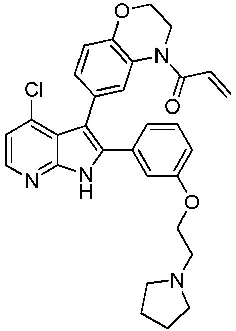 | 528 | 529 |

### Example 104:

### 1-(6-(4-ethoxy-2-(4-(4-methylpiperazin-1-yl)phenyl)-1H-pyrrolor2,3-blpyridin-3-yl)indolin-1-yl)prop-2-en-1-one 2,2,2-trifluoroacetate (K5)

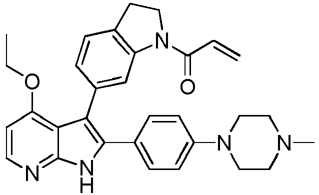

### Step 1: tert-butyl 6-(4-ethoxy-2-(trimethylsilyl)-1H-pyrrolor[2,3-b]pyridin-3-yl)indoline-1-carboxylate (K1)

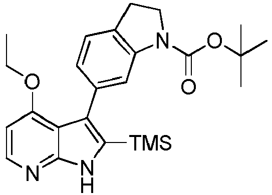

**K1** (3.74g, 8.29mmol, 73%, yellow solid) was prepared from 4-ethoxy-3-iodopyridin-2-amine and tert-butyl 6-((trimethylsilyl)ethynyl)indoline-1-carboxylate following the general procedure reported in Preparative Example 1 Step 1. MS (ES) C₂₅H₃₃N₃O₃Si requires: 451, found: 452 (M+H)⁺.

### Step 2: 4-ethoxy-3-(indolin-6-yl)-2-(trimethylsilyl)-1H-pyrrolo[2,3-b]pyridine (K2)

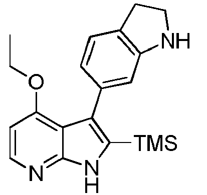

Tert-butyl 6-(4-ethoxy-2-(trimethylsilyl)-1H-pyrrolo[2,3-b]pyridin-3-yl)indoline-1-carboxylate **K1** (1.5g, 3.3mmol) in 30% TFA in DCM (60mL) at RT was stirred for 2h. Evaporation of the solvents yielded a brown solid. To the crude was added aq. sat. NaHCO₃-solution and the mixture were extracted three times with DCM. The organic phase was dried over MgSO₄ and solvent was removed in vacuo. The desired product **K2** which has an impurity of 4-ethoxy-3-(indolin-6-yl)-1H-pyrrolo[2,3-b]pyridine was used without further purification in the next step. MS (ES) C₂₀H₂₅N₃OSi requires: 351, found: 352 (M+H)⁺.

### Step 3: 1-(6-(4-ethoxy-2-(trimethylsilyl)-1H-pyrrolo[[2,3-b]pyridin-3-yl)indolin-1-yl)prop-2-en-1-one (K3)

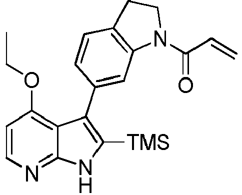

**K3** (371mg, 0.91mmol, 32%, white solid) was prepared from **K2** following the general procedure reported in Preparative Example 1 Step 3 Procedure A. MS (ES) C₂₃H₂N₃O₂Si requires: 405 found: 406 (M+H)⁺.

### Step 4: 1-(6-(4-ethoxy-2-iodo-1H-pyrrolo[[2,3-b]pyridin-3-yl)indolin--yl)prop-2-en-1-one (K4)

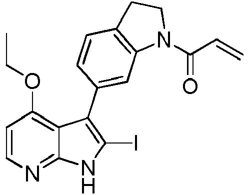

**K4** (380mg, 0.83mmol, yellow solid) was prepared from **K3** following the general procedure reported in Preparative Example 1 Step 4. MS (ES) C₂₀H₁₈IN₃O₂ requires: 459, found: 460 (M+H)⁺.

### Step 5: 1-(6-(4-ethoxy-2-(4-(4-methylpiperazin-1-yl)phenyl)-1H-pyrrolo[2,3-b]pyridin-3-yl)indolin-1-yl)prop-2-en-1-one 2,2,2-trifluoroacetate (K5)

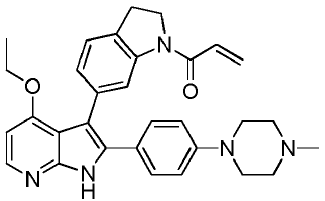

**K5** (14mg, 0.019mmol, 17%, yellow solid) was prepared from **K4** and 4-(4-methylpiperazin-1-yl)phenylboronic acid following the general procedure reported in Preparative Example 1 Step 5. ¹H NMR (400MHz, d₆-DMSO, 300K) δ 1.09 (t, J = 6.9 Hz, 3H), 2.85 (s, 3H), 2.97 (t, J = 12.6 Hz, 2H), 3.12 (m, 2H), 3.21 (t, J = 8.5 Hz, 2H), 3.51 (d, J = 11.9 Hz, 2H), 3.91 (d, J = 13.3 Hz, 2H), 4.11 (quart., J = 6.9 Hz, 2H), 4.26 (t, J = 8.5 Hz, 2H), 5.80 (d, J = 10.3 Hz, 1H), 6.25 (d, J = 16.5 Hz, 1H), 6.76 (m, 2H), 6.91 (d, J = 7.6 Hz, 1H), 6.96 (d, J = 8.5 Hz, 2H), 7.17 (d, J = 7.6 Hz, 1H), 7.34 (d, J = 8.5 Hz, 2H), 8.19 (d, J = 6.0 Hz, 1H), 8.25 (s, 1H), 9.79 (s, 1H), 12.39 (s, 1H). MS (ES) C₃₁H₃₃N₅O₂ requires: 507, found: 508 (M+H)⁺.

The Examples in the following table were prepared according to the procedure described for **K5** (Example 104).

| **Example** | **Name** | **Mwt** | **[M+H]⁺** |
|---|---|---|---|
| 105 | 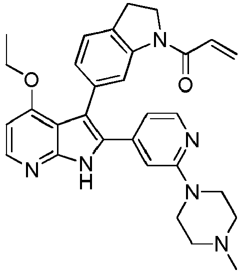 | 508 | 509 |
| 106 | 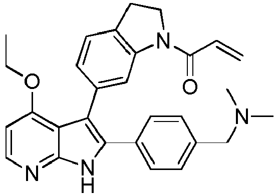 | 466 | 467 |
| 107 | 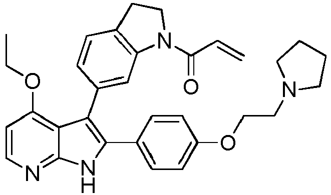 | 522 | 523 |
| 108 | 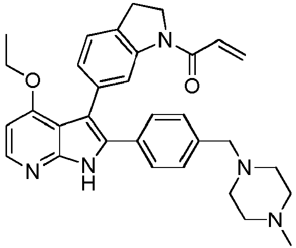 | 521 | 522 |
| 109 | 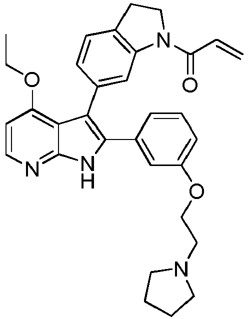 | 522 | 523 |

The Examples in the following table were prepared according to the procedure described for **K5** (Example 104) using 4-methoxy-3-iodopyridin-2-amine as a starting material in the step 1.

| **Example** | **Name** | **Mwt** | **[M+H]⁺** |
|---|---|---|---|
| 110 | 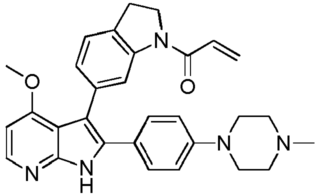 | 493 | 494 |
| 111 | 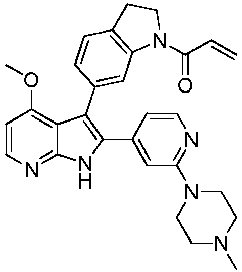 | 494 | 495 |
| 112 | 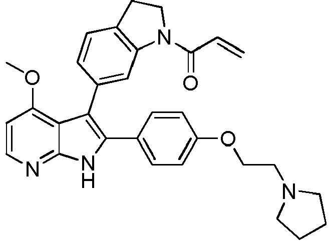 | 508 | 509 |
| 113 | 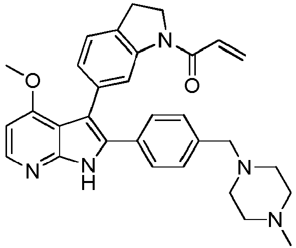 | 507 | 508 |
| 114 | 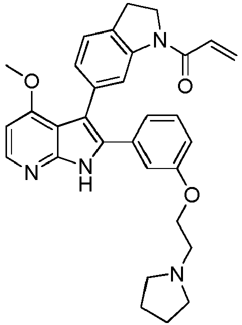 | 508 | 509 |

### Example 115:

### 1-(6-(4-isopropoxy-2-(4-(4-methylpiperazin-1-yl)phenyl)-1H-pyrrolo[2,3-b]pyridin-3-yl)indolin-1-yl)prop-2-en-1-one 2,2,2-trifluoroacetate (L1)

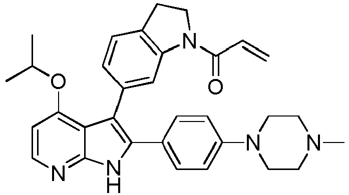

**L1** (15mg, 0.2mmol, yellow solid) was prepared according to the procedure described for **K5** (Example 104) using 3-iodo-4-isopropoxypyridin-2-amine as a starting material in the step 1. ¹H NMR (400MHz, d₆-DMSO, 300K) δ 1.13 (d, J = 6.0 Hz, 6H), 2.85 (s, 3H), 3.00 (m, 2H), 3.12 (m, 2H), 3.22 (t, J = 8.3 Hz, 2H), 3.50 (m, 2H), 3.92 (m, 2H), 4.27 (t, J = 8.3 Hz, 2H), 4.85 (quint., J = 6.0 Hz, 1H), 5.80 (d, J = 10.3 Hz, 1H), 6.25 (d, J = 16.6 Hz, 1H), 6.77 (d, J = 10.3 Hz, J = 16.6 Hz, 1H), 6.90 (d, J = 7.6 Hz, 1H), 6.94 (d, J = 6.5 Hz, 1H), 6.98 ( d, J = 8.5 Hz, 2H), 7.18 (d, J = 7.6 Hz, 1H), 7.35 (d, J = 8.5 Hz, 2H), 8.27 (m, 2H), 10.02 (s, 1H), 12.81 (s, 1H). MS (ES) C₃₂H₃₅N₅O₂ requires: 521, found: 522 (M+H)⁺.

### Example 116:

### 3-(3-acrylamido-4-methylphenyl)-2-(4-(4-methylpiperazin-1-yl)phenyl)-1H-pyrrolo[2,3-b]pyridine-5-carboxylic acid 2,2,2-trifluoroacetate (M3)

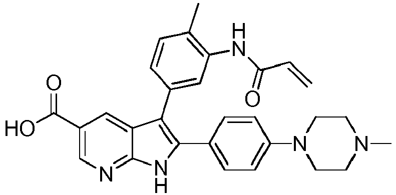

### Step 1: 3-(4-methyl-3-nitrophenyl)-2-(4-(4-methylpiperazin-1-yl)phenyl)-1H-pyrrolo[2,3-b]pyridine-5-carboxylic acid hydrochloric acid (M1)

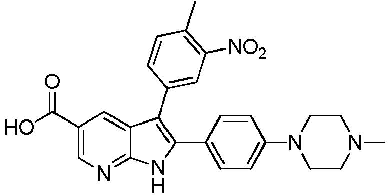

A mixture of 3-(4-methyl-3-nitrophenyl)-2-(4-(4-methylpiperazin-1-yl)phenyl)-1 H-pyrrolo[2,3-b]pyridine-5-carbonitrile I3 (3.0g, 6.6mmol) in conc. aq. HCl-solution (50mL) was heated for 3 h at 100⁰C. Solvent was removed in vacuo yielding the desired product **M1** as a brown solid (3.1g, 6.6mmol). MS (ES) C₂₆H₂₅N₅O₄ requires: 471, found: 472 (M+H)⁺.

### Step 2: 3-(3-amino-4-methylphenyl)-2-(4-(4-methylpiperazin-1 -yl)phenyl)-1H-pyrrolo[2,3-b]pyridine-5-carboxylic acid (M2)

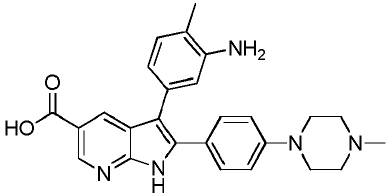

A mixture of 3-(4-methyl-3-nitrophenyl)-2-(4-(4-methylpiperazin-1-yl)phenyl)-1H-pyrrolo[2,3-b]pyridine-5-carboxylic acid **M1** (3.0g, 6.6mmol, 1.0eq.) and iron (1.8g, 31.8mmol, 5eq.) in EtOH (100mL) and sat. aq. NH₄CI-solution (20mL) was heated for 15h at 80⁰C. Solvents were removed in vacuo and the crude was purified by reverse phase RP-HPLC (column: C18), using H₂O (0.1%TFA) and ACN (0.1%TFA) as eluents. The desired fractions were lyophilized to yield the title compound **M2** (1.7g, 4.0mmol, 60%) as a yellow powder. MS (ES) C₂₆H₂₇N₅O₂ requires: 441, found: 442 (M+H)⁺.

### Step 3: 3-(3-acrylamido-4-methylphenyl)-2-(4-(4-methylpiperazin-1-yl)phenyl)-1H-pyrrolo[2,3-b]pyridine-5-carboxylic acid 2,2,2-trifluoroacetate (M3)

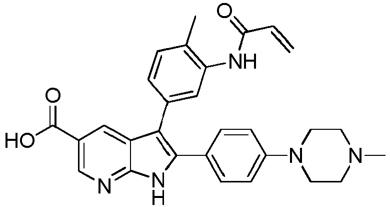

To a solution of 3-(3-amino-4-methylphenyl)-2-(4-(4-methylpiperazin-1-yl)phenyl)-1H-pyrrolo[2,3-b]pyridine-5-carboxylic acid **M2** (580mg, 1.31mmol, 1.0eq.) and DIPEA (2.3mL, 13.1mmol, 10eq.) in dry THF (10ml) at 0⁰C was added slowly acryloyl chloride (0.11mL, 1.31mmol, 1.0eq.) in dry THF (1mL). After 10min a drop of water was added and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **M3** (170mg, 0.34mmol, 26%) as a yellow powder. ¹H NMR (400MHz, d₆-DMSO, 300K) δ 2.27 (s, 3H), 2.85 (s, 3H), 3.01 (t, J = 12.5 Hz, 2H), 3.13 (m, 2H), 3.51 (d, J = 12.0 Hz, 2H), 3.93 (d, J = 13.3 Hz, 2H), 5.74 (dd, J = 10.1 Hz, J = 2.0 Hz, 1H), 6.22 (dd, J = 17.1 Hz, J = 2.0 Hz, 1H), 6.56 (dd, J = 17.1 Hz, J = 10.1 Hz, 1H), 7.03 (m, 3H), 7.27 (d, J = 7.9 Hz, 1H), 7.47 (d, J = 6.4 Hz, 2H), 7.60 (s, 1H), 8.26 (d, J = 2.0 Hz, 1H), 8.80 (d, J = 2.0 Hz, 1H), 9.55 (s, 1H), 9.72 (br s, 1H), 12.41 (s, 1H), 12.86 (br s, 1H). MS (ES) C₂₉H₂₉N₅O₃ requires: 495, found: 496 (M+H)⁺.

### Example 117:

### isopropyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (N3)

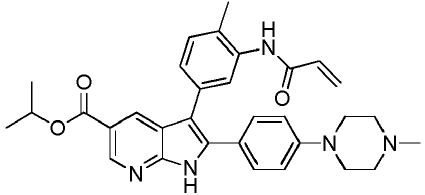

### Step 1: isopropyl 3-(4-methyl-3-nitrophenyl)-2-(4-(4-methylpiperazin-1-yl)phenyl)-1H-pyrrolo[2,3-b]pyridine-5-carboxylate (N1)

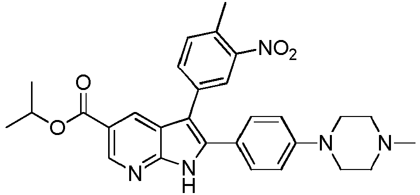

A mixture of 3-(4-methyl-3-nitrophenyl)-2-(4-(4-methylpiperazin-1-yl)phenyl)-1H-pyrrolo[2,3-b]pyridine-5-carbonitrile I3 (100mg, 0.2mmol) and conc. H₂SO₄ (1mL) in isopropyl alcohol (2mL) was heated for 48h at 80⁰C. After cooling to RT, EtOAc and sat. aq. NaHCO₃-solution was added. The aq. phase was extracted with EtOAc (3x) and the combined organic phase was dried over MgSO₄. Evaporation of solvents yielded the crude product of **N1** (28mg, 0.05mmol, 27%) as a yellow solid. The crude was used in the next step without further purification. MS (ES) C₂₉H₃₁N₅O₄ requires: 513, found: 514 (M+H)⁺.

### Step 2: isopropyl 3-(3-amino-4-methylphenyl)-2-(4-(4-methylpiperazin-1-yl)phenyl)-1H-pyrrolo[2,3-b]pyridine-5-carboxylate (N2)

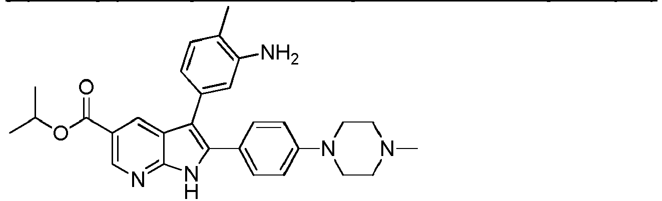

Isopropyl 3-(4-methyl-3-nitrophenyl)-2-(4-(4-methylpiperazin-1-yl)phenyl)-1H-pyrrolo[2,3-b]pyridine-5-carboxylate **N1** (28mg, 0.05mmol, 1.0eq.) and iron (15mg, 0.3mmol, 5eq.) in EtOH (5mL) and sat. aq. NH₄CI-solution (1mL) was heated for 3h at 80⁰C. Sat. aq. NaHCO₃-solution was added and the aq. phase was extracted with DCM (3x). The organic phase was dried over MgSO₄ and solvents were removed in vacuo. The crude product was purified by flash chromatography on silica gel (DCM/MeOH with 0.1% NEt₃ = 100:0 to 1:1) to yield the desired product **N2** (25mg, 0.05mmol, 94%) as a brown solid. MS (ES) C₂₉H₃₃N₅O₂ requires: 483, found: 484 (M+H)⁺.

### Step 3: isopropyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (N3)

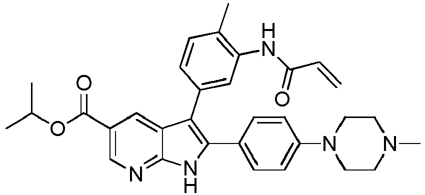

**N3** (2mg, 0.003mmol, 4%, yellow solid) was prepared from **N2** following the general procedure reported in Preparative Example 98 Step 3. The crude product was purified reverse phase RP-HPLC (column: C18), using H₂O (0.1%TFA) and ACN (0.1%TFA) as eluents. The desired fractions were lyophilized to yield the title compound **N3** as a yellow solid. ¹H NMR (400MHz, d₆-DMSO, 300K) δ 1.31 (d, J = 6.2 Hz, 6H), 2.26 (s, 3H), 2.85 (s, 3H), 2.98 (t, J = 13.0 Hz, 2H), 3.10 (m, 2H), 3.50 (d, J = 12.0 Hz, 2H), 3.92 (d, J = 13.0 Hz, 2H), 5.14 (quint., J = 6.2 Hz, 1H), 5.72 (dd, J = 10.2 Hz, J = 2.0 Hz, 1H), 6.21 (dd, J = 17.1 Hz, J = 2.0 Hz, 1H), 6.55 (dd, J = 10.2 Hz, J = 17.1 Hz, 1H), 6.99 (d, J = 8.6 Hz, 2H), 7.25 (d, J = 7.9 Hz, 1H), 7.45 (d, J = 8.6 Hz, 2H), 7.64 (s, 1H), 8.31 (d, J = 2.0 Hz, 1 H), 8.79 (d, J = 2.0 Hz, 1 H), 9.50 (s, 1 H), 9.58 (br. s, 1 H), 12.43 (s, 1H). MS (ES) C₃₂H₃₅N₅O₃ requires: 537 found: 538 (M+H)⁺.

### Example 118:

### 3-(3-acrylamido-4-methylphenyl)-N-benzyl-2-(4-(4-methylpiperazin-1-yl)phenyl)-1H-pyrrolor[2,3-b]pyridine-5-carboxamide 2,2,2-trifluoroacetate (O1)

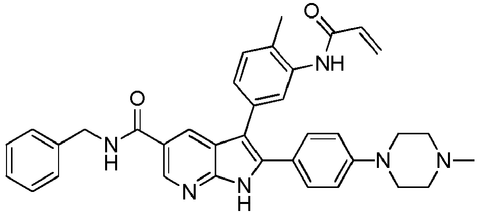

A solution of 3-(3-acrylamido-4-methylphenyl)-2-(4-(4-methylpiperazin-1-yl)phenyl)-1H-pyrrolo[2,3-b]pyridine-5-carboxylic acid **M3** (30mg, 0.06mmol, 1.0eq.), DIPEA (0.1mL, 0.60mmol, 10eq.) and HATU (46mg, 0.12mmol, 2.0eq.) in dry DMF (1mL) was stirred for 5min at 0⁰C. Then benzyl amine (13mg, 0.12mmol, 2.0eq.) was added and the mixture was stirred for 30min at 0⁰C. Sat. aq. NaHCO₃-solution was added and the mixture was extracted with EtOAc. The combined organic phase was dried over MgSO₄ and and solvents were removed in vacuo. The crude was purified by reverse phase RP-HPLC (column: C18), using H₂O (0.1%TFA) and ACN (0.1%TFA) as eluents. The desired fractions were lyophilized to yield the title compound O1 (10mg, 0.01mmol, 21%) as a yellow powder. ¹H NMR (400MHz, d₄-MeOH, 300K) δ 2.30 (s, 3H), 2.97 (s, 3H), 3.07 (t, J = 12.4 Hz, 2H), 3.26 (m, 2H), 3.61 (d, J = 12.4 Hz, 2H), 3.93 (d, J = 13.7 Hz, 2H), 4.59 (s, 2H), 5.77 (dd, J = 10.2 Hz, J = 1.7 Hz, 1H), 6.32 (dd, J = 17.0 Hz, J = 1.7 Hz, 1H), 6.50 (dd, J = 10.2 Hz, J = 17.0 Hz, 1H), 7.01 (d, J = 8.9 Hz, 2H), 7.12 (d, J = 8.1 Hz, 1H), 7.20 - 7.37 (m, 6H), 7.50 (d, J = 8.9 Hz, 2H), 7.54 (s, 1H), 8.48 ( d, J = 2.1 Hz, 1H), 8.75 (d, J = 2.1 Hz, 1H). MS (ES) C₃₆H₃₆N₆O₂ requires: 584 found: 585 (M+H)⁺.

The Examples in the following table were prepared according to the procedure described for O1 (Example 118).

| **Example** | **Name** | **Mwt** | **[M+H]⁺** |
|---|---|---|---|
| 119 | 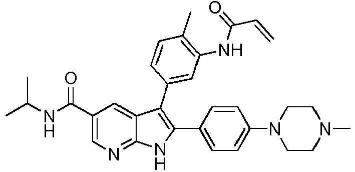 | 536 | 537 |
| 120 | 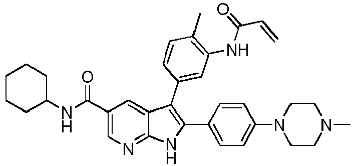 | 576 | 577 |
| 121 | 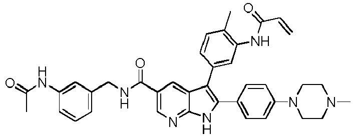 | 641 | 642 |
| 122 | 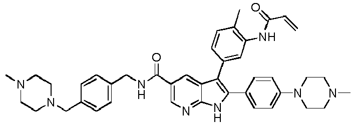 | 696 | 670 |
| 123 | 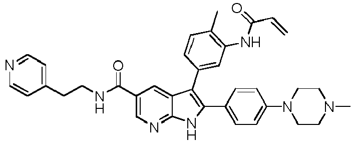 | 599 | 600 |
| 124 | 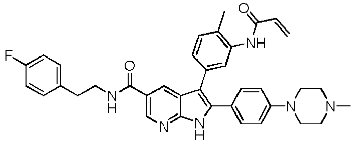 | 616 | 617 |
| 125 | 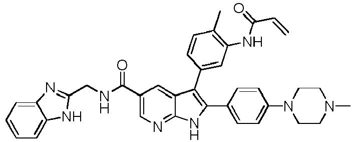 | 624 | 625 |
| 126 | 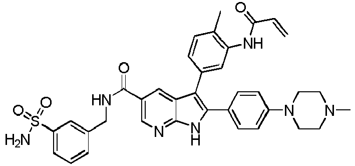 | 663 | 664 |
| 127 | 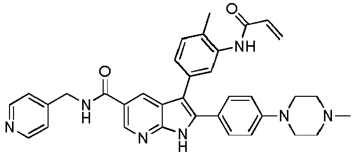 | 585 | 586 |
| 128 | 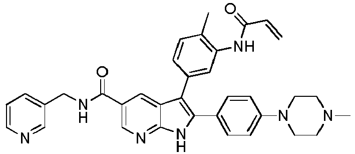 | 585 | 586 |
| 129 | 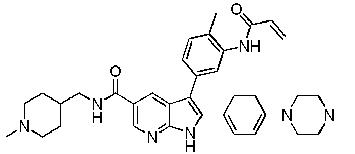 | 605 | 606 |
| 130 | 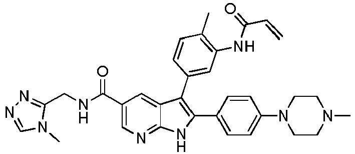 | 589 | 590 |
| 131 | 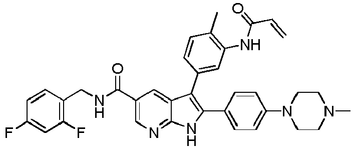 | 620 | 621 |
| 132 | 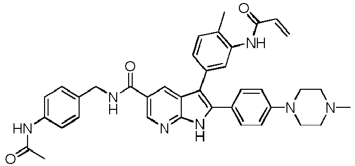 | 641 | 642 |
| 133 | 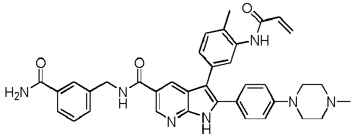 | 627 | 628 |

### Example 134:

### Methyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (P1)

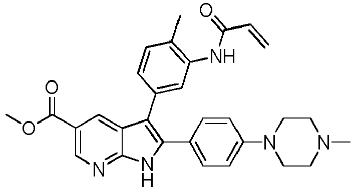

A solution of 3-(3-acrylamido-4-methylphenyl)-2-(4-(4-methylpiperazin-1-yl)phenyl)-1H-pyrrolo[2,3-b]pyridine-5-carboxylic acid **M3** (40mg, 0.08mmol, 1.0eq.), DMAP (5mg, 0.04mmol, 0.5eq.), *N*-(3-dimethylaminopropyl)-*N*'-ethylcarbodiimide hydrochloride (17mg, 0.09mmol, 1.1eq.) and dry methanol (7mg, 0.2mmol, 2.5eq.) in DCM (2mL) was stirred for 15h at RT. The crude solution was directly purified by reverse phase RP-HPLC (column: C18), using H₂O (0.1%TFA) and ACN (0.1%TFA) as eluents. The desired fractions were lyophilized to yield the title compound **P1** (20mg, 0.03mmol, 34%) as a yellow powder. ¹H NMR (400MHz, d₆-DMSO, 300K) δ 2.26 (s, 3H), 2.84 (s, 3H), 2.98 (t, J = 12.0 Hz, 2H), 3.11 (m, 2H), 3.50 (t, J = 12.0 Hz, 2H), 3.84 (s, 3H), 3.92 (d, J = 13.3 Hz, 2H), 5.72 (dd, J = 10.2 Hz, J = 2.0 Hz, 1H), 6.21 (dd, J = 17.0 Hz, J = 2.0 Hz, 1H), 6.54 (dd, J = 17.0 Hz, J = 10.2 Hz, 1H), 6.99 (d, J = 9.0 Hz, 2H), 7.02 (m, 1H), 7.26 (d, J = 7.9 Hz, 1H), 7.44 (d, J = 9.0 Hz, 2H), 7.58 (s, 1H), 8.26 (d, J = 2.1 Hz, 1H), 8.80 (d, J = 2.1 Hz, 1H), 9.52 (s, 1H), 9.72 (s, 1H), 12.45 (s, 1H). MS (ES) C₃₀H₃₁N₅O₃ requires: 509 found: 510 (M+H)⁺.

The Examples in the following table were prepared according to the procedure described for **P1** (Example 134).

| **Example** | **Name** | **Mwt** | **[M+H]⁺** |
|---|---|---|---|
| 135 | 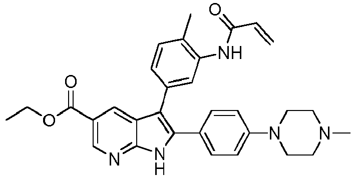 | 523 | 524 |
| 136 | 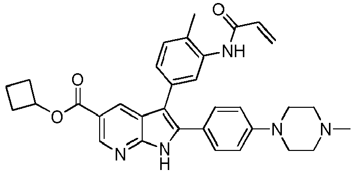 | 549 | 550 |
| 137 | 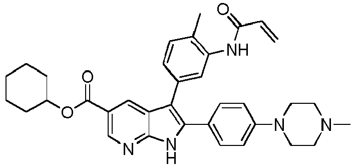 | 577 | 578 |
| 138 | 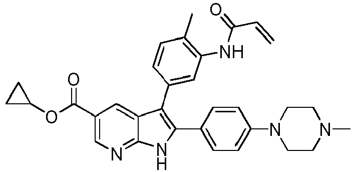 | 535 | 536 |
| 139 | 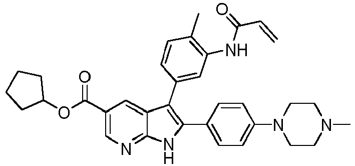 | 563 | 564 |
| 140 | 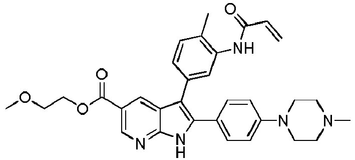 | 553 | 554 |
| 141 | 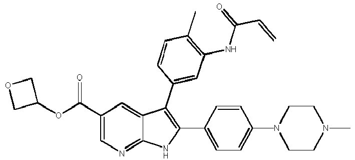 | 551 | 552 |

### Example 142:

### (E)-N-(5-(4-chloro-2-(4-(4-methylpiperazin-1-yl)phenyl)-1H-pyrrolo[2,3-b]pyridin-3-yl)-2-methylphenyl)but-2-enamide 2,2,2-trifluoroacetate (Q2)

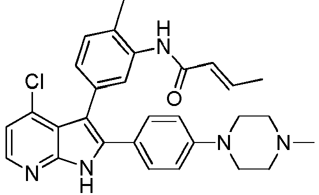

### Step 1: 5-(4-chloro-2-(4-(4-methylpiperazin-1-yl)phenyl)-1H-pyrrolor2,3-blpyridin-3-yl)-2-methylaniline (Q1)

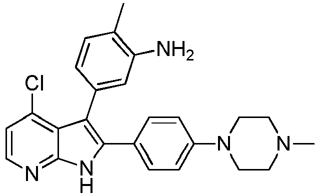

**Q1** was prepared from 4-chloro-3-(4-methyl-3-nitrophenyl)-2-(trimethylsilyl)-1H-pyrrolo[2,3-b]pyridine **A1** following the procedure reported in Preparative Example 97 Step 2 - 4. MS (ES) C₂₅H₂₆ClN₅ requires: 431 found: 432 (M+H)⁺.

### Step 2: (E)-N-(5-(4-chloro-2-(4-(4-methylpiperazin-1 -yl)phenyl)-1H-pyrrolo[2,3-b]pyridin-3-yl)-2-methylphenyl)but-2-enamide 222-trifluoroacetate (Q2)

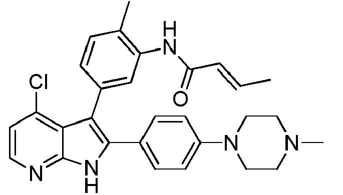

To a solution of **Q1** (20mg, 0.05mmol, 1.0eq.) and DIPEA (60mg, 0.46mmol, 10.0eq.) in dry THF (2mL) at 0⁰C was added slowly crotonoyl chloride (5mg, 0.05mmol, 1.1eq.) in dry THF (0.5mL). The mixture was stirred for 10min. The solution was diluted with EtOAc and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **Q2** (13mg, 0.02mmol, 36%) as a yellow powder. ¹H NMR (400MHz, d₆-DMSO, 300K) δ 1.82 (dd, J = 7.0 Hz, J = 1.7 Hz, 3H), 2.23 (s, 3H), 2.82 (s, 3H), 2.95 (t, J = 12.4 Hz, 2H), 3.07 (m, 2H), 3.43 (m, 2H), 3.90 (d, J = 13.3 Hz, 2H), 6.21 (d, J = 15.2 Hz, 1H), 6.70 (dq, J = 7.0 Hz, J = 15.2 Hz, 1H), 6.92 (d, J = 9.0 Hz, 2H), 7.00 (dd, J = 7.5 Hz, J = 1.8 Hz, 1H), 7.06 (d, J = 5.2 Hz, 1H, 7.18 (d, J = 7.9 Hz, 1H), 7.35 (d, J = 9.0 Hz, 2H), 7.52 (s, 1H), 8.12 (d, J = 5.2 Hz, 1H), 9.21 (s, 1H, 9.69 (s, 1H), 12.30 (s, 1H). MS (ES) C₂₉H₃₀CIN₅O requires: 500, found: 501 (M+H)⁺.

### Example 143:

### (E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide 2,2,2-trifluoroacetate (R1)

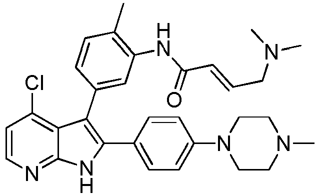

To a solution of trans-4-dimethylaminocrotonic acid hydrochlorid (46mg, 0.28mmol, 3eq.) and a drop of dry DMF in dry DCM (1mL) at 0⁰C was added slowly oxalyl chloride (20uL, 0.23mmol, 2.5eq.) in dry DCM (0.2mL). After 1h this mixture was added to a solution of **Q1** (40mg, 0.09mmol, 1.0eq.) in dry DCM (1mL) and dry NMP (1mL). The mixture was stirred for 10min. The solution was diluted with DCM and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **R1** (26mg, 0.03mmol, 33%) as a yellow powder. ¹H NMR (400MHz, d₆-DMSO, 300K) δ 2.28 (s, 3H), 2.80 (s, 6H), 2.84 (s, 3H), 2.97 (t, J = 12.6 Hz, 2H), 3.09 (m, 2H), 3.48 (m, 2H), 3.93 (m, 2H), 6.57(d, J = 15.4 Hz, 1H), 6.68 (dt, J = 7.0 Hz, J = 15.4 Hz, 1H), 6.94 (d, J = 9.0 Hz, 2H), 7.05 ( d, J = 7.7 Hz, 1H), 7.08 (d, J = 5.2 Hz, 1H), 7.23 ( d, J = 7.7 Hz, 1H), 7.36 (d, J = 9.0 Hz, 2H), 7.56 (s, 1H), 8.14 (d, J = 5.2 Hz, 1H), 9.65 (s, 1H), 9.74 (s, 1H), 12.34 (s, 1H). MS (ES) C₃₁H₃₅CIN₆O requires: 543, found: 544 (M+H)⁺.

### Example 144:

### N-(5-(4-chloro-2-(4-(4-methylpiperazin-1-yl)phenyl)-1H-pyrrolo[2,3-b]pyridin-3-yl)-2-methylphenyl)propiolamide 2,2,2-trifluoroacetate (S1)

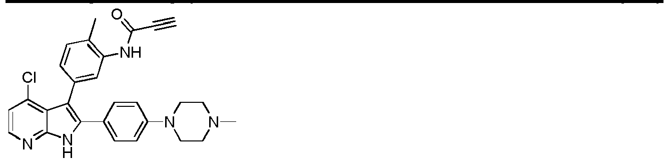

To a solution of **Q1** (70mg, 0.16mmol, 1.0eq.) and DIPEA (0.28mL, 1.62mmol, 10.0eq.) in dry THF (2mL) at 0⁰C was added slowly 3-(trimethylsilyl)propioloyl chloride (29mg, 0.18mmol, 1.1eq.) in dry THF (0.5mL). The mixture was stirred for 10min. Then K₂CO₃ (223mg, 1.62mmol, 10eq.) was added and the solution was stirred for 30min at at 0⁰C. The mixture was directly purified by reverse phase RP-HPLC (column: C18), using H₂O (0.1%TFA) and ACN (0.1%TFA) as eluents. The desired fractions were lyophilized to yield the title compound **S1** (30mg, 0.04mmol, 26%) as a yellow powder. ¹H NMR (400MHz, d₆-DMSO, 300K) δ 2.25 (s, 3H), 2.85 (s, 3H), 2.98 (t, J = 12.4 Hz, 2H), 3.10 (m, 2H), 3.48 (d, J = 12.0 Hz, 2H), 3.92 (d, J = 13.3 Hz, 2H), 4.32 (s, 1H), 6.93 (d, J = 8.8 Hz, 2H), 7.08 (m, 2H), 7.23 (d, J = 7.8 Hz, 1H), 7.31 (s, 1H), 7.35 (d, J = 8.8 Hz, 2H), 8.14 (d, J = 5.2 Hz, 1H), 9.78 (s, 1H), 10.23 (s, 1H), 12.35 (s, 1H). MS (ES) C₂₈H₂₆CIN₅O requires: 483, found: 484 (M+H)⁺.

### Example 145:

### N-(5-(4-chloro-2-(4-(4-methylpiperazin-1-yl)phenyl)-1H-pyrrolo[2,3-b]pyridin-3-yl)-2-methylphenyl)ethenesulfonamide 2,2,2-trifluoroacetate (T1)

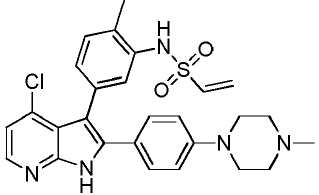

To a solution of **Q1** (25mg, 0.06mmol, 1.0eq.) and triethylamine (0.10mL, 0.58mmol, 10.0eq.) in dry THF (2mL) at -10⁰C was added slowly 2-chloroethanesulfonyl chloride (10mg, 0.06mmol, 1.1eq.) in dry THF (0.5mL). After 2h stirring at -10⁰C, the mixture was directly purified by reverse phase RP-HPLC (column: C18), using H₂O (0.1%TFA) and ACN (0.1%TFA) as eluents. The desired fractions were lyophilized to yield the title compound **T1** (18mg, 0.02mmol, 40%) as a yellow powder. ¹H NMR (400MHz, d₆-DMSO, 300K) δ 2.32 (s, 3H), 2.82 (s, 3H), 2.95 (m, 2H), 3.08 (m, 2H), 3.42 (m, 2H), 3.90 (m, 2H), 5.79 (d, J = 9.8 Hz, 1H), 5.80 (d, J = 16.4 Hz, 1H), 6.64 (dd, J = 9.8 Hz, J = 16.4 Hz, 1H), 6.91 (d, J = 9.0 Hz, 2H), 7.07 (d, J = 5.2 Hz, 1H), 7.08 (d, J = 1.8 Hz, 1H), 7.13 (d, J = 1.8 Hz, 1H), 7.21 (d, J = 7.7 Hz, 1H), 7.27 (d, J = 9.0 Hz, 2H), 8.12 (d, J = 5.2 Hz, 1H), 9.22 (s,1H), 9.85 (s, 1H), 12.33 (s, 1H). MS (ES) C₂₇H₂₈CIN₅O₂S requires: 522, found: 523 (M+H)⁺.

### Example146:

### 2-chloro-N-(5-(4-chloro-2-(4-(4-methylpiperazin-1-yl)phenyl)-1H-pyrrolo[2,3-b]pyridin-3-yl)-2-methylphenyl)acetamide 2,2,2-trifluoroacetate (U1)

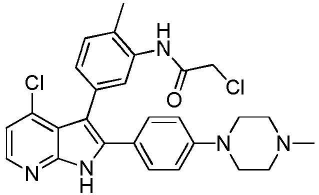

To a solution of **Q1** (30mg, 0.07mmol, 1.0eq.) and DIPEA (0.12mL, 0.69mmol, 10.0eq.) in dry THF (2mL) at 0⁰C was added slowly chloroacetyl chloride (39mg, 0.35mmol, 5.0eq.) in dry THF (0.5mL). The mixture was stirred for 10min. Then sat. aq. NaHCO₃-solution was added and the aq. phase was extracted with DCM. The combined organic phase was dried over MgSO₄ and solvents were removed in vacuo. The crude product was purified by reverse phase RP-HPLC (column: C18), using H₂O (0.1%TFA) and ACN (0.1%TFA) as eluents. The desired fractions were lyophilized to yield the title compound **U1** (13mg, 0.02mmol, 25%) as a yellow powder. ¹H NMR (400MHz, d₆-DMSO, 300K) δ 2.25 (s, 3H), 2.81 (s, 3H), 2.96 (t, J = 12.6 Hz, 2H), 3.08 (m, 2H), 3.46 (d, J = 12.0 Hz, 2H), 3.90 (d, J = 13.2 Hz, 2H), 4.26 (s, 2H), 6.91 (d, J = 8.9 Hz, 2H), 7.04 (d, J = 7.6 Hz, 1H), 7.07 (d, J = 5.2 Hz, 1H), 7.20 (d, J = 7.6 Hz, 1H), 7.34 (d, J = 8.9 Hz, 2H), 7.43 (s, 1H), 8.12 (d, J = 5.2 Hz, 1H), 9.62 (s, 1H), 9.73 (s, 1H), 12.32 (s, 1H). MS (ES) C₂₇H₂₇Cl₂N₅O requires: 507, found: 508 (M+H)⁺.

### Example 147:

### N-(5-(4-ethoxy-2-(4-(4-methylpiperazin-1-yl)phenyl)-1H-pyrrolo[2,3-b]pyridin-3-yl) 2-methylphenyl)acrylamide 2,2,2-trifluoroacetate (V1)

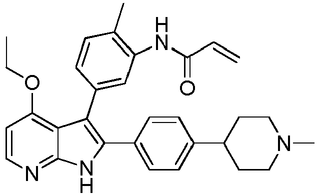

**V1** was prepared from 3-iodo-4-ethoxypyridin-2-amine, trimethyl((4-methyl-3-nitrophenyl)ethynyl)silane and 4-(1-methyl-4-piperidyl)phenylboronic acid pinacol Ester following the general procedure reported in Preparative Example 72 Step 1-5. MS (ES) C₃₁H₃₄N₄O₂ requires: 494, found: 495 (M+H)⁺.

### Example 148:

### (E)-isopropyl 3-(3-(4-(dimethylamino)but-2-enamido)-4-methylphenyl)-2-(4-(4-methylpiperazin-1-yl)phenyl)-1H-pyrrolo[2,3-b]pyridine-5-carboxylate 2,2,2-trifluoroacetate (W1)

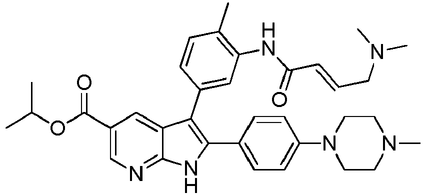

To a solution of trans-4-dimethylaminocrotonic acid hydrochlorid (30mg, 0.18mmol, 3.5eq.) and a drop of dry DMF in dry THF (1mL) at 0⁰C was added slowly oxalyl chloride (14uL, 0.16mmol, 3.0eq.) in dry THF (0.2mL). After 90min the mixture was added to a solution of **N2** (25mg, 0.05mmol, 1.0eq.) in dry NMP (1mL). The mixture was stirred for 10min. The solution was diluted with DCM and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **W1** (13mg, 0.01 mmol, 28%) as a yellow powder. ¹H NMR (400MHz, d₆-DMSO, 300K) δ 1.34 (d, J = 6.3 Hz, 6H), 2.29 (s, 3H), 2.81 (s, 6H), 2.86 (s, 3H), 3.04 (m, 2H), 3.13 (m, 2H), 3.43 (m, 2H), 3.90 (m, 2H), 3.94 (d, J = 7.0 Hz, 2H), 5.16 (sept., J = 6.3 Hz, 1H), 6.62 (d, J = 15.4 Hz, 1H), 6.73 (dt, J = 15.4 Hz, J = 7.0 Hz, 1H), 7.02 (m, 3H), 7.27 (d, J = 7.9 Hz, 1H), 7.47 (d, J = 8.9 Hz, 2H), 7.67 (s, 1H), 8.33 (d, J = 2.0 Hz, 1H), 8.82 (d, J = 2.0 Hz, 1H), 9.75 (s, 1H), 10.02 (s, 1H), 12.47 (s, 1H). MS (ES) C₃₅H₄₂N₆O₃ requires: 594, found: 595 (M+H)⁺.

### Example 149:

### isopropyl 3-(3-acrylamido-4-methylphenyl)-2-(4-(1-methylpiperidin-4-yl)phenyl)-1H-pyrrolo[2,3-b]pyridine-5-carboxylate 2,2,2-trifluoroacetate (AA1)

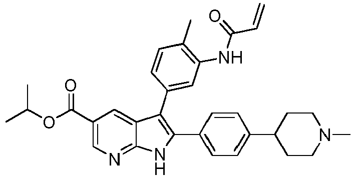

**AA1** was prepared following the procedure reported in Preparative Example 97 and 117. The crude product was purified by reverse phase RP-HPLC (column: C18), using H₂O (0.1%TFA) and ACN (0.1%TFA) as eluents. The desired fractions were lyophilized to yield the title compound **AA1** as a yellow solid. ¹H-NMR (400MHz, d₆-DMSO, 300K) δ 1.34 (d, J = 6.2 Hz, 6H), 1.82 (q, J = 13.2 Hz, 2H), 2.05 (d, J = 13.2 Hz, 2H), 2.28 (s, 3H), 2.82 (m, 4H), 3.08 (q, J = 12.0 Hz, 2H), 5.15 (septet, J = 6.2 Hz, 1H), 5.75 (dd, J = 2.0 Hz, J = 10.2 Hz, 1H), 6.21 (dd, J = 2.0 Hz, J = 17.0 Hz, 1H), 6.58 (dd, J = 10.2 Hz, J = 17.0 Hz, 1H), 7.00 (d, J = 7.8 Hz, 1H), 7.27 (m, 3H), 7.54 (d, J = 8.1 Hz, 2H), 7.66 (s, 1H), 8.40 (d, J = 1.9 Hz, 1H), 8.85 (d, J = 1.9 Hz, 1H), 9.41 (br s, 1H), 9.53 (s, 1H), 12.56 (s, 1H). MS (ES) C₃₃H₃₆N₄O₃ requires: 536 found: 537 (M+H)⁺.

The Example in the following table was prepared according to the procedure described for **AA1** (Example 149).

| **Example** | **Name** | **Mwt** | **[M+H]⁺** |
|---|---|---|---|
| 151 | 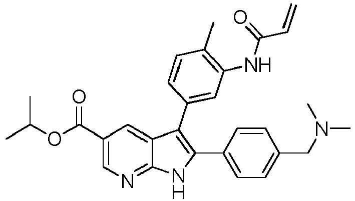 | 496 | 497 |

### Example 150:

### N-(5-(4-chloro-2-(4-(4-methylpiperazin-1-yl)phenyl)-1H-pyrrolo[2,3-b]pyridin-3-yl)-2,3-dimethylphenyl)acrylamide 2,2,2-trifluoroacetate (AB1)

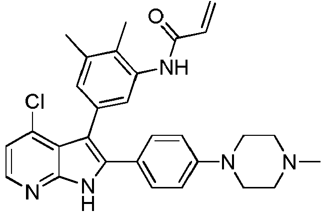

**AB1** was prepared was prepared from 4-chloro-3-iodopyridine-2-amine and ((3,4-dimethyl-5-nitrophenyl)ethynyl)trimethylsilane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AB1** as a yellow solid. ¹H-NMR (400MHz, d₆-DMSO, 300K) δ 2.14 (s, 3H), 2.26 (s, 3H), 2.84 (s, 3H), 2.87 (m, 2H), 3.01 (m,2H), 3.12 (m, 2H), 3.91 (m, 2H), 5.70 (dd, J = 2.0 Hz, J = 10.1, 1H), 6.18 (dd, J = 2.0 Hz, J = 17.2Hz, 1H), 6.52 (dd, J = 10.1 Hz, J = 17.2Hz, 1H), 6.93 (d, J = 8.7 Hz, 2H), 7.0 (s, 1H), 7.05 (d, J = 5.2 Hz, 1H), 7.26 (s, 1H), 7.41 (d, J = 8.7 Hz, 2H), 8.12 (d, J = 5.2 Hz, 1H), 9.54 (s, 1H), 9.81 (br s, 1H), 12.28 (s, 1H). MS (ES) C₂₉H₃₀ClN₅O requires: 500 found: 501 (M+H)⁺.

The Example in the following table was prepared according to the procedure described for **AB1** (Example 150).

| **Example** | **Name** | **Mwt** | **[M+H^{t}** |
|---|---|---|---|
| 152 | 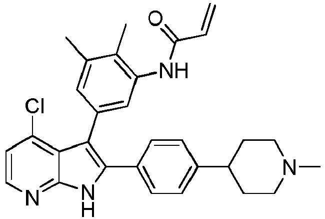 | 499 | 500 |

### Example 153:

### 3-(3-acrylamido-4-methy)pheny))-2-(4-((2-(dimethylamino)ethyl)(methyl)amino)phenly)-1H-pyrrolo[2,3-b]pyridine-5-carboxylic acid 2,2,2-trifluoroacetate (AC1)

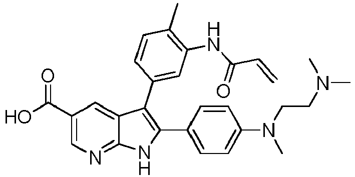

**AC1** was prepared was prepared following the general procedure reported in Preparative Example 97 and 116. The crude product was purified by reverse phase RP-HPLC (column: C18), using H₂O (0.1%TFA) and ACN (0.1%TFA) as eluents. The desired fractions were lyophilized to yield the title compound **AC1** as a yellow solid. ¹H-NMR (400MHz, d₆-DMSO, 300K) δ 2.27 (s, 3H), 2.83 (s, 3H), 2.84 (s, 3H), 2.94 (s, 3H), 3.23 (m,2H), 3.66 (m, 2H), 5.74 (dd, J = 2.1 Hz, J = 10.2 Hz, 1H), 6.22 (d, J = 2.1 Hz, J = 17.1Hz, 1H), 6.55 (dd, J = 10.2 Hz, 17.1 Hz, 1H), 6.79 (d, J = 9.1 Hz, 2H), 7.04 (d, J = 7.9 Hz, 1H), 7.28 (d, J = 7.9 Hz, 1H), 7.44 (d, J = 9.1 Hz, 2H), 7.58 (s, 1H), 8.23 (d, J = 2.0 Hz, 1H), 8.79 (d, J = 2.0 Hz, 1H), 9.34 (br s, 1H), 9.56 (s, 1H), 12.34 (s, 1H), 12.81 (br s, 1H). MS (ES) C₂₉H₃₁N₅O₃ requires: 497 found: 498 (M+H)⁺.

### Example 154:

### isopropyl 3-(3-acrylamido-4-methylphenyl)-2-(4-((2-(dimethylamino)ethyl)(methyl)amino)phenyl)-1H-pyrrolo[2,3-b]pyridine-5-carboxylate2,2,2-trifluoroacetate (AD1)

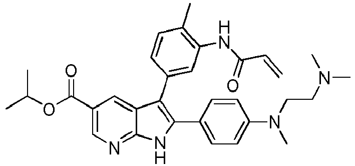

**AD1** (8mg, 0.01mmol, 26%) was prepared from **AC1** and isopropanol following the general procedure reported in Preparative Example 98 Step 3. The crude product was purified reverse phase RP-HPLC (column: C18), using H₂O (0.1%TFA) and ACN (0.1%TFA) as eluents. The desired fractions were lyophilized to yield the title compound **AD1** as a yellow solid. ¹H NMR (400MHz, d₆-DMSO, 300K) δ 1.33 (d, J = 6.3 Hz, 6H), 2.28 (s, 3H), 2.80 (s, 6H), 2.95 (s, 3H), 3.19 (m, 2H), 3.68 (t, J = 7.4 Hz, 2H), 5.15 (septet, J = 6.3 Hz, 1H), 5.75 (dd, J = 1.9 Hz, J = 10.0 Hz, 1H), 6.23 (dd, J = 1.9 Hz, J = 17.0 Hz, 1H), 6.57 (dd, J = 10.0Hz, J = 17.0 Hz, 1H), 6.79 (d, J = 8.8 Hz, 2H), 7.03 (d, J = 7.8 Hz, 1H), 7.27 (d, J = 7.8 Hz, 1H), 7.44 (d, J = 8.8 Hz, 2H), 7.63 (s, 1H), 8.29 (d, J = 2.0 Hz, 1H), 8.79 (d, J = 2.0 Hz, 1H), 9.55 (s, 1H), 9.75 (br s, 1H), 12.39 (s, 1H). (ES) C₃₂H₃₇N₅O₃ requires: 539, found: 540 (M+H)⁺.

### Example 155:

### N-(5-(5-chloro-4-methyl-2-(4-(4-methylpiperazin-1-yl)phenyl)-1H-pyrrolo[2,3-b]pyridin-3-yl)-2-methylphenyl)acrylamide 2,2,2-trifluoroacetate (AE1)

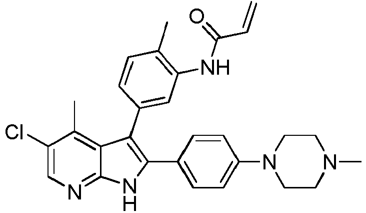

**AE1** was prepared was prepared from 3-bromo-5-chloro-4-methylpyridin-2-amine and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AE1** as a yellow solid. ¹H-NMR (400MHz, d₆-DMSO, 300K) δ 2.07 (s, 3H), 2.28 (s, 3H), 2.82 /2.83 (s, 3H), 2.94 (t, J = 12.6 Hz, 2H), 3.08 (m, 2H), 3.45 (m, 2H), 3.88 (d, J 13.2 Hz, 2H), 5.71 (dd, J = 2.0 Hz, J = 10.1 Hz, 1H), 6.20 (dd, J = 2.0 Hz, J = 17.1 Hz, 1 H), 6.52 (dd, J = 10.1 Hz, J = 17.1 Hz, 1H), 6.90 (d, J = 8.8 Hz, 2H), 7.02 ( d, J = 7.8 Hz, 1H), 7.24 (d, 7.8 Hz, 1H), 7.34 (d, J = 8.8 Hz, 2H), 7.56 (s, 1H), 8.14 (s, 1H), 9.46 (s, 1H), 9.54 (br s, 1H), 12,09 (s, 1H). MS (ES) C₂₉H₃₀ClN₅O requires: 500 found: 501 (M+H)⁺.

### Example 156:

### isopropyl 3-(3-acrvlamido-4-methylphenyl)-2-(2-(4-methylpiperazin-1-yl)pyridin-4-yl)-1H-pyrrolo[2,3-b]pyridine-5-carboxylate 2,2,2-trifluoroacetate (AF5)

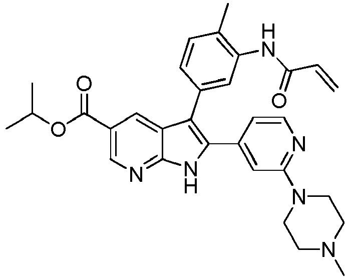

### Step 1: Isopropyl 3-(4-methyl-3-nitrophenyl)-2-(trimethylsilyl)-1H-pyrrolo[2,3-b]pyridine-5-carboxylate (AF1)

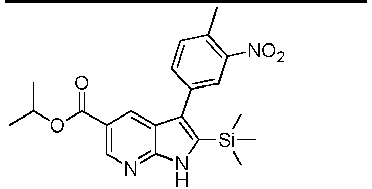

Isopropyl 6-amino-5-iodonicotinate (11.4g, 37.2mmol, 1.0eq.), trimethyl((4-methyl-3-nitrophenyl)ethynyl)silane (8.7g, 37.2mmol, 1.0eq.), 1,4-diazabicyclo[2.2.2]octane (7.1g, 63.2mmol, 1.7eq.) and dichlorobis(triphenylphosphine)palladium(II) (2.6g, 3.7mmol, 0.1eq.) in dry DMF (120mL) under N₂ atmosphere was splitted in six microwave vials. Each vial was heated at 145⁰C for 5 h. The solvent was removed in vacuo and the crude product was purified by flash chromatography on silica gel (cHex/EtOAc = 100:0 to 1:1) to yield the desired product **AF1** (7.1g, 17.3mmol, 46%) as a beige solid. ¹H-NMR (400MHz, d₆-DMSO, 300K) δ 0.23 (s, 9H), 1.33 (d, J = 6.2 Hz, 6H), 2.60 (s, 3H), 5.15 (septet, J = 6.2 Hz, 1H), 7.63 (d, J = 7.8 Hz, 1H), 7.71 (dd, J = 1.9 Hz, J = 7.8 Hz, 1H), 7.97 (d, J = 1.9 Hz, 1H), 8.25 (d, J = 1.9 Hz, 1H), 8.88 (d, J = 1.9 Hz, 1H), 12.17 (s, 1H). MS (ES) C₂₁H₂₅N₃O₄Si requires: 411, found: 412 (M+H)⁺.

### Step 2: Isopropyl 3-(3-amino-4-methylphenyl)-2-(trimethylsilyl)-1H-pyrrolo[2,3-b]pyridine-5-carboxylate (AF2)

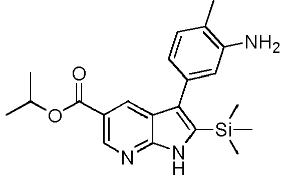

A solution of isopropyl 3-(4-methyl-3-nitrophenyl)-2-(trimethylsilyl)-1H-pyrrolo[2,3-b]pyridine-5-carboxylate **AF1** (7.1g, 17.3mmol, 1.0eq.) and iron (2g, 36.4mmol, 2.1 eq.) in EtOH (360mL) and aq. sat. NH₄Cl-solution (36mL) was stirred for 8h at 80°C. The solution was filtered through a pad of Celite^{®}. Solvents were removed in vacuo. The crude product was purified by flash chromatography on silica gel (cHex/EtOAc = 100:0 to 0:100) to yield the desired product **AF2** (5.4g, 14.2mmol, 82%) as a beige solid. MS (ES) C₂₁H₂₇N₃O₂Si requires: 381, found: 382 (M+H)⁺.

### Step 3: Isopropyl 3-(3-acrylamido-4-methylphenyl)-2-(trimethylsilyl)-1H-pyrrolo[2,3-b]pyridine-5-carboxylate (AF3)

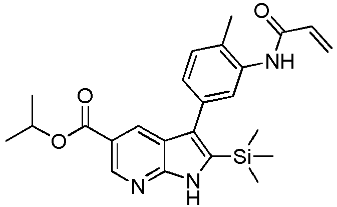

To a solution of **AF2** (5.4g, 14.2mmol, 1.0eq.) and DIPEA (24.7mL, 141.7mmol, 10.0eq.) in dry THF (200mL) at -78°C was added acrylolyl chloride (1.53g, 17.0mmol, 1.2eq.) in dry THF (20mL). The mixture was stirred for 20min at -40°C and then a few drops of water were added. Solvents were removed in vacuo. The crude product was purified by flash chromatography on silica gel (cHex/EtOAc = 100:0 to 1:1) to yield the desired product **AF3** (6.2g, 14.2mmol, quant.) as a yellow solid. MS (ES) C₂₄H₂₉N₃O₃Si requires: 435, found: 436 (M+H)⁺.

### Step 4: Isopropyl 3-(3-acrylamido-4-methylphenyl)-2-iodo-1H-pyrrolo[2,3-b]pyridine-5-carboxylate (AF4)

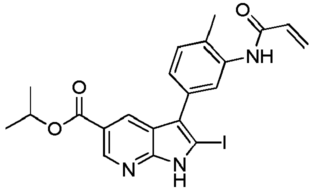

Isopropyl 3-(3-acrylamido-4-methylphenyl)-2-(trimethylsilyl)-1H-pyrrolo[2,3-b]pyridine-5-carboxylate **AF3** (6.2g, 14.2mmol, 1.0eq.) and N-iodosuccinimide (4.2g, 18.5mmol, 1.3eq.) were solved in dry dichloromethane (700mL) and stirred for 15h at RT. The organic phase was washed once with aq. sat. Na₂S₂O₃-solution and twice with aq. sat. NaHCO₃-solution. The organic phase was dried over Na₂SO₄ and solvents were removed in vacuo. The crude was stirred in DCM (30mL) for 1h at RT, the precipitate was filtered off and dried in vacuo yielding desired product **AF4** (3.9g, 8.0mmol, 56%) as a beige solid. The crude was used without purification in the next step. ¹H-NMR (400MHz, d₆-DMSO, 300K) δ 1.33 (d, J = 6.2 Hz, 6H), 2.31 (s, 3H), 5.16 (septet, J = 6.2 Hz, 1H), 5.77 (dd, J = 2.2 Hz, J = 10.1 Hz, 1H), 6.27 (dd, J = 2.2 Hz, 17.0 Hz, 1H), 6.60 (dd, J = 10.1 Hz, J = 17.0 Hz, 1H), 7.32 ( J = 1.8 Hz, J = 7.8 Hz, 1H), 7.39 (d, J = 7.8 Hz, 1H), 7.78 (s, 1H), 8.43 (d, J = 2.0 Hz, 1H), 8.79 (d, J = 2.0 Hz, 1H), 9.57 (s, 1H), 12.84 (s, 1H). MS (ES) C₂₁H₂₀IN₃O₃ requires: 489, found: 490 (M+H)⁺.

### Step 5: Isopropyl 3-(3-acrylamido-4-methylphenyl)-2-(2-(4-methylpiperazin-1-yl)pyridin-4-yl)-1H-pyrrolo[2,3-b]pyridine-5-carboxylate 2,2,2-trifluoroacetate (AF5)

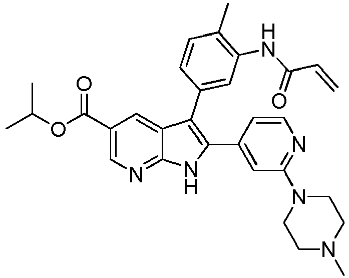

A mixture of isopropyl 3-(3-acrylamido-4-methylphenyl)-2-iodo-1H-pyrrolo[2,3-b]pyridine-5-carboxylate **AF4** (30mg, 0.06mmol, 1.0eq.), 2-(4-methylpiperazino)pyridine-4-boronic acid pinacol ester (24mg, 0.08mmol, 1.3eq.) and K₃PO₄ (26mg, 0.12mmol, 2.0eq.) in dioxane/H₂O (5mL, 10/1) was degassed with a stream of N₂ for 5min. [1,1'-Bis(diphenylphosphino)ferrocene]palladium dichloride dichloromethane adduct (5mg, 0.006mmol, 0.1eq.) was added and the reaction mixture heated to 130°C for 2 h under N₂ atmosphere in the microwave oven. The reaction mixture was diluted with EtOAc, washed three times with aq. sat. NaHCO₃-SOlution. The organic phase was dried over MgSO₄ and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **AF5** (8mg, 0.01mmol, 17%) as a yellow powder. ¹H-NMR (400MHz, d₆-DMSO, 300K) δ 1.34 (d, J = 6.2 Hz, 6H), 2.31 (s, 3H), 2.85 (s, 3H), 3.07 (m, 2H), 3.13 (m, 2H), 3.50 (d, J = 11.7 Hz, 2 H), 4.35 (d, J = 13.8 Hz, 2H), 5.17 (septet, J = 6.2 Hz, 1H), 5.76 (dd, J = 2.1 Hz, J = 10.2 Hz, 1H), 6.23 (dd, J = 2.1 Hz, J = 17.1 Hz, 1H), 6.56 (dd, J = 10.2 Hz, J = 17.1 Hz, 1H), 6.78 (dd, J = 1.3 Hz, J = 5.3 Hz, 1H), 7.10 (dd, J = 1.8 Hz, J = 7.8 Hz, 1H), 7.16 (s, 1H), 7.35 (d, J = 7.8 Hz, 1H), 7.64 (s, 1H), 8.10 (d, J = 5.3 Hz, 1H), 8.38 (d, J = 2.0 Hz, 1H), 8.91 (d, J = 2.0 Hz, 1H), 9.58 (s, 1H), 9.78 (br s, 1H), 12.75 (s, 1H). MS (ES) C₃₁H₃₄N₆O₃ requires: 538, found: 539 (M+H)⁺.

The Example in the following table was prepared according to the procedure described for **AF5** (Example 156).

| **Example** | **Name** | **Mwt** | **[M+H]⁺** |
|---|---|---|---|
| 157 | 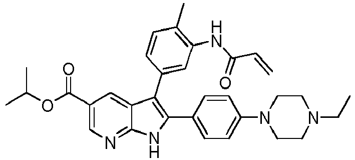 | 551 | 552 |
| 158 | 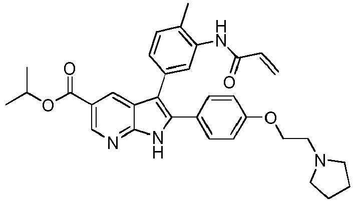 | 552 | 553 |
| 159 | 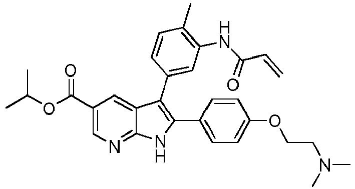 | 526 | 527 |
| 160 | 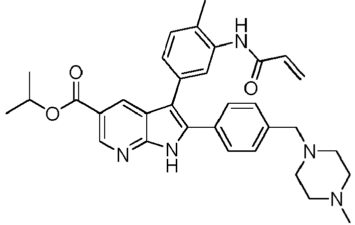 | 551 | 552 |
| 161 | 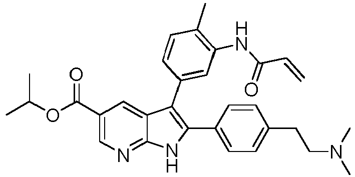 | 510 | 511 |
| 162 | 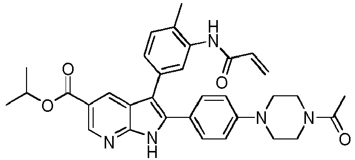 | 565 | 566 |
| 163 | 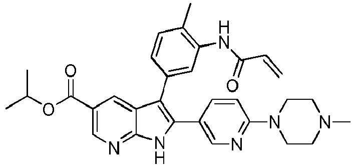 | 538 | 539 |
| 164 | 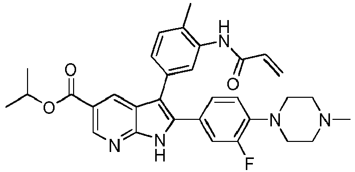 | 555 | 556 |
| 166 | 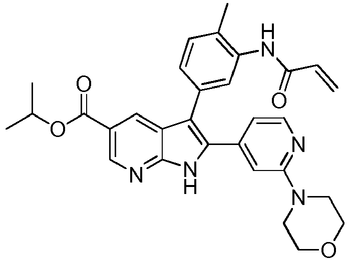 | 525 | 526 |
| 167 | 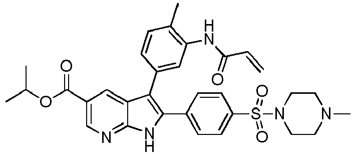 | 601 | 602 |
| 168 | 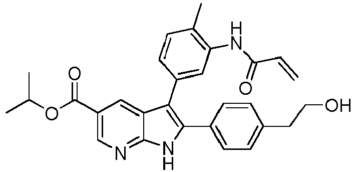 | 483 | 484 |
| 169 | 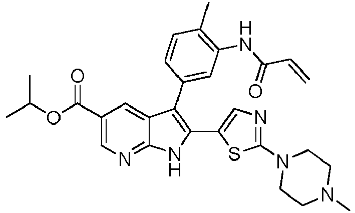 | 544 | 545 |
| 170 | 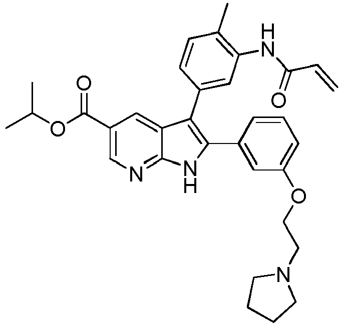 | 552 | 553 |
| 171 | 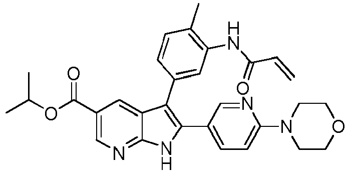 | 525 | 526 |
| 173 | 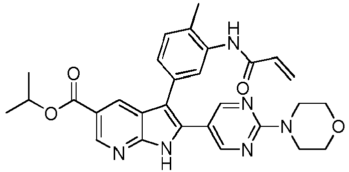 | 526 | 527 |
| 175 | 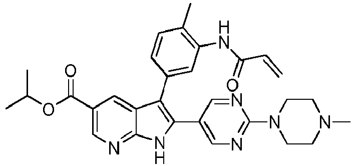 | 539 | 540 |
| 176 | 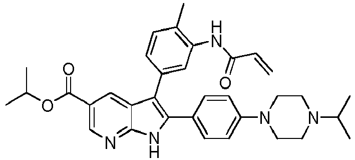 | 565 | 566 |
| 178 | 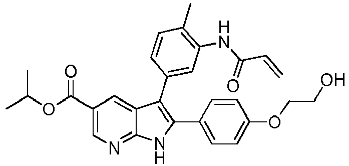 | 499 | 500 |
| 181 | 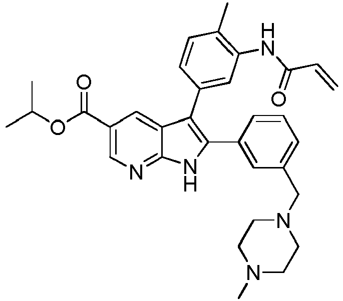 | 551 | 552 |
| 183 | 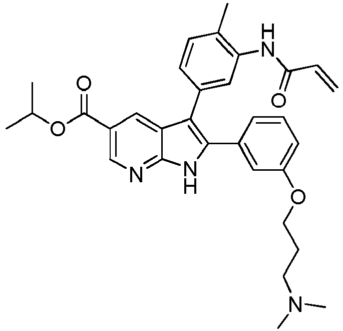 | 540 | 541 |
| 184 | 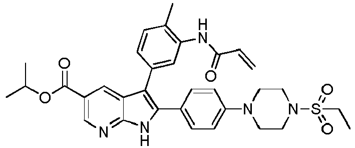 | 615 | 616 |
| 185 | 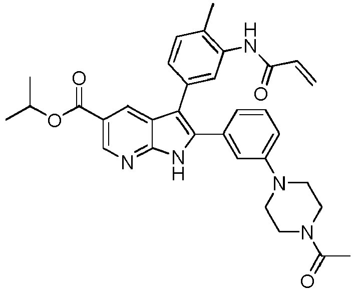 | 565 | 566 |
| 186 | 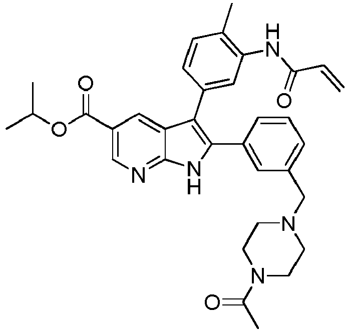 | 579 | 580 |
| 187 | 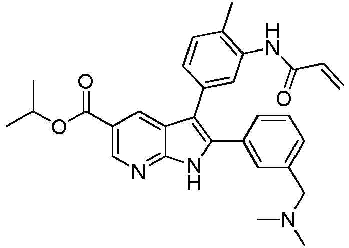 | 496 | 497 |

### Example 165:

### isopropyl 3-(3-acrylamido-5-fluoro-4-methylphenyl)-2-(4-(4-methylpiperazin-1-yl)phenyl)-1H-pyrrolo[2,3-b]pyridine-5-carboxylate 2,2,2-trifluoroacetate (AG1)

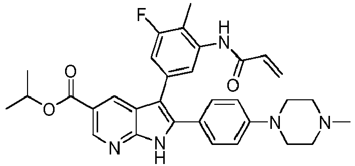

**AG1** was prepared from isopropyl 6-amino-5-iodonicotinate and ((3-fluoro-4-methyl-5-nitrophenyl)ethynyl)trimethylsilane following the general procedure reported in Preparative Example 156. The crude product was purified by reverse phase RP-HPLC (column: C18), using H₂O (0.1%TFA) and ACN (0.1%TFA) as eluents. The desired fractions were lyophilized to yield the title compound **AG1** as a yellow solid. ¹H-NMR (400MHz, d₆-DMSO, 300K) δ 1.34 (d, J = 6.2 Hz, 6H), 2.18 (s, 3H), 2.85/2.87 (s, 3H), 3.02 (t, J = 12.4 Hz, 2H), 3.14 (q, J = 12.4 Hz, 2H), 3.51 (m, 2H), 3.96 (d, J = 13.3 Hz, 2H), 5.17 (septet, J = 6.2 Hz, 1H), 5.87 (dd, J = 2.0 Hz, J = 10.2 Hz, 1H), 6.25 (dd, J = 2.0 Hz, J = 17.1 Hz, 1H), 6.57 (dd, J = 10.2 Hz, J = 17.1 Hz, 1H), 6.87 (d, J = 10.4 Hz, 1H), 7.05 (d, J = 8.8 Hz, 2H), 7.47 (d, J = 8.8 Hz, 2H), 7.54 (s, 1H), 8.38 (d, J = 2.0 Hz, 1H), 8.83 (d, J = 2.0 Hz, 1H), 9.64 (br s, 1H), 9.72 (s, 1H), 12.53 (s, 1H). MS (ES) C₃₂H₃₄FN₅O₃ requires: 555 found: 556 (M+H)⁺.

The Example in the following table was prepared according to the procedure described for **AG1** (Example 165).

| **Example** | **Name** | **Mwt** | **[M+H]⁺** |
|---|---|---|---|
| 172 | 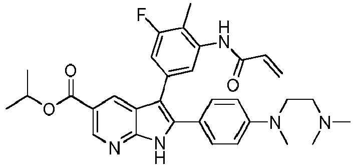 | 557 | 558 |
| 174 | 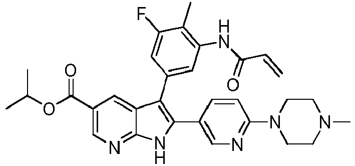 | 556 | 557 |
| 177 | 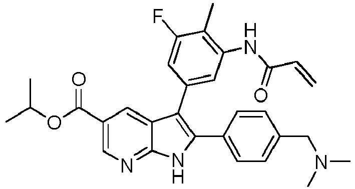 | 514 | 515 |
| 179 | 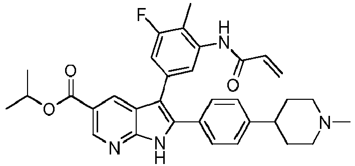 | 554 | 555 |
| 180 | 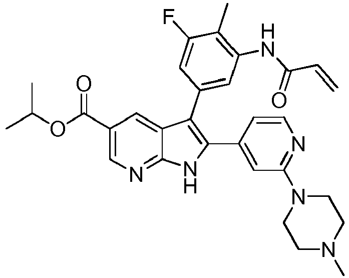 | 556 | 557 |
| 182 | 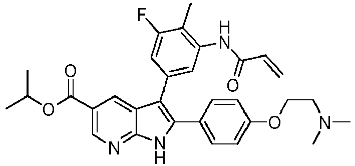 | 544 | 545 |

### Example 188:

### N-(2-methyl-5-(2-(4-(4-methylpiperazin-1-yl)phenyl)-5-(trifluoromethoxy)-1H-pyrrolo[2,3-b]pridin-3-yl)phenyl)acrylamide 2,2,2-trifluoroacetate (AH1)

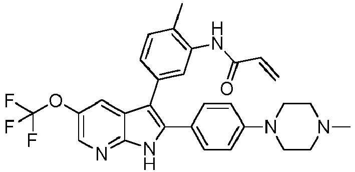

**AH1** was prepared from 3-bromo-5-(trifluoromethoxy)pyridin-2-amine and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AH1** as a yellow solid. ¹H-NMR (400MHz, d₆-DMSO, 300K) δ 2.26 (s, 3H), 2.86/2.87 (s, 3H), 3.00 (t, J = 12.8 Hz, 2H), 3.13 (m, 2H), 3.52 (d, J = 12.0 Hz, 2H), 3.96 (d, J = 13.3 Hz, 2H), 5.75 (dd, J = 2.0 Hz, J = 10.0 Hz, 1H), 6.25 (dd, J = 2.0 Hz, J = 17.1 Hz, 1H), 6.57 (dd, J = 10.0 Hz, J = 17.1 Hz, 1H), 6.98 (d, J = 7.8 Hz, 1H), 7.02 (d, J = 9.0 Hz, 2 H), 7.23 (d, J = 7.8 Hz, 1H), 7.46 (d, J = 9.0 Hz, 2H), 7.63 (s, 1H), 7.78 (d, J = 1.9 Hz, 1H), 8.29 (d, J = 1.9 Hz, 1H), 9.51 (s, 1H), 9.66 (br s, 1H), 12.36 (s, 1H). MS (ES) C₂₉H₂₈F₃N₅O₂ requires: 535 found: 536 (M+H)⁺.

### Example 189:

### 2-(3-(3-acrylamido-4-methylphenyl)-2-(4-(4-methylpiperazin-1-yl)phenyl)-1H-pyrrolo[2,3-b]pyridin-5-yl)acetic acid 2,2,2-trifluoroacetate (AI2)

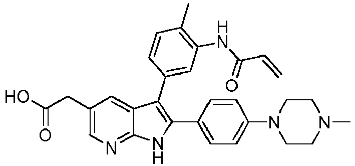

### Step 1: tert-butyl 2-(3-(3-acrylamido-4-methylphenyl)-2-(4-(4-methylpiperazin-1-yl)phenyl)-1H-pyrrolo[2,3-b]pyridin-5-yl)acetate 2,2,2-trifluoroacetate (AI1)

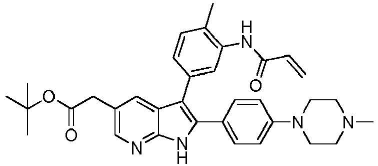

**AI1** was prepared from tert-butyl 2-(6-amino-5-bromopyridin-3-yl)acetate and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AI1** as a yellow solid. MS (ES) C₃₄H₃₉N₅O₃ requires: 565 found: 566 (M+H)⁺.

### Step 2: 2-(3-(3-acrylamido-4-methylphenyl)-2-(4-(4-methylpiperazin-1-yl)phenyl)-1H-pyrrolo[2,3-b]pyridin-5-yl)acetic acid 2,2,2-trifluoroacetate (AI2)

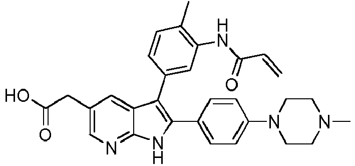

A solution of **AI1** (245mg, 0.31mmol) and TFA (2.5mL) in DCM (7.5mL) was stirred for 3h at RT. Solvents were removed in vacuo. The crude product was purified by reverse phase RP-HPLC (column: C18), using H₂O (0.1%TFA) and ACN (0.1%TFA) as eluents. The desired fractions were lyophilized to yield the title compound **AI2** (172mg, 0.23mmol, 74%) as a yellow solid. ¹H-NMR (400MHz, d₆-DMSO, 300K) δ 2.26 (s, 3H), 2.86/2.87 (s, 3H), 2.98 (t, J =12.1 Hz, 2H), 3.13 (m, 2H), 3.52 (m,2H), 3.65 (s, 2H), 3.92 (d, J = 13.3 Hz, 2H), 5.73 (dd, J = 2.0 Hz, J = 10.2 Hz, 1H), 6.22 (dd, J = 2.0 Hz, J = 17.1 Hz, 1H), 6.55 (dd, J = 10.2 Hz, J = 17.1 Hz, 1H), 6.99 (m, 3H), 7.24 (d, J = 7.9 Hz, 1H), 7.45 (d, J = 8.9 Hz, 2H), 7.56 (s, 1H), 7.67 (d, J = 2.1 Hz, 1H), 8.11 (d, J = 2.1 Hz, 1H), 9.50 (s, 1H), 9.60 (br s, 1H), 11.95 (s, 1H), 12.30 (br s, 1H). MS (ES) C₃₀H₃₁N₅O₃ requires: 509 found: 510 (M+H)⁺.

### Example 190:

### isopropyl 2-(3-(3-acrylamido-4-methylphenyl)-2-(4-(4-methylpiperazin-1-yl)phenyl)-1H-pyrrolo[2,3-b]pyridin-5-yl)acetate 2,2,2-trifluoroacetate (AJ1)

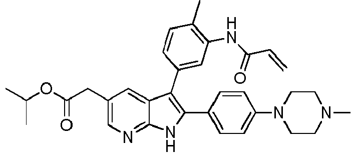

**AD1** (2mg, 0.003mmol, 26%) was prepared from **AI2** and isopropanol following the general procedure reported in Preparative Example 134. The crude product was purified reverse phase RP-HPLC (column: C18), using H₂O (0.1%TFA) and ACN (0.1%TFA) as eluents. The desired fractions were lyophilized to yield the title compound **AD1** as a yellow solid. ¹H NMR (400MHz, d₄-MeOD, 300K) δ 1.22 (d, J = 6.3 Hz, 6H), 2.30 (s, 3H), 2.98 (s, 3H), 3.07 (m,2H), 3.27 (m, 2H), 3.60 (m, 2H), 3.73 (s, 2H), 3.93 (m,2H), 4.98 (septet, J = 6.3 Hz, 1H), 5.79 (dd, J = 1.7 Hz, 10.0 Hz, 1H), 6.35 (dd, J = 1.7 Hz, J = 17.0 Hz, 1H), 6.51 (dd, J = 10.0 Hz, J = 17.0 Hz, 1H), 7.01 (d, J = 9.0 Hz, 2H), 7.09 (d, J = 7.9 Hz, 1H), 7.23 (d, J = 7.9 Hz, 1H), 7.50 (m, 3H), 7.91 (d, J = 1.9 Hz, 1H), 8.13 (d, J = 1.9 Hz, 1H). MS (ES) C₃₃H₃₇N₅O₃ requires: 551 found: 552 (M+H)⁺.

The Examples in the following table were prepared according to the procedure described for **AJ1** (Example 190).

| **Example** | **Name** | **Mwt** | **[M+H]⁺** |
|---|---|---|---|
| 196 | 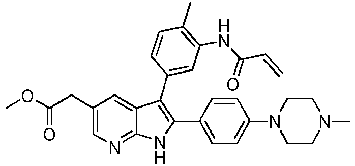 | 523 | 524 |
| 198 | 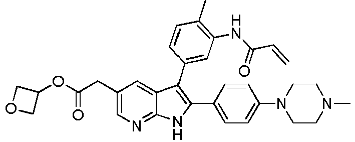 | 565 | 566 |

### Example 191:

### N-(5-(5-ethyl-2-(4-(4-methylpiperazin-1-yl)phenyl)-1H-pyrrolo[2,3-b]pyridin-3-yl)-2-methylphenyl)acrylamide 2,2,2-trifluoroacetate (AK1)

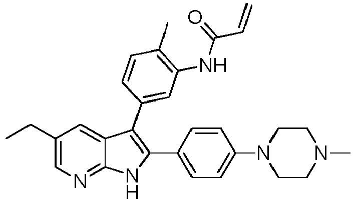

**AK1** was prepared from 3-bromo-5-ethylpyridin-2-amine and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AK1** as a yellow solid. ¹H-NMR (400MHz, d₆-DMSO, 300K) δ 1.23 (t, J = 7.6 Hz, 3H), 2.27 (s, 3H), 2.69 (q, J = 7.6 Hz, 2H), 2.86/2.87 (s, 3H), 3.00 (t, J = 12.7 Hz, 2H), 3.14 (m, 2H), 3.51 (m, 2H), 3.93 (d, J = 13.5 Hz, 2H), 5.75 (dd, J = 2.0 Hz, J = 10.0 Hz, 1H), 6.24 (dd, J = 2.0 Hz, J = 17.0 Hz, 1H), 6.57 (dd, J = 10.0 Hz, J = 17.0 Hz, 1H), 7.00 (m, 3H), 7.23 (d, J = 7.7 Hz, 1H), 7.44 (d, J = 7.0 Hz, 2H), 7.61 (s, 1H), 7.68 (d, J = 2.0 Hz, 1H), 8.13 (d, J = 2.0 Hz, 1H), 9.51 (s, 1H), 9.70 (br s, 1H), 11.90 (s, 1H). MS (ES) C₃₀H₃₃N₅O requires: 479 found: 480 (M+H)⁺.

### Example 192:

### isopropyl 3-(1-acryloylindolin-6-yl)-2-(4-(4-methylpiperazin-1-yl)phenyl)-1H-pyrrolo[2,3-b]pyridine-5-carboxylate 2,2,2-trifluoroacetate (AL1)

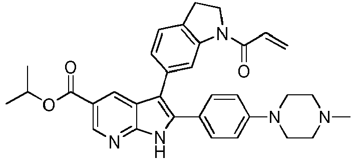

**AL1** was prepared from isopropyl 6-amino-5-iodonicotinate and tert-butyl 6-((trimethylsilyl)ethynyl)indoline-1-carboxylate following the general procedure reported in Preparative Example 104. The crude product was purified by reverse phase RP-HPLC (column: C18), using H₂O (0.1%TFA) and ACN (0.1%TFA) as eluents. The desired fractions were lyophilized to yield the title compound **AL1** as a yellow solid. ¹H NMR (400MHz, d₄-MeOD, 300K) δ 1.30 (d, J = 6.1 Hz, 6H), 2.84 (s, 3H), 2.98 (t, J = 12.6 Hz, 2H), 3.10 (m, 2H), 3.23 (m, 2H), 3.48 (d, J = 12.0 Hz, 2H), 3.92 (d, J = 13.4 Hz, 2H), 4.27 (m, 2H), 5.13 (septet, J = 6.1 Hz, 1H), 5.79 (d, J = 10.2 Hz, 1H), 6.24 (d, J = 16.6 Hz, 1H), 6.74 (dd, J = 10.2 Hz, J = 16.6 Hz, 1H), 6.98 (m, 3H), 7.29 (d, J = 7.7 Hz, 1H), 7.44 (d, J = 8.7 Hz, 2H), 8.21 (s, 1H), 8.25 (s, 1H), 8.80 (d, J = 2.0 Hz, 1H), 9.65 (br s, 1H), 12.44 (s, 1H). MS (ES) C₃₃H₃₅N₅O₃ requires: 549 found: 550 (M+H)⁺.

### Example 193:

### N-(5-(5-ethoxy-2-(4-(4-methylpiperazin-1-yl)phenyl)-1H-pyrrolo[2,3-b]pyridin-3-yl) 2-methylphenyl)acrylamide 2,2,2-trifluoroacetate (AM1)

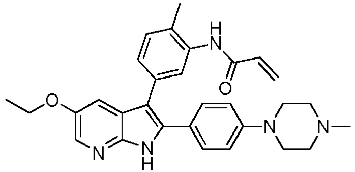

**AM1** was prepared from 3-bromo-5-ethoxypyridin-2-amine and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AM1** as a yellow solid. ¹H NMR (400MHz, d₄-MeOD, 300K) δ 1.32 (t, J = 7.0 Hz, 3H), 2.24 (s, 3H), 2.84 (s, 3H), 2.97 (t, J = 12.6 Hz, 2H), 3.12 (m, 2H), 3.50 (d, J = 12.0 Hz, 2H), 3.88 (d, J = 13.4 Hz, 2H), 4.05 (q, J = 7.0 Hz, 2H), 5.72 (dd, J = 2.0 Hz, J = 10.1 Hz, 1H), 6.22 (dd, J = 2.0 Hz, J = 17.1 Hz, 1H), 6.55 (dd, J = 10.1Hz, J = 17.1 Hz, 1H), 6.94 (d, J = 8.0 Hz, 1H), 6.98 (d, J = 9.0 Hz, 2H), 7.18 (d, J = 8.0 Hz, 1H), 7.42 (m, 3H), 7.62 (s, 1H), 7.96 (d, J = 2.7 Hz, 1H), 9.49 (s, 1H), 9.73 (br s, 1H), 11.79 (s, 1H). MS (ES) C₃₀H₃₃N₅O₂ requires: 495 found: 496 (M+H)⁺.

### Example 194:

### N-(5-(5-(2-(dimethylamino)-2-oxoethyl)-2-(4-(4-methylpiperazin-1-yl)phenyl)-1H-pyrrolo[2,3-b]pyridin-3-yl)-2-methylphenyl)acrylamide 2,2,2-trifluoroacetate (AN1)

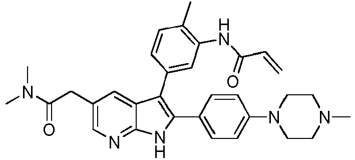

**AN1** (2mg, 0.003mmol, 26%) was prepared from **AI2** and dimethylamine following the general procedure reported in Preparative Example Example 118. The crude product was purified reverse phase RP-HPLC (column: C18), using H₂O (0.1%TFA) and ACN (0.1%TFA) as eluents. The desired fractions were lyophilized to yield the title compound **AN1** as a yellow solid. ¹H NMR (400MHz, d₄-MeOD, 300K) δ 2.26 (s, 3H), 2.81 (s, 3H), 2.86 (s, 3H), 2.99 (t, J = 12.6 Hz, 2H), 3.04 (s, 3H), 3.13 (m, 2H), 3.51 (m, 2H), 3.76 (s, 2H), 3.92 (d, J = 13.3 Hz, 2H), 5.74 (dd, J = 2.0 Hz, J =10.1 Hz, 1H), 6.22 (dd, J = 2.0 Hz, J = 17.0 Hz, 1H), 6.55 (dd, J = 10.1 Hz, J = 17.0 Hz, 1H), 6.99 (m, 3H), 7.23 (d, J = 7.8 Hz, 1H), 7.43 (d, J = 8.9 Hz, 2H), 7.57 (s, 1H), 7.62 (d, J = 2.0 Hz, 1H), 8.08 (d, J = 2.0 Hz, 1H), 9.50 (s, 1H), 9.65 (br s, 1H), 11.93 (s, 1H). MS (ES) C₃₂H₃₆N₆O₂ requires: 536 found: 537 (M+H)⁺.

The Example in the following table was prepared according to the procedure described for **AN1** (Example 194).

| **Example** | **Name** | **Mwt** | **[M+H]⁺** |
|---|---|---|---|
| 196 | 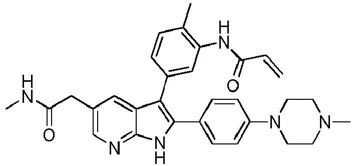 | 522 | 523 |

### Example 198:

### isopropyl 3-(3-acrylamido-4-methylphenyl)-4-methyl-2-(4-(4-methylpiperazin-1-yl)phenyl)-1H-pyrrolo[2,3-b]pyridine-5-carboxylate 2,2,2-trifluoroacetate (AO1)

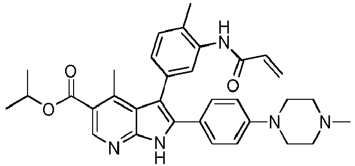

**AO1** was prepared from isopropyl 6-amino-5-iodo-4-methylnicotinate and trimethyl((4-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AO1** as a yellow solid. ¹H NMR (400MHz, d₆-DMSO, 300K) δ 1.32 (d, J = 6.2 Hz, 6H), 2.27 (s, 3H), 2.30 (s, 3H), 2.84/2.85 (s, 3H), 2.96 (t, J = 12.8 Hz, 2H), 3.08 (m, 2H), 3.49 (m, 2H), 3.90 (d, J = 13.4 Hz, 2H), 5.12 (septet, J = 6.2 Hz, 1H), 5.73 (dd, J = 2.0 Hz, J = 10.1 Hz, 1H), 6.20 (dd, J = 2.0 Hz, J = 17.0 Hz, 1H), 6.55 (dd, J = 10.1 Hz, J = 17.0 Hz, 1H), 6.93 (d, J = 9.0 Hz, 2H), 7.05 (d, J = 7.9 Hz, 1H), 7.26 (d, J = 7.9 Hz, 1H), 7.36 (d, J = 9.0 Hz, 2H), 7.58 (s, 1H), 8.60 (s, 1H), 9.49 (s, 1H), 9.59 (br s, 1H), 12.28 (s, 1H). MS (ES) C₃₃H₃₇N₅O₃ requires: 551 found: 552 (M+H)⁺.

The Examples in the following table were prepared according to the procedure described for **AO1** (Example 198).

| **Example** | **Name** | **Mwt** | **[M+H]⁺** |
|---|---|---|---|
| 204 | 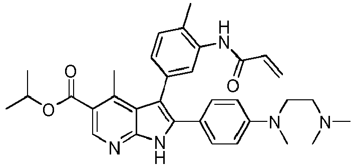 | 553 | 554 |
| 205 | 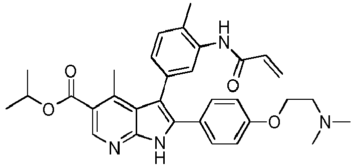 | 540 | 541 |

### Example 199:

### N-(5-(5-(hydroxymethyl)-2-(4-(4-methylpiperazin-1-yl)phenyl)-1H-pyrrolo[2,3-b]pyridin-3-yl)-2-methylphenyl)acrylamide 2,2,2-trifluoroacetate (AP3)

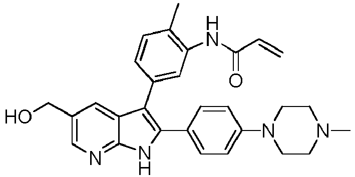

### Step 1: methyl 3-(3-amino-4-methylphenyl)-2-(4-(4-methylpiperazin-1-yl)phenyl)-1H-pyrrolo[2,3-b]pyridine-5-carboxylate 2,2,2-trifluoroacetate (AP1)

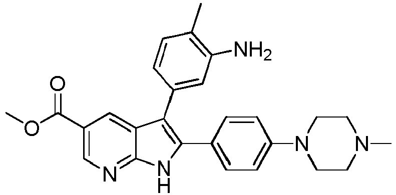

To a solution of 3-(3-amino-4-methylphenyl)-2-(4-(4-methylpiperazin-1-yl)phenyl)-1H-pyrrolo[2,3-b]pyridine-5-carboxylic acid **M2** (800mg, 1.8mmol, 1.0eq.) in dry methanol (12mL) was added thionyl chloride (0.4mL). The mixture was stirred at 70°C for 15h. Solvent were removed and the crude was purified by reverse phase RP-HPLC (column: C18), using H₂O (0.1%TFA) and ACN (0.1%TFA) as eluents. The desired fractions were lyophilized to yield the title compound **AP1** (306mg, 0.45mmol, 25%) as a yellow powder. ¹H NMR (400MHz, d₆-DMSO, 300K) δ 2.15 (s, 3H), 2.84 (s, 3H), 2.98 (t, J = 12.6 Hz, 2H), 3.11 (m, 2H), 3.49 (d, J = 11.8 Hz, 2H), 3.84 (s, 3H), 3.91 (d, J = 13.2 Hz, 2H), 6.61 (m, 1H), 6.86 (s, 1H), 6.99 (d, J = 9.0 Hz, 2H), 7.06 (d, J = 7.6 Hz, 1H), 7.45 (d, J = 9.0 Hz, 2H), 8.23 (d, J = 2.1 Hz, 1H), 8.80 (d, J = 2.1 Hz, 1H), 9.70 (br s, 1H), 12.39 (s, 1H). MS (ES) C₂₇H₂₉N₅O₂ requires: 455, found: 456 (M+H)⁺.

### Step 2: (3-(3-amino-4-methylphenyl)-2-(4-(4-methylpiperazin-1-yl)phenyl)-1H-pyrrolo[2,3-b]pyridin-5-yl)methanol (AP2)

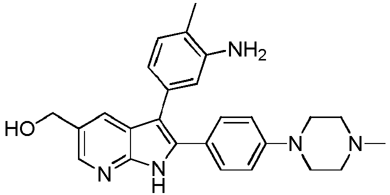

To a solution of **AP1** (306mg, 0.45mmol, 1.0eq.) in dry THF (24mL) at 0°C was added LiAIH₄-solution (1M, 1.34mL, 1.34mmol, 3.0eq.). The mixture was stirred 2h at 0°C and 0.5 h at RT. The mixture was cooled to 0°C and again LiAIH₄-solution (1M, 0.45mL, 0.45mmol, 1.0eq.) was added. After 1h aq. sat. NaHCO₃-solution was carefully added and then EtOAc was added. The precipitate was filtered off and dried in vacuo yielding the title compound **AP2** (25mg, 0.06mmol, 13%) as a beige powder. MS (ES) C₂ₛH₂₉N₅O requires: 427, found: 428 (M+H)⁺.

### Step 3: N-(5-(5-(hydroxymethyl)-2-(4-(4-methylpiperazin-1-yl)phenyl)-1H-pyrrolo[2,3-b]pyridin-3-yl)-2-methylphenyl)acrylamide 2,2,2-trifluoroacetate (AP3)

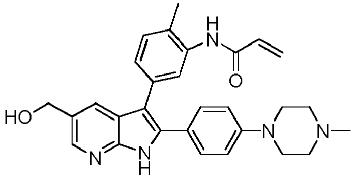

To a solution of **AP2** (25mg, 0.06mmol, 1.0eq.), DIPEA (30uL, 0.18mmol, 3.0eq.) and HATU (34mg, 0.09mmol, 1.5eq.) in dry DMF (1.5mL) at 0°C was added a solution of acrylic acid (4mg, 0.06mmol, 1.0eq.) in dry DMF (0.5mL). The mixture was stirred for 1h at 0°C. The mixture was diluted with EtOAc and then was washed with with aq. sat. NaHCO₃-solution and brine. The organic phase was dried over MgSO₄ and solvents were removed in vacuo. The crude product was purified by reverse phase RP-HPLC (column: C18), using H₂O (0.1%TFA) and ACN (0.1%TFA) as eluents. The desired fractions were lyophilized to yield the title compound **AP3** (3mg, 0.004mmol, 73%) as a yellow solid. ¹H NMR (400MHz, d₆-DMSO, 300K) δ 2.24 (s, 3H), 2.84 (s, 3H), 2.97 (t, J = 12.6 Hz, 2H), 3.13 (m, 2H), 3.48 (m, 2H), 3.90 (d, J = 13.4 Hz, 2H), 4.54 (s, 2H), 5.72 (dd, J = 2.0 Hz, J = 10.1 Hz, 1H), 6.20 (dd, J = 2.0 Hz, J = 17.1 Hz, 1H), 6.53 (dd, J = 10.1 Hz, J = 17.1 Hz, 1H), 6.97 (m, 3H), 6.22 (d, J = 7.8 Hz, 1H), 7.43 (d, J = 8.9 Hz, 2H), 7.56 (s, 1H), 7.73 (d, J = 2.0 Hz, 1H), 8.17 (d, J = 2.0 Hz, 1H), 9.50 (s, 1H), 9.65 (br s, 1H), 11.93 (s, 1H). MS (ES) C₂₉H₃₁N₅O₂ requires: 481 found: 482 (M+H)⁺.

### Example 200:

### N-(5-(5-isobutyramido-2-(4-(4-methylpiperazin-1-yl)phenyl)-1H-pyrrolo[2,3-b]pyridin-3-yl)-2-methylphenyl)acrylamide 2,2,2-trifluoroacetate (AQ5)

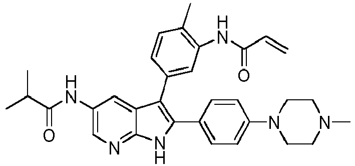

### Step 1: tert-butyl (2-methyl-5-(2-(4-(4-methylpiperazin-1-yl)phenyl)-5-nitro-1H-pyrrolo[2,3-b]pyridin-3-vl)phenyl)carbamate (AQ1)

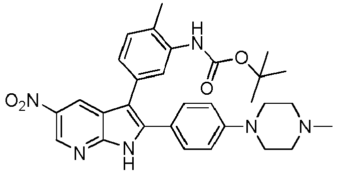

**AQ1** was prepared from 3-iodo-5-nitropyridin-2-amine and tert-butyl (2-methyl-5-((trimethylsilyl)ethynyl)phenyl)carbamate following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AQ1** as an orange solid. ¹H NMR (400MHz, d₆-DMSO, 300K) δ 1.42 (s, 9H), 2.24 (s, 3H), 2.84 (s, 3H), 3.00 (m, 2H), 3.11 (m, 2H), 3.48 (m, 2H), 3.94 (d, J = 13.2 Hz, 2H), 6.95 (dd, J = 1.8 Hz, J = 7.7 Hz, 1H), 7.01 (d, J = 8.9 Hz, 2H), 7.20 (d, J = 7.7 Hz, 1H), 7.44 (m, 1H), 7.46 (d, J = 8.9 Hz, 2H), 8.47 (d, J = 2.4 Hz, 1H), 8.61 (s, 1H), 9.09 (d, J = 2.4 Hz, 1H), 9.75 (br s, 1H), 12.83 (s, 1H). MS (ES) C₃₀H₃₄N₆O₄ requires: 542 found: 543 (M+H)⁺.

### Step 2: tert-butyl (5-(5-amino-2-(4-(4-methylpiperazin-1-yl)phenyl)-1H-pyrrolo[2,3-b]pyridin-3-yl)-2-methylphenyl)carbamate (AQ2)

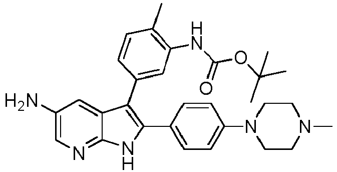

A solution of tert-butyl (2-methyl-5-(2-(4-(4-methylpiperazin-1-yl)phenyl)-5-nitro-1H-pyrrolo[2,3-b]pyridin-3-yl)phenyl)carbamate **AQ1** (40mg, 0.05mmol, 1.0eq.) and iron (15mg, 0.26mmol, 5.0eq.) in EtOH (1mL) and aq. sat. NH₄Cl-solution (0.1mL) was stirred for 3h at 80°C. The solution was filtered through a pad of Celite^{®}. Solvents were removed in vacuo. The crude was solved in DCM and was washed twice with aq. sat. NaHCO₃-solution and once with brine. The organic phase was dried over MgSO₄ and solvents were removed in vacuo yielding the desired product **AQ2** (23mg, 0.04mmol, 85%) as a beige solid. MS (ES) C₃₀H₃₆N₆O₂ requires: 512, found: 513 (M+H)⁺.

### Step 3: tert-butyl (5-(5-isobutyramido-2-(4-(4-methylpiperazin-1-yl)phenyl-1H-pyrrolo[2,3-b]pyridin-3-yl)-2-methylphenyl)carbamate (AQ3)

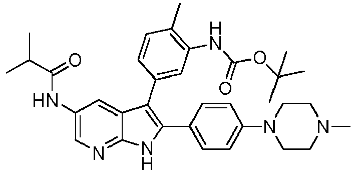

To a solution of **AQ2** (20mg, 0.04mmol, 1.0eq.) and DIPEA (17uL, 0.10mmol, 2.50eq.) in dry DCM (1mL) at 0°C was added isobutyryl chloride (4mg, 0.04mmol, 1.0eq.) in dry DCM (0.5mL). The mixture was stirred for 1h at 0°C and 1h at RT. The mixture was diluted with DCM and then was washed with with aq. sat. NaHCO₃-solution and brine. The organic phase was dried over MgSO₄ and solvents were removed in vacuo. The crude product was purified by reverse phase RP-HPLC (column: C18), using H₂O (0.1%TFA) and ACN (0.1%TFA) as eluents. The desired fractions were lyophilized to yield the title compound **AQ3** (20mg, 0.02mmol, 63%) as a yellow solid. MS (ES) C₃₄H₄₂N₆O₃ requires: 582, found: 583 (M+H)⁺.

### Step 4: N-(3-(3-amino-4-methylphenyl)-2-(4-(4-methylpiperazin-1-yl)phenyl)-1H-pyrrolo[2,3-b]pyridin-5-yl)isobutyramide (AQ4)

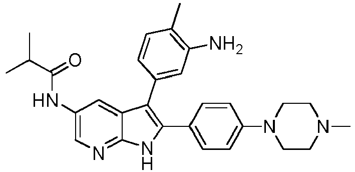

A solution of **AQ3** (19mg, 0.02mmol) and TFA (1mL) in DCM (4mL) was stirred for 0.5h at RT. Solvents were removed in vacuo yielding the title compound **AQ4** (23mg, 0.03mmol, quant.) as a yellow solid. MS (ES) C₂₉H₃₄N₆O requires: 482, found: 483 (M+H)⁺.

### Step 5: N-(5-(5-isobutyramido-2-(4-(4-methylpiperazin-1-yl)phenyl)-1H-pyrrolor2,3-b]pyridin-3-yl)-2-methylphenyl)acrylamide 2,2,2-trifluoroacetate (AQ5)

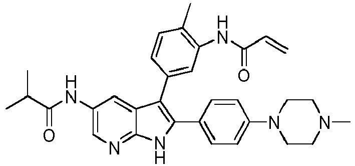

To a solution of **AQ4** (23mg, 0.03mmol, 1.0eq.) and DIPEA (47.4uL, 0.28mmol, 10.0eq.) in dry DCM (2mL) at -78°C was added acrylolyl chloride (3mg, 0.03mmol, 1.0eq.) in dry DCM (0.5mL). The mixture was stirred for 15min at -78°C and then 15min at 0°C. Again acrylolyl chloride (3mg, 0.03mmol, 1.0eq.) in dry DCM (0.5mL) was added. After 15min a few drops of water were added and the mixture was purified by reverse phase RP-HPLC (column: C18), using H₂O (0.1%TFA) and ACN (0.1%TFA) as eluents. The desired fractions were lyophilized to yield the title compound **AQ5** (4mg, 0.005mmol, 19%) as a yellow solid. ¹H NMR (400MHz, d₆-DMSO, 300K) δ 1.10 (d, J = 6.8 Hz, 6H), 2.27 (s, 3H), 2.58 (septet, J = 6.8 Hz, 1H), 2.85/2.86 (s, 3H), 2.98 (t, J = 13.0 Hz, 2H), 3.13 (m, 2H), 3.51 (d, J = 12.1 Hz, 2H), 3.91 (d, J = 13.3 Hz, 2H), 5.73 (dd, J = 2.0 Hz, J = 10.2 Hz, 1H), 6.20 (dd, J = 2.0 Hz, J = 17.0 Hz, 1H), 6.55 (dd, J = 10.2 Hz, J = 17.0 Hz, 1H), 6.98 (d, J = 9.0 Hz, 2H), 7.00 (m, 1H), 7.26 (d, J = 8.8 Hz, 1H), 7.43 (d, J = 9.0 Hz, 2H), 7.53 (s, 1H), 8.06 (d, J = 2.3 Hz, 1H), 8.36 (d, J = 2.3 Hz, 1H), 9.51 (s, 1H), 9.67 (br s, 1H), 9.82 (s, 1H), 11.87 (s, 1H). MS (ES) C₃₃H₃₆N₆O₂ requires: 536 found: 537 (M+H)⁺.

The Example in the following table was prepared according to the procedure described for **AQ5** (Example 200).

| **Example** | **Name** | **Mwt** | **[M+H]⁺** |
|---|---|---|---|
| 201 | 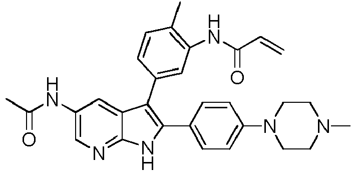 | 508 | 509 |

### Example 202:

### isopropyl 3-(5-acrylamido-2,4-dimethylphenyl)-2-(4-(4-methylpiperazin-1-yl)phenyl)-1H-pyrrolo[2,3-b]pyridine-5-carboxylate 2,2,2-trifluoroacetate (AR1)

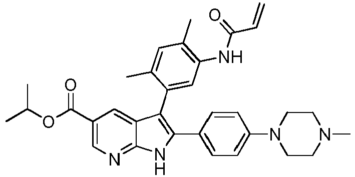

**AR1** was prepared from isopropyl 6-amino-5-iodonicotinate and ((2,4-dimethyl-5-nitrophenyl)ethynyl)trimethylsilane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AR1** as a yellow solid. ¹H NMR (400MHz, d₆-DMSO, 300K) δ 1.31 (d, J = 6.2 Hz, 3H), 1.32 (d, J = 6.2 Hz, 3H), 1.86 (s, 3H), 2.29 (s, 3H), 2.86/2.87 (s, 3H), 2.99 (t, J = 12.8 Hz, 2H), 3.10 (m, 2H), 3.50 (d, J = 12.1 Hz, 2H), 3.95 (d, J = 13.6 Hz, 2H), 5.15 (septet, J = 6.2 Hz, 1H), 5.73 (dd, J = 2.0 Hz, J = 10.0 Hz, 1H), 6.20 (dd, J = 2.0 Hz, J = 17.0 Hz, 1H), 6.54 (dd, J = 10.0 Hz, J = 17.0 Hz, 1H), 7.00 (d, J = 8.6 Hz, 2H), 7.22 (s, 1H), 7.43 (d, J = 8.6 Hz, 2H), 7.46 (s, 1H), 7.96 (d, J = 2.1 Hz, 1H), 8.81 (d, J = 2.1 Hz, 1H), 9.51 (s, 1H), 9.68 (br s, 1H), 12.51 (s, 1H). MS (ES) C₃₃H₃₇N₅O₃ requires: 551 found: 552 (M+H)⁺.

### Example 203:

### isopropyl 3-(3-(acrylamidomethyl)phenyl)-2-(4-(4-methylpiperazin-1-yl)phenyl)-1H-pyrrolo[2,3-b]pyridine-5-carboxylate 2,2,2-trifluoroacetate (AS1)

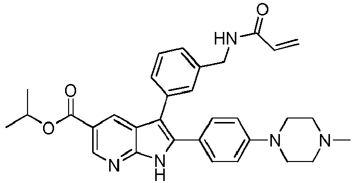

**AS1** was prepared from isopropyl 6-amino-5-iodonicotinate and tert-butyl 3-((trimethylsilyl)ethynyl)benzylcarbamate following the general procedure reported in Preparative Example 83. The crude product was purified by reverse phase RP-HPLC (column: C18), using H₂O (0.1%TFA) and ACN (0.1%TFA) as eluents. The desired fractions were lyophilized to yield the title compound **AS1** as a yellow solid. ¹H NMR (400MHz, d₆-DMSO, 300K) δ 1.34 (d, J = 6.2 Hz, 6H), 2.87/2.88 (s, 3H), 3.00 (t, J = 12.6 Hz, 2H), 3.13 (m, 2H), 3.51 (m, 2H), 3.94 (d, J = 13.5 Hz, 2H), 4.39 (d, J = 5.8 Hz, 2H), 5.16 (septet, J = 6.2 Hz, 1H), 5.61 (dd, J = 2.2 Hz, J = 10.2 Hz, 1H), 6.11 (dd, J = 2.2 Hz, J = 17.0 Hz, 1H), 6.26 (dd, J = 10.2 Hz, J = 17.0 Hz, 1H), 7.00 (d, J = 9.2 Hz, 2H), 7.23 (d, J = 7.6 Hz, 1H), 7.27 (d, J = 7.6 Hz, 1H), 7.34 (s, 1H), 7.42 (m, 3H), 8.24 (d, J = 2.1 Hz, 1H), 8.64 (t, J = 5.8 Hz, 1H), 8.83 (d, J = 2.1 Hz, 1H), 9.66 (br s, 1H), 12.51 (s, 1H). MS (ES) C₃₂H₃₅N₅O₃ requires: 537 found: 538 (M+H)⁺.

### Example 206:

### isopropyl 3-(3-acrylamido-4-ethylphenyl)-2-(4-(4-methylpiperazin-1-yl)phenyl)-1H-pyrrolo[2,3-b]pyridine-5-carboxylate 2,2,2-trifluoroacetate (AT1)

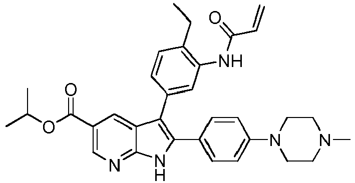

**AT1** was prepared from isopropyl 6-amino-5-iodonicotinate and ((4-ethyl-3-nitrophenyl)ethynyl)trimethylsilane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AT1** as a yellow solid. ¹H NMR (400MHz, d₆-DMSO, 300K) δ 1.18 (t, J = 7.5 Hz, 3H), 1.34 (d, J = 6.3 Hz, 6 H), 2.67 (q, J = 7.5 Hz, 2H), 2.87 (s, 3H), 3.01 (t, J =12.6 Hz, 2H), 3.11 (m, 2H), 3.54 (m, 2H), 3.96 (d, J = 13.4 Hz, 2H), 5.17 (septet, J = 6.3 Hz, 1H), 5.76 (d, J = 10.2 Hz, 1H), 6.24 (d, J = 17.1 Hz, 1H), 6.58 (dd, J = 10.2 Hz, J = 17.1 Hz, 1H), 7.02 (d, J = 8.5 Hz, 2H), 7.08 (d, J = 7.8 Hz, 1H), 7.29 (d, J = 7.8 Hz, 1H), 7.46 (d, J = 8.5 Hz, 2H), 7.61 (s, 1H), 8.38 (d, J = 2.0 Hz, 1H), 8.82 (d, J = 2.0 Hz, 1H), 9.95 (s, 1H), 9.69 (br s, 1H), 12.49 (s, 1H). MS (ES) C₃₂H₃₇N₅O₃ requires: 551 found: 552 (M+H)⁺.

### Example 207:

### isopropyl 3-(3-acrylamido-2-methylphenyl)-2-(4-(4-methylpiperazin-1-yl)phenyl)-1H-pyrrolo[2,3-b]pyridine-5-carboxylate2,2,2-trifluoroacetate (AU1)

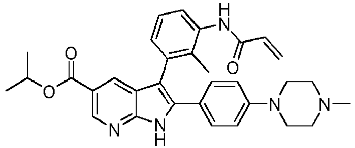

**AU1** was prepared from isopropyl 6-amino-5-iodonicotinate and trimethyl((2-methyl-3-nitrophenyl)ethynyl)silane following the general procedure reported in Preparative Example 1. The crude product was purified by reverse phase RP-HPLC (column: C18), using H₂O (0.1%TFA) and ACN (0.1%TFA) as eluents. The desired fractions were lyophilized to yield the title compound **AU1** as a yellow solid. ¹H NMR (400MHz, d₆-DMSO, 300K) δ 1.31 (d, J = 6.2 Hz, 6H), 1.88 (s, 3H), 2.85 (s, 3H), 2.98 (t, J = 12.6 Hz, 2H), 3.10 (m, 2H), 3.50 (m, 2H), 3.93 (d, J = 13.4 Hz, 2H), 5.14 (septet, J = 6.2 Hz, 1H), 5.75 (dd, J = 2.0 Hz, J = 10.1 Hz, 1H), 6.26 (dd, J = 2.0 Hz, J = 17.0 Hz, 1H), 6.55 (dd, J = 10.1 Hz, J = 17.0 Hz, 1H), 6.98 (d, J = 9.0 Hz, 2H), 7.10 (d, J = 7.8 Hz, 1H), 7.30 (t, J = 7.8 Hz, 1H), 7.39 (d, J = 9.0 Hz, 2H), 7.61 (d, J = 7.8 Hz, 1H), 7.88 (d, J = 2.0 Hz, 1H), 8.81 (d, J = 2.0 Hz, 1H), 9.58 (s, 1H), 9.68 (br s, 1H), 12.52 (s, 1H). MS (ES) C₃₂H₃₅N₅O₃ requires: 537 found: 538 (M+H)⁺.

The Examples in the following table were prepared according to the procedure described for **AU1** (Example 207).

| **Example** | **Name** | **Mwt** | **[M+H]⁺** |
|---|---|---|---|
| 208 | 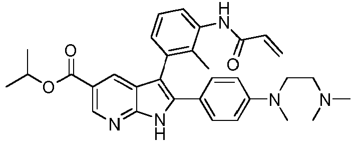 | 539 | 540 |
| 209 | 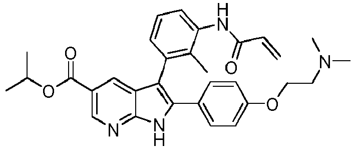 | 526 | 527 |

### Biological Assays

The exemplified compounds described herein were tested for activity and were found to have an IC₅₀ value less than 10uM, particularly less than 500nM, in one of the following assays:

### 1. Measurement of HER2 INS YVMA kinase activity

This protocol describes how the Lance Kinase Activity Assay was performed to determine IC₅₀ values of compounds of general formula (I) against HER2 INS YVMA. The principle behind this enzymatic assay is based upon the phosphorylation of the Ulight-peptide substrate. It is detected by using a specific EU-labeled anti-phospho peptide antibody. The binding of the Eu labeled anti-phospho peptide antibody to the phosphorylated ULight labeled peptide gives rise to a FRET-signal.

Binding of an inhibitor to the kinase prevents phosphorylation of the Ulight-substrate, resulting in a loss of FRET. In table 2 is summarized the relevant information for the LANCE assay.

**Table 2: Reagents, stock concentrations and final assay concentrations for Her2 INS YVMA.**

| Reagents | Stock concentration | Working concentration | Final assay concentration | Supplier |
|---|---|---|---|---|
| ULight^{™}-Poly GT substrate | 10 µM | 250 nM | 100 nM | Perkin Elmer |
| Eu-Anti-PT66 Antibody (AB) | 3125 nM | 4nM | 2 nM | Perkin Elmer |
| Her2 ins YVMA | 41,83 µM | 2,5 nM | 1 nM | DPF |
| ATP | 100 mM | 8 µM | 1,6 µM | Sigma |

The compounds of general formula (I) summarized in **Table 3** were serial diluted from a 10 mM DMSO stock solution 1:3 over 8 steps in a total volume of 20 µl.

For every sample, 8 µl of kinase-substrate mix was transferred into a suitable assay plate (e.g. Corning #3673). Compound was added via pintool transfer (10nl/well) using a Biomek FX robot (BeckmanCoulter). Reaction was started by addition of 2µl ATP working solution and mixed using variomag teleshaker (Thermo Fischer Scientific). After 1 h incubation at room temperature the reaction was stopped with 10µl detection mix containing the Eu-labeled phosphospecific antibody and 10mM EDTA. After a second incubation period of 1 h at room temperature the FRET signal was measured at 340 nm excitation, 665 nm and 615 nm emission (for the ULight-substrate and Eu-AB, respectively) with an Envision spectrophotometer (Perkin Elmer, Waltham, MA, USA) with 50 µs delay and 300 µs integration time. IC₅₀ values were determined from the sigmoidal dose response curves with the software Quattro Workflow (Quattro GmbH, Munich, Germany).

### 2. Measurement of cellular activity

The CellTiter-Glo Luminescent Cell Viability Assay (Promega) is a homogeneous method of determining the number of viable cells in culture. It is based on quantification of ATP, indicating the presence of metabolically active cells. Cells are seeded on day 1 at cell numbers that assure assay linearity and optimal signal intensity. After incubation for 24h in humidified chambers at 37°C and 5% CO₂, compounds in DMSO are added at different concentrations. Cells are further incubated for 72 h at 37°C and 5% CO₂. Cells treated with the compound vehicle DMSO are used as positive controls and cells treated with 10 µM Staurosporine serve as negative controls. At day 5 the CellTiter Glo Reagent is prepared according to the instructions of the kit (Promega Inc.): Reagent is mixed 1:1 with cell culture medium. Thereon, mixture and assay plates are equilibrated at room temperature for 20 min. Equal volumes of the reagent-medium-mixture is added to the volume of culture medium present in each well. The plates are mixed at ∼200 rpm for 2 minutes on an orbital shaker. The microplates are then incubated at room temperature for 10 minutes for stabilization of the luminescent signal. Following incubation the luminescence is recorded on a Victor microplate reader (Perkin Elmer) using a 200 ms integration time. The data is then analyzed with Excel using the XLFIT Plugin (dose response Fit 205) for IC₅₀-determination. As quality control the Z'-factor is calculated from 16 positive and negative control values. Only assay results showing a Z'-factor ≥ 0.5 are used for further analysis.

**Table 3** shows activity data in the biochemical Her2 Exon 20 INSYVMA Lance assay and cellular Her2 Exon 20 INSYVMA Ba/F3 CellTiter-Glo and EGFR Exon 20 INSNPH Ba/F3 CellTiter-Glo assays. Inhibition is indicated as IC₅₀ [nM] ("-" = not measured). Compounds having an activity designated as "A" provided an IC₅₀ ≤ 100nM; compounds having an activity designated as "B" provided an 100nM < IC₅₀ ≤ 500nM; compounds having an activity designated as "C" provided an 500nM < IC₅₀ ≤ 1000nM; compounds having an activity designated as "D" provided an 1000nM < IC₅₀ ≤ 10000nM; and compounds having an activity designated as "E" provided an IC₅₀ > 10000nM.

**Table 3:**

| **Example** | **Her2 Exon 20 INSYVMA Lance IC₅₀ / [nM]** | **Her2 Exon 20 INSYVMA Ba/F3 CTG IC₅₀ / [nM]** | **EGFR Exon 20 INSNPH Ba/F3 CTG IC₅₀ / [nM]** |
|---|---|---|---|
| **1** | A | B | A |
| **2** | A | A | A |
| **3** | A | A | A |
| **4** | A | B | B |
| **5** | A | B | B |
| **6** | C | C | c |
| **7** | c | c | D |
| **8** | A | B | B |
| **9** | A | B | B |
| **10** | A | A | A |
| **11** | A | B | B |
| **12** | B | B | B |
| **13** | A | B | B |
| **14** | B | C | C |
| **15** | B | B | B |
| **16** | A | B | B |
| **17** | A | B | B |
| **18** | A | B | A |
| **19** | B | C | C |
| **20** | A | C | C |
| **21** | B | C | C |
| **22** | B | C | D |
| **23** | A | B | B |
| **24** | B | C | C |
| **25** | A | B | B |
| **26** | A | B | B |
| **27** | C | D | D |
| **28** | A | B | B |
| **29** | A | C | C |
| **30** | B | D | C |
| **31** | B | D | D |
| **32** | B | C | C |
| **33** | D | D | D |
| **34** | D | D | D |
| **35** | B | C | C |
| **36** | C | D | D |
| **37** | A | B | B |
| **38** | A | A | A |
| **39** | B | C | D |
| **40** | A | A | A |
| **41** | A | A | A |
| **42** | A | B | B |
| **43** | A | A | A |
| **44** | B | D | D |
| **45** | E | D | D |
| **46** | D | D | D |
| **47** | A | D | D |
| **48** | A | B | B |
| **49** | c | C | D |
| **50** | E | D | D |
| **51** | B | C | C |
| **52** | B | D | D |
| **53** | A | D | D |
| **54** | B | D | D |
| **55** | A | C | C |
| **56** | A | B | B |
| **57** | A | B | C |
| **58** | A | D | D |
| **59** | A | B | B |
| **60** | A | B | B |
| **61** | A | B | B |
| **62** | A | B | B |
| **63** | A | B | B |
| **64** | A | B | B |
| **65** | A | B | C |
| **66** | A | B | C |
| **67** | A | B | B |
| **68** | A | C | D |
| **69** | A | B | B |
| **70** | A | B | C |
| **71** | A | B | C |
| **72** | A | A | A |
| **73** | A | A | A |
| **74** | A | C | B |
| **75** | A | B | A |
| **76** | A | B | B |
| **77** | A | B | B |
| **78** | B | B | C |
| **79** | A | B | B |
| **80** | C | D | D |
| **81** | D | D | D |
| **82** | D | D | D |
| **83** | A | B | B |
| **84** | A | B | B |
| **85** | A | B | B |
| **86** | A | C | C |
| **87** | A | C | C |
| **88** | A | D | D |
| **89** | A | B | B |
| **90** | A | C | D |
| **91** | A | C | C |
| **92** | A | C | D |
| **93** | B | C | D |
| **94** | A | D | D |
| **95** | A | D | D |
| **96** | A | B | B |
| **97** | A | B | A |
| **98** | A | B | B |
| **99** | A | B | B |
| **100** | A | B | C |
| **101** | A | B | B |
| **102** | A | B | B |
| **103** | A | B | B |
| **104** | A | B | B |
| **105** | A | B | B |
| **106** | A | B | B |
| **107** | A | B | B |
| **108** | A | B | B |
| **109** | A | B | B |
| **110** | A | B | B |
| **111** | A | A | A |
| **112** | A | C | C |
| **113** | A | A | A |
| **114** | - | - | - |
| **115** | A | B | B |
| **116** | A | E | E |
| **117** | A | A | A |
| **118** | A | B | B |
| **119** | A | B | B |
| **120** | A | B | B |
| **121** | A | E | E |
| **122** | A | D | D |
| **123** | C | D | D |
| **124** | B | B | C |
| **125** | D | D | D |
| **126** | B | E | E |
| **127** | A | D | D |
| **128** | A | D | D |
| **129** | C | E | E |
| **130** | B | E | E |
| **131** | B | C | D |
| **132** | C | E | E |
| **133** | B | E | E |
| **134** | A | B | B |
| **135** | A | B | B |
| **136** | A | B | B |
| **137** | B | C | C |
| **138** | A | B | B |
| **139** | B | C | C |
| **140** | A | B | B |
| **141** | A | C | C |
| **142** | C | D | D |
| **143** | A | B | B |
| **144** | A | D | D |
| **145** | A | C | C |
| **146** | A | B | B |
| **147** | A | C | C |
| **148** | A | B | C |
| **149** | A | B | B |
| **150** | A | C | B |
| **151** | A | B | B |
| **152** | A | B | B |
| **153** | A | E | E |
| **154** | A | B | B |
| **155** | A | C | B |
| **156** | A | B | B |
| **157** | A | B | B |
| **158** | A | B | B |
| **159** | A | B | B |
| **160** | A | B | B |
| **161** | A | B | B |
| **162** | A | D | C |
| **163** | A | C | C |
| **164** | A | B | B |
| **165** | A | B | B |
| **166** | C | D | D |
| **167** | B | C | C |
| **168** | B | C | C |
| **169** | A | C | B |
| **170** | A | B | B |
| **171** | D | D | D |
| **172** | A | B | B |
| **173** | E | D | D |
| **174** | A | C | B |
| **175** | B | D | D |
| **176** | A | B | B |
| **177** | A | B | B |
| **178** | B | D | C |
| **179** | A | B | B |
| **180** | A | B | B |
| **181** | A | C | C |
| **182** | A | B | B |
| **183** | A | B | B |
| **184** | C | D | D |
| **185** | D | D | D |
| **186** | B | D | D |
| **187** | A | B | B |
| **188** | A | B | B |
| **189** | A | E | E |
| **190** | A | B | B |
| **191** | A | B | B |
| **192** | A | B | C |
| **193** | A | A | B |
| **194** | A | D | D |
| **195** | A | B | B |
| **196** | A | A | D |
| **197** | A | D | B |
| **198** | A | A | A |
| **199** | A | C | B |
| **200** | A | B | B |
| **201** | A | D | D |
| **202** | D | D | D |
| **203** | D | D | D |
| **204** | A | A | A |
| **205** | A | B | A |
| **206** | - | - | - |
| **207** | - | - | - |
| **208** | - | - | - |
| **209** | - | - | - |

**Table 4** shows activity data in the biochemical EGFR T790ML858R Lance and EGFR wt Lance assays and the cellular H1975 CellTiter-Glo, H1781 CellTiter-Glo and A431 CellTiter-Glo assays. Inhibition is indicated as IC₅₀ [nM] ("-" = not measured). Compounds having an activity designated as "A" provided an IC₅₀ ≤ 100nM; compounds having an activity designated as "B" provided an 100nM < IC₅₀ ≤ 500nM; compounds having an activity designated as "C" provided an 500nM < IC₅₀ ≤ 1000nM; compounds having an activity designated as "D" provided an 1000nM < IC₅₀ ≤ 10000nM; and compounds having an activity designated as "E" provided an IC₅₀ > 10000nM.

**Table 4:**

| **Example** | **EGFR T790M L858R Lance IC₅₀ [nM]** | **EGFR wt Lance IC₅₀ [nM]** | **H1975 CTG IC₅₀ [nM]** | **H1781 CTG IC₅₀ [nM]** | **A431 CTG IC₅₀ [nM]** |
|---|---|---|---|---|---|
| **1** | A | A | A | B | B |
| **2** | A | A | A | B | B |
| **3** | A | A | A | B | B |
| **4** | A | A | A | B | B |
| **5** | A | A | A | E | E |
| **6** | A | A | B | - | - |
| **7** | A | B | B | - | - |
| **8** | A | A | A | B | B |
| **9** | A | A | A | - | - |
| **10** | A | A | A | B | C |
| **11** | A | A | A | C | B |
| **12** | A | A | A | - | - |
| **13** | A | A | A | B | B |
| **14** | A | A | B | - | - |
| **15** | A | A | A | C | B |
| **16** | A | A | A | C | C |
| **17** | A | A | A | B | B |
| **18** | A | A | A | B | B |
| **19** | A | A | B | - | D |
| **20** | A | A | A | - | - |
| **21** | A | A | A | - | - |
| **22** | A | B | B | - | - |
| **23** | A | A | A | B | B |
| **24** | A | B | B | - | - |
| **25** | A | A | A | C | B |
| **26** | A | A | A | C | B |
| **27** | A | B | B | - | - |
| **28** | A | A | A | B | B |
| **29** | A | A | A | - | - |
| **30** | A | A | B | - | - |
| **31** | A | C | B | - | - |
| **32** | A | A | A | - | - |
| **33** | B | C | B | - | - |
| **34** | B | D | C | - | - |
| **35** | A | A | A | - | - |
| **36** | A | A | B | - | D |
| **37** | A | A | A | - | B |
| **38** | A | A | A | - | B |
| **39** | A | A | B | - | D |
| **40** | A | A | A | - | B |
| **41** | A | A | A | - | B |
| **42** | A | A | A | - | B |
| **43** | A | A | A | - | B |
| **44** | A | B | B | - | - |
| **45** | B | D | D | - | - |
| **46** | A | C | D | - | - |
| **47** | A | A | A | - | C |
| **48** | A | A | A | B | B |
| **49** | A | B | B | - | - |
| **50** | B | D | C | - | - |
| **51** | A | A | A | - | - |
| **52** | A | B | B | - | - |
| **53** | A | A | B | - | - |
| **54** | A | A | B | - | D |
| **55** | A | A | A | D | D |
| **56** | A | A | A | C | C |
| **57** | A | A | B | D | D |
| **58** | A | A | B | D | D |
| **59** | A | A | A | B | B |
| **60** | A | A | B | C | C |
| **61** | A | A | A | C | B |
| **62** | A | A | A | - | C |
| **63** | A | A | A | - | C |
| **64** | A | A | A | - | C |
| **65** | A | A | A | - | C |
| **66** | A | A | B | - | D |
| **67** | A | A | B | - | D |
| **68** | A | A | B | - | D |
| **69** | A | A | A | - | C |
| **70** | A | A | B | - | C |
| **71** | A | A | B | - | C |
| **72** | A | A | A | B | B |
| **73** | A | A | A | B | B |
| **74** | A | A | A | - | C |
| **75** | A | A | A | B | B |
| **76** | A | A | A | C | C |
| **77** | A | A | A | B | B |
| **78** | A | A | A | C | B |
| **79** | A | A | A | C | B |
| **80** | A | B | B | - | D |
| **81** | A | B | D | - | D |
| **82** | A | B | C | - | D |
| **83** | A | A | B | D | D |
| **84** | A | A | B | - | C |
| **85** | A | A | B | - | C |
| **86** | A | A | B | D | D |
| **87** | A | A | B | - | D |
| **88** | A | A | B | - | D |
| **89** | A | A | B | - | C |
| **90** | A | A | B | - | D |
| **91** | A | A | B | - | D |
| **92** | A | A | C | - | D |
| **93** | A | A | C | - | D |
| **94** | A | A | B | - | D |
| **95** | A | A | C | - | D |
| **96** | A | A | A | B | B |
| **97** | A | A | A | - | B |
| **98** | A | A | B | - | C |
| **99** | A | A | B | - | D |
| **100** | A | A | B | - | D |
| **101** | A | A | B | - | D |
| **102** | A | A | B | - | D |
| **103** | A | A | B | - | C |
| **104** | A | A | A | - | C |
| **105** | A | A | A | - | B |
| **106** | A | A | A | - | C |
| **107** | A | A | A | - | C |
| **108** | A | A | A | - | C |
| **109** | A | A | A | - | C |
| **110** | A | A | A | - | C |
| **111** | A | A | A | - | B |
| **112** | A | A | B | - | D |
| **113** | A | A | A | - | B |
| **114** | - | - | - | - | - |
| **115** | A | A | A | - | C |
| **116** | A | A | E | - | E |
| **117** | A | A | A | - | B |
| **118** | A | A | C | - | C |
| **119** | A | A | C | - | C |
| **120** | A | A | B | - | B |
| **121** | B | A | E | - | E |
| **122** | A | A | D | - | D |
| **123** | A | A | E | - | D |
| **124** | A | A | C | - | C |
| **125** | B | A | B | - | D |
| **126** | A | A | E | - | E |
| **127** | A | A | D | - | D |
| **128** | A | A | D | - | D |
| **129** | B | B | E | - | E |
| **130** | B | A | E | - | E |
| **131** | C | A | D | - | D |
| **132** | B | A | E | - | E |
| **133** | B | A | E | - | E |
| **134** | A | A | B | - | D |
| **135** | A | A | A | - | D |
| **136** | A | A | B | - | D |
| **137** | A | A | B | - | D |
| **138** | A | A | A | - | D |
| **139** | A | A | B | - | D |
| **140** | A | A | B | - | D |
| **141** | A | A | B | - | D |
| **142** | A | A | B | - | D |
| **143** | A | A | B | - | D |
| **144** | A | A | D | - | D |
| **145** | A | A | B | - | D |
| **146** | A | A | A | - | B |
| **147** | A | A | A | - | - |
| **148** | A | A | C | - | D |
| **149** | A | A | B | - | D |
| **150** | A | A | A | - | C |
| **151** | A | A | B | - | D |
| **152** | A | A | A | - | C |
| **153** | A | A | E | - | E |
| **154** | A | A | B | - | D |
| **155** | A | A | A | - | D |
| **156** | A | A | C | - | D |
| **157** | A | A | B | - | D |
| **158** | A | A | B | - | D |
| **159** | A | A | B | - | D |
| **160** | A | A | B | - | D |
| **161** | A | A | B | - | D |
| **162** | A | A | D | - | D |
| **163** | A | A | C | - | D |
| **164** | A | A | B | - | D |
| **165** | A | A | B | - | D |
| **166** | B | A | D | - | D |
| **167** | A | A | C | - | D |
| **168** | B | A | D | - | D |
| **169** | A | A | D | - | D |
| **170** | A | A | B | - | D |
| **171** | C | A | D | - | D |
| **172** | A | A | B | - | D |
| **173** | D | C | D | - | D |
| **174** | A | A | C | - | D |
| **175** | B | A | D | - | D |
| **176** | A | A | B | - | D |
| **177** | A | A | B | - | D |
| **178** | B | A | D | - | D |
| **179** | A | A | B | - | D |
| **180** | A | A | C | - | D |
| **181** | A | A | C | - | D |
| **182** | A | A | B | - | D |
| **183** | A | A | B | - | D |
| **184** | B | A | D | - | E |
| **185** | C | A | D | - | E |
| **186** | B | A | D | - | D |
| **187** | A | A | B | - | D |
| **188** | A | A | B | - | D |
| **189** | A | A | E | - | E |
| **190** | A | A | B | - | D |
| **191** | A | A | A | - | D |
| **192** | A | A | D | - | D |
| **193** | A | A | B | - | D |
| **194** | A | A | D | - | D |
| **195** | A | A | B | - | D |
| **196** | A | A | D | - | D |
| **197** | A | A | C | - | D |
| **198** | A | A | B | - | C |
| **199** | A | A | B | - | D |
| **200** | A | A | D | - | D |
| **201** | A | A | D | - | D |
| **202** | B | A | D | - | E |
| **203** | C | B | D | - | D |
| **204** | A | A | B | - | D |
| **205** | A | A | B | - | D |
| **206** | - | - | - | - | - |
| **207** | - | - | - | - | - |
| **208** | - | - | - | - | - |
| **209** | - | - | - | - | - |

### 3. Metabolic stability

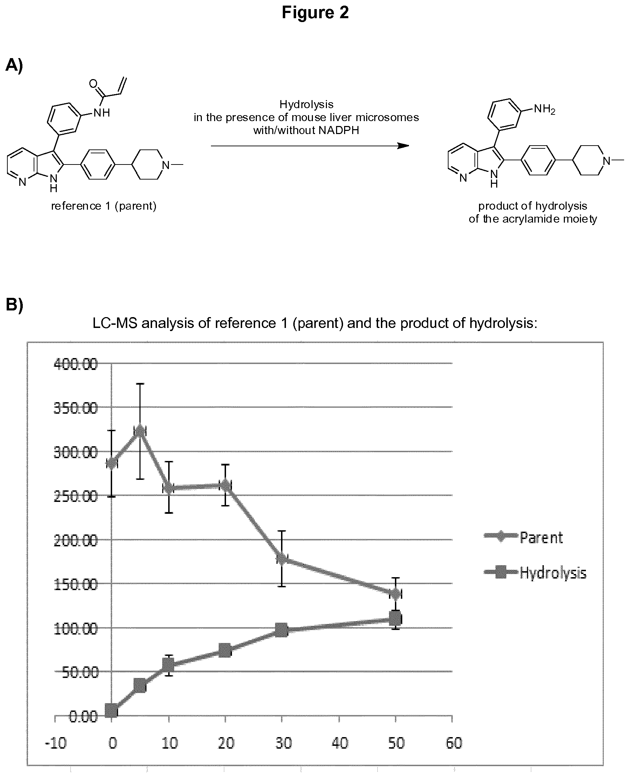

There are two major groups of enzyme reactions catalyzed by drug metabolizing enzymes, the so called Phase I and Phase II reactions. The basic processes in phase I reactions are oxidation, reduction and/or hydrolysis mostly catalyzed by the cytochrome P450 (CYP) family of enzymes. Exploring the metabolic stability of reference 1 we have observed as a major metabolite the hydrolysis of the acrylamide moiety in the presence of mouse liver microsomes with and without the cofactor NADPH. Figure 2 shows the hydrolysis of the acrylamide moiety of reference 1 in the presence of mouse liver microsomes after 50 minutes.

The following procedures describe the determination of the in vitro Phase I metabolic stability of test compounds using liver microsomes by measuring compound depletion over time. Test compound at the concentration of 3 |jM was incubated with the liver microsomes from the mouse species and the experiment was performed without NADPH. The depletion of the test compounds was measured over time up to 50 minutes at 37 °C by LC-MS/MS. Different compounds were tested under these conditions for microsomal stability (see Table 5). Prevention of hydrolysis of the acrylamide moiety was achieved either by the introduction of substituents like a methyl group (as in Example 1) next to the acrylamide moiety or by modifying the secondary acrylamide of reference 1 to a tertiary acrylamide (like Example 55).

**Table 5: Stability without cofactor NADPH in mouse liver microsomes**

| **Example** | **Structure** | **Stability in presence of mouse liver microsomes without cofactor NADPH [% remaining after 50min]** |
|---|---|---|
| **Reference 1** | 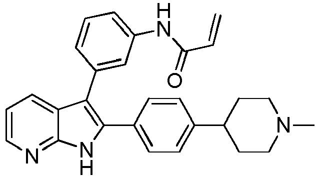 | 33 |
| **Reference 2** | 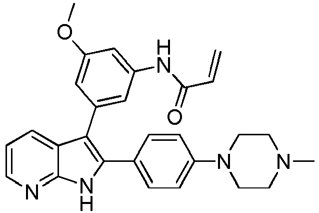 | 48 |
| **1** | 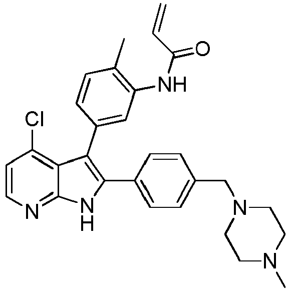 | >99 |
| **2** | 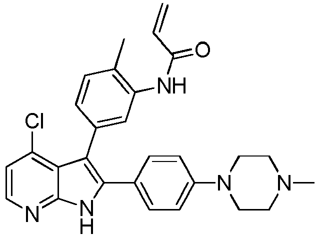 | >99 |
| **3** | 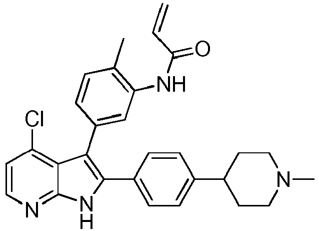 | >99 |
| **4** | 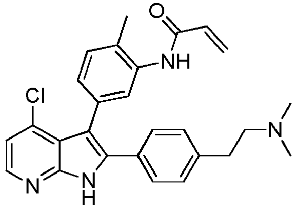 | >99 |
| **5** | 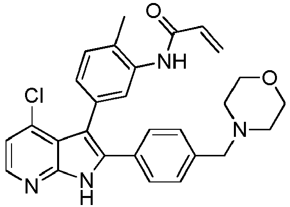 | >99 |
| **6** | 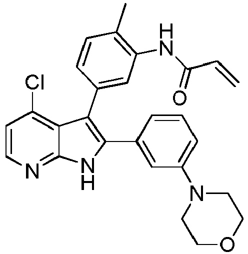 | >99 |
| **7** | 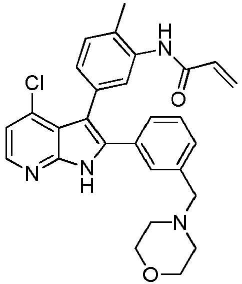 | >99 |
| **8** | 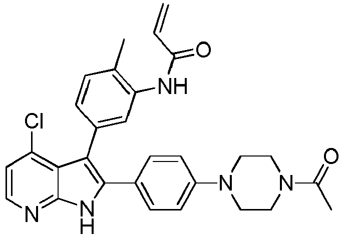 | >99 |
| **9** | 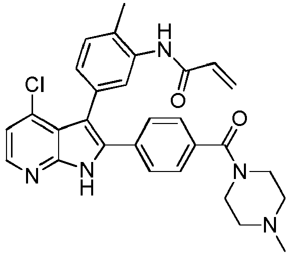 | >99 |
| **10** | 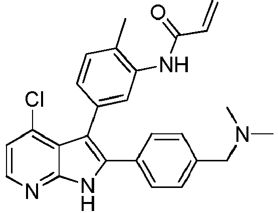 | >99 |
| **11** | 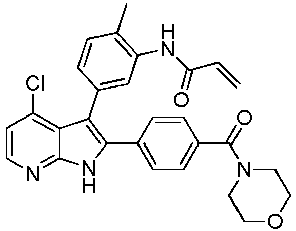 | >99 |
| **12** | 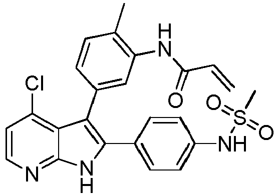 | >99 |
| **13** | 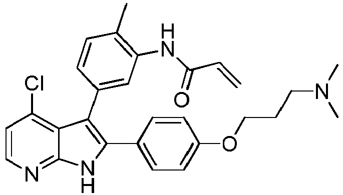 | 95 |
| **14** | 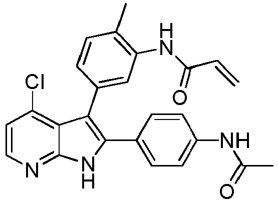 | 68 |
| **15** | 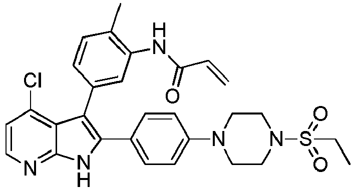 | >99 |
| **16** | 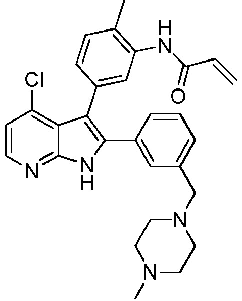 | >99 |
| **17** | 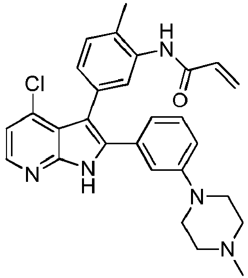 | 90 |
| **18** | 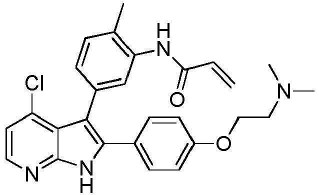 | 90 |
| **19** | 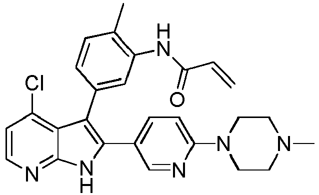 | >99 |
| **20** | 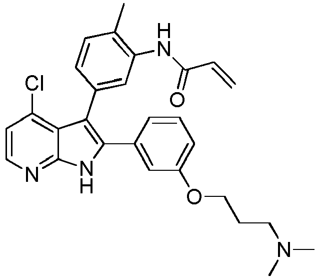 | >99 |
| **21** | 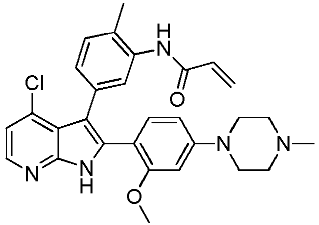 | 93 |
| **22** | 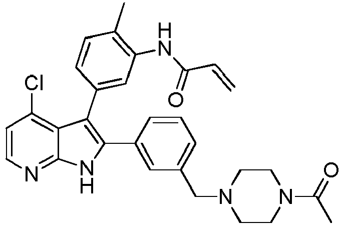 | >99 |
| **23** | 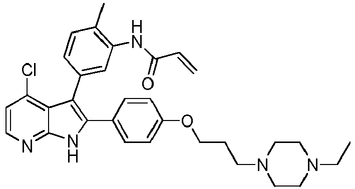 | >99 |
| **24** | 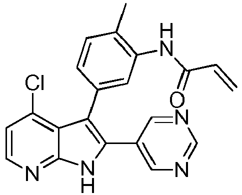 | >99 |
| **25** | 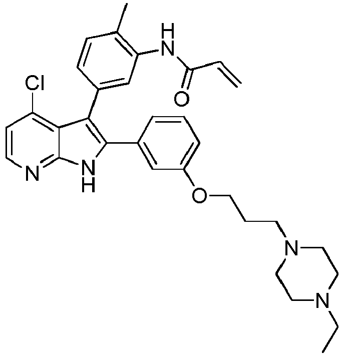 | >99 |
| **26** | 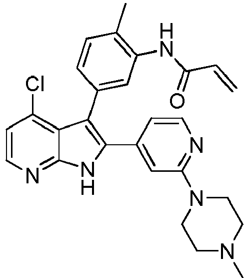 | >99 |
| **27** | 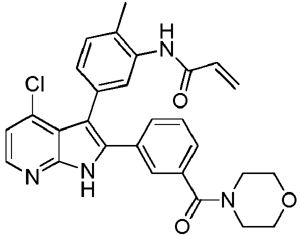 | >99 |
| **28** | 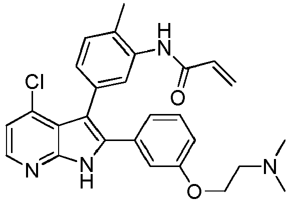 | >99 |
| **29** | 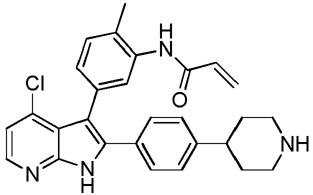 | >99 |
| **30** | 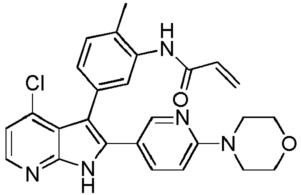 | >99 |
| **31** | 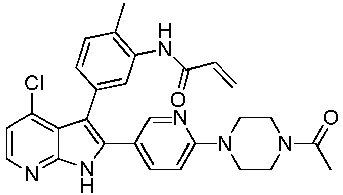 | >99 |
| **32** | 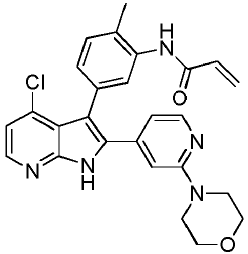 | >99 |
| **36** | 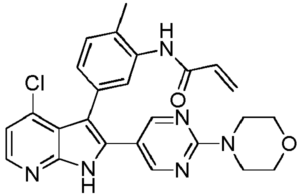 | >99 |
| **37** | 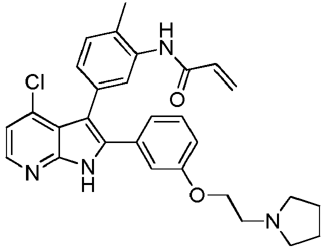 | >99 |
| **38** | 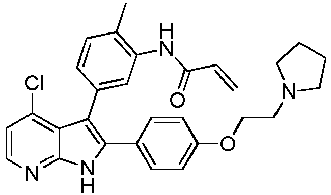 | >99 |
| **39** | 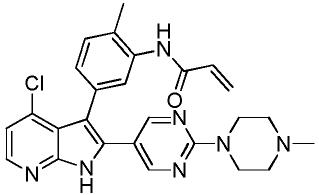 | >99 |
| **40** | 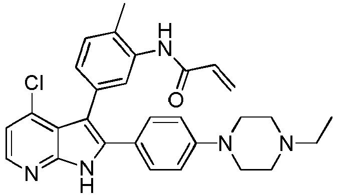 | >99 |
| **41** | 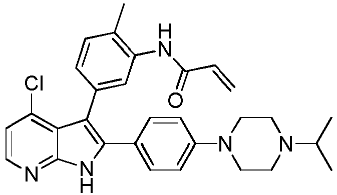 | >99 |
| **42** | 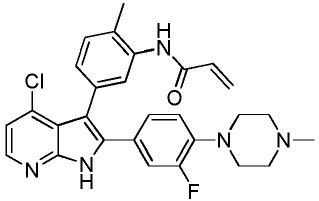 | >99 |
| **43** | 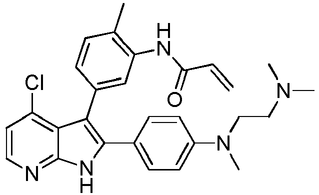 | >99 |
| **55** | 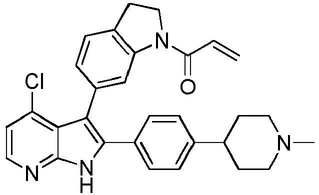 | >99 |
| **56** | 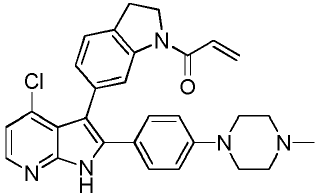 | >99 |
| **57** | 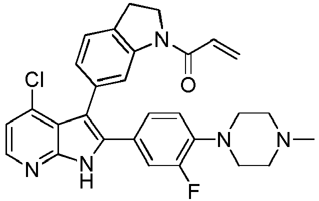 | >99 |
| **58** | 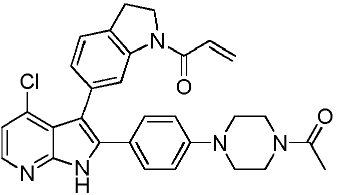 | >99 |
| **59** | 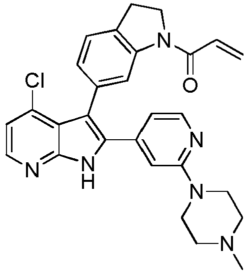 | >99 |
| **60** | 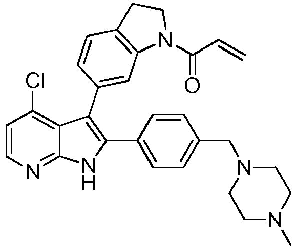 | >99 |
| **61** | 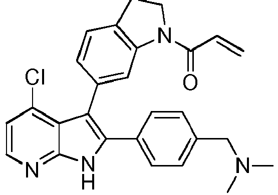 | >99 |
| **62** | 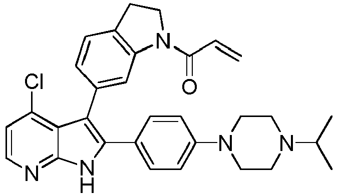 | >99 |
| **63** | 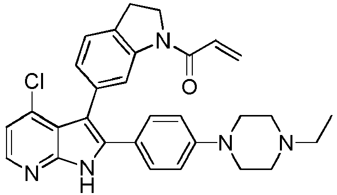 | >99 |
| **64** | 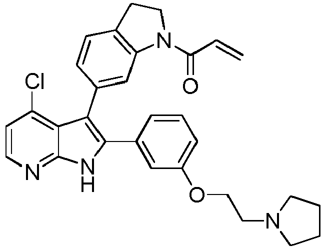 | >99 |
| **65** | 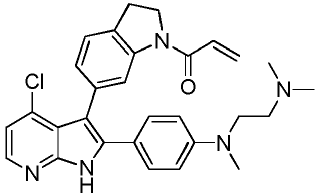 | >99 |
| **66** | 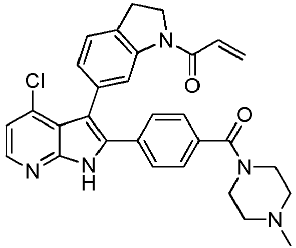 | >99 |
| **67** | 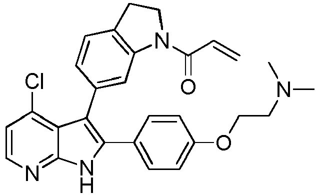 | >99 |
| **68** | 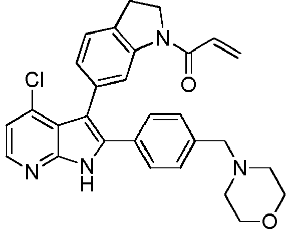 | >99 |
| **69** | 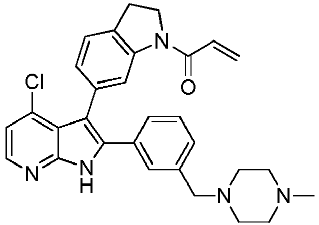 | 98 |
| **70** | 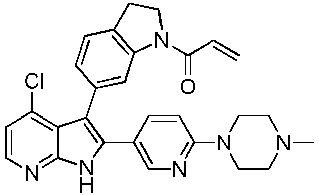 | >99 |
| **71** | 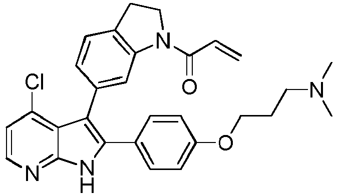 | >99 |
| **72** | 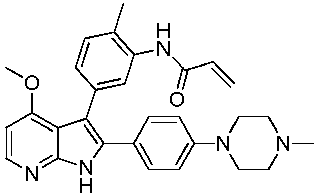 | >99 |
| **73** | 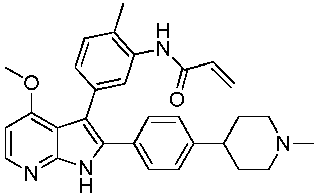 | 99 |
| **74** | 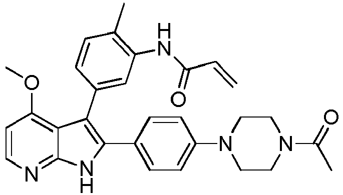 | 90 |
| **75** | 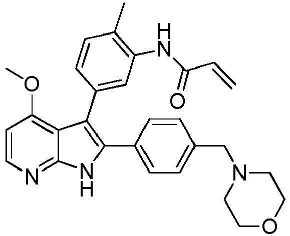 | >99 |
| **76** | 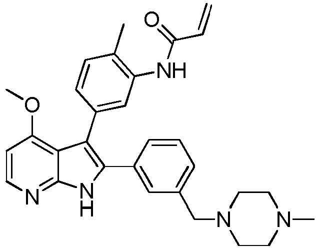 | >99 |
| **77** | 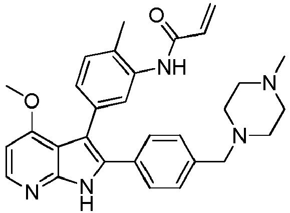 | >99 |
| **78** | 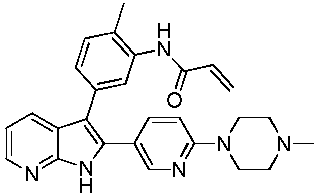 | >99 |
| **79** | 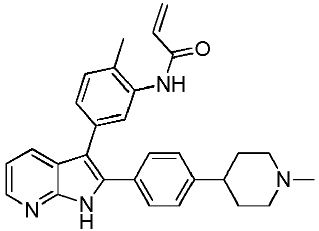 | >99 |
| **80** | 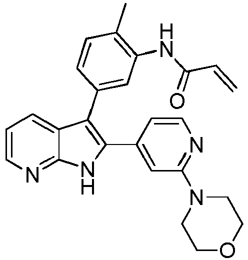 | >99 |
| **81** | 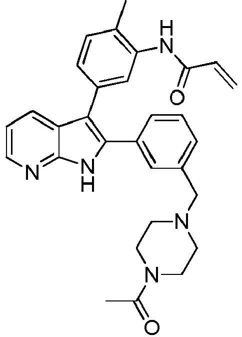 | 80 |
| **82** | 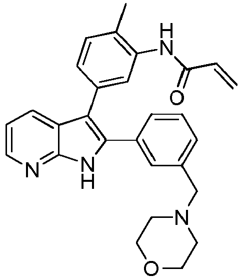 | 97 |
| **83** | 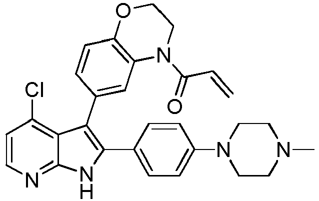 | >99 |
| **84** | 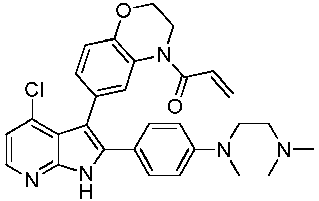 | 94 |
| **85** | 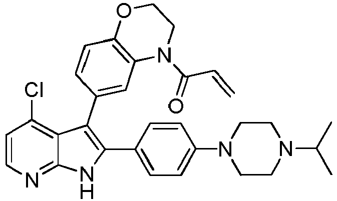 | >99 |
| **86** | 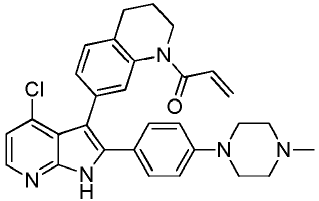 | >99 |
| **88** | 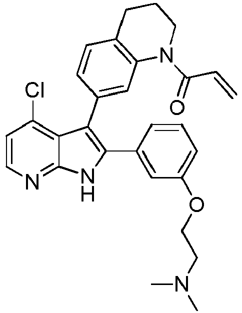 | >99 |
| **91** | 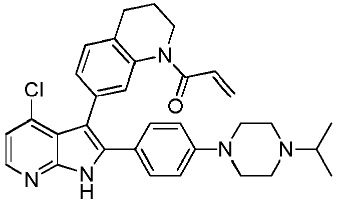 | >99 |
| **92** | 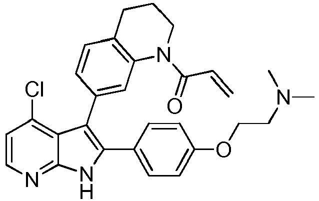 | >99 |
| **93** | 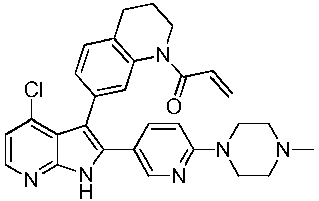 | >99 |
| **95** | 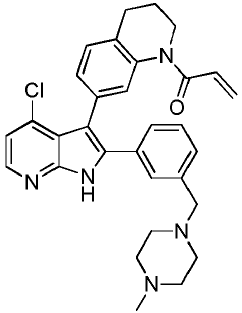 | >99 |
| **96** | 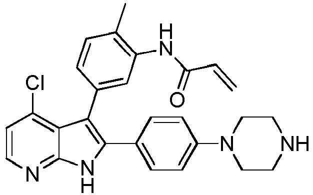 | 92 |
| **97** | 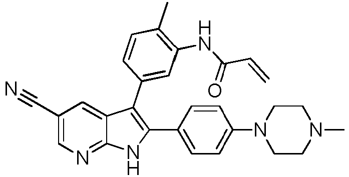 | >99 |
| **98** | 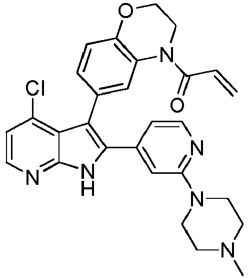 | >99 |
| **99** | 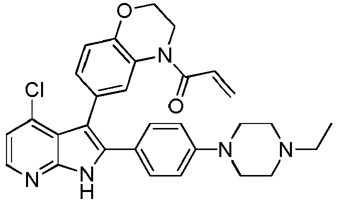 | >99 |
| **100** | 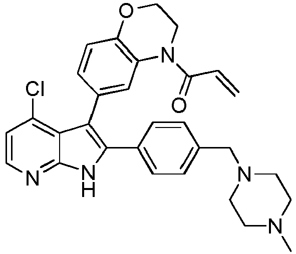 | >99 |
| **104** | 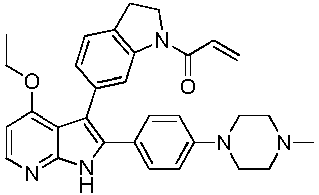 | 89 |
| **108** | 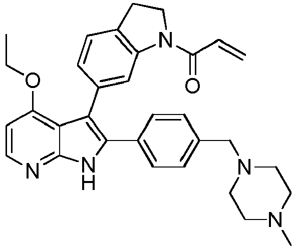 | 86 |
| **109** | 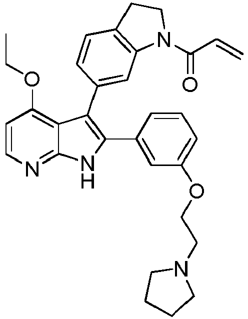 | 77 |
| **110** | 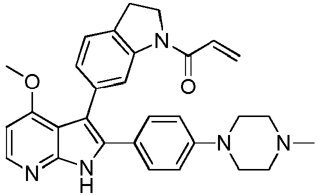 | 93 |
| **111** | 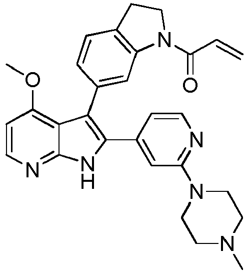 | 90 |
| **112** | 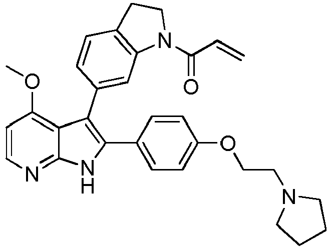 | 79 |
| **113** | 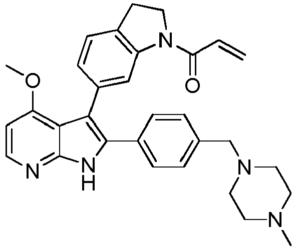 | 96 |
| **115** | 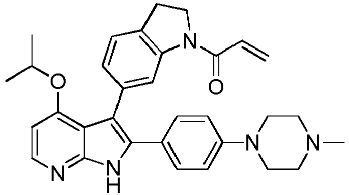 | >99 |
| **116** | 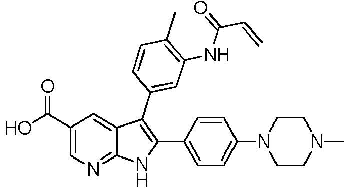 | 92 |
| **117** | 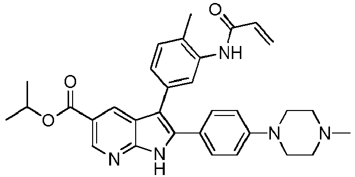 | 100 |
| **119** | 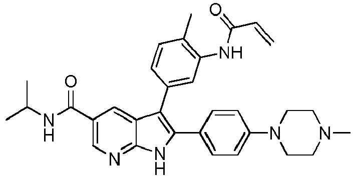 | >99 |
| **120** | 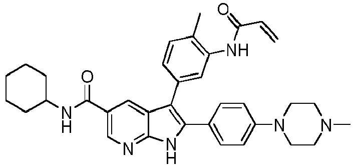 | >99 |
| **134** | 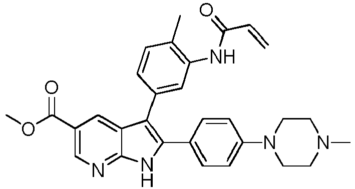 | >99 |
| **135** | 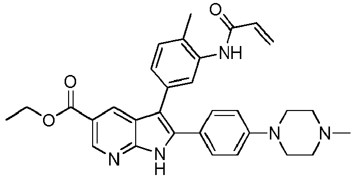 | 95 |
| **138** | 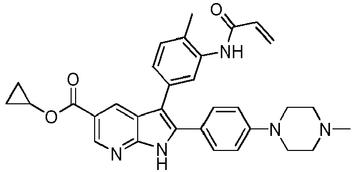 | >99 |
| **146** | 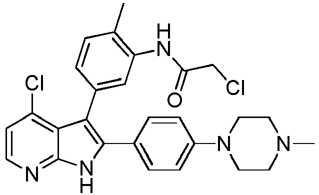 | 9 |
| **147** | 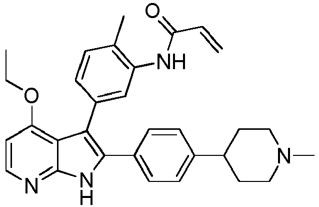 | 94 |
| **154** | 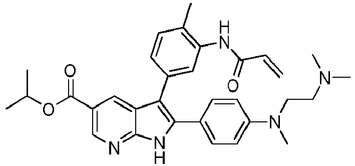 | >99 |
| **155** | 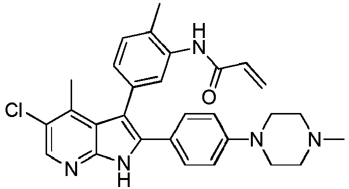 | >99 |
| **157** | 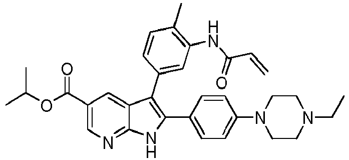 | >99 |
| **158** | 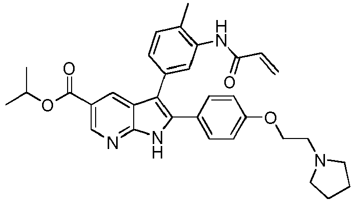 | >99 |
| **159** | 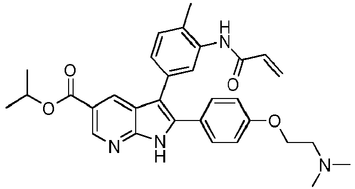 | >99 |
| **160** | 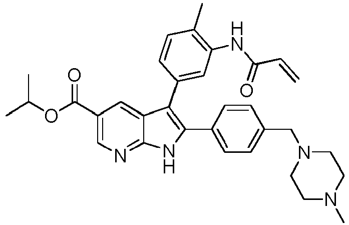 | >99 |
| **161** | 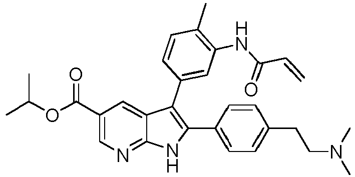 | >99 |
| **164** | 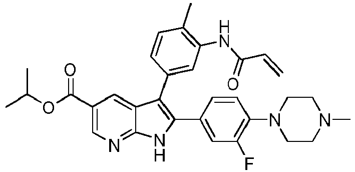 | >99 |
| **165** | 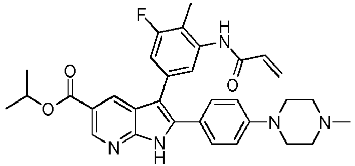 | >99 |
| **170** | 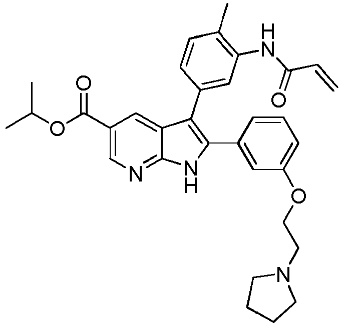 | >99 |
| **172** | 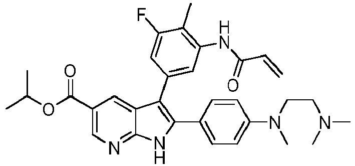 | >99 |
| **176** | 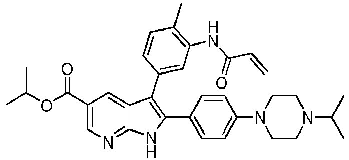 | >99 |
| **177** | 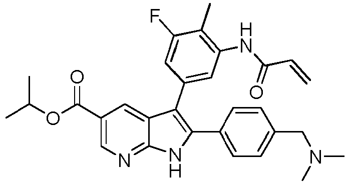 | >99 |
| **179** | 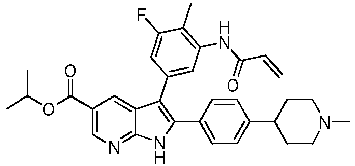 | >99 |
| **182** | 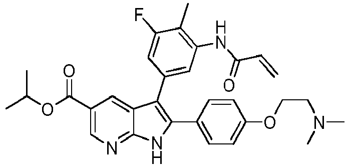 | >99 |
| **187** | 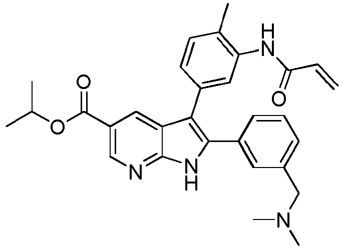 | >99 |

### 4. SAR

The structure activity relationship (SAR) of the compounds of the present invention as represented by the Formula (Ia and Ib) shows covalent inhibitor as cellular potent mutant-selective ErbB inhibitors. The covalent binding mode, obtained for example through the introduction of an acrylic moiety, is crucial for improved biochemical and cellular activity. The direct comparison of examples from this invention having a acryl moiety for the covalent binding with corresponding molecules having a propionic moiety are showing the improved cellular activities for the covalent binder.

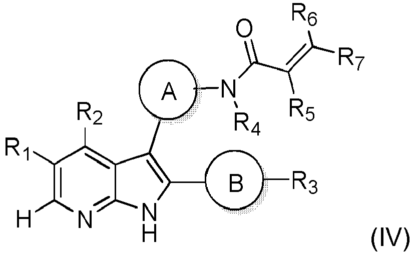

**Table 6** shows comparison of covalent inhibitors with the corresponding reversible analogues. In all cases the covalent inhibitor shows improved cellular activities compared to the reversible analogues.

**Table 6: Comparison covalent inhibitors with corresponding reversible one.**

| **Binding Mode** | **Structure** | **Her2 Exon 20 INSYVMA Lance IC₅₀ [nM]** | **Her2 Exon 20 INSYVM A Ba/F3 CTG IC₅₀ [nM]** | **EGFR Exon 20 INSNPH Ba/F3 CTG IC₅₀ [nM]** |
|---|---|---|---|---|
| Covalent | 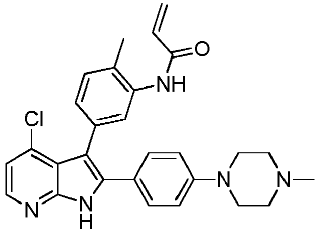 | 5 | 64 | 35 |
| | Example 2 | | | |
| Reversibel | 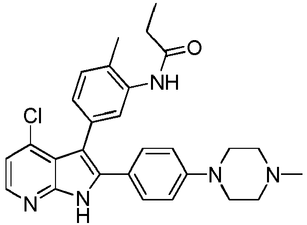 | 916 | 754 | 1311 |
| | Reference 3 | | | |
| Covalent | 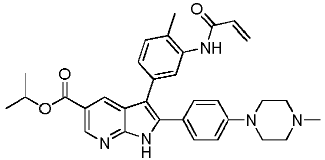 | 13 | 208 | 200 |
| | Example 148 | | | |
| Reversibel | 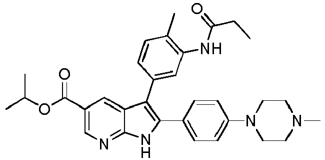 | 4023 | 2391 | 2344 |
| | Reference 4 | | | |

## Claims

1. A compound of the formula (I) 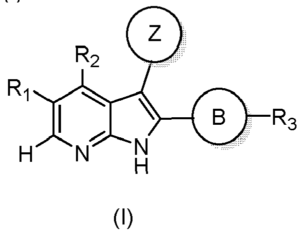 wherein
**Z** represents 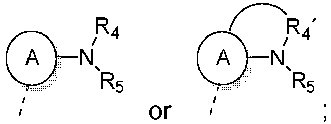
**A** represents 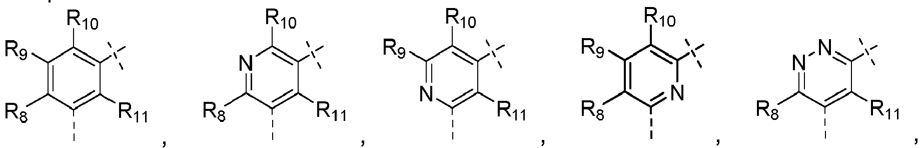 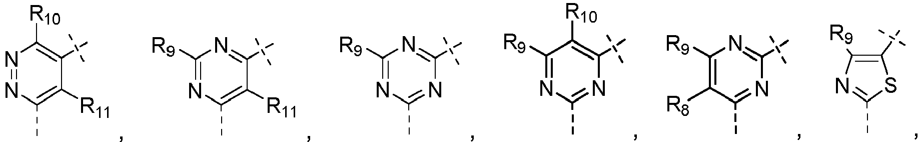 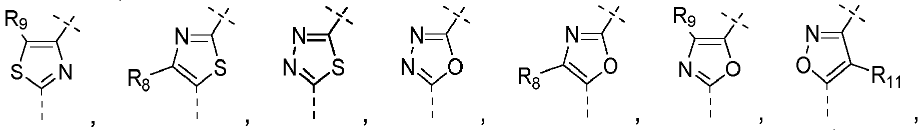 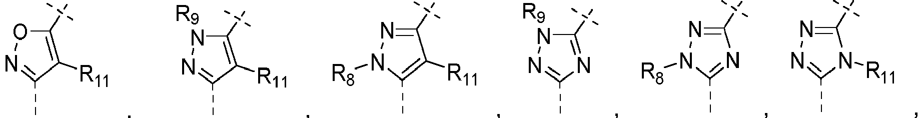 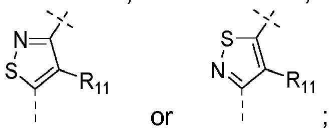 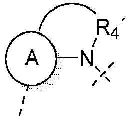 represents 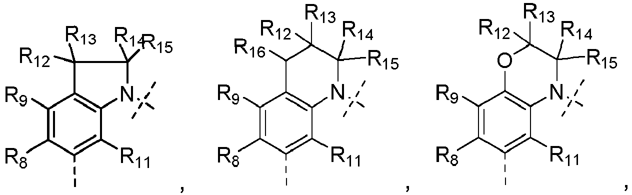 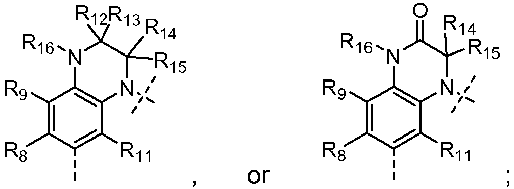
B represents 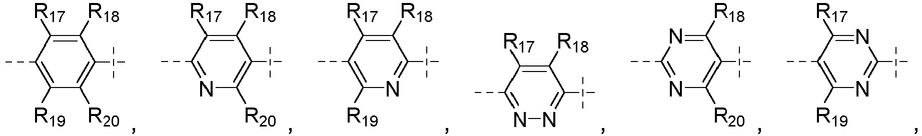 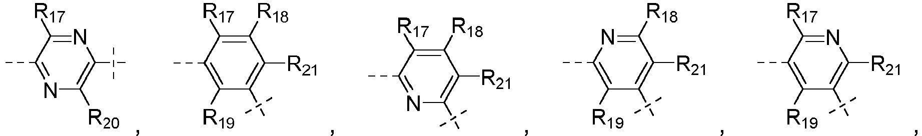 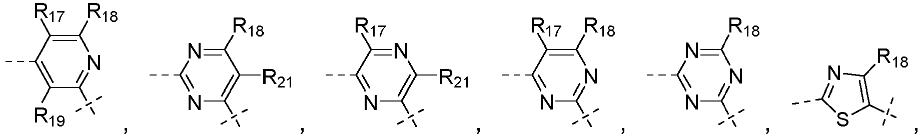 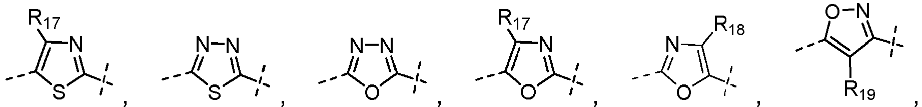 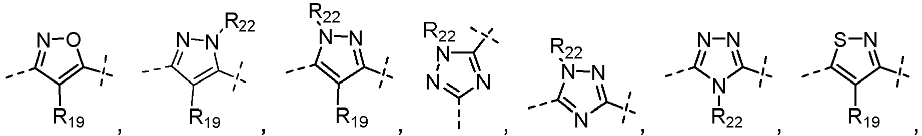 or 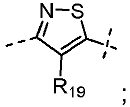
**R₁** represents -H, -**R***, -CH₂-**R***, -CHR^{a}-**R***, -CR^{a}R^{b}-**R***, -CH₂CH₂-**R***, -CR^{a}R^{b}-CR^{c}R^{d}-**R***, -CH=CH-**R***, -CR^{a}=CR^{b}-**R***, -CH₂CH₂CH₂-**R*** or -CR^{a}R^{b}-CR^{c}R^{d}-CR^{e}R^{f}-**R***;
**R**^{a} - **R**^{f} represent independently of each other -H, -F, -Cl, -CH₃, -OCH₃, -CH₂CH₃, -CH₂CH₂CH₃, -CH(CH₃)₂, -CH₂CH(CH₃)₂, or -cyclo-C₃H₅;
**R*** represents -H, -F, -Cl, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂₃ -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHSO₂CH₃, -NHSO₂C₂H₅, -NHSO₂C₃H₇, -NHSO₂CH(CH₃)₂, -NHSO₂-cyclco-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclco-C₃H₅, -SO(NH)CH₃, -SO(NH)C₂H₅, -SO(NH)C₃H₇, -SO(NH)CH(CH₃)₂, -SO(NH)-cyclco-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclco-C₃H₅, 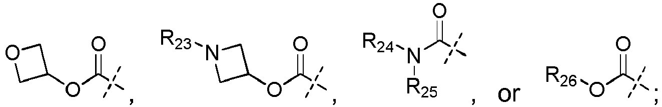
**R₂** represents -H, -F, -Cl, -Br, -I, -OH, -CN, -CH₃, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅, or -O-C₂H₄-cyclo-C₃H₅;
**R₃** represents -H, -CH₃, -C₂H₄OH, -OC₂H₄OH, -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇R₂₈, -OCH₂CH₂NR₂₇R₂₈, -OCH₂CH₂CH₂NR₂₇R₂₈, -NR₂₉CH₂NR₂₇R₂₈, -NR₂₉CH₂CH₂NR₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 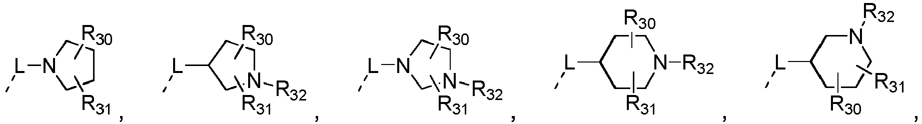 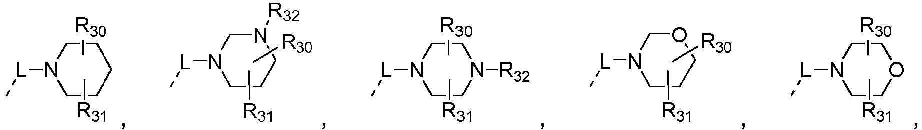 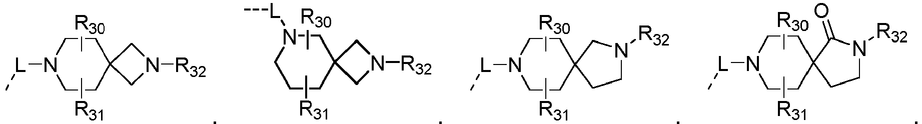 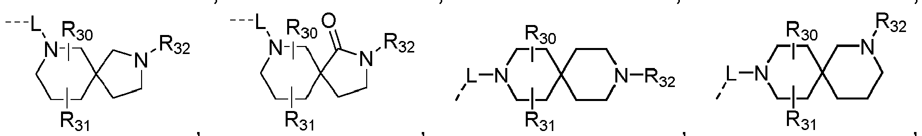 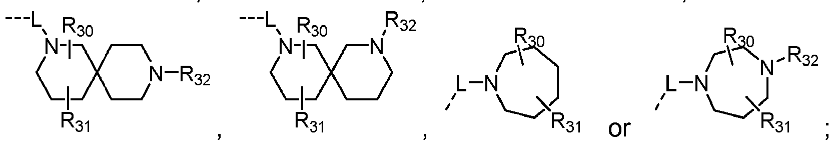
L is a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO- or -CH₂CH₂OCO-
**R₄** represents -H, -CH₃, or -C₂H₅;
**R₅** represents 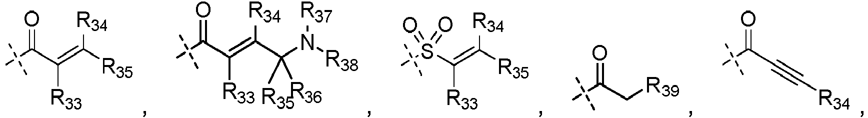 or 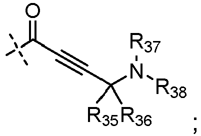
**R₈** - **R₁₁** represent independently of each other -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇,
or **R₈** and **R₉** or **R₉** and **R₁₀** form together -CH₂-, -CH₂CH₂-, -CH₂CH₂CH₂-, -CH₂CH₂CH₂CH₂-, -CH₂OCH₂- -CH₂OCH₂CH₂-, -OCH₂O-, or -OCH₂CH₂O-;
**R₁₂** - **R₁₆** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₁₇** - **R₂₁** represent independently of each other -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₂₂** represents -H, -CH₃, -CF₃, -C₂H₅, -C₃H₇, -COCH₃, -COC₂H₅, -SO₂CH₃, or -SO₂C₂H₅;
**R₂₃** represents -H, -CH₃, -CF₃, -C₂H₅, -C₃H₇, -CH₂CH₂OCH₃, or -C₄H₉;
**R₂₄** and **R₂₅** represent independently of each other -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 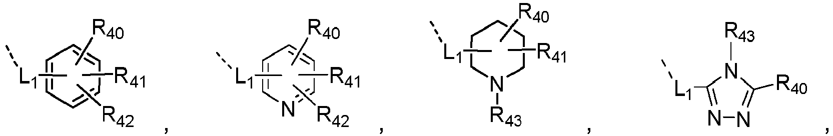 or 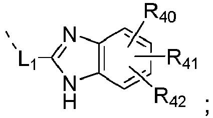
or **R²⁴** and **R²⁵** form together -CH₂-, -CH₂CH₂-, -CH₂CH₂CH₂-, -CH₂CH₂CH₂CH₂-, -CH₂OCH₂- or -CH₂OCH₂CH₂-;
**L₁** represents a bond, -CH₂-, -CH₂CH₂-, or -CH₂CH₂CH₂-;
**R₂₆** represents -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph, or -CH₂Ph;
**R₂₇** - **R₃₁** represent independently of each other -H, -F, -CI, -OH, -CN, -NH₂, -OCH₃, -OC₂H₅, -OC₃H₇, -OCH(CH₃)₂, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, or -cyclo-C₆H₁₁;
**R₃₂**, **R₃₇**, **R₃₈**, and **R₄₃** represent independently of each other -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃, -SO₂C₂H₅, -SO₂CF₃, or -SO₂Ph;
**R₃₃**, **R₃₄**, **R₃₅**, and **R₃₆** represent independently of each other -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, or -COOC(CH₃)₃;
**R₃₉** represents -F, -Br, -Cl, or -I;
**R₄₀**, **R₄₁**, and **R₄₂** represent independently of each other -H, -F, -Cl, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂,-SO₂CH₃, -SO₂C₂H₅, -SO₂CF₃, -SO₂Ph, -SO₂NH₂, -SO₂NHCH₃, 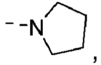 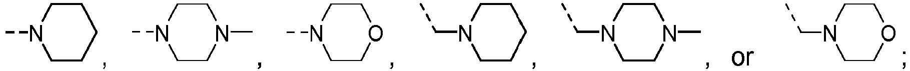 or an enantiomer, a mixture of enantiomers, a diastereomer, a mixture of diastereomers, a tautomer, a hydrate, a solvate, or pharmaceutically acceptable salts thereof.

2. The compound according to Claim 1, wherein
A represents 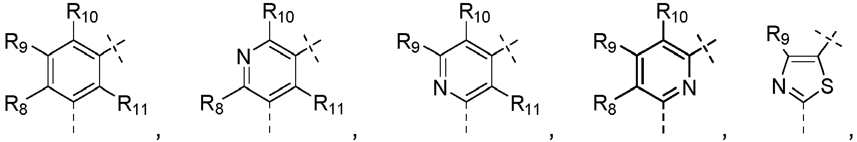 or 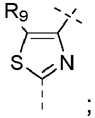 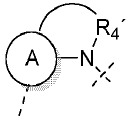 represents 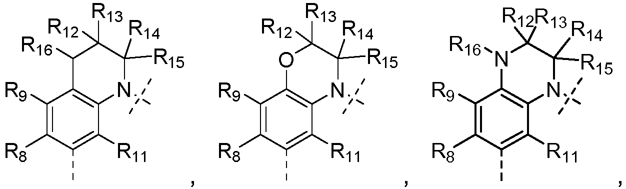 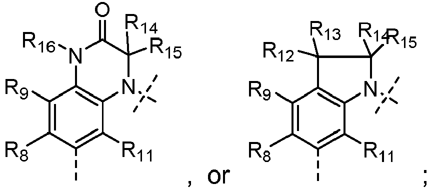 B represents 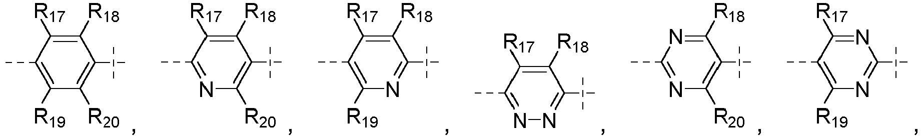 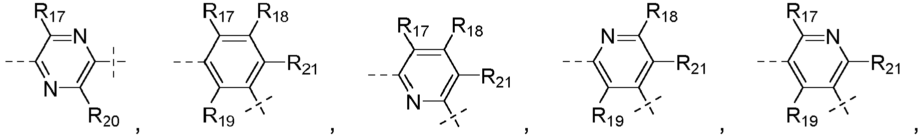 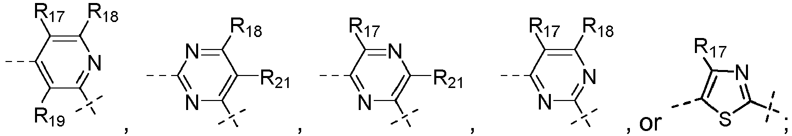 and **R₈** - **R₂₁** have the same meanings as defined in claim 1.

3. The compound according to Claim 1 or 2, wherein
A represents 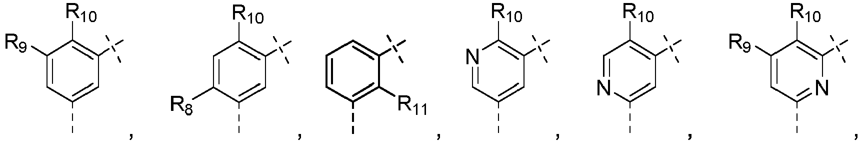 or 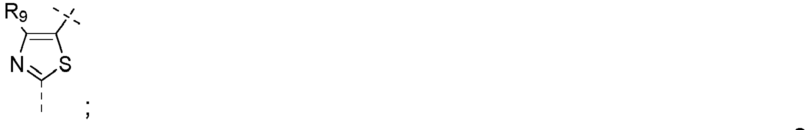 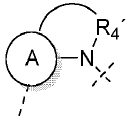 represents 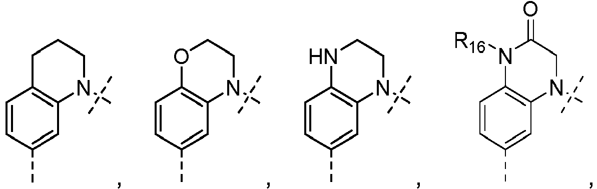 or 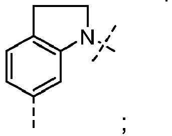
**B** represents 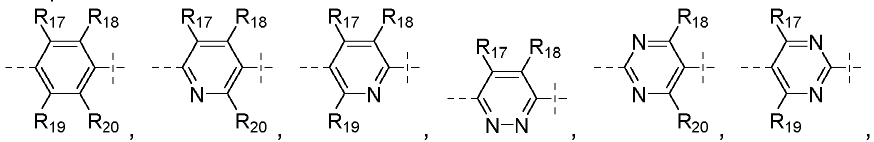 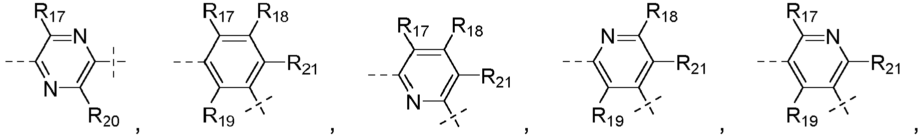 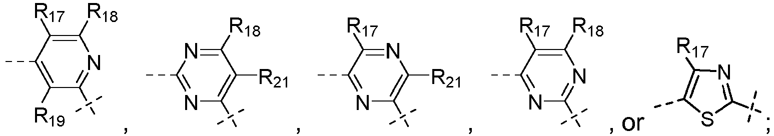 and **R₈**- **R₁₁**, and **R₁₆**- **R₂₁** have the meanings as defined in Claim 1.

4. The compound according to Claim 1, wherein the compound has the formula (**Ia**) or (**Ib**): 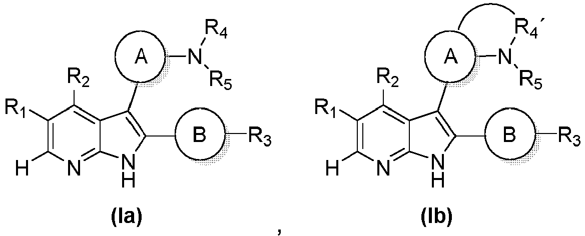 wherein
A represents 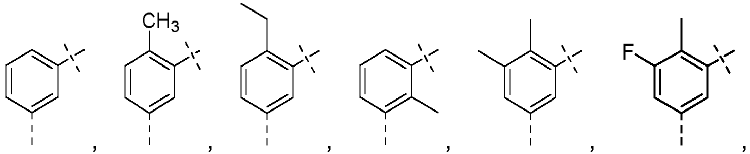 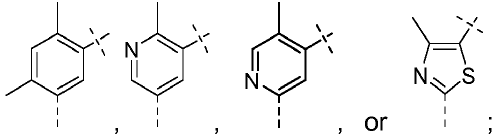 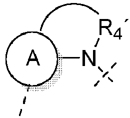 represents 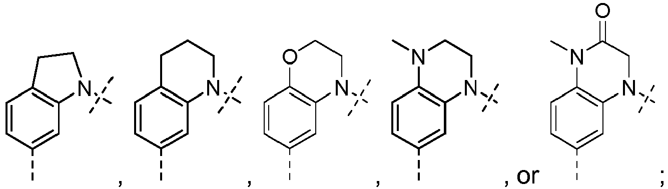
B represents 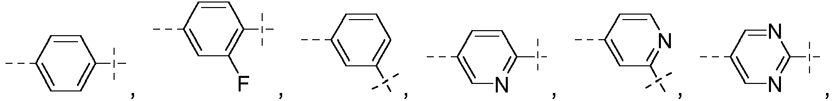 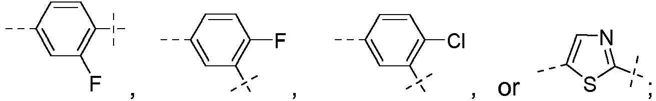 and **R₁**, **R₂**, **R₃**, **R₄**, and **R₅** have the same meanings as defined in claim 1.

5. The compound according to any one of claim 1 - 4, wherein
**Z** represents 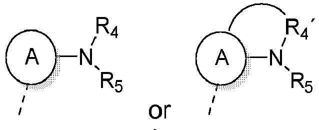 and is selected from 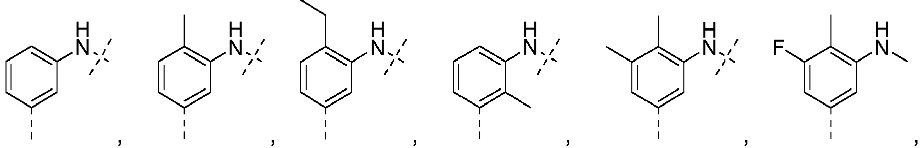 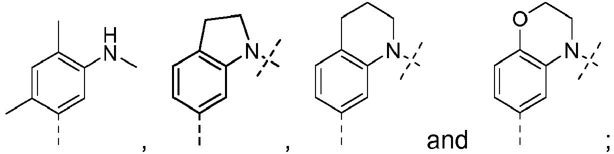
**R₁** represents -H, -CN, -C₂H₅, -OC₂H₅, -OCF₃, -CH₂OH, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, -CH₂COOH, -CH₂COOCH₃, -CH₂COOCH(CH₃)₂, -CH₂CONH(CH₃), -CH₂CON(CH₃)₂, -NHCOCH₃, -NHCOCH(CH₃)₂, 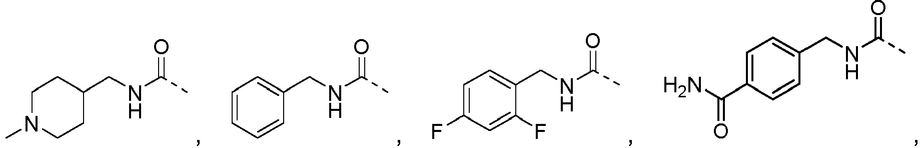 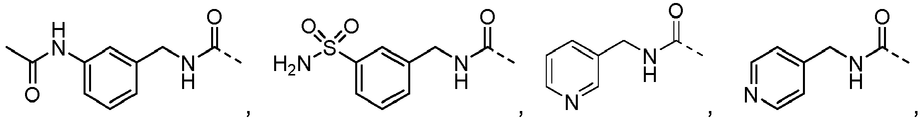 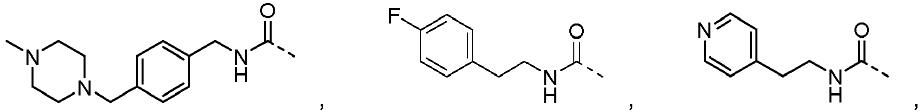 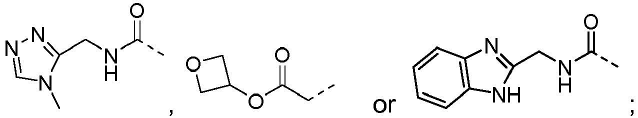
**R₂** represents -H, -Cl, -OCH₃, -OC₂H₅, or -OCH(CH₃)₂;
B represents 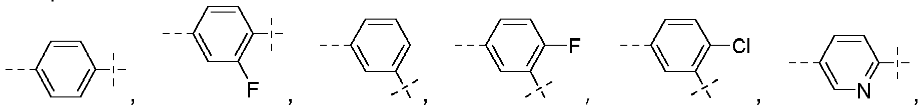 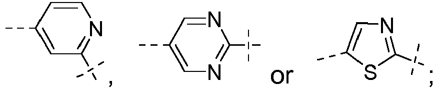
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅,-CONH-cyclo-C₆H₁₁, -CONHCH₂CH₂N(CH₃)CH₂Ph, -CH₂CH₂OH, -OCH₂CH₂OH, 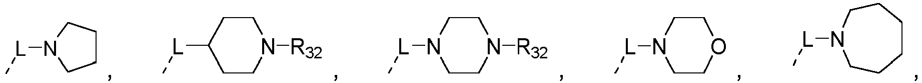 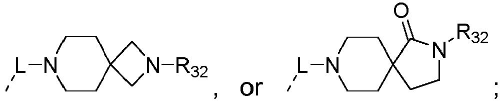
**R₅** represents 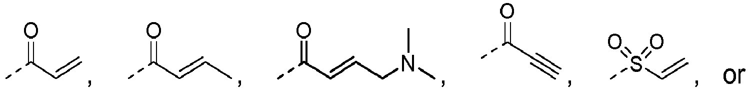 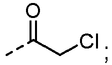
L is a bond, -CH₂-, -OCH₂CH₂-, -CO-, -CONH-, -SO₂-, or -CONHCH₂CH₂CH₂-;
and
**R₃₂** represents -H, -CH₃, -C₂H₅, -CH(CH₃)₂, -CH₂Ph, -COCH₃, or -SO₂C₂H₅.

6. The compound according to Claim 1 selected from the group consisting of:
N-(2-methyl-5-(2-(4-(1-methylpiperidin-4-yl)phenyl)-1H-pyrrolo[2,3-b]pyridin-3-yl)phenyl)acrylamide;
N-(5-(4-chloro-2-(4-(4-methylpiperazin-1-yl)phenyl)-1H-pyrrolo[2,3-b]pyridin-3-yl)-2-methylphenyl)acrylamide;
N-(5-(4-chloro-2-(4-(1-methylpiperidin-4-yl)phenyl)-1H-pyrrolo[2,3-b]pyridin-3-yl)-2-methylphenyl)acrylamide;
1-(6-(2-(4-(4-methylpiperazin-1-yl)phenyl)-1H-pyrrolo[2,3-b]pyridin-3-yl)indolin-1-yl)prop-2-en-1-one;
N-(5-(4-chloro-2-(4-(2-(dimethylamino)ethyl)phenyl)-1H-pyrrolo[2,3-b]pyridin-3-yl)-2-methylphenyl)acrylamide;
N-(5-(4-chloro-2-(4-(morpholinomethyl)phenyl)-1H-pyrrolo[2,3-b]pyridin-3-yl)-2-methylphenyl)acrylamide;
N-(5-(4-chloro-2-(3-morpholinophenyl)-1H-pyrrolo[2,3-b]pyridin-3-yl)-2-methylphenyl)acrylamide;
N-(5-(4-chloro-2-(3-(morpholinomethyl)phenyl)-1H-pyrrolo[2,3-b]pyridin-3-yl)-2-methylphenyl)acrylamide;
N-(5-(2-(4-(4-acetylpiperazin-1-yl)phenyl)-4-chloro-1H-pyrrolo[2,3-b]pyridin-3-yl)-2-methylphenyl)acrylamide;
N-(5-(4-chloro-2-(4-((4-methylpiperazin-1-yl)methyl)phenyl)-1H-pyrrolo[2,3-b]pyridin-3-yl)-2-methylphenyl)acrylamide;
N-(5-(4-chloro-2-(4-(4-methylpiperazine-1-carbonyl)phenyl)-1H-pyrrolo[2,3-b]pyridin-3-yl)-2-methylphenyl)acrylamide;
N-(5-(4-chloro-2-(4-((dimethylamino)methyl)phenyl)-1H-pyrrolo[2,3-b]pyridin-3-yl)-2-methylphenyl)acrylamide;
N-(5-(4-chloro-2-(4-(morpholine-4-carbonyl)phenyl)-1H-pyrrolo[2,3-b]pyridin-3-yl)-2-methylphenyl)acrylamide;
N-(5-(4-chloro-2-(4-(methylsulfonamido)phenyl)-1H-pyrrolo[2,3-b]pyridin-3-yl)-2-methylphenyl)acrylamide;
N-(5-(4-chloro-2-(4-(3-(dimethylamino)propoxy)phenyl)-1H-pyrrolo[2,3-b]pyridin-3-yl)-2-methylphenyl)acrylamide;
N-(5-(2-(4-acetamidophenyl)-4-chloro-1H-pyrrolo[2,3-b]pyridin-3-yl)-2-methylphenyl)acrylamide;
N-(5-(4-chloro-2-(4-(4-(ethylsulfonyl)piperazin-1-yl)phenyl)-1H-pyrrolo[2,3-b]pyridin-3-yl)-2-methylphenyl)acrylamide;
N-(5-(4-chloro-2-(3-((4-methylpiperazin-1-yl)methyl)phenyl)-1H-pyrrolo[2,3-b]pyridin-3-yl)-2-methylphenyl)acrylamide;
N-(5-(4-chloro-2-(3-(4-methylpiperazin-1-yl)phenyl)-1H-pyrrolo[2,3-b]pyridin-3-yl)-2-methylphenyl)acrylamide;
N-(5-(4-chloro-2-(4-(2-(dimethylamino)ethoxy)phenyl)-1H-pyrrolo[2,3-b]pyridin-3-yl)-2-methylphenyl)acrylamide;
N-(5-(4-methoxy-2-(4-(1-methylpiperidin-4-yl)phenyl)-1H-pyrrolo[2,3-b]pyridin-3-yl)-2-methylphenyl)acrylamide;
N-(5-(4-methoxy-2-(4-(4-methylpiperazin-1-yl)phenyl)-1H-pyrrolo[2,3-b]pyridin-3-yl)-2-methylphenyl)acrylamide;
N-(5-(2-(4-(4-acetylpiperazin-1-yl)phenyl)-4-methoxy-1H-pyrrolo[2,3-b]pyridin-3-yl)-2-methylphenyl)acrylamide;
N-(5-(4-methoxy-2-(4-(morpholinomethyl)phenyl)-1H-pyrrolo[2,3-b]pyridin-3-yl)-2-methylphenyl)acrylamide;
N-(5-(4-methoxy-2-(3-((4-methylpiperazin-1-yl)methyl)phenyl)-1H-pyrrolo[2,3-b]pyridin-3-yl)-2-methylphenyl)acrylamide;
N-(5-(4-methoxy-2-(4-((4-methylpiperazin-1-yl)methyl)phenyl)-1H-pyrrolo[2,3-b]pyridin-3-yl)-2-methylphenyl)acrylamide;
N-(5-(4-chloro-2-(6-(4-methylpiperazin-1-yl)pyridin-3-yl)-1H-pyrrolo[2,3-b]pyridin-3-yl)-2-methylphenyl)acrylamide;
N-(5-(4-ethoxy-2-(4-(1-methylpiperidin-4-yl)phenyl)-1H-pyrrolo[2,3-b]pyridin-3-yl)-2-methylphenyl)acrylamide;
N-(5-(4-chloro-2-(3-(3-(dimethylamino)propoxy)phenyl)-1H-pyrrolo[2,3-b]pyridin-3-yl)-2-methylphenyl)acrylamide;
N-(5-(4-chloro-2-(2-methoxy-4-(4-methylpiperazin-1-yl)phenyl)-1H-pyrrolo[2,3-b]pyridin-3-yl)-2-methylphenyl)acrylamide;
N-(5-(2-(3-((4-acetylpiperazin-1-yl)methyl)phenyl)-4-chloro-1H-pyrrolo[2,3-b]pyridin-3-yl)-2-methylphenyl)acrylamide;
N-(5-(4-chloro-2-(4-(3-(4-ethylpiperazin-1-yl)propoxy)phenyl)-1H-pyrrolo[2,3-b]pyridin-3-yl)-2-methylphenyl)acrylamide;
N-(5-(4-chloro-2-(pyrimidin-5-yl)-1H-pyrrolo[2,3-b]pyridin-3-yl)-2-methylphenyl)acrylamide; N-(5-(4-chloro-2-(3-(3-(4-ethylpiperazin-1-yl)propoxy)phenyl)-1H-pyrrolo[2,3-b]pyridin-3-yl)-2-methylphenyl)acrylamide;
N-(5-(4-chloro-2-(2-(4-methylpiperazin-1-yl)pyridin-4-yl)-1H-pyrrolo[2,3-b]pyridin-3-yl)-2-methylphenyl)acrylamide;
N-(5-(4-chloro-2-(3-(morpholine-4-carbonyl)phenyl)-1H-pyrrolo[2,3-b]pyridin-3-yl)-2-methylphenyl)acrylamide;
N-(5-(4-chloro-2-(3-(2-(dimethylamino)ethoxy)phenyl)-1H-pyrrolo[2,3-b]pyridin-3-yl)-2-methylphenyl)acrylamide;
N-(5-(4-chloro-2-(4-(piperidin-4-yl)phenyl)-1H-pyrrolo[2,3-b]pyridin-3-yl)-2-methylphenyl)acrylamide;
N-(5-(4-chloro-2-(6-morpholinopyridin-3-yl)-1H-pyrrolo[2,3-b]pyridin-3-yl)-2-methylphenyl)acrylamide;
N-(5-(2-(6-(4-acetylpiperazin-1-yl)pyridin-3-yl)-4-chloro-1H-pyrrolo[2,3-b]pyridin-3-yl)-2-methylphenyl)acrylamide;
N-(5-(4-chloro-2-(2-morpholinopyridin-4-yl)-1H-pyrrolo[2,3-b]pyridin-3-yl)-2-methylphenyl)acrylamide;
1-(6-(4-chloro-2-(4-(1-methylpiperidin-4-yl)phenyl)-1H-pyrrolo[2,3-b]pyridin-3-yl)indolin-1-yl)prop-2-en-1-one;
N-(5-(4-chloro-2-(3-(4-methylpiperazine-1-carbonyl)phenyl)-1H-pyrrolo[2,3-b]pyridin-3-yl)-2-methylphenyl)acrylamide;
N-(5-(2-(3-(4-acetylpiperazin-1-yl)phenyl)-4-chloro-1H-pyrrolo[2,3-b]pyridin-3-yl)-2-methylphenyl)acrylamide; 3-(3-(3-acrylamido-4-methylphenyl)-4-chloro-1H-pyrrolo[2,3-b]pyridin-2-yl)-N-(3-(pyrrolidin-1-yl)propyl)benzamide;
3-(3-(3-acrylamido-4-methylphenyl)-4-chloro-1H-pyrrolo[2,3-b]pyridin-2-yl)-N-(1-benzylpiperidin-4-yl)benzamide;
3-(3-(3-acrylamido-4-methylphenyl)-4-chloro-1H-pyrrolo[2,3-b]pyridin-2-yl)-N-cyclopropylbenzamide;
3-(3-(3-acrylamido-4-methylphenyl)-4-chloro-1H-pyrrolo[2,3-b]pyridin-2-yl)-N-(3-(benzyl(methyl)amino)propyl)benzamide;
N-(5-(2-(3-(4-acetylpiperazine-1-carbonyl)phenyl)-4-chloro-1H-pyrrolo[2,3-b]pyridin-3-yl)-2-methylphenyl)acrylamide;
N-(5-(4-chloro-2-(3-(2-methyl-2,7-diazaspiro[3.5]nonane-7-carbonyl)phenyl)-1H-pyrrolo[2,3-b]pyridin-3-yl)-2-methylphenyl)acrylamide;
3-(3-(3-acrylamido-4-methylphenyl)-4-chloro-1H-pyrrolo[2,3-b]pyridin-2-yl)-N-(3-(4-ethylpiperazin-1-yl)propyl)benzamide;
1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one;
1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one;
N-(5-(4-chloro-2-(2-morpholinopyrimidin-5-yl)-1H-pyrrolo[2,3-b]pyridin-3-yl)-2-methylphenyl)acrylamide;
N-(5-(4-chloro-2-(3-(2-(pyrrolidin-1-yl)ethoxy)phenyl)-1H-pyrrolo[2,3-b]pyridin-3-yl)-2-methylphenyl)acrylamide;
N-(5-(4-chloro-2-(4-(2-(pyrrolidin-1-yl)ethoxy)phenyl)-1H-pyrrolo[2,3-b]pyridin-3-yl)-2-methylphenyl)acrylamide;
1-(7-(4-chloro-2-(4-(1-methylpiperidin-4-yl)phenyl)-1H-pyrrolo[2,3-b]pyridin-3-yl)-3,4-dihydroquinolin-1 (2H)-yl)prop-2-en-1-one;
1-(7-(4-chloro-2-(3-(2-(dimethylamino)ethoxy)phenyl)-1H-pyrrolo[2,3-b]pyridin-3-yl)-3,4-dihydroquinolin-1(2H)-yl)prop-2-en-1-one;
1-(7-(4-chloro-2-(6-((4-methylpiperazin-1-yl)methyl)pyridin-3-yl)-1H-pyrrolo[2,3-b]pyridin-3-yl)-3,4-dihydroquinolin-1 (2H)-yl)prop-2-en-1-one;
N-(5-(4-chloro-2-(2-(4-methylpiperazin-1-yl)pyrimidin-5-yl)-1H-pyrrolo[2,3-b]pyridin-3-yl)-2-methylphenyl)acrylamide;
N-(5-(4-chloro-2-(4-(4-ethylpiperazin-1-yl)phenyl)-1H-pyrrolo[2,3-b]pyridin-3-yl)-2-methylphenyl)acrylamide;
N-(5-(4-chloro-2-(4-(4-isopropylpiperazin-1-yl)phenyl)-1H-pyrrolo[2,3-b]pyridin-3-yl)-2-methylphenyl)acrylamide;
1-(7-(4-chloro-2-(4-(2-(pyrrolidin-1-yl)ethoxy)phenyl)-1H-pyrrolo[2,3-b]pyridin-3-yl)-3,4-dihydroquinolin-1(2H)-yl)prop-2-en-1-one;
1-(7-(4-chloro-2-(4-(4-isopropylpiperazin-1-yl)phenyl)-1H-pyrrolo[2,3-b]pyridin-3-yl)-3,4-dihydroquinolin-1(2H)-yl)prop-2-en-1-one;
1-(7-(4-chloro-2-(4-(2-(dimethylamino)ethoxy)phenyl)-1H-pyrrolo[2,3-b]pyridin-3-yl)-3,4-dihydroquinolin-1(2H)-yl)prop-2-en-1-one;
1-(7-(4-chloro-2-(6-(4-methylpiperazin-1-yl)pyridin-3-yl)-1H-pyrrolo[2,3-b]pyridin-3-yl)-3,4-dihydroquinolin-1(2H)-yl)prop-2-en-1-one;
1-(7-(4-chloro-2-(3-(2-(pyrrolidin-1-yl)ethoxy)phenyl)-1H-pyrrolo[2,3-b]pyridin-3-yl)-3,4-dihydroquinolin-1 (2H)-yl)prop-2-en-1-one;
1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one;
N-(5-(4-chloro-2-(4-(piperazin-1-yl)phenyl)-1H-pyrrolo[2,3-b]pyridin-3-yl)-2-methylphenyl)acrylamide;
1-(6-(4-chloro-2-(4-(4-methylpiperazin-1-yl)phenyl)-1H-pyrrolo[2,3-b]pyridin-3-yl)indolin-1-yl)prop-2-en-1-one;
1-(6-(4-chloro-2-(3-fluoro-4-(4-methylpiperazin-1-yl)phenyl)-1H-pyrrolo[2,3-b]pyridin-3-yl)indolin-1-yl)prop-2-en-1-one;
1-(6-(2-(4-(4-acetylpiperazin-1-yl)phenyl)-4-chloro-1H-pyrrolo[2,3-b]pyridin-3-yl)indolin-1-yl)prop-2-en-1-one;
1-(6-(4-chloro-2-(2-(4-methylpiperazin-1-yl)pyridin-4-yl)-1H-pyrrolo[2,3-b]pyridin-3-yl)indolin-1-yl)prop-2-en-1-one;
1-(6-(4-chloro-2-(4-((4-methylpiperazin-1-yl)methyl)phenyl)-1H-pyrrolo[2,3-b]pyridin-3-yl)indolin-1-yl)prop-2-en-1-one;
1-(6-(4-chloro-2-(4-((dimethylamino)methyl)phenyl)-1H-pyrrolo[2,3-b]pyridin-3-yl)indolin-1-yl)prop-2-en-1-one;
1-(6-(4-chloro-2-(4-(4-isopropylpiperazin-1-yl)phenyl)-1H-pyrrolo[2,3-b]pyridin-3-yl)indolin-1-yl)prop-2-en-1-one;
1-(6-(4-chloro-2-(4-(4-ethylpiperazin-1-yl)phenyl)-1H-pyrrolo[2,3-b]pyridin-3-yl)indolin-1-yl)prop-2-en-1-one;
1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one;
1-(6-(4-chloro-2-(4-((2-(dimethylamino)ethyl)(methyl)amino)phenyl)-1H-pyrrolo[2,3-b]pyridin-3-yl)indolin-1-yl)prop-2-en-1-one;
1-(6-(4-chloro-2-(4-(4-methylpiperazine-1-carbonyl)phenyl)-1H-pyrrolo[2,3-b]pyridin-3-yl)indolin-1-yl)prop-2-en-1-one;
1-(6-(4-chloro-2-(4-(2-(dimethylamino)ethoxy)phenyl)-1H-pyrrolo[2,3-b]pyridin-3-yl)indolin-1-yl)prop-2-en-1-one;
1-(6-(4-chloro-2-(4-(morpholinomethyl)phenyl)-1H-pyrrolo[2,3-b]pyridin-3-yl)indolin-1-yl)prop-2-en-1-one;
1-(6-(4-chloro-2-(3-((4-methylpiperazin-1-yl)methyl)phenyl)-1H-pyrrolo[2,3-b]pyridin-3-yl)indolin-1-yl)prop-2-en-1-one;
1-(6-(4-chloro-2-(6-(4-methylpiperazin-1-yl)pyridin-3-yl)-1H-pyrrolo[2,3-b]pyridin-3-yl)indolin-1-yl)prop-2-en-1-one;
1-(6-(4-chloro-2-(4-(3-(dimethylamino)propoxy)phenyl)-1H-pyrrolo[2,3-b]pyridin-3-yl)indolin-1-yl)prop-2-en-1-one;
N-(5-(4-chloro-2-(3-fluoro-4-(4-methylpiperazin-1-yl)phenyl)-1H-pyrrolo[2,3-b]pyridin-3-yl)-2-methylphenyl)acrylamide;
1-(6-(4-chloro-2-(4-((2-(dimethylamino)ethyl)(methyl)amino)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one;
N-(5-(4-chloro-2-(4-((2-(dimethylamino)ethyl)(methyl)amino)phenyl)-1H-pyrrolo[2,3-b]pyridin-3-yl)-2-methylphenyl)acrylamide;
N-(5-(5-cyano-2-(4-(4-methylpiperazin-1-yl)phenyl)-1H-pyrrolo[2,3-b]pyridin-3-yl)-2-methylphenyl)acrylamide;
3-(3-acrylamido-4-methylphenyl)-N-isopropyl-2-(4-(4-methylpiperazin-1-yl)phenyl)-1H-pyrrolo[2,3-b]pyridine-5-carboxamide;
1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one;
1-(6-(4-chloro-2-(4-(4-ethylpiperazin-1-yl)phenyl)-1H-pyrrolo[2,3-b]pyridin-3-yl)-2H-benzo[b][1,4]oxazin-4(3H)-yl)prop-2-en-1 -one;
1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-((dimethylamino)methyl)phenyl)-1H-pyrrolo[2,3-b]pyridin-3-yl)-2H-benzo[b][1,4]oxazin-4(3H)-yl)prop-2-en-1-one;
1-(6-(4-chloro-2-(3-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one;
Isopropyl 3-(3-acrylamido-4-methylphenyl)-2-(4-(4-methylpiperazin-1-yl)phenyl)-1H-pyrrolo[2,3-b]pyridine-5-carboxylate;
1-(6-(4-ethoxy-2-(2-(4-methylpiperazin-1-yl)pyridin-4-yl)-1 H-pyrrolo[2,3-b]pyridin-3-yl)indolin-1-yl)prop-2-en-1-one;
1-(6-(2-(4-((dimethylamino)methyl)phenyl)-4-ethoxy-1H-pyrrolo[2,3-b]pyridin-3-yl)indolin-1-yl)prop-2-en-1-one;
1-(6-(4-ethoxy-2-(4-(2-(pyrrolidin-1-yl)ethoxy)phenyl)-1H-pyrrolo[2,3-b]pyridin-3-yl)indolin-1-yl)prop-2-en-1-one;
1-(6-(4-ethoxy-2-(4-((4-methylpiperazin-1-yl)methyl)phenyl)-1H-pyrrolo[2,3-b]pyridin-3-yl)indolin-1-yl)prop-2-en-1-one;
1 -(6-(4-ethoxy-2-(4-(4-methylpiperazin-1-yl)phenyl)-1 H-pyrrolo[2,3-b]pyridin-3-yl)indolin-1-yl)prop-2-en-1-one;
1-(6-(4-ethoxy-2-(3-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-cyclohexyl-2-(4-(4-methylpiperazin-1-yl)phenyl)-1H-pyrrolo[2,3-b]pyridine-5-carboxamide;
1-(6-(4-methoxy-2-(4-(4-methylpiperazin-1-yl)phenyl)-1H-pyrrolo[2,3-b]pyridin-3-yl)indolin-1-yl)prop-2-en-1-one;
1-(6-(4-methoxy-2-(2-(4-methylpiperazin-1-yl)pyridin-4-yl)-1 H-pyrrolo[2,3-b]pyridin-3-yl)indolin-1-yl)prop-2-en-1-one;
1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-benzyl-2-(4-(4-methylpiperazin-1-yl)phenyl)-1H-pyrrolo[2,3-b]pyridine-5-carboxamide;
N-(3-acetamidobenzyl)-3-(3-acrylamido-4-methylphenyl)-2-(4-(4-methylpiperazin-1 -yl)phenyl)-1H-pyrrolo[2,3-b]pyridine-5-carboxamide;
1-(6-(4-isopropoxy-2-(4-(4-methylpiperazin-1-yl)phenyl)-1H-pyrrolo[2,3-b]pyridin-3-yl)indolin-1-yl)prop-2-en-1-one;
1-(6-(4-methoxy-2-(4-((4-methylpiperazin-1-yl)methyl)phenyl)-1H-pyrrolo[2,3-b]pyridin-3-yl)indolin-1-yl)prop-2-en-1-one;
1-(6-(4-methoxy-2-(3-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-(4-((4-methylpiperazin-1-yl)methyl)benzyl)-2-(4-(4-methylpiperazin-1-yl)phenyl)-1H-pyrrolo[2,3-b]pyridine-5-carboxamide;
3-(3-acrylamido-4-methylphenyl)-2-(4-(4-methylpiperazin-1-yl)phenyl)-N-(2-(pyridin-4-yl)ethyl)-1H-pyrrolo[2,3-b]pyridine-5-carboxamide;
3-(3-acrylamido-4-methylphenyl)-N-(4-fluorophenethyl)-2-(4-(4-methylpiperazin-1-yl)phenyl)-1H-pyrrolo[2,3-b]pyridine-5-carboxamide;
N-((1H-benzo[d]imidazol-2-yl)methyl)-3-(3-acrylamido-4-methylphenyl)-2-(4-(4-methylpiperazin-1-yl)phenyl)-1H-pyrrolo[2,3-b]pyridine-5-carboxamide;
N-(2-methyl-5-(2-(2-morpholinopyridin-4-yl)-1H-pyrrolo[2,3-b]pyridin-3-yl)phenyl)acrylamide;
N-(2-methyl-5-(2-(6-(4-methylpiperazin-1-yl)pyridin-3-yl)-1 H-pyrrolo[2,3-b]pyridin-3-yl)phenyl)acrylamide;
3-(3-acrylamido-4-methylphenyl)-2-(4-(4-methylpiperazin-1-yl)phenyl)-N-(3-sulfamoylbenzyl)-1H-pyrrolo[2,3-b]pyridine-5-carboxamide;
3-(3-acrylamido-4-methylphenyl)-2-(4-(4-methylpiperazin-1-yl)phenyl)-N-(pyridin-4-ylmethyl)-1H-pyrrolo[2,3-b]pyridine-5-carboxamide;
3-(3-acrylamido-4-methylphenyl)-2-(4-(4-methylpiperazin-1-yl)phenyl)-N-(pyridin-3-ylmethyl)-1H-pyrrolo[2,3-b]pyridine-5-carboxamide;
3-(3-acrylamido-4-methylphenyl)-2-(4-(4-methylpiperazin-1-yl)phenyl)-N-((1-methylpiperidin-4-yl)methyl)-1H-pyrrolo[2,3-b]pyridine-5-carboxamide;
3-(3-acrylamido-4-methylphenyl)-N-((4-methyl-4H-1,2,4-triazol-3-yl)methyl)-2-(4-(4-methylpiperazin-1-yl)phenyl)-1H-pyrrolo[2,3-b]pyridine-5-carboxamide;
3-(3-acrylamido-4-methylphenyl)-N-(2,4-difluorobenzyl)-2-(4-(4-methylpiperazin-1-yl)phenyl)-1H-pyrrolo[2,3-b]pyridine-5-carboxamide;
N-(4-acetamidobenzyl)-3-(3-acrylamido-4-methylphenyl)-2-(4-(4-methylpiperazin-1 -yl)phenyl)-1H-pyrrolo[2,3-b]pyridine-5-carboxamide;
3-(3-acrylamido-4-methylphenyl)-N-(3-carbamoylbenzyl)-2-(4-(4-methylpiperazin-1-yl)phenyl)-1H-pyrrolo[2,3-b]pyridine-5-carboxamide;
3-(3-acrylamido-4-methylphenyl)-2-(4-(4-methylpiperazin-1-yl)phenyl)-1H-pyrrolo[2,3-b]pyridine-5-carboxylic acid;
2-Chloro-N-(5-(4-chloro-2-(4-(4-methylpiperazin-1-yl)phenyl)-1H-pyrrolo[2,3-b]pyridin-3-yl)-2-methylphenyl)acetamide;
(E)-N-(5-(4-chloro-2-(4-(4-methylpiperazin-1-yl)phenyl)-1H-pyrrolo[2,3-b]pyridin-3-yl)-2-methylphenyl)but-2-enamide;
N-(5-(4-chloro-2-(4-(4-methylpiperazin-1-yl)phenyl)-1H-pyrrolo[2,3-b]pyridin-3-yl)-2-methylphenyl)ethenesulfonamide;
(E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide;
Methyl 3-(3-acrylamido-4-methylphenyl)-2-(4-(4-methylpiperazin-1-yl)phenyl)-1H-pyrrolo[2,3-b]pyridine-5-carboxylate;
Ethyl 3-(3-acrylamido-4-methylphenyl)-2-(4-(4-methylpiperazin-1-yl)phenyl)-1H-pyrrolo[2,3-b]pyridine-5-carboxylate; cyclobutyl 3-(3-acrylamido-4-methylphenyl)-2-(4-(4-methylpiperazin-1-yl)phenyl)-1H-pyrrolo[2,3-b]pyridine-5-carboxylate;
Cyclohexyl 3-(3-acrylamido-4-methylphenyl)-2-(4-(4-methylpiperazin-1-yl)phenyl)-1H-pyrrolo[2,3-b]pyridine-5-carboxylate;
Cyclopropyl 3-(3-acrylamido-4-methylphenyl)-2-(4-(4-methylpiperazin-1-yl)phenyl)-1H-pyrrolo[2,3-b]pyridine-5-carboxylate;
Cyclopentyl 3-(3-acrylamido-4-methylphenyl)-2-(4-(4-methylpiperazin-1-yl)phenyl)-1H-pyrrolo[2,3-b]pyridine-5-carboxylate;
2-methoxyethyl 3-(3-acrylamido-4-methylphenyl)-2-(4-(4-methylpiperazin-1-yl)phenyl)-1H-pyrrolo[2,3-b]pyridine-5-carboxylate; N-(5-(4-chloro-2-(4-(4-methylpiperazin-1-yl)phenyl)-1H-pyrrolo[2,3-b]pyridin-3-yl)-2-methylphenyl)propiolamide; oxetan-3-yl 3-(3-acrylamido-4-methylphenyl)-2-(4-(4-methylpiperazin-1-yl)phenyl)-1H-pyrrolo[2,3-b]pyridine-5-carboxylate;
(E)-N-(5-(4-chloro-2-(4-(4-methylpiperazin-1-yl)phenyl)-1H-pyrrolo[2,3-b]pyridin-3-yl)-2-methylphenyl)but-2-enamide;
(E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide;
N-(5-(4-chloro-2-(4-(4-methylpiperazin-1-yl)phenyl)-1H-pyrrolo[2,3-b]pyridin-3-yl)-2-methylphenyl)-ethenesulfonamide;
2-chloro-N-(5-(4-chloro-2-(4-(4-methylpiperazin-1-yl)phenyl)-1H-pyrrolo[2,3-b]pyridin-3-yl)-2-methylphenyl)acetamide;
N-(5-(4-ethoxy-2-(4-(1-methylpiperidin-4-yl)phenyl)-1H-pyrrolo[2,3-b]pyridin-3-yl)-2-methylphenyl)acrylamide;
(E)-isopropyl 3-(3-(4-(dimethylamino)but-2-enamido)-4-methylphenyl)-2-(4-(4-methylpiperazin-1-yl)phenyl)-1H-pyrrolo[2,3-b]pyridine-5-carboxylate;
isopropyl 3-(3-acrylamido-4-methylphenyl)-2-(4-(1-methylpiperidin-4-yl)phenyl)-1H-pyrrolo[2,3-b]pyridine-5-carboxylate;
N-(5-(4-chloro-2-(4-(4-methylpiperazin-1-yl)phenyl)-1H-pyrrolo[2,3-b]pyridin-3-yl)-2,3-dimethylphenyl)acrylamide; isopropyl 3-(3-acrylamido-4-methylphenyl)-2-(4-((dimethylamino)methyl)phenyl)-1H-pyrrolo[2,3-b]pyridine-5-carboxylate;
N-(5-(4-chloro-2-(4-(1-methylpiperidin-4-yl)phenyl)-1H-pyrrolo[2,3-b]pyridin-3-yl)-2,3-dimethylphenyl)acrylamide;
3-(3-acrylamido-4-methylphenyl)-2-(4-((2-(dimethylamino)ethyl)(methyl)amino)phenyl)-1H-pyrrolo[2,3-b]pyridine-5-carboxylic acid;
isopropyl 3-(3-acrylamido-4-methylphenyl)-2-(4-((2-(dimethylamino)ethyl)(methyl)amino)phenyl)-1H-pyrrolo[2,3-b]pyridine-5-carboxylate;
N-(5-(5-chloro-4-methyl-2-(4-(4-methylpiperazin-1-yl)phenyl)-1H-pyrrolo[2,3-b]pyridin-3-yl)-2-methylphenyl)acrylamide;
isopropyl 3-(3-acrylamido-4-methylphenyl)-2-(2-(4-methylpiperazin-1-yl)pyridin-4-yl)-1H-pyrrolo[2,3-b]pyridine-5-carboxylate;
isopropyl 3-(3-acrylamido-4-methylphenyl)-2-(4-(4-ethylpiperazin-1-yl)phenyl)-1H-pyrrolo[2,3-b]pyridine-5-carboxylate;
isopropyl 3-(3-acrylamido-4-methylphenyl)-2-(4-(2-(pyrrolidin-1-yl)ethoxy)phenyl)-1H-pyrrolo[2,3-b]pyridine-5-carboxylate;
isopropyl 3-(3-acrylamido-4-methylphenyl)-2-(4-(2-(dimethylamino)ethoxy)phenyl)-1H-pyrrolo[2,3-b]pyridine-5-carboxylate; isopropyl 3-(3-acrylamido-4-methylphenyl)-2-(4-((4-methylpiperazin-1-yl)methyl)phenyl)-1H-pyrrolo[2,3-b]pyridine-5-carboxylate;
isopropyl 3-(3-acrylamido-4-methylphenyl)-2-(4-(2-(dimethylamino)ethyl)phenyl)-1H-pyrrolo[2,3-b]pyridine-5-carboxylate; isopropyl 2-(4-(4-acetylpiperazin-1-yl)phenyl)-3-(3-acrylamido-4-methylphenyl)-1H-pyrrolo[2,3-b]pyridine-5-carboxylate;
isopropyl 3-(3-acrylamido-4-methylphenyl)-2-(6-(4-methylpiperazin-1-yl)pyridin-3-yl)-1H-pyrrolo[2,3-b]pyridine-5-carboxylate;
isopropyl 3-(3-acrylamido-4-methylphenyl)-2-(3-fluoro-4-(4-methylpiperazin-1-yl)phenyl)-1H-pyrrolo[2,3-b]pyridine-5-carboxylate; isopropyl 3-(3-acrylamido-5-fluoro-4-methylphenyl)-2-(4-(4-methylpiperazin-1-yl)phenyl)-1H-pyrrolo[2,3-b]pyridine-5-carboxylate; isopropyl 3-(3-acrylamido-4-methylphenyl)-2-(2-morpholinopyridin-4-yl)-1H-pyrrolo[2,3-b]pyridine-5-carboxylate;
isopropyl 3-(3-acrylamido-4-methylphenyl)-2-(4-((4-methylpiperazin-1-yl)sulfonyl)phenyl)-1H-pyrrolo[2,3-b]pyridine-5-carboxylate;
isopropyl 3-(3-acrylamido-4-methylphenyl)-2-(4-(2-hydroxyethyl)phenyl)-1H-pyrrolo[2,3-b]pyridine-5-carboxylate;
isopropyl 3-(3-acrylamido-4-methylphenyl)-2-(2-(4-methylpiperazin-1-yl)thiazol-5-yl)-1H-pyrrolo[2,3-b]pyridine-5-carboxylate;
isopropyl 3-(3-acrylamido-4-methylphenyl)-2-(3-(2-(pyrrolidin-1-yl)ethoxy)phenyl)-1H-pyrrolo[2,3-b]pyridine-5-carboxylate;
isopropyl 3-(3-acrylamido-4-methylphenyl)-2-(6-morpholinopyridin-3-yl)-1H-pyrrolo[2,3-b]pyridine-5-carboxylate;
isopropyl 3-(3-acrylamido-5-fluoro-4-methylphenyl)-2-(4-((2-(dimethylamino)ethyl)(methyl)amino)phenyl)-1H-pyrrolo[2,3-b]pyridine-5-carboxylate;
isopropyl 3-(3-acrylamido-4-methylphenyl)-2-(2-morpholinopyrimidin-5-yl)-1H-pyrrolo[2,3-b]pyridine-5-carboxylate;
isopropyl 3-(3-acrylamido-5-fluoro-4-methylphenyl)-2-(6-(4-methylpiperazin-1-yl)pyridin-3-yl)-1H-pyrrolo[2,3-b]pyridine-5-carboxylate;
isopropyl 3-(3-acrylamido-4-methylphenyl)-2-(2-(4-methylpiperazin-1-yl)pyrimidin-5-yl)-1H-pyrrolo[2,3-b]pyridine-5-carboxylate;
isopropyl 3-(3-acrylamido-4-methylphenyl)-2-(4-(4-isopropylpiperazin-1-yl)phenyl)-1H-pyrrolo[2,3-b]pyridine-5-carboxylate;
isopropyl 3-(3-acrylamido-5-fluoro-4-methylphenyl)-2-(4-((dimethylamino)methyl)phenyl)-1H-pyrrolo[2,3-b]pyridine-5-carboxylate; isopropyl 3-(3-acrylamido-4-methylphenyl)-2-(4-(2-hydroxyethoxy)phenyl)-1H-pyrrolo[2,3-b]pyridine-5-carboxylate;
isopropyl 3-(3-acrylamido-5-fluoro-4-methylphenyl)-2-(4-(1-methylpiperidin-4-yl)phenyl)-1H-pyrrolo[2,3-b]pyridine-5-carboxylate; isopropyl 3-(3-acrylamido-5-fluoro-4-methylphenyl)-2-(2-(4-methylpiperazin-1-yl)pyridin-4-yl)-1H-pyrrolo[2,3-b]pyridine-5-carboxylate; isopropyl 3-(3-acrylamido-4-methylphenyl)-2-(3-((4-methylpiperazin-1-yl)methyl)phenyl)-1H-pyrrolo[2,3-b]pyridine-5-carboxylate;
isopropyl 3-(3-acrylamido-5-fluoro-4-methylphenyl)-2-(4-(2-(dimethylamino)ethoxy)phenyl)-1H-pyrrolo[2,3-b]pyridine-5-carboxylate;
isopropyl 3-(3-acrylamido-4-methylphenyl)-2-(3-(3-(dimethylamino)propoxy)phenyl)-1H-pyrrolo[2,3-b]pyridine-5-carboxylate; isopropyl 3-(3-acrylamido-4-methylphenyl)-2-(4-(4-(ethylsulfonyl)piperazin-1-yl)phenyl)-1H-pyrrolo[2,3-b]pyridine-5-carboxylate;
isopropyl 2-(3-(4-acetylpiperazin-1-yl)phenyl)-3-(3-acrylamido-4-methylphenyl)-1H-pyrrolo[2,3-b]pyridine-5-carboxylate;
isopropyl 2-(3-((4-acetylpiperazin-1-yl)methyl)phenyl)-3-(3-acrylamido-4-methylphenyl)-1H-pyrrolo[2,3-b]pyridine-5-carboxylate;
isopropyl 3-(3-acrylamido-4-methylphenyl)-2-(3-((dimethylamino)methyl)phenyl)-1H-pyrrolo[2,3-b]pyridine-5-carboxylate; N-(2-methyl-5-(2-(4-(4-methylpiperazin-1-yl)phenyl)-5-(trifluoromethoxy)-1H-pyrrolo[2,3-b]pyridin-3-yl)phenyl)acrylamide;
2-(3-(3-acrylamido-4-methylphenyl)-2-(4-(4-methylpiperazin-1-yl)phenyl)-1H-pyrrolo[2,3-b]pyridin-5-yl)acetic acid;
isopropyl 2-(3-(3-acrylamido-4-methylphenyl)-2-(4-(4-methylpiperazin-1-yl)phenyl)-1H-pyrrolo[2,3-b]pyridin-5-yl)acetate; N-(5-(5-ethyl-2-(4-(4-methylpiperazin-1-yl)phenyl)-1H-pyrrolo[2,3-b]pyridin-3-yl)-2-methylphenyl)acrylamide;
isopropyl 3-(1-acryloylindolin-6-yl)-2-(4-(4-methylpiperazin-1-yl)phenyl)-1H-pyrrolo[2,3-b]pyridine-5-carboxylate;
N-(5-(5-ethoxy-2-(4-(4-methylpiperazin-1-yl)phenyl)-1H-pyrrolo[2,3-b]pyridin-3-yl)-2-methylphenyl)acrylamide;
N-(5-(5-(2-(dimethylamino)-2-oxoethyl)-2-(4-(4-methylpiperazin-1-yl)phenyl)-1 H-pyrrolo[2,3-b]pyridin-3-yl)-2-methylphenyl)acrylamide; methyl 2-(3-(3-acrylamido-4-methylphenyl)-2-(4-(4-methylpiperazin-1-yl)phenyl)-1H-pyrrolo[2,3-b]pyridin-5-yl)acetate;
N-(2-methyl-5-(5-(2-(methylamino)-2-oxoethyl)-2-(4-(4-methylpiperazin-1-yl)phenyl)-1H-pyrrolo[2,3-b]pyridin-3-yl)phenyl)acrylamide;
oxetan-3-yl 2-(3-(3-acrylamido-4-methylphenyl)-2-(4-(4-methylpiperazin-1-yl)phenyl)-1H-pyrrolo[2,3-b]pyridin-5-yl)acetate;
isopropyl 3-(3-acrylamido-4-methylphenyl)-4-methyl-2-(4-(4-methylpiperazin-1-yl)phenyl)-1H-pyrrolo[2,3-b]pyridine-5-carboxylate;
N-(5-(5-(hydroxymethyl)-2-(4-(4-methylpiperazin-1-yl)phenyl)-1 H-pyrrolo[2,3-b]pyridin-3-yl)-2-methylphenyl)acrylamide;
N-(5-(5-isobutyramido-2-(4-(4-methylpiperazin-1-yl)phenyl)-1H-pyrrolo[2,3-b]pyridin-3-yl)-2-methylphenyl)acrylamide;
N-(5-(5-acetamido-2-(4-(4-methylpiperazin-1-yl)phenyl)-1H-pyrrolo[2,3-b]pyridin-3-yl)-2-methylphenyl)acrylamide;
isopropyl 3-(5-acrylamido-2,4-dimethylphenyl)-2-(4-(4-methylpiperazin-1-yl)phenyl)-1H-pyrrolo[2,3-b]pyridine-5-carboxylate;
isopropyl 3-(3-(acrylamidomethyl)phenyl)-2-(4-(4-methylpiperazin-1-yl)phenyl)-1H-pyrrolo[2,3-b]pyridine-5-carboxylate;
isopropyl 3-(3-acrylamido-4-methylphenyl)-2-(4-((2-(dimethylamino)ethyl)(methyl)amino)phenyl)-4-methyl-1H-pyrrolo[2,3-b]pyridine-5-carboxylate;
isopropyl 3-(3-acrylamido-4-methylphenyl)-2-(4-(2-(dimethylamino)ethoxy)phenyl)-4-methyl-1H-pyrrolo[2,3-b]pyridine-5-carboxylate;
isopropyl 3-(3-acrylamido-4-ethylphenyl)-2-(4-(4-methylpiperazin-1-yl)phenyl)-1H-pyrrolo[2,3-b]pyridine-5-carboxylate;
isopropyl 3-(3-acrylamido-2-methylphenyl)-2-(4-(4-methylpiperazin-1-yl)phenyl)-1H-pyrrolo[2,3-b]pyridine-5-carboxylate;
isopropyl 3-(3-acrylamido-2-methylphenyl)-2-(4-((2-(dimethylamino)ethyl)(methyl)amino)phenyl)-1H-pyrrolo[2,3-b]pyridine-5-carboxylate, and
isopropyl 3-(3-acrylamido-2-methylphenyl)-2-(4-(2-(dimethylamino)ethoxy)phenyl)-1H-pyrrolo[2,3-b]pyridine-5-carboxylate
or an enantiomer, a mixture of enantiomers, a diastereomer, a mixture of diastereomers, a tautomer, a hydrate, a solvate of the above mentioned compounds, or pharmaceutically acceptable salts thereof.

7. A compound according to any one of claims 1 - 6 as selective inhibitor of Exon 20 mutations of EGFR and Her2.

8. A compound according to any one of claims 1 - 6 for use as a medicament.

9. A compound according to any one of claims 1 - 6 for use in the treatment of cancer or for use in the treatment of cancer, wherein the cancer has an activating mutation of a receptor belonging to an ErbB family of receptors.

10. The compound for use according to claim 9, wherein the activating mutation of the receptor is an insertion within exon 20 of epidermal growth factor receptor (EGFR) or within exon 20 of human epidermal growth factor receptor (HER) or wherein the activating mutation of the receptor is selected from the group consisting of Her2 A775_G776insYVMA, EGFR D770_N771insSVD, EGFR H773_V774insNPH, EGFR V769_D770insASV, EGFR P772_H773insPR, EGFR T790M and EGFR T790ML858R..

11. The compound for use according to claim 9, wherein the cancer is selected from the group consisting of breast cancer, colon cancer, prostate cancer, lung cancer, gastric cancer, ovarian cancer, renal cancer, hepatocellular cancer, thyroid cancer, uterine cancer, esophagus cancer, squamous cell cancer, leukemia, lymphoma, osteosarcoma, melanoma, glioblastoma and neuroblastoma.

12. The compound for use according to any one of claims 9 - 11, wherein the cancer is non-small cell lung cancer or mamma carcinoma.

13. A compound according to claim 1 - 12 in combination with at least one anticancer drug for use in treatment of cancer.

14. A method for producing a compound of the formula (**Ia-1**), comprising:
**Step A1:** perfoming a first cross coupling reaction of pyridine compound 1* with alkyne comopund **2a*** 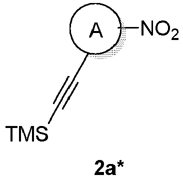 to obtain a compound **3*** 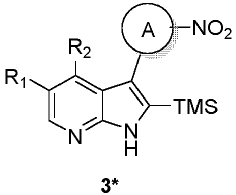 in the presence of a first palladium catalyst, and a first base;
**Step B1:** converting a trimethylsilyl group of the compound **3*** to a halide like an iodide to obtain a compound **4*** 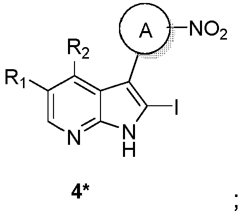
**Step C1:** perfoming a second cross coupling reaction of **4*** with a compound **5*** 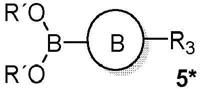 in the presence of a second palladium catalyst, and a second base to obtain a compound **6*** 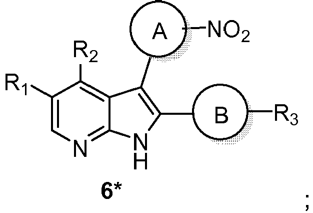
**Step D1:** reducing nitro (NO₂) group of the compound **6*** to a primary amine (NH₂) group to obtain a compound **7***; and
**Step E1:** perfoming a coupling reaction of the compound **7*** 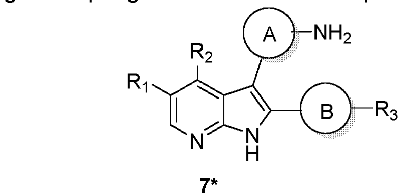 with a compound HO-**R₅** or AG-**R₅** to obtain a product compound of the formula (**Ia-1**) 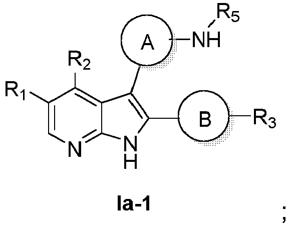 a method for producing a compound of the formula (**Ia-1**), comprising:
**Step A1:** perfoming a first cross coupling reaction of pyridine compound 1* with alkyne compound **2a*** 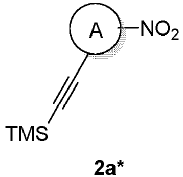 to obtain a compound **3*** 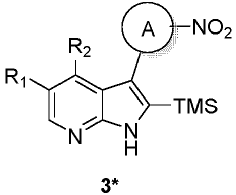 in the presence of a first palladium catalyst, and a first base;
**Step D2:** reducing nitro (NO₂) group of the compound **3*** to a primary amine (NH₂) group to obtain a compound **10*** 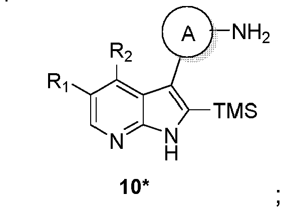
**Step E2:** perfoming a coupling reaction of the compound **10*** with a compound HO-**R₅** or AG-**R₅** to obtain a compound **11*** 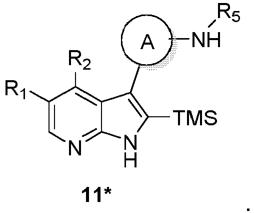
**Step B2:** converting a trimethylsilyl group of the compound **11*** to a halide like an iodide to obtain a compound **12*** 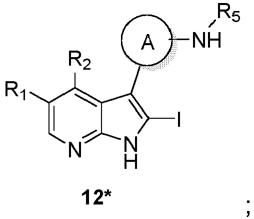 and
**Step C2:** perfoming a second cross coupling reaction of the compound **12*** with a compound **5*** in the presence of a second palladium catalyst, and a second base to obtain a product compound of the formula (la-1) 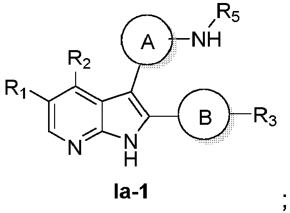 or
a method for producing a compound of the formula **(Ib),** comprising:
**Step A3:**
i) perfoming a first cross coupling reaction of pyridine compound **1*** 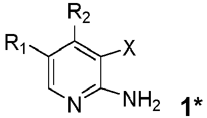 with alkyne comopund **2b*** 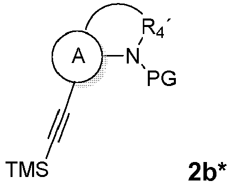 in the presence of a first palladium catalyst, and a first base; and
ii) removing a protecting group PG of a resulting comopund after the step i) to obtain a compound **3b*** 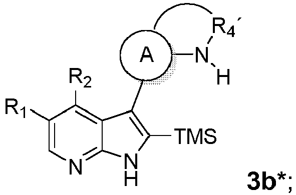
**Step** E3: perfoming a coupling reaction of the compound **3b*** with a compound HO-**R₅** or AG-**R₅** to obtain a compound **11b*** 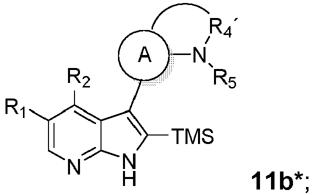
**Step** B3: converting a trimethylsilyl group of the compound **11b*** to a halide like an iodide to obtain a compound **12b*** 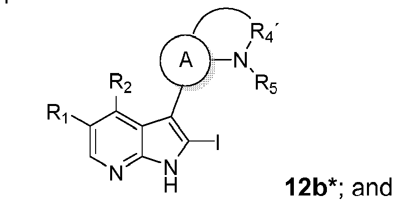
**Step C3:** perfoming a second cross coupling reaction of the compound **12b*** with a compound **5*** 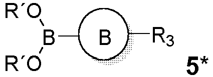 in the presence of a second palladium catalyst, and a second base to obtain a product compound of the formula (**Ib**) 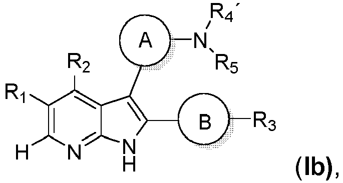 wherein A, B, R₁, R₂, R₃, R₄, R₄', R₅ and 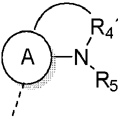 have the same meanings as defined in the formula (I) of claim 1;
X is a leaving group and represents Cl, Br, I, or OTf;
AG is an activating group of carboxylic acid;
PG is an amino protecting group;
TMS is a trimethylsilyl group;
R' is H or an alkyl chain with 1-10 carbon atoms or a cycloalkyl chain with 3 to 12 carbon atoms or both residues R' represent together a residue derived from pinacol..

15. An intermediate compound selected from the group consisting of the compounds **3***, **3b***, **4***, **7***, **10***, **11***, **11b***, **12*** and **12b***: 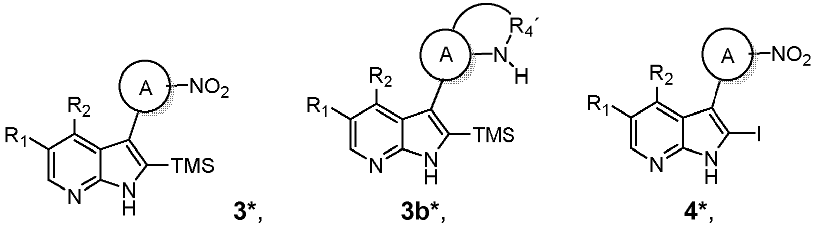 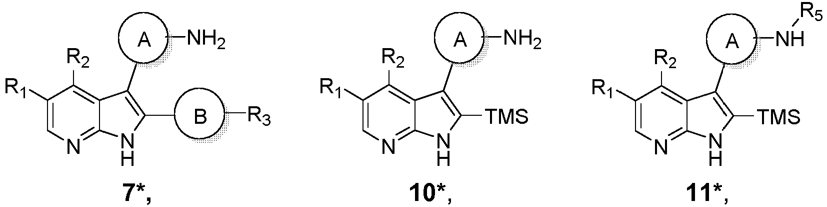 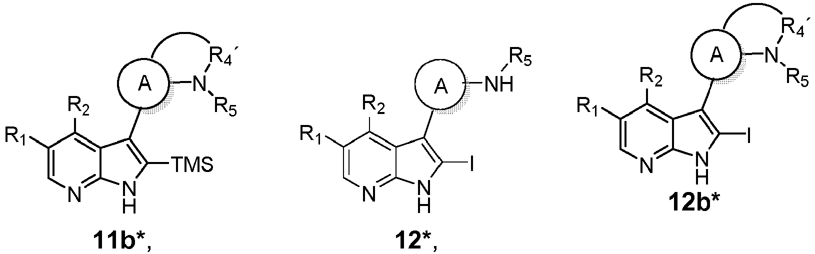 wherein A, B, R₁, R₂, R₃, R₄, R_{4'}, R₅, and 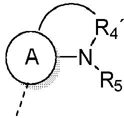 have the same meanings as defined in claim 1 and TMS is trimethyl silyl group.

## Patentansprüche

1. Eine Verbindung der Formel (I) 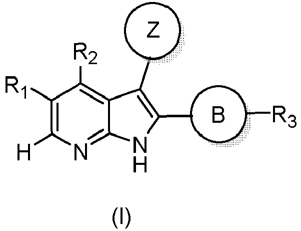 wobei 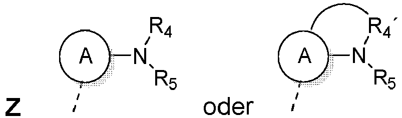 darstellt; 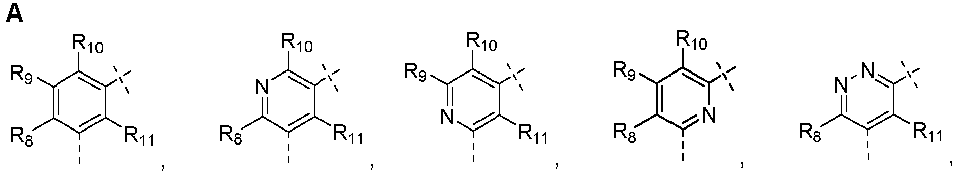 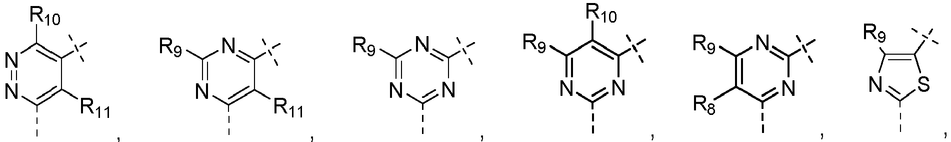 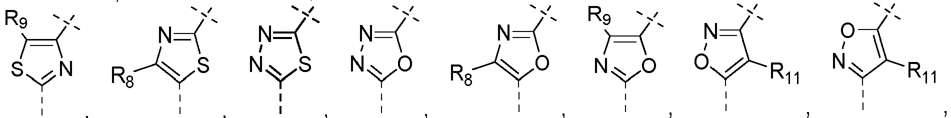 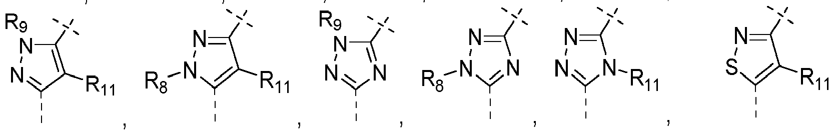 oder 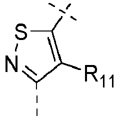 darstellt; 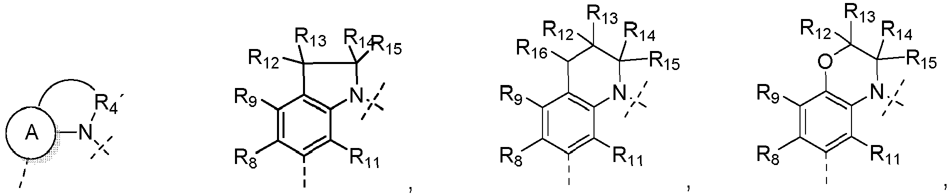 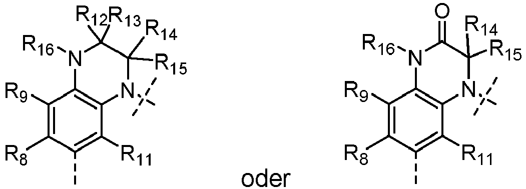 darstellt;
B 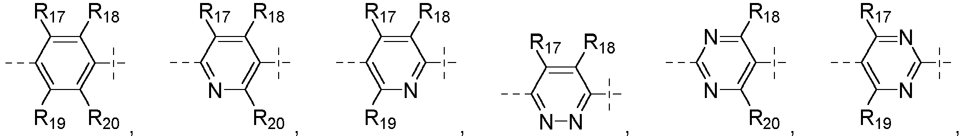 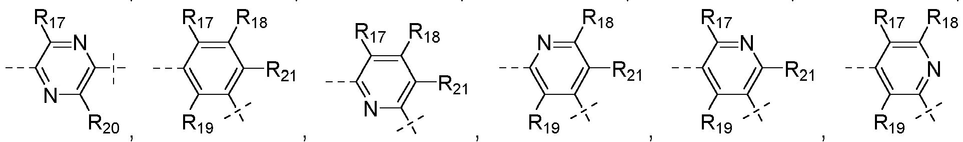 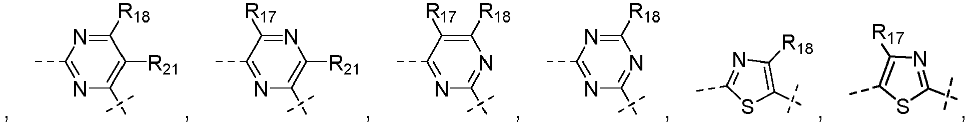 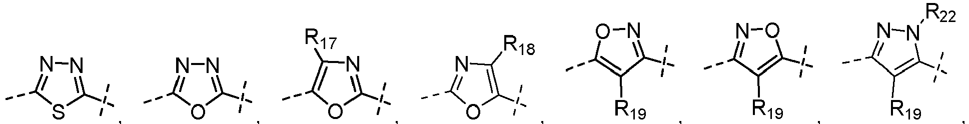 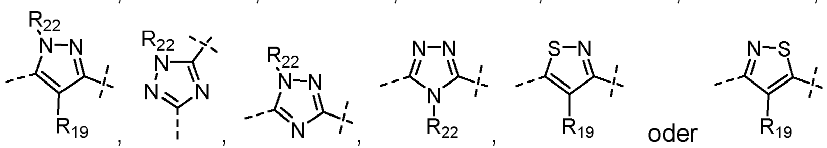 darstellt; **R₁** -H, -**R***, -CH₂-**R***, -CHR^{a}-**R***, -CR^{a}R^{b}-**R***, -CH₂CH₂-**R***, -CR^{a}R^{b}-CR^{c}R^{d}-**R***, -CH=CH-**R***, -CR^{a}=CR^{b}-**R***, -CH₂CH₂CH₂-**R*** oder -CR^{a}R^{b}-CR^{c}R^{d}-CR^{e}R^{f}-**R*** darstellt;
**R**^{a} - **R**^{f} unabhängig voneinander-H, -F, -Cl, -CH₃, -OCH₃, -CH₂CH₃, -CH₂CH₂CH₃, -CH(CH₃)₂, -CH₂CH(CH₃)₂ oder -cyclo-C₃H₅ darstellen;
**R*** -H, -F, -Cl, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHSO₂CH₃, -NHSO₂C₂H₅, -NHSO₂C₃H₇, -NHSO₂CH(CH₃)₂, -NHSO₂-cyclco-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclco-C₃H₅, -SO(NH)CH₃, -SO(NH)C₂H₅, -SO(NH)C₃H₇, -SO(NH)CH(CH₃)₂, -SO(NH)-cyclco-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclco-C₃H₅, 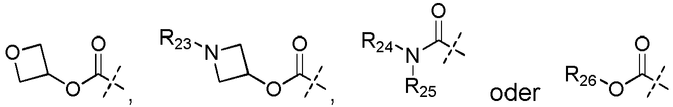 darstellt;
**R₂** -H, -F, -Cl, -Br, -I, -OH, -CN, -CH₃, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅ oder -O-C₂H₄-cyclo-C₃H₅ darstellt;
**R₃** -H, -CH₃, -C₂H₄OH, -OC₂H₄OH -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇R₂₈, -OCH₂CH₂NR₂₇R₂₈, -OCH₂CH₂CH₂NR₂₇R₂₈, -NR₂₉CH₂NR₂₇R₂₈, -NR₂₉CH₂CH₂NR₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 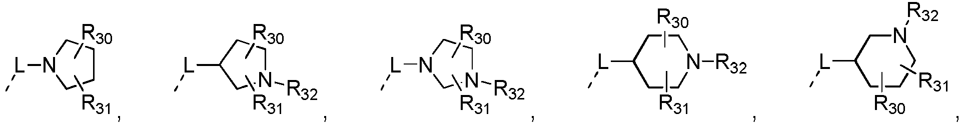 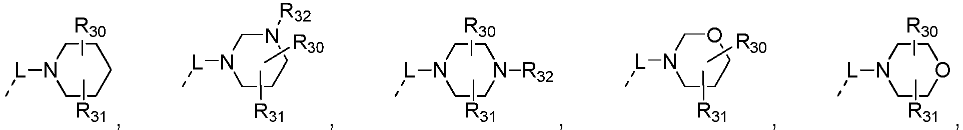 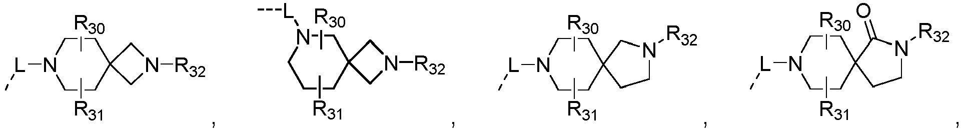 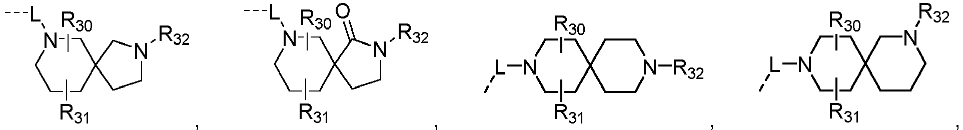 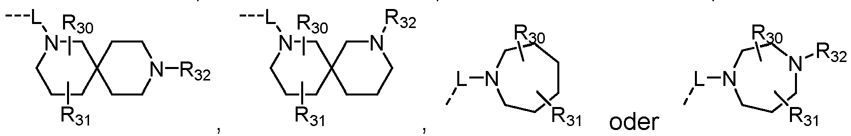 darstellt;
L eine Bindung , -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO- oder -CH₂CH₂OCO- ist;
**R₄** -H, -CH₃ oder -C₂H₅ darstellt;
**R₅** 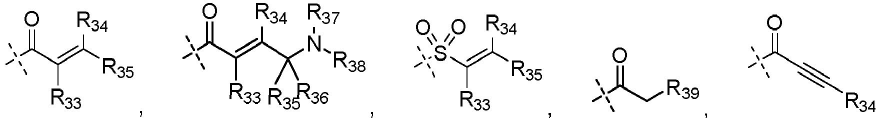 oder 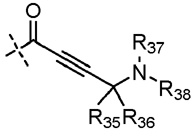 darstellt;
**R₈** - **R₁₁** unabhängig voneinander -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ oder -C₃H₇ darstellen,
oder **R₈** und **R₉** oder **R₉** und **R₁₀** bilden gemeinsam -CH₂-, -CH₂CH₂-, -CH₂CH₂CH₂-, -CH₂CH₂CH₂CH₂-, -CH₂OCH₂-, -CH₂OCH₂CH₂--OCH₂O- oder -OCH₂CH₂O-;
**R₁₂** - **R₁₆** unabhängig voneinander -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ oder -C₃H₇ darstellen;
**R₁₇** - **R₂₁** unabhängig voneinander -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ oder -C₃H₇ darstellen;
**R₂₂** -H, -CH₃, -CF₃, -C₂H₅, -C₃H₇, -COCH₃, -COC₂H₅, -SO₂CH₃ oder -SO₂C₂H₅ darstellt;
**R₂₃** -H, -CH₃, -CF₃, -C₂H₅, -C₃H₇, -CH₂CH₂OCH₃ oder -C₄H₉ darstellt;
**R₂₄** und **R₂₅** unabhängig voneinander -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 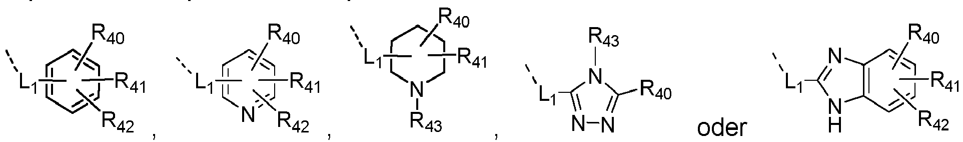 darstellen;
oder **R²⁴** und **R²⁵** gemeinsam -CH₂-, -CH₂CH₂-, -CH₂CH₂CH₂-, -CH₂CH₂CH₂CH₂-, -CH₂OCH₂- oder -CH₂OCH₂CH₂- bilden;
**L₁** eine Bindung, -CH₂-, -CH₂CH₂- oder -CH₂CH₂CH₂- darstellt;
**R₂₆** -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph oder -CH₂Ph darstellt;
**R₂₇** - **R₃₁** unabhängig voneinander -H, -F, -Cl, -OH, -CN, -NH₂, -OCH₃, -OC₂H₅, -OC₃H₇, -OCH(CH₃)₂, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉ oder -cyclo-C₆H₁₁ darstellen;
**R₃₂**, **R₃₇**, **R₃₈**, und **R₄₃** unabhängig voneinander -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃,-SO₂C₂H₅, -SO₂CF₃ oder -SO₂Ph darstellen;
**R₃₃**, **R₃₄**, **R₃₅**, und **R₃₆** unabhängig voneinander -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂ oder -COOC(CH₃)₃ darstellen;
**R₃₉** -F, -Br, -Cl oder -I darstellt;
**R₄₀**, **R₄₁**, und **R₄₂** unabhängig voneinander -H, -F, -Cl, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂, -SO₂CH₃, -SO₂C₂H₅, -SO₂CF₃, -SO₂Ph, -SO₂NH₂, -SO₂NHCH₃, 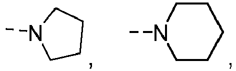 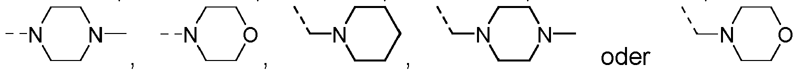 darstellen;
oder ein Enantiomer, ein Gemisch von Enantiomeren, ein Diastereomer, ein Gemisch von Diastereomeren, ein Tautomer, ein Hydrat, ein Solvat oder ein pharmazeutisch verträgliches Salz davon.

2. Die Verbindung gemäß Anspruch 1, wobei
A 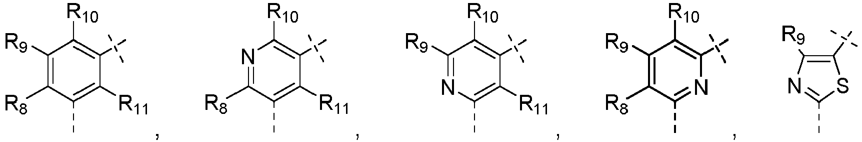 oder 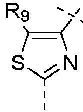 darstellt; 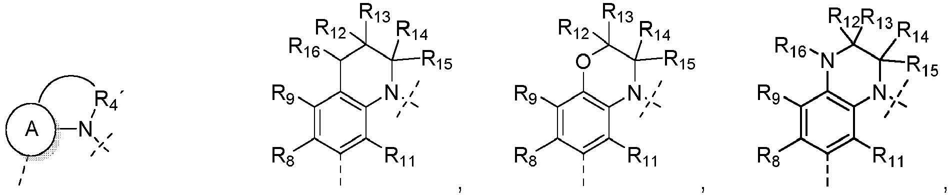 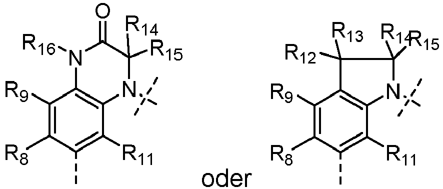 darstellt;
B 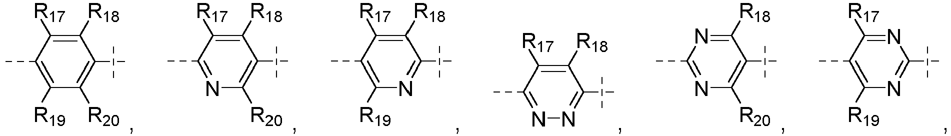 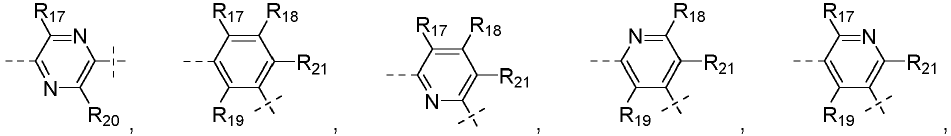 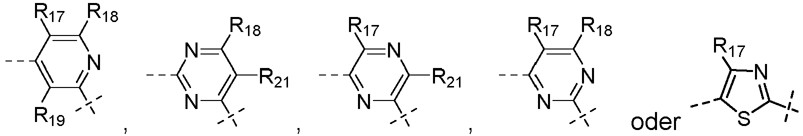 darstellt;
und **R₈** - **R₂₁** die gleichen Bedeutungen wie in Anspruch 1 angegeben haben.

3. Die Verbindung gemäß Anspruch 1 oder 2, wobei
A 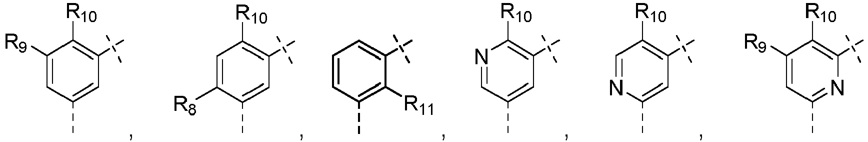 oder 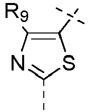 darstellt; 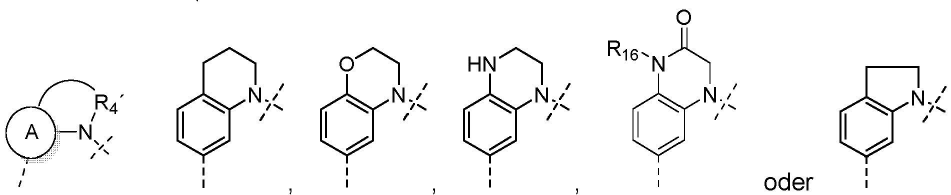 darstellt;
**B** 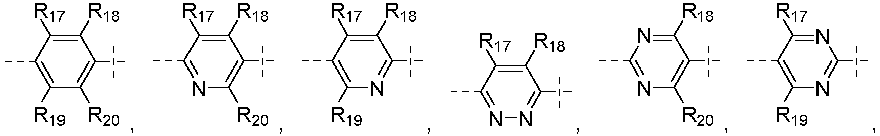 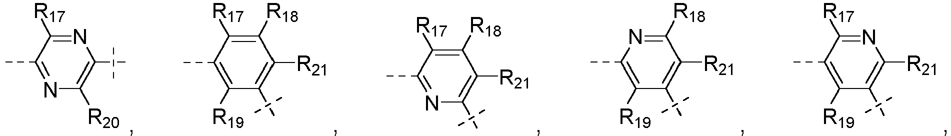 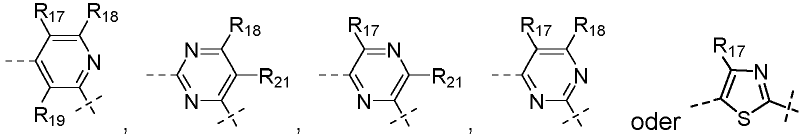 darstellt;
und **R₈**- **R₁₁**, und **R₁₆**- **R₂₁** die gleichen Bedeutungen wie in Anspruch 1 angegeben haben.

4. Die Verbindung gemäß Anspruch 1,wobei die Verbindung die Formel (**Ia**) oder (**Ib**) aufweist: 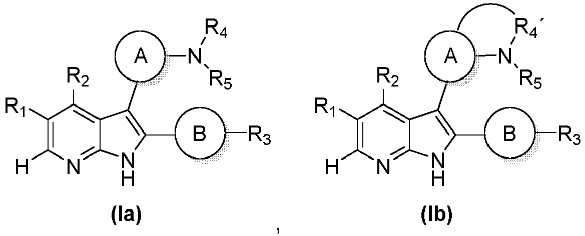 wobei 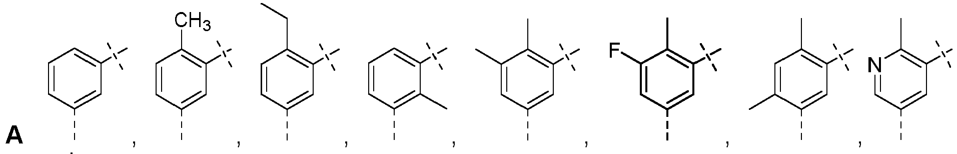 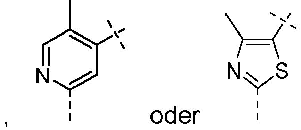 darstellt; 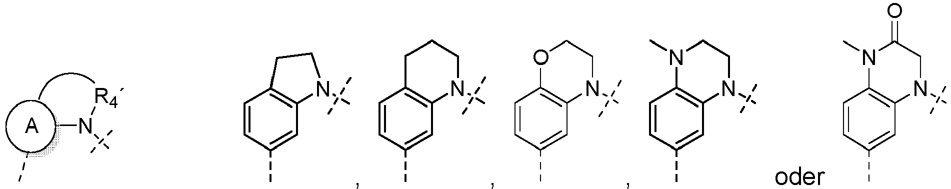 darstellt;
**B** 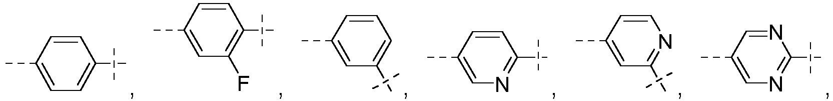 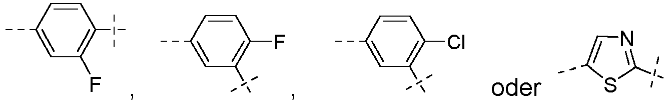 darstellt;
und **R₁**, **R₂**, **R₃**, **R₄**, und **R₅** die gleichen Bedeutungen wie in Anspruch 1 definiert haben.

5. Die Verbindung gemäß einem der Ansprüche 1 - 4, wobei 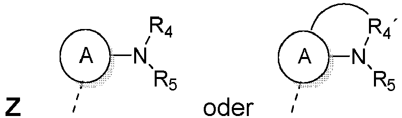 darstellt und ausgewählt wird aus 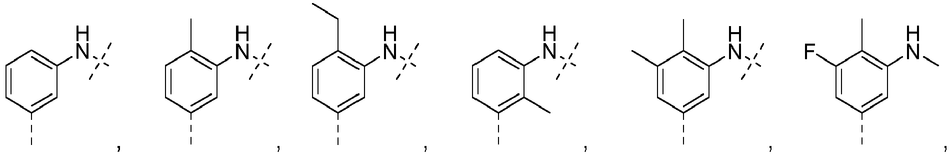 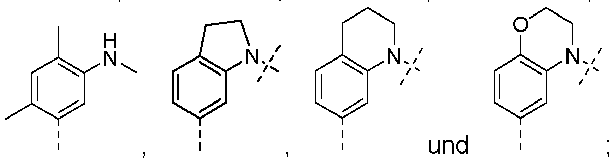 **R₁** -H, -CN, -C₂H₅, -OC₂H₅, -OCF₃, -CH₂OH, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, -CH₂COOH, -CH₂COOCH₃, -CH₂COOCH(CH₃)₂, -CH₂CONH(CH₃), -CH₂CON(CH₃)₂, -NHCOCH₃, -NHCOCH(CH₃)₂, 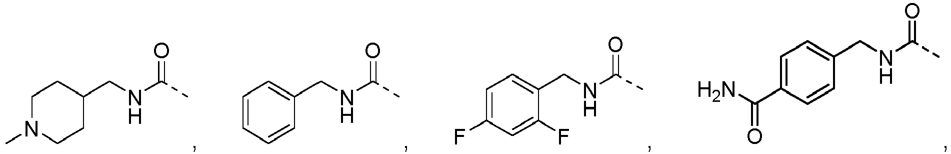 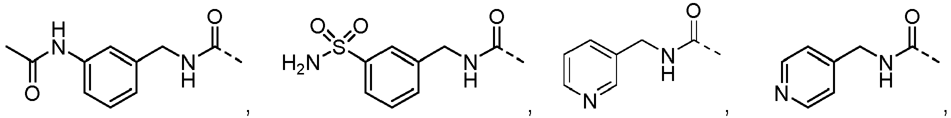 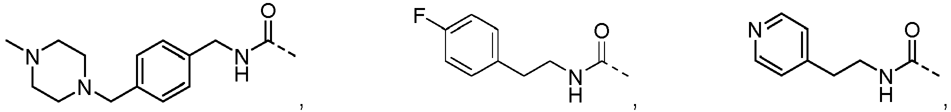 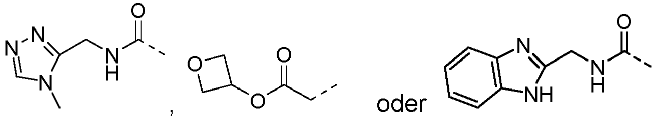 darstellt;
**R₂** -H, -Cl, -OCH₃, -OC₂H₅, oder -OCH(CH₃)₂ darstellt;
B 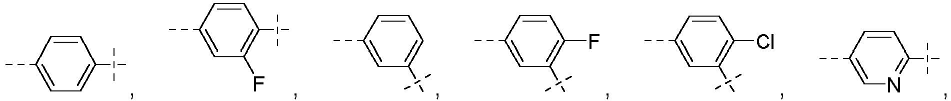 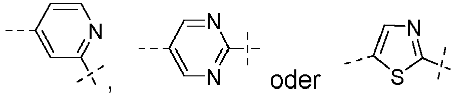 darstellt;
**R₃** -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, -CH₂CH₂OH, -OCH₂CH₂OH, 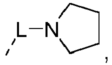 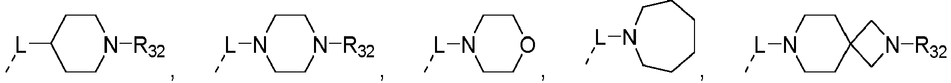 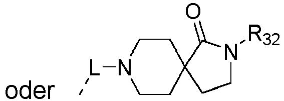 darstellt; 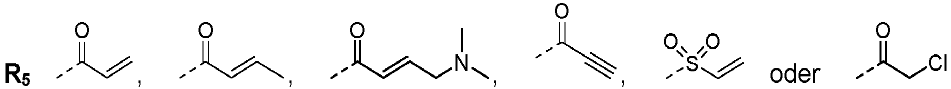 darstellt;
**L** eine Bindung, -CH₂-, -OCH₂CH₂-, -CO-, -CONH-, -SO₂- oder -CONHCH₂CH₂CH₂- ist;
und
**R₃₂** -H, -CH₃, -C₂H₅, -CH(CH₃)₂, -CH₂Ph, -COCH₃ oder -SO₂C₂H₅ darstellt.

6. Die Verbindung gemäß Anspruch 1 ausgewählt aus der Gruppe bestehend aus:
N-(2-Methyl-5-(2-(4-(1-methylpiperidin-4-yl)phenyl)-1H-pyrrolo[2,3-b]pyridin-3-yl)phenyl)acrylamid;
N-(5-(4-Chlor-2-(4-(4-methylpiperazin-1-yl)phenyl)-1H-pyrrolo[2,3-b]pyridin-3-yl)-2-methylphenyl)acrylamid;
N-(5-(4-Chlor-2-(4-(1-methylpiperidin-4-yl)phenyl)-1H-pyrrolo[2,3-b]pyridin-3-yl)-2-methylphenyl)acrylamid;
1-(6-(2-(4-(4-Methylpiperazin-1-yl)phenyl)-1H-pyrrolo[2,3-b]pyridin-3-yl)indolin-1-yl)prop-2-en-1-on;
N-(5-(4-Chlor-2-(4-(2-(dimethylamino)ethyl)phenyl)-1H-pyrrolo[2,3-b]pyridin-3-yl)-2-methylphenyl)acrylamid;
N-(5-(4-Chlor-2-(4-(morpholinomethyl)phenyl)-1H-pyrrolo[2,3-b]pyridin-3-yl)-2-methylphenyl)acrylamid;
N-(5-(4-Chlor-2-(3-morpholinophenyl)-1H-pyrrolo[2,3-b]pyridin-3-yl)-2-methylphenyl)acrylamid;
N-(5-(4-Chloro-2-(3-(morpholinomethyl)phenyl)-1H-pyrrolo[2,3-b]pyridin-3-yl)-2-methylphenyl)acrylamid;
N-(5-(2-(4-(4-Acetylpiperazin-1-yl)phenyl)-4-chlor-1H-pyrrolo[2,3-b]pyridin-3-yl)-2-methylphenyl)acrylamid;
N-(5-(4-Chlor-2-(4-((4-methylpiperazin-1-yl)methyl)phenyl)-1H-pyrrolo[2,3-b]pyridin-3-yl)-2-methylphenyl)acrylamid;
N-(5-(4-Chlor-2-(4-(4-methylpiperazine-1-carbonyl)phenyl)-1H-pyrrolo[2,3-b]pyridin-3-yl)-2-methylphenyl)acrylamid;
N-(5-(4-Chlor-2-(4-((dimethylamino)methyl)phenyl)-1H-pyrrolo[2,3-b]pyridin-3-yl)-2-methylphenyl)acrylamid;
N-(5-(4-Chlor-2-(4-(morpholin-4-carbonyl)phenyl)-1H-pyrrolo[2,3-b]pyridin-3-yl)-2-methylphenyl)acrylamid;
N-(5-(4-Chlor-2-(4-(methylsulfonamido)phenyl)-1H-pyrrolo[2,3-b]pyridin-3-yl)-2-methylphenyl)acrylamid;
N-(5-(4-Chlor-2-(4-(3-(dimethylamino)propoxy)phenyl)-1H-pyrrolo[2,3-b]pyridin-3-yl)-2-methylphenyl)acrylamid;
N-(5-(2-(4-Acetamidophenyl)-4-chlor-1H-pyrrolo[2,3-b]pyridin-3-yl)-2-methylphenyl)acrylamid;
N-(5-(4-Chlor-2-(4-(4-(ethylsulfonyl)piperazin-1-yl)phenyl)-1H-pyrrolo[2,3-b]pyridin-3-yl)-2-methylphenyl)acrylamid;
N-(5-(4-Chlor-2-(3-((4-methylpiperazin-1-yl)methyl)phenyl)-1H-pyrrolo[2,3-b]pyridin-3-yl)-2-methylphenyl)acrylamid;
N-(5-(4-Chlor-2-(3-(4-methylpiperazin-1-yl)phenyl)-1H-pyrrolo[2,3-b]pyridin-3-yl)-2-methylphenyl)acrylamid;
N-(5-(4-Chlor-2-(4-(2-(dimethylamino)ethoxy)phenyl)-1H-pyrrolo[2,3-b]pyridin-3-yl)-2-methylphenyl)acrylamid;
N-(5-(4-Methoxy-2-(4-(1-methylpiperidin-4-yl)phenyl)-1H-pyrrolo[2,3-b]pyridin-3-yl)-2-methylphenyl)acrylamid;
N-(5-(4-Methoxy-2-(4-(4-methylpiperazin-1-yl)phenyl)-1H-pyrrolo[2,3-b]pyridin-3-yl)-2-methylphenyl)acrylamid;
N-(5-(2-(4-(4-Acetylpiperazin-1-yl)phenyl)-4-methoxy-1H-pyrrolo[2,3-b]pyridin-3-yl)-2-methylphenyl)acrylamid;
N-(5-(4-Methoxy-2-(4-(morpholinomethyl)phenyl)-1H-pyrrolo[2,3-b]pyridin-3-yl)-2-methylphenyl)acrylamid;
N-(5-(4-Methoxy-2-(3-((4-methylpiperazin-1-yl)methyl)phenyl)-1H-pyrrolo[2,3-b]pyridin-3-yl)-2-methylphenyl)acrylamid;
N-(5-(4-Methoxy-2-(4-((4-methylpiperazin-1-yl)methyl)phenyl)-1H-pyrrolo[2,3-b]pyridin-3-yl)-2-methylphenyl)acrylamid;
N-(5-(4-Chlor-2-(6-(4-methylpiperazin-1-yl)pyridin-3-yl)-1H-pyrrolo[2,3-b]pyridin-3-yl)-2-methylphenyl)acrylamid;
N-(5-(4-Ethoxy-2-(4-(1-methylpiperidin-4-yl)phenyl)-1H-pyrrolo[2,3-b]pyridin-3-yl)-2-methylphenyl)acrylamid;
N-(5-(4-Chlor-2-(3-(3-(dimethylamino)propoxy)phenyl)-1H-pyrrolo[2,3-b]pyridin-3-yl)-2-methylphenyl)acrylamid;
N-(5-(4-Chlor-2-(2-methoxy-4-(4-methylpiperazin-1-yl)phenyl)-1H-pyrrolo[2,3-b]pyridin-3-yl)-2-methylphenyl)acrylamid;
N-(5-(2-(3-((4-Acetylpiperazin-1-yl)methyl)phenyl)-4-chloro-1H-pyrrolo[2,3-b]pyridin-3-yl)-2-methylphenyl)acrylamid;
N-(5-(4-Chlor-2-(4-(3-(4-ethylpiperazin-1-yl)propoxy)phenyl)-1H-pyrrolo[2,3-b]pyridin-3-yl)-2-methylphenyl)acrylamid;
N-(5-(4-Chlor-2-(pyrimidin-5-yl)-1H-pyrrolo[2,3-b]pyridin-3-yl)-2-methylphenyl)acrylamid;
N-(5-(4-Chlor-2-(3-(3-(4-ethylpiperazin-1-yl)propoxy)phenyl)-1H-pyrrolo[2,3-b]pyridin-3-yl)-2-methylphenyl)acrylamid;
N-(5-(4-Chlor-2-(2-(4-methylpiperazin-1-yl)pyridin-4-yl)-1H-pyrrolo[2,3-b]pyridin-3-yl)-2-methylphenyl)acrylamid;
N-(5-(4-Chlor-2-(3-(morpholine-4-carbonyl)phenyl)-1H-pyrrolo[2,3-b]pyridin-3-yl)-2-methylphenyl)acrylamid;
N-(5-(4-Chlor-2-(3-(2-(dimethylamino)ethoxy)phenyl)-1H-pyrrolo[2,3-b]pyridin-3-yl)-2-methylphenyl)acrylamid;
N-(5-(4-Chlor-2-(4-(piperidin-4-yl)phenyl)-1H-pyrrolo[2,3-b]pyridin-3-yl)-2-methylphenyl)acrylamid;
N-(5-(4-Chlor-2-(6-morpholinopyridin-3-yl)-1H-pyrrolo[2,3-b]pyridin-3-yl)-2-methylphenyl)acrylamid;
N-(5-(2-(6-(4-Acetylpiperazin-1-yl)pyridin-3-yl)-4-chlor-1H-pyrrolo[2,3-b]pyridin-3-yl)-2-methylphenyl)acrylamid;
N-(5-(4-Chlor-2-(2-morpholinopyridin-4-yl)-1H-pyrrolo[2,3-b]pyridin-3-yl)-2-methylphenyl)acrylamid;
1 -(6-(4-Chlor-2-(4-(1-methylpiperidin-4-yl)phenyl)-1H-pyrrolo[2,3-b]pyridin-3-yl)indolin-1-yl)prop-2-en-1-on;
N-(5-(4-Chlor-2-(3-(4-methylpiperazine-1-carbonyl)phenyl)-1H-pyrrolo[2,3-b]pyridin-3-yl)-2-methylphenyl)acrylamid;
N-(5-(2-(3-(4-Acetylpiperazin-1-yl)phenyl)-4-chlor-1H-pyrrolo[2,3-b]pyridin-3-yl)-2-methylphenyl)acrylamid;
3-(3-(3-Acrylamido-4-methylphenyl)-4-chlor-1H-pyrrolo[2,3-b]pyridin-2-yl)-N-(3-(pyrrolidin-1-yl)propyl)benzamid;
3-(3-(3-Acrylamido-4-methylphenyl)-4-chlor-1H-pyrrolo[2,3-b]pyridin-2-yl)-N-(1-benzylpiperidin-4-yl)benzamid;
3-(3-(3-Acrylamido-4-methylphenyl)-4-chlor-1H-pyrrolo[2,3-b]pyridin-2-yl)-N-cyclopropylbenzamid;
3-(3-(3-Acrylamido-4-methylphenyl)-4-chlor-1H-pyrrolo[2,3-b]pyridin-2-yl)-N-(3-(benzyl(methyl)amino)propyl)benzamid;
N-(5-(2-(3-(4-Acetylpiperazine-1-carbonyl)phenyl)-4-chlor-1H-pyrrolo[2,3-b]pyridin-3-yl)-2-methylphenyl)acrylamid;
N-(5-(4-Chlor-2-(3-(2-methyl-2,7-diazaspiro[3.5]nonane-7-carbonyl)phenyl)-1H-pyrrolo[2,3-b]pyridin-3-yl)-2-methylphenyl)acrylamid;
3-(3-(3-Acrylamido-4-methylphenyl)-4-chlor-1H-pyrrolo[2,3-b]pyridin-2-yl)-N-(3-(4-ethylpiperazin-1-yl)propyl)benzamid;
1-(6-(4-Chlor-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-on;
1-(7-(4-Chlor-2-(4-(4-methylpiperazin-1-yl)phenyl)-1H-pyrrolo[2,3-b]pyridin-3-yl)-3,4-dihydrochinolin-1 (2H)-yl)prop-2-en-1-on;
N-(5-(4-Chlor-2-(2-morpholinopyrimidin-5-yl)-1H-pyrrolo[2,3-b]pyridin-3-yl)-2-methylphenyl)acrylamid;
N-(5-(4-Chlor-2-(3-(2-(pyrrolidin-1-yl)ethoxy)phenyl)-1H-pyrrolo[2,3-b]pyridin-3-yl)-2-methylphenyl)acrylamid;
N-(5-(4-Chlor-2-(4-(2-(pyrrolidin-1-yl)ethoxy)phenyl)-1H-pyrrolo[2,3-b]pyridin-3-yl)-2-methylphenyl)acrylamid;
1 -(7-(4-Chlor-2-(4-(1-methylpiperidin-4-yl)phenyl)-1H-pyrrolo[2,3-b]pyridin-3-yl)-3,4-dihydrochinolin-1 (2H)-yl)prop-2-en-1-on;
1-(7-(4-Chlor-2-(3-(2-(dimethylamino)ethoxy)phenyl)-1H-pyrrolo[2,3-b]pyridin-3-yl)-3,4-dihydrochinolin-1(2H)-yl)prop-2-en-1-one;
1 -(7-(4-Chlor-2-(6-((4-methylpiperazin-1-yl)methyl)pyridin-3-yl)-1H-pyrrolo[2,3-b]pyridin-3-yl)-3,4-dihydroquinolin-1 (2H)-yl)prop-2-en-1-on;
N-(5-(4-Chlor-2-(2-(4-methylpiperazin-1-yl)pyrimidin-5-yl)-1H-pyrrolo[2,3-b]pyridin-3-yl)-2-methylphenyl)acrylamid;
N-(5-(4-Chlor-2-(4-(4-ethylpiperazin-1-yl)phenyl)-1H-pyrrolo[2,3-b]pyridin-3-yl)-2-methylphenyl)acrylamid;
N-(5-(4-Chlor-2-(4-(4-isopropylpiperazin-1-yl)phenyl)-1H-pyrrolo[2,3-b]pyridin-3-yl)-2-methylphenyl)acrylamid;
1-(7-(4-Chlor-2-(4-(2-(pyrrolidin-1-yl)ethoxy)phenyl)-1H-pyrrolo[2,3-b]pyridin-3-yl)-3,4-dihydrochinolin-1 (2H)-yl)prop-2-en-1-on;
1 -(7-(4-Chlor-2-(4-(4-isopropylpiperazin-1-yl)phenyl)-1H-pyrrolo[2,3-b]pyridin-3-yl)-3,4-dihydrochinolin-1(2H)-yl)prop-2-en-1-on;
1-(7-(4-Chlor-2-(4-(2-(dimethylamino)ethoxy)phenyl)-1H-pyrrolo[2,3-b]pyridin-3-yl)-3,4-dihydrochinolin-1(2H)-yl)prop-2-en-1-on;
1-(7-(4-Chlor-2-(6-(4-methylpiperazin-1-yl)pyridin-3-yl)-1H-pyrrolo[2,3-b]pyridin-3-yl)-3,4-dihydrochinolin-1(2H)-yl)prop-2-en-1-one;
1-(7-(4-Chlor-2-(3-(2-(pyrrolidin-1-yl)ethoxy)phenyl)-1H-pyrrolo[2,3-b]pyridin-3-yl)-3,4-dihydrochinolin-1 (2H)-yl)prop-2-en-1-on;
1-(7-(4-Chlor-2-(3-((4-methylpiperazin-1-yl)methyl)phenyl)-1H-pyrrolo[2,3-b]pyridin-3-yl)-3,4-dihydrochinolin-1(2H)-yl)prop-2-en-1-on;
N-(5-(4-Chlor-2-(4-(piperazin-1-yl)phenyl)-1H-pyrrolo[2,3-b]pyridin-3-yl)-2-methylphenyl)acrylamid;
1-(6-(4-Chlor-2-(4-(4-methylpiperazin-1-yl)phenyl)-1H-pyrrolo[2,3-b]pyridin-3-yl)indolin-1-yl)prop-2-en-1-on;
1-(6-(4-Chlor-2-(3-fluor-4-(4-methylpiperazin-1-yl)phenyl)-1H-pyrrolo[2,3-b]pyridin-3-yl)indolin-1-yl)prop-2-en-1-on;
1-(6-(2-(4-(4-Acetylpiperazin-1-yl)phenyl)-4-chloro-1H-pyrrolo[2,3-b]pyridin-3-yl)indolin-1-yl)prop-2-en-1-one;
1-(6-(4-Chlor-2-(2-(4-methylpiperazin-1-yl)pyridin-4-yl)-1H-pyrrolo[2,3-b]pyridin-3-yl)indolin-1-yl)prop-2-en-1-on;
1-(6-(4-Chlor-2-(4-((4-methylpiperazin-1-yl)methyl)phenyl)-1H-pyrrolo[2,3-b]pyridin-3-yl)indolin-1-yl)prop-2-en-1-on;
1-(6-(4-Chlor-2-(4-((dimethylamino)methyl)phenyl)-1H-pyrrolo[2,3-b]pyridin-3-yl)indolin-1-yl)prop-2-en-1-on;
1-(6-(4-Chlor-2-(4-(4-isopropylpiperazin-1-yl)phenyl)-1H-pyrrolo[2,3-b]pyridin-3-yl)indolin-1-yl)prop-2-en-1-on;
1-(6-(4-Chlor-2-(4-(4-ethylpiperazin-1-yl)phenyl)-1H-pyrrolo[2,3-b]pyridin-3-yl)indolin-1-yl)prop-2-en-1-on;
1-(6-(4-Chlor-2-(3-(2-(pyrrolidin-1-yl)ethoxy)phenyl)-1H-pyrrolo[2,3-b]pyridin-3-yl)indolin-1-yl)prop-2-en-1-on;
1-(6-(4-Chlor-2-(4-((2-(dimethylamino)ethyl)(methyl)amino)phenyl)-1H-pyrrolo[2,3-b]pyridin-3-yl)indolin-1-yl)prop-2-en-1-on;
1-(6-(4-Chlor-2-(4-(4-methylpiperazine-1-carbonyl)phenyl)-1H-pyrrolo[2,3-b]pyridin-3-yl)indolin-1-yl)prop-2-en-1-on;
1-(6-(4-Chlor-2-(4-(2-(dimethylamino)ethoxy)phenyl)-1H-pyrrolo[2,3-b]pyridin-3-yl)indolin-1-yl)prop-2-en-1-on;
1-(6-(4-Chlor-2-(4-(morpholinomethyl)phenyl)-1H-pyrrolo[2,3-b]pyridin-3-yl)indolin-1-yl)prop-2-en-1-on;
1-(6-(4-Chlor-2-(3-((4-methylpiperazin-1-yl)methyl)phenyl)-1H-pyrrolo[2,3-b]pyridin-3-yl)indolin-1-yl)prop-2-en-1-on;
1-(6-(4-Chlor-2-(6-(4-methylpiperazin-1-yl)pyridin-3-yl)-1H-pyrrolo[2,3-b]pyridin-3-yl)indolin-1-yl)prop-2-en-1-on;
1-(6-(4-Chlor-2-(4-(3-(dimethylamino)propoxy)phenyl)-1H-pyrrolo[2,3-b]pyridin-3-yl)indolin-1-yl)prop-2-en-1-on;
N-(5-(4-Chloro2-(3-fluor-4-(4-methylpiperazin-1-yl)phenyl)-1H-pyrrolo[2,3-b]pyridin-3-yl)-2-methylphenyl)acrylamid;
1-(6-(4-Chlor-2-(4-((2-(dimethylamino)ethyl)(methyl)amino)phenyl)-1H-pyrrolo[2,3-b]pyridin-3-yl)-2H-benzo[b][1,4]oxazin-4(3H)-yl)prop-2-en-1-on;
1-(6-(4-Chlor-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-on;
N-(5-(4-Chlor-2-(4-((2-(dimethylamino)ethyl)(methyl)amino)phenyl)-1H-pyrrolo[2,3-b]pyridin-3-yl)-2-methylphenyl)acrylamid;
N-(5-(5-Cyano-2-(4-(4-methylpiperazin-1-yl)phenyl)-1H-pyrrolo[2,3-b]pyridin-3-yl)-2-methylphenyl)acrylamid;
3-(3-Acrylamido-4-methylphenyl)-N-isopropyl-2-(4-(4-methylpiperazin-1-yl)phenyl)-1H-pyrrolo[2,3-b]pyridin-5-carboxamid;
1-(6-(4-Chlor-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-on;
1-(6-(4-Chlor-2-(4-(4-ethylpiperazin-1-yl)phenyl)-1H-pyrrolo[2,3-b]pyridin-3-yl)-2H-benzo[b][1,4]oxazin-4(3H)-yl)prop-2-en-1-on;
1-(6-(4-Chlor-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-on;
1-(6-(4-Chlor-2-(4-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-on;
1-(6-(4-Chlor-2-(4-((dimethylamino)methyl)phenyl)-1H-pyrrolo[2,3-b]pyridin-3-yl)-2H-benzo[b][1,4]oxazin-4(3H)-yl)prop-2-en-1-on;
1-(6-(4-Chlor-2-(3-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-on;
Isopropyl-3-(3-acrylamido-4-methylphenyl)-2-(4-(4-methylpiperazin-1-yl)phenyl)-1H-pyrrolo[2,3-b]pyridin-5-carboxylat;
1-(6-(4-Ethoxy-2-(2-(4-methylpiperazin-1-yl)pyridin-4-yl)-1H-pyrrolo[2,3-b]pyridin-3-yl)indolin-1-yl)prop-2-en-1-on;
1-(6-(2-(4-((Dimethylamino)methyl)phenyl)-4-ethoxy-1H-pyrrolo[2,3-b]pyridin-3-yl)indolin-1-yl)prop-2-en-1-on;
1-(6-(4-Ethoxy-2-(4-(2-(pyrrolidin-1-yl)ethoxy)phenyl)-1H-pyrrolo[2,3-b]pyridin-3-yl)indolin-1-yl)prop-2-en-1-on;
1-(6-(4-Ethoxy-2-(4-((4-methylpiperazin-1-yl)methyl)phenyl)-1H-pyrrolo[2,3-b]pyridin-3-yl)indolin-1-yl)prop-2-en-1-on;
1-(6-(4-Ethoxy-2-(4-(4-methylpiperazin-1-yl)phenyl)-1H-pyrrolo[2,3-b]pyridin-3-yl)indolin-1-yl)prop-2-en-1-on;
1-(6-(4-Ethoxy-2-(3-(2-(pyrrolidin-1-yl)ethoxy)phenyl)-1H-pyrrolo[2,3-b]pyridin-3-yl)indolin-1-yl)prop-2-en-1-on;
3-(3-Acrylamido-4-methylphenyl)-N-cyclohexyl-2-(4-(4-methylpiperazin-1-yl)phenyl)-1H-pyrrolo[2,3-b]pyridin-5-carboxamid;
1-(6-(4-Methoxy-2-(4-(4-methylpiperazin-1-yl)phenyl)-1H-pyrrolo[2,3-b]pyridin-3-yl)indolin-1-yl)prop-2-en-1-on;
1-(6-(4-Methoxy-2-(2-(4-methylpiperazin-1-yl)pyridin-4-yl)-1H-pyrrolo[2,3-b]pyridin-3-yl)indolin-1-yl)prop-2-en-1-on;
1-(6-(4-Methoxy-2-(4-(2-(pyrrolidin-1-yl)ethoxy)phenyl)-1H-pyrrolo[2,3-b]pyridin-3-yl)indolin-1-yl)prop-2-en-1-on;
3-(3-Acrylamido-4-methylphenyl)-N-benzyl-2-(4-(4-methylpiperazin-1-yl)phenyl)-1H-pyrrolo[2,3-b]pyridin-5-carboxamid;
N-(3-Acetamidobenzyl)-3-(3-acrylamido-4-methylphenyl)-2-(4-(4-methylpiperazin-1-yl)phenyl)-1H-pyrrolo[2,3-b]pyridin-5-carboxamid;
1-(6-(4-Isopropoxy-2-(4-(4-methylpiperazin-1-yl)phenyl)-1H-pyrrolo[2,3-b]pyridin-3-yl)indolin-1-yl)prop-2-en-1-on;
1-(6-(4-Methoxy-2-(4-((4-methylpiperazin-1-yl)methyl)phenyl)-1H-pyrrolo[2,3-b]pyridin-3-yl)indolin-1-yl)prop-2-en-1-on;
1-(6-(4-Methoxy-2-(3-(2-(pyrrolidin-1-yl)ethoxy)phenyl)-1H-pyrrolo[2,3-b]pyridin-3-yl)indolin-1-yl)prop-2-en-1-on;
3-(3-Acrylamido-4-methylphenyl)-N-(4-((4-methylpiperazin-1-yl)methyl)benzyl)-2-(4-(4-methylpiperazin-1-yl)phenyl)-1H-pyrrolo[2,3-b]pyridin-5-carboxamid;
3-(3-Acrylamido-4-methylphenyl)-2-(4-(4-methylpiperazin-1-yl)phenyl)-N-(2-(pyridin-4-yl)ethyl)-1H-pyrrolo[2,3-b]pyridin-5-carboxamid;
3-(3-Acrylamido-4-methylphenyl)-N-(4-fluorophenethyl)-2-(4-(4-methylpiperazin-1-yl)phenyl)-1H-pyrrolo[2,3-b]pyridin-5-carboxamid;
N-((1H-Benzo[d]imidazol-2-yl)methyl)-3-(3-acrylamido-4-methylphenyl)-2-(4-(4-methylpiperazin-1-yl)phenyl)-1H-pyrrolo[2,3-b]pyridine-5-carboxamid;
N-(2-Methyl-5-(2-(2-morpholinopyridin-4-yl)-1H-pyrrolo[2,3-b]pyridin-3-yl)phenyl)acrylamid;
N-(2-Methyl-5-(2-(6-(4-methylpiperazin-1-yl)pyridin-3-yl)-1H-pyrrolo[2,3-b]pyridin-3-yl)phenyl)acrylamid;
3-(3-Acrylamido-4-methylphenyl)-2-(4-(4-methylpiperazin-1-yl)phenyl)-N-(3-sulfamoylbenzyl)-1H-pyrrolo[2,3-b]pyridin-5-carboxamid;
3-(3-Acrylamido-4-methylphenyl)-2-(4-(4-methylpiperazin-1-yl)phenyl)-N-(pyridin-4-ylmethyl)-1H-pyrrolo[2,3-b]pyridin-5-carboxamid;
3-(3-Acrylamido-4-methylphenyl)-2-(4-(4-methylpiperazin-1-yl)phenyl)-N-(pyridin-3-ylmethyl)-1H-pyrrolo[2,3-b]pyridin-5-carboxamid;
3-(3-Acrylamido-4-methylphenyl)-2-(4-(4-methylpiperazin-1-yl)phenyl)-N-((1-methylpiperidin-4-yl)methyl)-1H-pyrrolo[2,3-b]pyridin-5-carboxamid;
3-(3-Acrylamido-4-methylphenyl)-N-((4-methyl-4H-1,2,4-triazol-3-yl)methyl)-2-(4-(4-methylpiperazin-1-yl)phenyl)-1H-pyrrolo[2,3-b]pyridine-5-carboxamid;
3-(3-Acrylamido-4-methylphenyl)-N-(2,4-difluorbenzyl)-2-(4-(4-methylpiperazin-1-yl)phenyl)-1H-pyrrolo[2,3-b]pyridin-5-carboxamid;
N-(4-Acetamidobenzyl)-3-(3-acrylamido-4-methylphenyl)-2-(4-(4-methylpiperazin-1-yl)phenyl)-1H-pyrrolo[2,3-b]pyridin-5-carboxamid;
3-(3-Acrylamido-4-methylphenyl)-N-(3-carbamoylbenzyl)-2-(4-(4-methylpiperazin-1-yl)phenyl)-1H-pyrrolo[2,3-b]pyridin-5-carboxamid;
3-(3-Acrylamido-4-methylphenyl)-2-(4-(4-methylpiperazin-1-yl)phenyl)-1H-pyrrolo[2,3-b]pyridin-5-carbonsäure;
2-Chlor-N-(5-(4-chlor-2-(4-(4-methylpiperazin-1-yl)phenyl)-1H-pyrrolo[2,3-b]pyridin-3-yl)-2-methylphenyl)acetamid;
(E)-N-(5-(4-Chlor-2-(4-(4-methylpiperazin-1-yl)phenyl)-1H-pyrrolo[2,3-b]pyridin-3-yl)-2-methylphenyl)but-2-enamid;
N-(5-(4-Chlor-2-(4-(4-methylpiperazin-1-yl)phenyl)-1H-pyrrolo[2,3-b]pyridin-3-yl)-2-methylphenyl)ethensulfonamid;
(E)-N-(5-(4-Chlor-2-(4-(4-methylpiperazin-1-yl)phenyl)-1H-pyrrolo[2,3-b]pyridin-3-yl)-2-methylphenyl)-4-(dimethylamino)but-2-enamid;
Methyl-3-(3-acrylamido-4-methylphenyl)-2-(4-(4-methylpiperazin-1-yl)phenyl)-1H-pyrrolo[2,3-b]pyridin-5-carboxylat;
Ethyl-3-(3-acrylamido-4-methylphenyl)-2-(4-(4-methylpiperazin-1-yl)phenyl)-1H-pyrrolo[2,3-b]pyridin-5-carboxylat;
Cyclobutyl-3-(3-acrylamido-4-methylphenyl)-2-(4-(4-methylpiperazin-1-yl)phenyl)-1H-pyrrolo[2,3-b]pyridin-5-carboxylat;
Cyclohexyl-3-(3-acrylamido-4-methylphenyl)-2-(4-(4-methylpiperazin-1-yl)phenyl)-1H-pyrrolo[2,3-b]pyridin-5-carboxylat;
Cyclopropyl-3-(3-acrylamido-4-methylphenyl)-2-(4-(4-methylpiperazin-1-yl)phenyl)-1H-pyrrolo[2,3-b]pyridin-5-carboxylat;
Cyclopentyl-3-(3-acrylamido-4-methylphenyl)-2-(4-(4-methylpiperazin-1-yl)phenyl)-1H-pyrrolo[2,3-b]pyridin-5-carboxylat;
2-Methoxyethyl-3-(3-acrylamido-4-methylphenyl)-2-(4-(4-methylpiperazin-1-yl)phenyl)-1H-pyrrolo[2,3-b]pyridin-5-carboxylat;
N-(5-(4-Chlor-2-(4-(4-methylpiperazin-1-yl)phenyl)-1H-pyrrolo[2,3-b]pyridin-3-yl)-2-methylphenyl)propiolamid;
Oxetan-3-yl-3-(3-acrylamido-4-methylphenyl)-2-(4-(4-methylpiperazin-1-yl)phenyl)-1H-pyrrolo[2,3-b]pyridin-5-carboxylat;
(E)-N-(5-(4-Chlor-2-(4-(4-methylpiperazin-1-yl)phenyl)-1H-pyrrolo[2,3-b]pyridin-3-yl)-2-methylphenyl)but-2-enamid;
(E)-N-(5-(4-Chlor-2-(4-(4-methylpiperazin-1-yl)phenyl)-1H-pyrrolo[2,3-b]pyridin-3-yl)-2-methylphenyl)-4-(dimethylamino)but-2-enamid;
N-(5-(4-Chlor-2-(4-(4-methylpiperazin-1-yl)phenyl)-1H-pyrrolo[2,3-b]pyridin-3-yl)-2-methylphenyl)-ethensulfonamid;
2-Chlor-N-(5-(4-chlor-2-(4-(4-methylpiperazin-1-yl)phenyl)-1H-pyrrolo[2,3-b]pyridin-3-yl)-2-methylphenyl)acetamid;
N-(5-(4-Ethoxy-2-(4-(1-methylpiperidin-4-yl)phenyl)-1H-pyrrolo[2,3-b]pyridin-3-yl)-2-methylphenyl)acrylamid;
(E)-Isopropyl-3-(3-(4-(dimethylamino)but-2-enamido)-4-methylphenyl)-2-(4-(4-methylpiperazin-1-yl)phenyl)-1H-pyrrolo[2,3-b]pyridin-5-carboxylat;
Isopropyl-3-(3-acrylamido-4-methylphenyl)-2-(4-(1-methylpiperidin-4-yl)phenyl)-1H-pyrrolo[2,3-b]pyridin-5-carboxylat;
N-(5-(4-Chlor-2-(4-(4-methylpiperazin-1-yl)phenyl)-1H-pyrrolo[2,3-b]pyridin-3-yl)-2,3-dimethylphenyl)acrylamid;
Isopropyl-3-(3-acrylamido-4-methylphenyl)-2-(4-((dimethylamino)methyl)phenyl)-1H-pyrrolo[2,3-b]pyridin-5-carboxylat;
N-(5-(4-Chlor-2-(4-(1-methylpiperidin-4-yl)phenyl)-1H-pyrrolo[2,3-b]pyridin-3-yl)-2,3-dimethylphenyl)acrylamid;
3-(3-Acrylamido-4-methylphenyl)-2-(4-((2-(dimethylamino)ethyl)(methyl)amino)phenyl)-1H-pyrrolo[2,3-b]pyridin-5-carbonsäure;
Isopropyl-3-(3-acrylamido-4-methylphenyl)-2-(4-((2-(dimethylamino)ethyl)(methyl)amino)phenyl)-1H-pyrrolo[2,3-b]pyridin-5-carboxylat;
N-(5-(5-chlor-4-methyl-2-(4-(4-methylpiperazin-1-yl)phenyl)-1H-pyrrolo[2,3-b]pyridin-3-yl)-2-methylphenyl)acrylamid;
Isopropyl-3-(3-acrylamido-4-methylphenyl)-2-(2-(4-methylpiperazin-1-yl)pyridin-4-yl)-1H-pyrrolo[2,3-b]pyridin-5-carboxylat;
Isopropyl-3-(3-acrylamido-4-methylphenyl)-2-(4-(4-ethylpiperazin-1-yl)phenyl)-1H-pyrrolo[2,3-b]pyridin-5-carboxylat;
Isopropyl-3-(3-acrylamido-4-methylphenyl)-2-(4-(2-(pyrrolidin-1-yl)ethoxy)phenyl)-1H-pyrrolo[2,3-b]pyridin-5-carboxylat;
Isopropyl-3-(3-acrylamido-4-methylphenyl)-2-(4-(2-(dimethylamino)ethoxy)phenyl)-1H-pyrrolo[2,3-b]pyridin-5-carboxylat;
Isopropyl-3-(3-acrylamido-4-methylphenyl)-2-(4-((4-methylpiperazin-1-yl)methyl)phenyl)-1H-pyrrolo[2,3-b]pyridin-5-carboxylat;
Isopropyl-3-(3-acrylamido-4-methylphenyl)-2-(4-(2-(dimethylamino)ethyl)phenyl)-1H-pyrrolo[2,3-b]pyridin-5-carboxylat;
Isopropyl-2-(4-(4-acetylpiperazin-1-yl)phenyl)-3-(3-acrylamido-4-methylphenyl)-1H-pyrrolo[2,3-b]pyridin-5-carboxylat;
Isopropyl-3-(3-acrylamido-4-methylphenyl)-2-(6-(4-methylpiperazin-1-yl)pyridin-3-yl)-1H-pyrrolo[2,3-b]pyridin-5-carboxylat;
Isopropyl-3-(3-acrylamido-4-methylphenyl)-2-(3-fluoro-4-(4-methylpiperazin-1-yl)phenyl)-1H-pyrrolo[2,3-b]pyridin-5-carboxylat;
Isopropyl-3-(3-acrylamido-5-fluor-4-methylphenyl)-2-(4-(4-methylpiperazin-1-yl)phenyl)-1H-pyrrolo[2,3-b]pyridin-5-carboxylate;
Isopropyl-3-(3-acrylamido-4-methylphenyl)-2-(2-morpholinopyridin-4-yl)-1H-pyrrolo[2,3-b]pyridin-5-carboxylat;
Isopropyl-3-(3-acrylamido-4-methylphenyl)-2-(4-((4-methylpiperazin-1-yl)sulfonyl)phenyl)-1H-pyrrolo[2,3-b]pyridin-5-carboxylat;
Isopropyl-3-(3-acrylamido-4-methylphenyl)-2-(4-(2-hydroxyethyl)phenyl)-1H-pyrrolo[2,3-b]pyridin-5-carboxylat;
Isopropyl-3-(3-acrylamido-4-methylphenyl)-2-(2-(4-methylpiperazin-1-yl)thiazol-5-yl)-1H-pyrrolo[2,3-b]pyridin-5-carboxylat;
Isopropyl-3-(3-acrylamido-4-methylphenyl)-2-(3-(2-(pyrrolidin-1-yl)ethoxy)phenyl)-1H-pyrrolo[2,3-b]pyridin-5-carboxylat;
Isopropyl-3-(3-acrylamido-4-methylphenyl)-2-(6-morpholinopyridin-3-yl)-1H-pyrrolo[2,3-b]pyridin-5-carboxylat;
Isopropyl-3-(3-acrylamido-5-fluor-4-methylphenyl)-2-(4-((2-(dimethylamino)ethyl)(methyl)amino)phenyl)-1H-pyrrolo[2,3-b]pyridin-5-carboxylat;
Isopropyl-3-(3-acrylamido-4-methylphenyl)-2-(2-morpholinopyrimidin-5-yl)-1H-pyrrolo[2,3-b]pyridin-5-carboxylat;
Isopropyl-3-(3-acrylamido-5-fluor-4-methylphenyl)-2-(6-(4-methylpiperazin-1-yl)pyridin-3-yl)-1H-pyrrolo[2,3-b]pyridin-5-carboxylat;
Isopropyl-3-(3-acrylamido-4-methylphenyl)-2-(2-(4-methylpiperazin-1-yl)pyrimidin-5-yl)-1H-pyrrolo[2,3-b]pyridin-5-carboxylat;
Isopropyl-3-(3-acrylamido-4-methylphenyl)-2-(4-(4-isopropylpiperazin-1-yl)phenyl)-1H-pyrrolo[2,3-b]pyridin-5-carboxylat;
Isopropyl-3-(3-acrylamido-5-fluor-4-methylphenyl)-2-(4-((dimethylamino)methyl)phenyl)-1H-pyrrolo[2,3-b]pyridin-5-carboxylat;
Isopropyl-3-(3-acrylamido-4-methylphenyl)-2-(4-(2-hydroxyethoxy)phenyl)-1H-pyrrolo[2,3-b]pyridin-5-carboxylat;
Isopropyl-3-(3-acrylamido-5-fluor-4-methylphenyl)-2-(4-(1-methylpiperidin-4-yl)phenyl)-1H-pyrrolo[2,3-b]pyridin-5-carboxylat;
Isopropyl-3-(3-acrylamido-5-fluor-4-methylphenyl)-2-(2-(4-methylpiperazin-1-yl)pyridin-4-yl)-1H-pyrrolo[2,3-b]pyridin-5-carboxylat;
Isopropyl-3-(3-acrylamido-4-methylphenyl)-2-(3-((4-methylpiperazin-1-yl)methyl)phenyl)-1H-pyrrolo[2,3-b]pyridin-5-carboxylat;
Isopropyl-3-(3-acrylamido-5-fluor-4-methylphenyl)-2-(4-(2-(dimethylamino)ethoxy)phenyl)-1H-pyrrolo[2,3-b]pyridin-5-carboxylat;
Isopropyl-3-(3-acrylamido-4-methylphenyl)-2-(3-(3-(dimethylamino)propoxy)phenyl)-1H-pyrrolo[2,3-b]pyridin-5-carboxylat;
Isopropyl-3-(3-acrylamido-4-methylphenyl)-2-(4-(4-(ethylsulfonyl)piperazin-1-yl)phenyl)-1H-pyrrolo[2,3-b]pyridin-5-carboxylat;
Isopropyl-2-(3-(4-acetylpiperazin-1-yl)phenyl)-3-(3-acrylamido-4-methylphenyl)-1H-pyrrolo[2,3-b]pyridin-5-carboxylat;
Isopropyl-2-(3-((4-acetylpiperazin-1-yl)methyl)phenyl)-3-(3-acrylamido-4-methylphenyl)-1H-pyrrolo[2,3-b]pyridin-5-carboxylat;
Isopropyl-3-(3-acrylamido-4-methylphenyl)-2-(3-((dimethylamino)methyl)phenyl)-1H-pyrrolo[2,3-b]pyridin-5-carboxylat;
N-(2-Methyl-5-(2-(4-(4-methylpiperazin-1-yl)phenyl)-5-(trifluormethoxy)-1H-pyrrolo[2,3-b]pyridin-3-yl)phenyl)acrylamid;
2-(3-(3-Acrylamido-4-methylphenyl)-2-(4-(4-methylpiperazin-1-yl)phenyl)-1H-pyrrolo[2,3-b]pyridin-5-yl)essigsäure;
Isopropyl 2-(3-(3-acrylamido-4-methylphenyl)-2-(4-(4-methylpiperazin-1-yl)phenyl)-1H-pyrrolo[2,3-b]pyridin-5-yl)acetat;
N-(5-(5-Ethyl-2-(4-(4-methylpiperazin-1-yl)phenyl)-1H-pyrrolo[2,3-b]pyridin-3-yl)-2-methylphenyl)acrylamid;
Isopropyl-3-(1-acryloylindolin-6-yl)-2-(4-(4-methylpiperazin-1-yl)phenyl)-1H-pyrrolo[2,3-b]pyridin-5-carboxylat;
N-(5-(5-Ethoxy-2-(4-(4-methylpiperazin-1-yl)phenyl)-1H-pyrrolo[2,3-b]pyridin-3-yl)-2-methylphenyl)acrylamid;
N-(5-(5-(2-(Dimethylamino)-2-oxoethyl)-2-(4-(4-methylpiperazin-1-yl)phenyl)-1H-pyrrolo[2,3-b]pyridin-3-yl)-2-methylphenyl)acrylamid;
Methyl-2-(3-(3-acrylamido-4-methylphenyl)-2-(4-(4-methylpiperazin-1-yl)phenyl)-1H-pyrrolo[2,3-b]pyridin-5-yl)acetat;
N-(2-Methyl-5-(5-(2-(methylamino)-2-oxoethyl)-2-(4-(4-methylpiperazin-1-yl)phenyl)-1H-pyrrolo[2,3-b]pyridin-3-yl)phenyl)acrylamid;
Oxetan-3-yl 2-(3-(3-acrylamido-4-methylphenyl)-2-(4-(4-methylpiperazin-1-yl)phenyl)-1H-pyrrolo[2,3-b]pyridin-5-yl)acetat;
Isopropyl-3-(3-acrylamido-4-methylphenyl)-4-methyl-2-(4-(4-methylpiperazin-1-yl)phenyl)-1H-pyrrolo[2,3-b]pyridin-5-carboxylat;
N-(5-(5-(Hydroxymethyl)-2-(4-(4-methylpiperazin-1-yl)phenyl)-1H-pyrrolo[2,3-b]pyridin-3-yl)-2-methylphenyl)acrylamid;
N-(5-(5-Isobutyramido-2-(4-(4-methylpiperazin-1-yl)phenyl)-1H-pyrrolo[2,3-b]pyridin-3-yl)-2-methylphenyl)acrylamid;
N-(5-(5-Acetamido-2-(4-(4-methylpiperazin-1-yl)phenyl)-1H-pyrrolo[2,3-b]pyridin-3-yl)-2-methylphenyl)acrylamid;
Isopropyl-3-(5-acrylamido-2,4-dimethylphenyl)-2-(4-(4-methylpiperazin-1-yl)phenyl)-1H-pyrrolo[2,3-b]pyridin-5-carboxylat;
Isopropyl-3-(3-(acrylamidomethyl)phenyl)-2-(4-(4-methylpiperazin-1-yl)phenyl)-1H-pyrrolo[2,3-b]pyridin-5-carboxylat;
Isopropyl-3-(3-acrylamido-4-methylphenyl)-2-(4-((2-(dimethylamino)ethyl)(methyl)amino)phenyl)-4-methyl-1H-pyrrolo[2,3-b]pyridin-5-carboxylat;
Isopropyl-3-(3-acrylamido-4-methylphenyl)-2-(4-(2-(dimethylamino)ethoxy)phenyl)-4-methyl-1H-pyrrolo[2,3-b]pyridin-5-carboxylat;
Isopropyl-3-(3-acrylamido-4-ethylphenyl)-2-(4-(4-methylpiperazin-1-yl)phenyl)-1H-pyrrolo[2,3-b]pyridin-5-carboxylat;
Isopropyl-3-(3-acrylamido-2-methylphenyl)-2-(4-(4-methylpiperazin-1-yl)phenyl)-1H-pyrrolo[2,3-b]pyridin-5-carboxylat;
Isopropyl-3-(3-acrylamido-2-methylphenyl)-2-(4-((2-(dimethylamino)ethyl)(methyl)amino)phenyl)-1H-pyrrolo[2,3-b]pyridin-5-carboxylat, und
Isopropyl-3-(3-acrylamido-2-methylphenyl)-2-(4-(2-(dimethylamino)ethoxy)phenyl)-1H-pyrrolo[2,3-b]pyridin-5-carboxylat
oder ein Enantiomer, ein Gemisch von Enantiomeren, ein Diastereomer, ein Gemisch von Diastereomeren, ein Tautomer, ein Hydrat, ein Solvat der oben genannten Verbindungen oder pharmazeutisch verträgliche Salze davon.

7. Eine Verbindung gemäß einem der Ansprüche 1 - 6 als selektiver Inhibitor von Exon 20 Mutationen von EGFR und Her2.

8. Eine Verbindung gemäß einem der Ansprüche 1 - 6 zur Verwendung als ein Medikament.

9. Eine Verbindung gemäß einem der Ansprüche 1 - 6 zur Verwendung in der Behandlung von Krebs oder zur Verwendung in der Behandlung von Krebs, wobei der Krebs eine aktivierende Mutation eines Rezeptors aufweist, der zu einer ErbB-Rezeptorfamilie gehört.

10. Die Verbindung zur Verwendung gemäß Anspruch 9, wobei die aktivierende Mutation des Rezeptors eine Insertion innerhalb des Exons 20 des epidermalen Wachstumsfaktorrezeptors (EGFR) oder innerhalb des Exons 20 des humanen epidermalen Wachstumsfaktorrezeptors (HER) ist, oder wobei die aktivierende Mutation des Rezeptors ausgewählt wird aus der Gruppe bestehend aus Her2 A775_G776insYVMA, EGFR D770_N771insSVD, EGFR H773_V774insNPH, EGFR V769_D770insASV, EGFR P772_H773insPR, EGFR T790M und EGFR T790ML858R.

11. Verbindung zur Verwendung gemäß Anspruch 9, wobei der Krebs ausgewählt wird aus der Gruppe bestehend aus Brustkrebs, Dickdarmkrebs, Prostatakrebs, Lungenkrebs, Magenkrebs, Eierstockkrebs, Nierenkrebs, Leberzellkrebs, Schilddrüsenkrebs, Gebärmutterkrebs, Speiseröhrenkrebs, Plattenepithelkrebs, Leukämie, Lymphom, Osteosarkom, Melanom, Glioblastom und Neuroblastom.

12. Die Verbindung zur Verwendung gemäß einem der Ansprüche 9 bis 11, wobei der Krebs ein nicht-kleinzelliger Lungenkrebs oder Brustkrebs ist.

13. Die Verbindung gemäß Anspruch 1 bis 12 in Kombination mit mindestens einem Antikrebsmittel zur Verwendung bei der Behandlung von Krebs.

14. Ein Verfahren zum Herstellen einer Verbindung der Formel **(Ia-1),** umfassend:
**Schritt A1:** Durchführen einer ersten Kreuzkupplungsreaktion von Pyridinverbindung 1* mit Alkinverbindung **2a*** 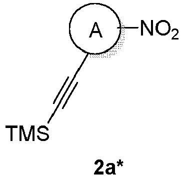
um eine Verbindung **3*** zu erhalten 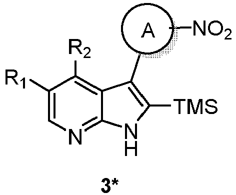 in Gegenwart eines ersten Palladiumkatalysators und einer ersten Base;
**Schritt B1:** Umwandeln einer Trimethylsilylgruppe der Verbindung **3*** zu einem Halogenid wie einem lodid, um eine Verbindung **4*** zu erhalten 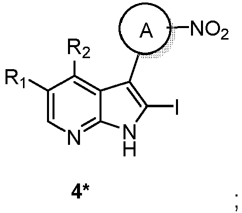
**Schritt C1:** Durchführen einer zweiten Kreuzkupplungsreaktion von **4*** mit einer Verbindung **5*** 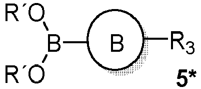 in Gegenwart eines zweiten Palladiumkatalysators und einer zweiten Base, um eine Verbindung **6*** zu erhalten 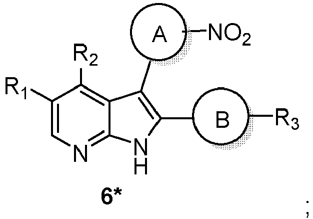
**Schritt D1:** Reduzieren der Nitro(NO₂)-Gruppe der Verbindung **6*** zu einer primären Amino(NH₂)-Gruppe, um eine Verbindung **7*** zu erhalten; und
**Schritt E1:** Durchführen einer Kupplungsreaktion von Verbindung **7*** 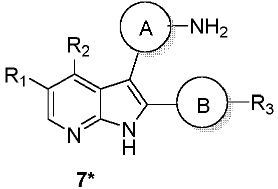 mit einer Verbindung HO-**R₅** oder AG-**R₅**, um eine Produktverbindung der Formel **(Ia-1)** zu erhalten 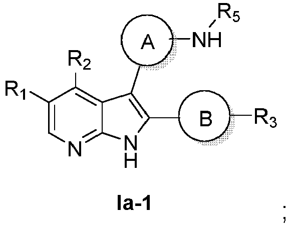 ein Verfahren zum Herstellen einer Verbindung der Formel **(la-1),** umfassend
**Schritt A1:** Durchführen einer ersten Kreuzkupplungsreaktion von Pyridinverbindung 1* mit Alkinverbindung **2a*** 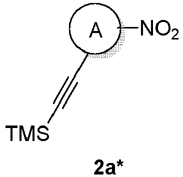 um eine Verbindung **3*** zu erhalten 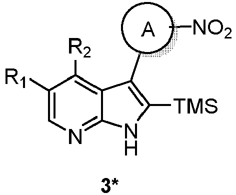 in Gegenwart eines ersten Palladiumkatalysators und einer ersten Base;
**Schritt D2:** Reduzieren der Nitro(NO₂)-Gruppe der Verbindung **3*** zu einer primären Amino(NH₂)-Gruppe, um eine Verbindung **10*** zu erhalten 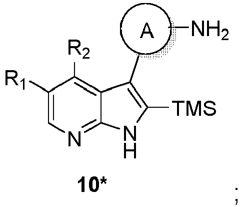
**Schritt E2:** Durchführen einer Kupplungsreaktion von der Verbindung **10*** mit einer Verbindung HO-**R₅** oder AG-**R₅**, um eine Verbindung **11*** zu erhalten 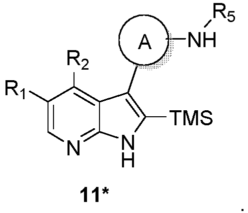
**Schritt B2:** Umwandeln einer Trimethylsilylgruppe der Verbindung **11*** zu einem Halogenid wie einem lodid, um eine Verbindung **12*** zu erhalten 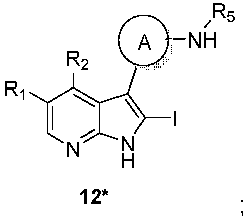 und
**Schritt C2:** Durchführen einer zweiten Kupplungsreaktion von der Verbindung **12*** mit einer Verbindung **5*** in Gegenwart eines zweiten Palladiumkatalysators und einer zweiten Base, um eine Produktverbindung der Formel **(Ia-1)** zu erhalten 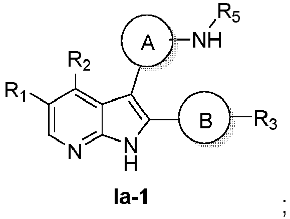 oder
ein Verfahren zum Herstellen einer Verbindung der Formel **(Ib),** umfassend:
**Schritt A3:**
i) Durchführen einer ersten Kupplungsreaktion von Pyridinverbindung **1*** 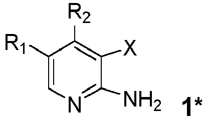 mit Alkinverbindung **2b*** 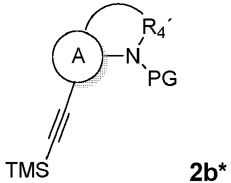 in der Gegenwart eines ersten Palladiumkatalysators und einer ersten Base; und
ii) Entfernen einer Schutzgruppe PG von einer aus Schritt i) resultierenden Verbindung, um eine Verbindung **3b*** zu erhalten 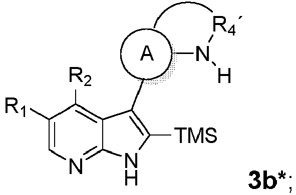
**Schritt E3:** Durchführen einer Kupplungsreaktion von der Verbindung **3b*** mit einer Verbindung HO-**R₅** oder AG-**R₅**, um eine Verbindung **11b*** zu erhalten 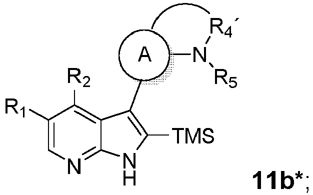
**Schritt B3:** Umwandeln einer Trimethylsilylgruppe der Verbindung **11b*** zu einem Halogenid wie einem lodid, um eine Verbindung **12b*** zu erhalten 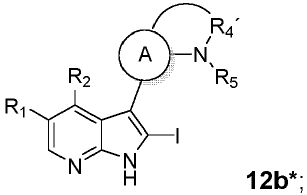 und
**Schritt C3:** Durchführen einer zweiten Kreuzkupplungsreaktion von der Verbindung **12b*** mit einer Verbindung **5*** 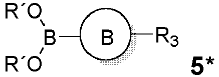 in der Gegenwart eines zweiten Palladiumkatalysators und einer zweiten Base, um eine Produktverbindung der Formel **(Ib)** zu erhalten 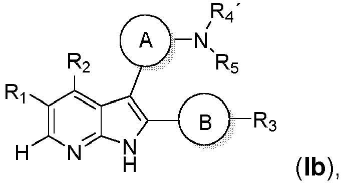
wobei A, B, R₁, R₂, R₃, R₄, R₄', R₅ und 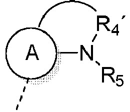 die gleichen Bedeutungen wie in Formel (I) von Anspruch 1 angegeben haben;
X eine Abgangsgruppe ist und Cl, Br, I oder OTf darstellt;
AG eine Aktivierungsgruppe der Carbonsäure ist;
PG eine Aminschutzgruppe ist;
TMS eine Trimethylsilylgruppe ist;
R' H oder eine Alkylkette mit 1-10 Kohlenstoffatomen oder eine Cycloalkylkette mit 3 bis 12 Kohlenstoffatomen ist oder beide Reste R' gemeinsam einen vom Pinacol abgeleiteten Rest darstellen.

15. Eine Zwischenverbindung ausgewählt aus der Gruppe bestehend aus den Verbindungen **3*, 3b*, 4*, 7*, 10*, 11*, 11b*, 12*** und **12b*:** 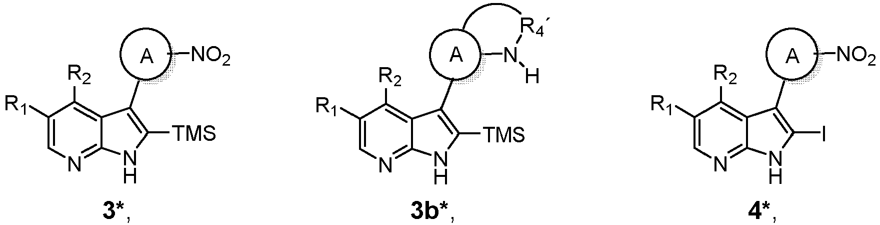 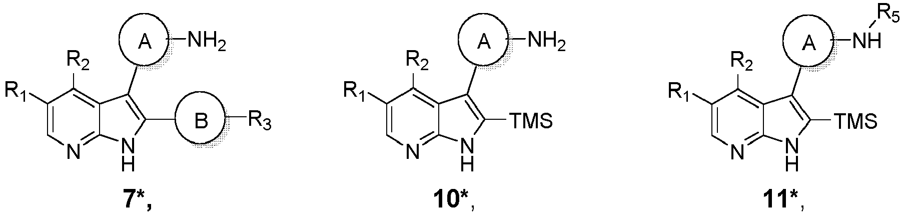 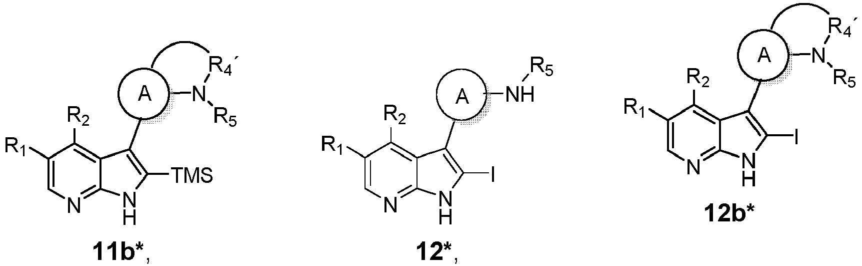 wobei A, B, R₁, R₂, R₃, R₄, R_{4'}, R₅, und 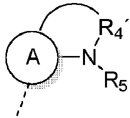 die gleichen Bedeutungen wie in Anspruch 1 angegeben haben und TMS eine Trimethylsilylgruppe ist.

## Revendications

1. Un composé de la formule (I) 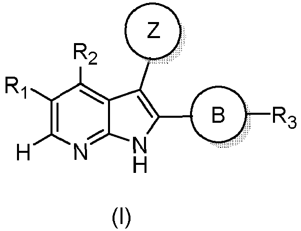 où
**Z** représente 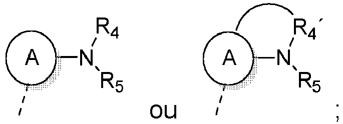
**A** représente 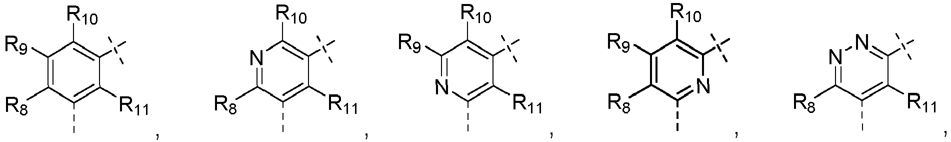 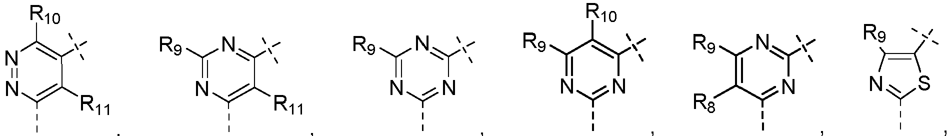 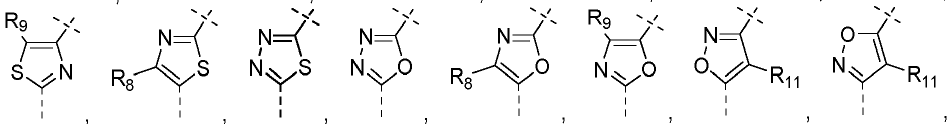 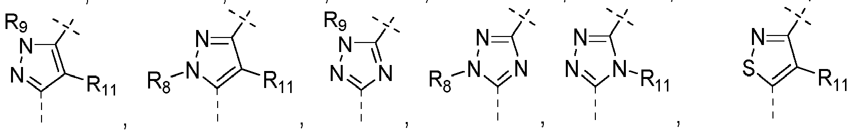 ou 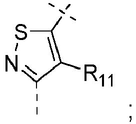 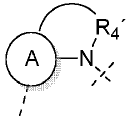 représente 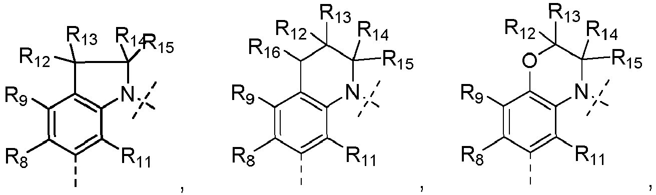 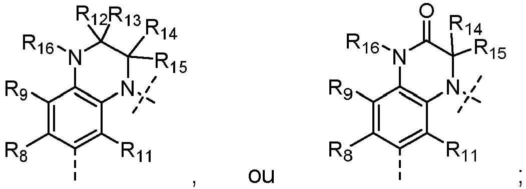
**B** représente 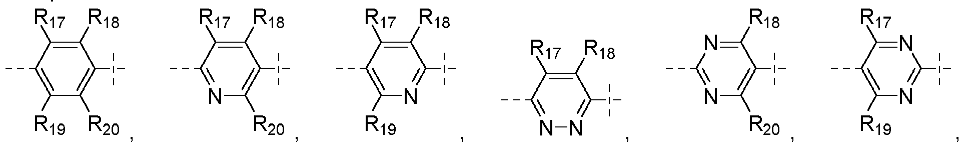 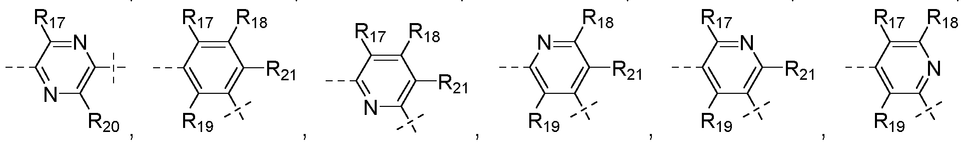 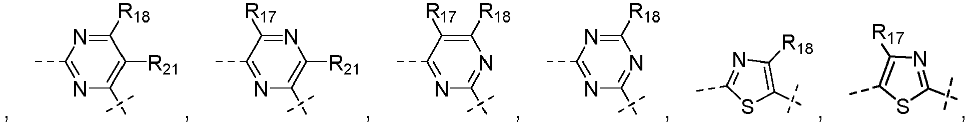 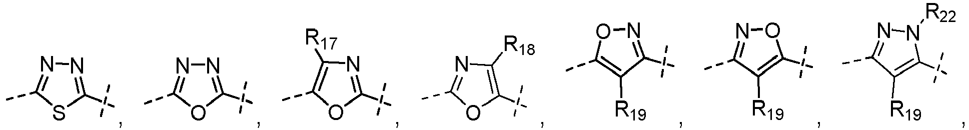 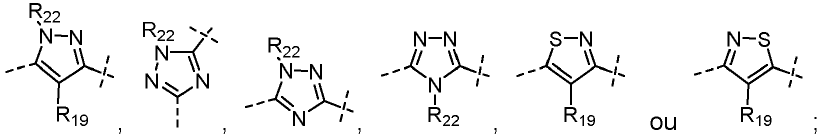
**R₁** représente -H, **-R*,** -CH₂-**R***, -CHR^{a}-**R***, -CR^{a}R^{b}-**R***, -CH₂CH₂-**R***, -CR^{a}R^{b}-CR^{c}R^{d}-**R***, -CH=CH-**R***, -CR^{a}=CR^{b}-**R***, -CH₂CH₂CH₂-**R*** ou -CR^{a}R^{b}-CR^{c}R^{d}-CR^{e}R^{f}-**R***;
**R**^{a} - **R**^{f} représentent indépendamment l'un de l'autre -H, -F, -Cl, -CH₃, -OCH₃, -CH₂CH₃, -CH₂CH₂CH₃, -CH(CH₃)₂, -CH₂CH(CH₃)₂ ou -cyclo-C₃H₅;
**R*** représente -H, -F, -Cl, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHSO₂CH₃, -NHSO₂C₂H₅, -NHSO₂C₃H₇, -NHSO₂CH(CH₃)₂, -NHSO₂-cyclco-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclco-C₃H₅, -SO(NH)CH₃, -SO(NH)C₂H₅, -SO(NH)C₃H₇, -SO(NH)CH(CH₃)₂, -SO(NH)-cyclco-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclco-C₃H₅, 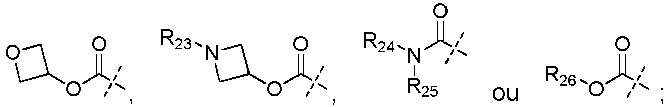
**R₂** représente -H, -F, -Cl, -Br, -I, -OH, -CN, -CH₃, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅ ou -O-C₂H₄-cyclo-C₃H₅;
**R₃** représente -H, -CH₃, -C₂H₄OH, -OC₂H₄OH, -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇R₂₈, -OCH₂CH₂NR₂₇R₂₈, -OCH₂CH₂CH₂NR₂₇R₂₈, -NR₂₉CH₂NR₂₇R₂₈, -NR₂₉CH₂CH₂NR₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 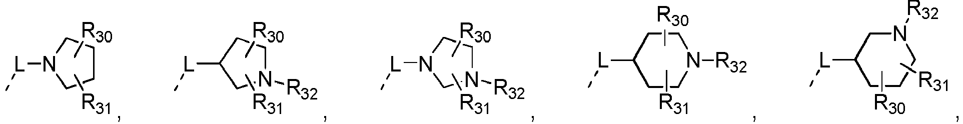 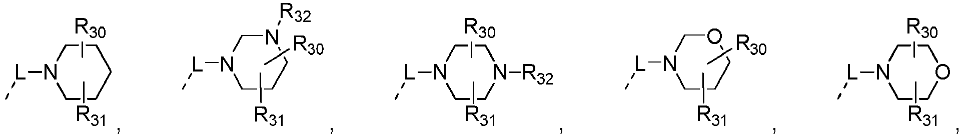 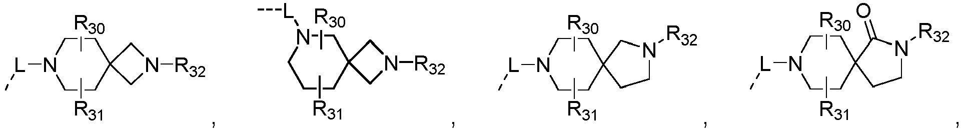 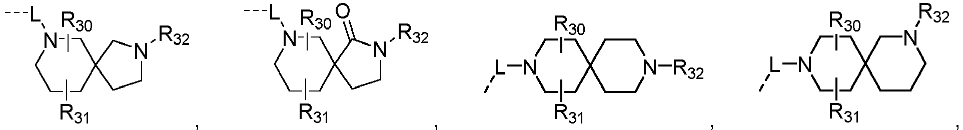 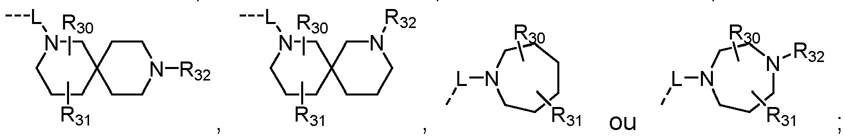
L est une liaison, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO- ou -CH₂CH₂OCO-;
**R₄** représente -H, -CH₃ ou -C₂H₅;
**R₅** représente 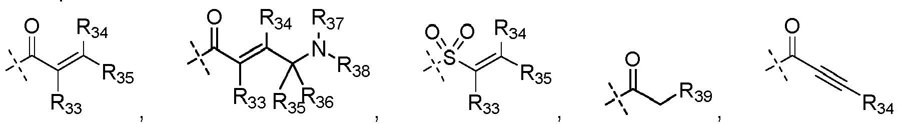 ou 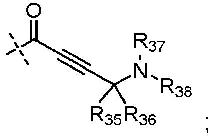
**R₈** - **R₁₁** représentent indépendamment l'un de l'autre -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ ou -C₃H₇,
ou **R₈** et **R₉** ou **R₉** et **R₁₀** forment ensemble -CH₂-, -CH₂CH₂-, -CH₂CH₂CH₂-, -CH₂CH₂CH₂CH₂-, -CH₂OCH₂-, -CH₂OCH₂CH₂-, -OCH₂O-, ou -OCH₂CH₂O-;
**R₁₂** - **R₁₆** représentent indépendamment l'un de l'autre -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ou -C₃H₇;
**R₁₇** - **R₂₁** représentent indépendamment l'un de l'autre -H, -F, -Cl, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅ou -C₃H₇;
**R₂₂** représente -H, -CH₃, -CF₃, -C₂H₅, -C₃H₇, -COCH₃, -COC₂H₅, -SO₂CH₃ ou -SO₂C₂H₅;
**R₂₃** représente -H, -CH₃, -CF₃, -C₂H₅, -C₃H₇, -CH₂CH₂OCH₃ ou -C₄H₉;
**R₂₄** et **R₂₅** représentent indépendamment l'un de l'autre -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 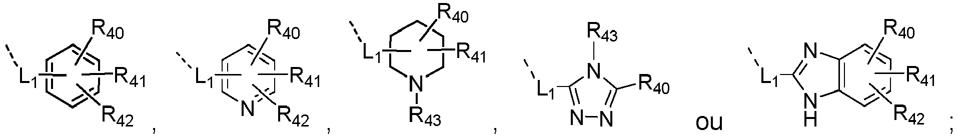 ou **R²⁴** et **R²⁵** forment ensemble -CH₂-, -CH₂CH₂-, -CH₂CH₂CH₂-, -CH₂CH₂CH₂CH₂-, -CH₂OCH₂- ou -CH₂OCH₂CH₂-;
**L₁** représente une liaison, -CH₂-, -CH₂CH₂- ou -CH₂CH₂CH₂-;
**R₂₆** représente -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph ou -CH₂Ph;
**R₂₇** - **R₃₁** représentent indépendamment l'un de l'autre -H, -F, -Cl, -OH, -CN, -NH₂, -OCH₃, -OC₂H₅, -OC₃H₇, -OCH(CH₃)₂, -CH₃, -CF₃, -C₂H₅, - C₃H₇, -CH₂CH₂OCH₃, -CH(CH₃)₂, -C₄H₉, -cyclo-C₃H₅, -cyclo-C₄H₇, -cyclo-C₅H₉ ou -cyclo-C₆H₁₁;
**R₃₂, R₃₇, R₃₈** et **R₄₃** représentent indépendamment l'un de l'autre -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃, -SO₂C₂H₅, -SO₂CF₃ ou -SO₂Ph;
**R₃₃, R₃₄, R₃₅** et **R₃₆** représentent indépendamment l'un de l'autre -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂ ou -COOC(CH₃)₃;
**R₃₉** représente -F, -Br, -Cl ou -I;
**R₄₀, R₄₁** et **R₄₂** représentent indépendamment l'un de l'autre -H, -F, -Cl, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂, -SO₂CH₃, -SO₂C₂H₅, -SO₂CF₃, -SO₂Ph, -SO₂NH₂, -SO₂NHCH₃, 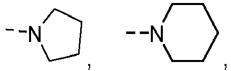 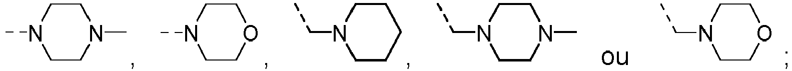 ou un énantiomère, un mélange d'énantiomères, un diastéréomère, un mélange de diastéréomères, un tautomère, un hydrate, un solvate, ou leurs sels pharmaceutiquement acceptables.

2. Le composé conforme à la revendication 1, où
**A** représente 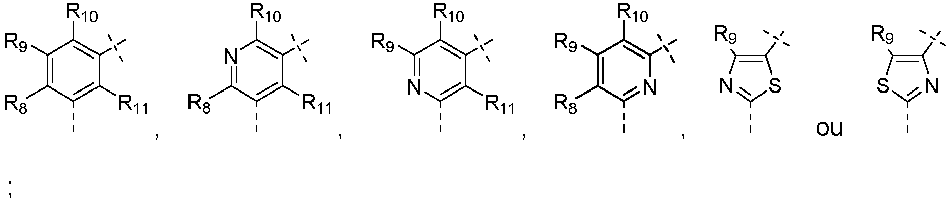 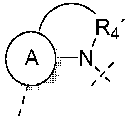 représente 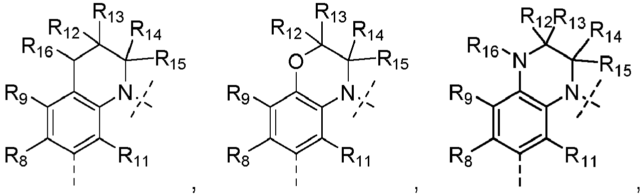 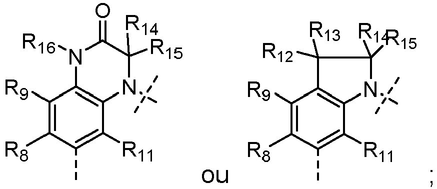
**B** représente 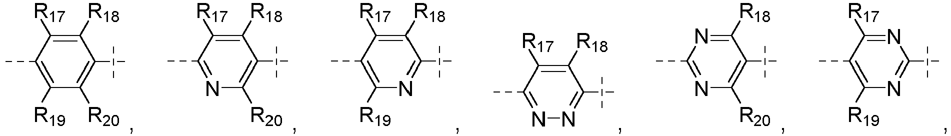 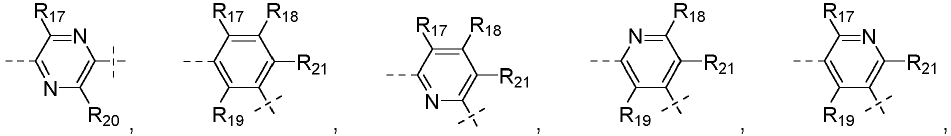 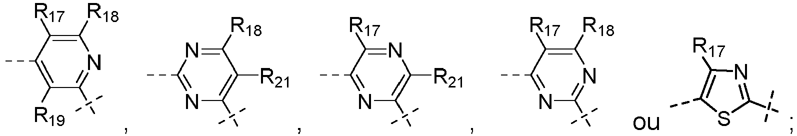 et **R₈** - **R₂₁** ont la signification définie dans la revendication 1.

3. Le composé conforme à la revendication 1 ou 2, où A représente 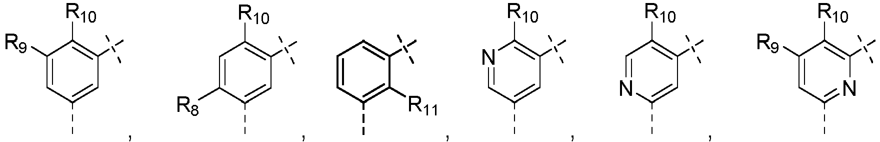 ou 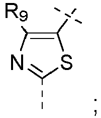 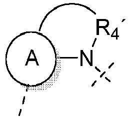 représente 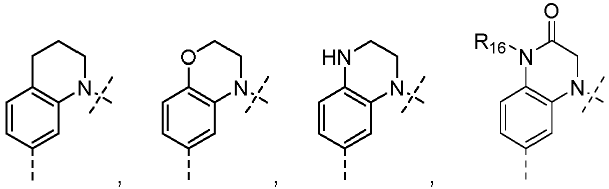 ou 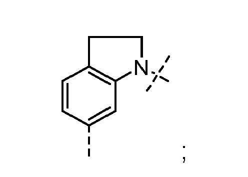 **B** représente 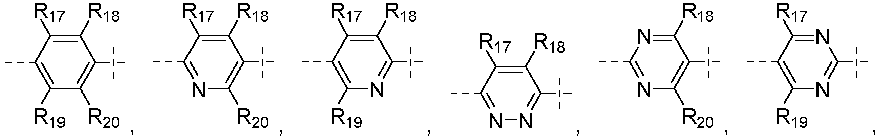 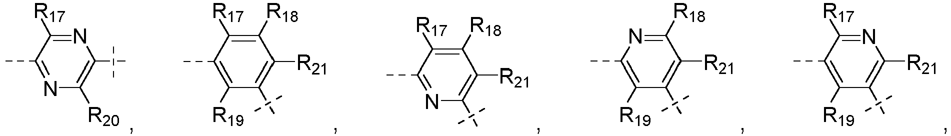 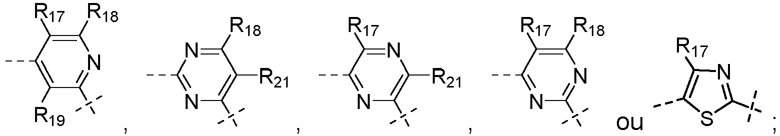 et **R₈- R₁₁** et **R₁₆- R₂₁** ont la signification définie dans la revendication 1.

4. Le composé conforme à la revendication 1, où ledit composé a la formule (la) ou **(Ib)**: 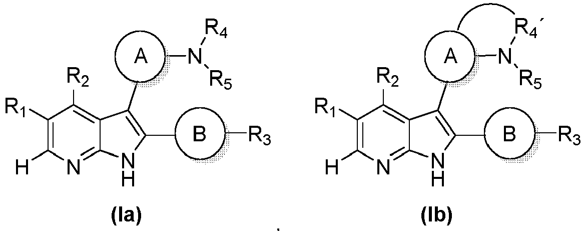 où
**A** représente 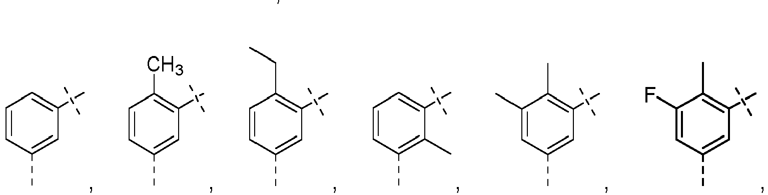 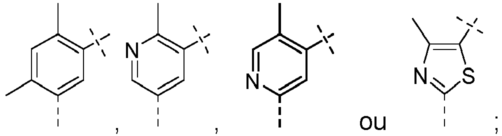 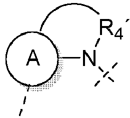 représente 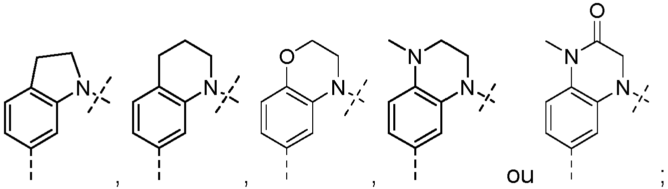
**B** représente 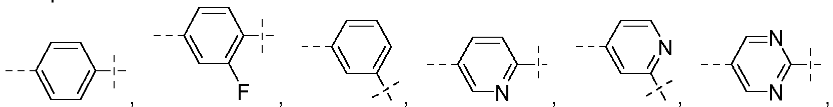 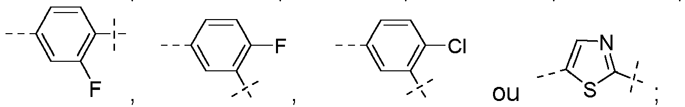 et **R₁, R₂, R₃, R₄** et **R₅** ont la signification définie dans la revendication 1.

5. Le composé conforme à l'une des revendications 1 à 4, où
**Z** représente 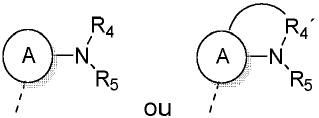 et est sélectionné parmi 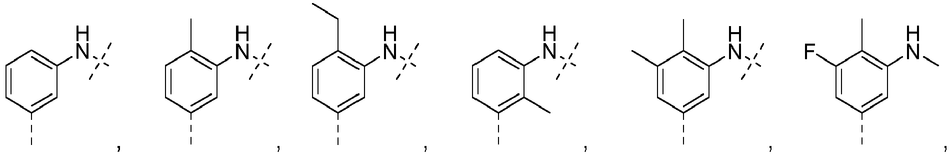 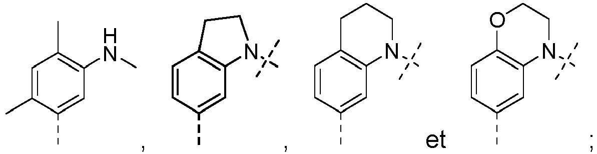
**R₁** représente -H, -CN, -C₂H₅, -OC₂H₅, -OCF₃, -CH₂OH, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, -CH₂COOH, -CH₂COOCH₃, -CH₂COOCH(CH₃)₂, -CH₂CONH(CH₃), -CH₂CON(CH₃)₂, -NHCOCH₃, -NHCOCH(CH₃)₂, 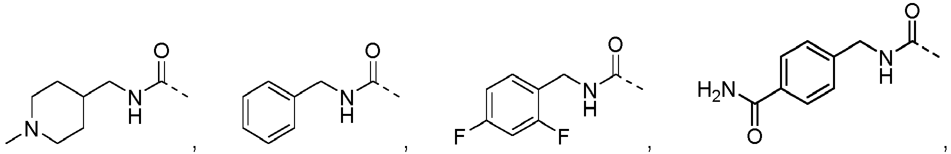 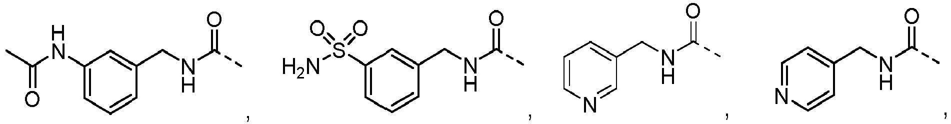 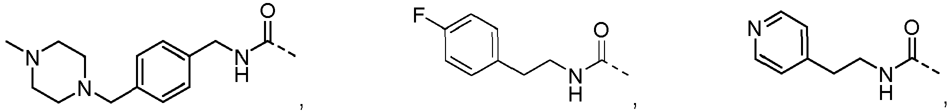 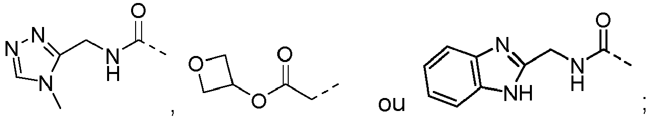
**R₂** représente -H, -Cl, -OCH₃, -OC₂H₅ ou -OCH(CH₃)₂;
**B** représente 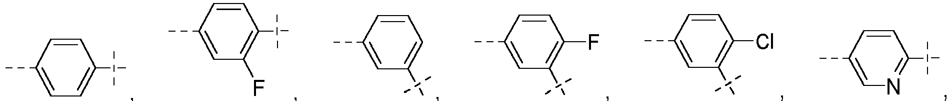 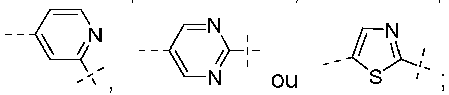
**R₃** représente -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅,-CONH-cyclo-C₆H₁₁, -CONHCH₂CH₂N(CH₃)CH₂Ph, -CH₂CH₂OH, -OCH₂CH₂OH, 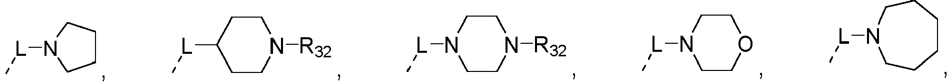 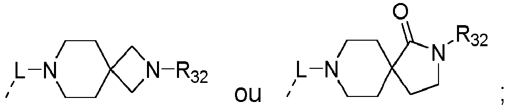
**R₅** représente 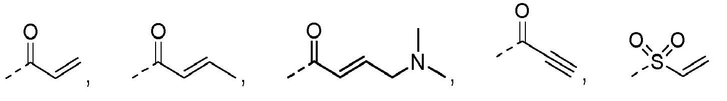 ou 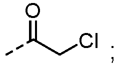
**L** est une liaison, -CH₂-, -OCH₂CH₂-, -CO-, -CONH-, -SO₂- ou -CONHCH₂CH₂CH₂-;
et
**R₃₂** représente -H, -CH₃, -C₂H₅, -CH(CH₃)₂, -CH₂Ph, -COCH₃ ou -SO₂C₂H₅.

6. Le composé conforme à la revendication 1, sélectionné parmi le groupe constitué de :
N-(2-méthyl-5-(2-(4-(1-méthylpipéridine-4-yl)phényl)-1H-pyrrolo[2,3-b]pyridine-3-yl)phényl)acrylamide;
N-(5-(4-chloro-2-(4-(4-méthylpipérazine-1-yl)phényl)-1H-pyrrolo[2,3-b]pyridine-3-yl)-2-méthylphényl)acrylamide;
N-(5-(4-chloro-2-(4-(1-méthylpipéridine-4-yl)phényl)-1H-pyrrolo[2,3-b]pyridine-3-yl)-2-méthylphényl)acrylamide;
1-(6-(2-(4-(4-méthylpipérazine-1-yl)phényl)-1H-pyrrolo[2,3-b]pyridine-3-yl)indoline-1-yl)prop-2-ène-1-one ;
N-(5-(4-chloro-2-(4-(2-(diméthylamino)éthyl)phényl)-1H-pyrrolo[2,3-b]pyridine-3-yl)-2-méthylphényl)acrylamide;
N-(5-(4-chloro-2-(4-(morpholinométhyl)phényl)-1H-pyrrolo[2,3-b]pyridine-3-yl)-2-méthylphényl)acrylamide;
N-(5-(4-chloro-2-(3-morpholinophényl)-1H-pyrrolo[2,3-b]pyridine-3-yl)-2-méthylphényl)acrylamide;
N-(5-(4-chloro-2-(3-(morpholinométhyl)phényl)-1H-pyrrolo[2,3-b]pyridine-3-yl)-2-méthylphényl)acrylamide;
N-(5-(2-(4-(4-acétylpipérazine-1-yl)phényl)-4-chloro-1H-pyrrolo[2,3-b]pyridine-3-yl)-2-méthylphényl)acrylamide;
N-(5-(4-chloro-2-(4-((4-méthylpipérazine-1-yl)méthyl)phényl)-1H-pyrrolo[2,3-b]pyridine-3-yl)-2-méthylphényl)acrylamide;
N-(5-(4-chloro-2-(4-(4-méthylpipérazine-1-carbonyl)phényl)-1H-pyrrolo[2,3-b]pyridine-3-yl)-2-méthylphényl)acrylamide;
N-(5-(4-chloro-2-(4-((diméthylamino)méthyl)phényl)-1H-pyrrolo[2,3-b]pyridine-3-yl)-2-méthylphényl)acrylamide;
N-(5-(4-chloro-2-(4-(morpholine-4-carbonyl)phényl)-1H-pyrrolo[2,3-b]pyridine-3-yl)-2-méthylphényl)acrylamide;
N-(5-(4-chloro-2-(4-(méthylsulfonamido)phényl)-1H-pyrrolo[2,3-b]pyridine-3-yl)-2-méthylphényl)acrylamide;
N-(5-(4-chloro-2-(4-(3-(diméthylamino)propoxy)phényl)-1H-pyrrolo[2,3-b]pyridine-3-yl)-2-méthylphényl)acrylamide ;
N-(5-(2-(4-acétamidophényl)-4-chloro-1H-pyrrolo[2,3-b]pyridine-3-yl)-2-méthylphényl)acrylamide;
N-(5-(4-chloro-2-(4-(4-(éthylsulfonyl)pipérazine-1-yl)phényl)-1H-pyrrolo[2,3-b]pyridine-3-yl)-2-méthylphényl)acrylamide ;
N-(5-(4-chloro-2-(3-((4-méthylpipérazine-1-yl)méthyl)phényl)-1H-pyrrolo[2,3-b]pyridine-3-yl)-2-méthylphényl)acrylamide ;
N-(5-(4-chloro-2-(3-(4-méthylpipérazine-1-yl)phényl)-1H-pyrrolo[2,3-b]pyridine-3-yl)-2-méthylphényl)acrylamide;
N-(5-(4-chloro-2-(4-(2-(diméthylamino)éthoxy)phényl)-1H-pyrrolo[2,3-b]pyridine-3-yl)-2-méthylphényl)acrylamide;
N-(5-(4-méthoxy-2-(4-(1-méthylpipéridine-4-yl)phényl)-1H-pyrrolo[2,3-b]pyridine-3-yl)-2-méthylphényl)acrylamide;
N-(5-(4-méthoxy-2-(4-(4-méthylpipérazine-1-yl)phényl)-1H-pyrrolo[2,3-b]pyridine-3-yl)-2-méthylphényl)acrylamide ;
N-(5-(2-(4-(4-acétylpipérazine-1-yl)phényl)-4-méthoxy-1H-pyrrolo[2,3-b]pyridine-3-yl)-2-méthylphényl)acrylamide;
N-(5-(4-méthoxy-2-(4-(morpholinométhyl)phényl)-1H-pyrrolo[2,3-b]pyridine-3-yl)-2-méthylphényl)acrylamide;
N-(5-(4-méthoxy-2-(3-((4-méthylpipérazine-1-yl)méthyl)phényl)-1H-pyrrolo[2,3-b]pyridine-3-yl)-2-méthylphényl)acrylamide ;
N-(5-(4-méthoxy-2-(4-((4-méthylpipérazine-1-yl)méthyl)phényl)-1H-pyrrolo[2,3-b]pyridine-3-yl)-2-méthylphényl)acrylamide ;
N-(5-(4-chloro-2-(6-(4-méthylpipérazine-1-yl)pyridine-3-yl)-1H-pyrrolo[2,3-b]pyridine-3-yl)-2-méthylphényl)acrylamide;
N-(5-(4-éthoxy-2-(4-(1-méthylpipéridine-4-yl)phényl)-1H-pyrrolo[2,3-b]pyridine-3-yl)-2-méthylphényl)acrylamide;
N-(5-(4-chloro-2-(3-(3-(diméthylamino)propoxy)phényl)-1H-pyrrolo[2,3-b]pyridine-3-yl)-2-méthylphényl)acrylamide ;
N-(5-(4-chloro-2-(2-méthoxy-4-(4-méthylpipérazine-1-yl)phényl)-1H-pyrrolo[2,3-b]pyridine-3-yl)-2-méthylphényl)acrylamide ;
N-(5-(2-(3-((4-acétylpipérazine-1-yl)méthyl)phényl)-4-chloro-1H-pyrrolo[2,3-b]pyridine-3-yl)-2-méthylphényl)acrylamide ;
N-(5-(4-chloro-2-(4-(3-(4-éthylpipérazine-1-yl)propoxy)phényl)-1H-pyrrolo[2,3-b]pyridine-3-yl)-2-méthylphényl)acrylamide ;
N-(5-(4-chloro-2-(pyrimidine-5-yl)-1H-pyrrolo[2,3-b]pyridine-3-yl)-2-méthylphényl)acrylamide; N-(5-(4-chloro-2-(3-(3-(4-éthylpipérazine-1-yl)propoxy)phényl)-1H-pyrrolo[2,3-b]pyridine-3-yl)-2-méthylphényl)acrylamide;
N-(5-(4-chloro-2-(2-(4-méthylpipérazine-1-yl)pyridine-4-yl)-1H-pyrrolo[2,3-b]pyridine-3-yl)-2-méthylphényl)acrylamide;
N-(5-(4-chloro-2-(3-(morpholine-4-carbonyl)phényl)-1H-pyrrolo[2,3-b]pyridine-3-yl)-2-méthylphényl)acrylamide;
N-(5-(4-chloro-2-(3-(2-(diméthylamino)éthoxy)phényl)-1H-pyrrolo[2,3-b]pyridine-3-yl)-2-méthylphényl)acrylamide;
N-(5-(4-chloro-2-(4-(pipéridine-4-yl)phényl)-1H-pyrrolo[2,3-b]pyridine-3-yl)-2-méthylphényl)acrylamide;
N-(5-(4-chloro-2-(6-morpholinopyridine-3-yl)-1H-pyrrolo[2,3-b]pyridine-3-yl)-2-méthylphényl)acrylamide;
N-(5-(2-(6-(4-acétylpipérazine-1-yl)pyridine-3-yl)-4-chloro-1H-pyrrolo[2,3-b]pyridine-3-yl)-2-méthylphényl)acrylamide ;
N-(5-(4-chloro-2-(2-morpholinopyridine-4-yl)-1H-pyrrolo[2,3-b]pyridine-3-yl)-2-méthylphényl)acrylamide;
1-(6-(4-chloro-2-(4-(1-méthylpipéridine-4-yl)phényl)-1H-pyrrolo[2,3-b]pyridine-3-yl)indoline-1-yl)prop-2-ène-1-one;
N-(5-(4-chloro-2-(3-(4-méthylpipérazine-1-carbonyl)phényl)-1H-pyrrolo[2,3-b]pyridine-3-yl)-2-méthylphényl)acrylamide;
N-(5-(2-(3-(4-acétylpipérazine-1-yl)phényl)-4-chloro-1H-pyrrolo[2,3-b]pyridine-3-yl)-2-méthylphényl)acrylamide; 3-(3-(3-acrylamido-4-méthylphényl)-4-chloro-1H-pyrrolo[2,3-b]pyridine-2-yl)-N-(3-(pyrrolidine-1-yl)propyl)benzamide;
3-(3-(3-acrylamido-4-méthylphényl)-4-chloro-1H-pyrrolo[2,3-b]pyridine-2-yl)-N-(1-benzylpipéridine-4-yl)benzamide;
3-(3-(3-acrylamido-4-méthylphényl)-4-chloro-1H-pyrrolo[2,3-b]pyridine-2-yl)-N-cyclopropylbenzamide ;
3-(3-(3-acrylamido-4-méthylphényl)-4-chloro-1H-pyrrolo[2,3-b]pyridine-2-yl)-N-(3-(benzyl(méthyl)amino)propyl)benzamide ;
N-(5-(2-(3-(4-acétylpipérazine-1-carbonyl)phényl)-4-chloro-1H-pyrrolo[2,3-b]pyridine-3-yl)-2-méthylphényl)acrylamide;
N-(5-(4-chloro-2-(3-(2-méthyl-2,7-diazaspiro[3.5]nonane-7-carbonyl)phényl)-1H-pyrrolo[2,3-b]pyridine-3-yl)-2-méthylphényl)acrylamide;
3-(3-(3-acrylamido-4-méthylphényl)-4-chloro-1H-pyrrolo[2,3-b]pyridine-2-yl)-N-(3-(4-éthylpipérazine-1-yl)propyl)benzamide ;
1-(6-(4-chloro-2-(4-(4-méthylpipérazine-1-yl)phényl)-1H-pyrrolo[2,3-b]pyridine-3-yl)-2H-benzo[b][1,4]oxazine-4(3H)-yl)prop-2-ène-1-one ;
1-(7-(4-chloro-2-(4-(4-méthylpipérazine-1-yl)phényl)-1H-pyrrolo[2,3-b]pyridine-3-yl)-3,4-dihydroquinoline-1(2H)-yl)prop-2-ène-1-one ;
N-(5-(4-chloro-2-(2-morpholinopyrimidine-5-yl)-1H-pyrrolo[2,3-b]pyridine-3-yl)-2-méthylphényl)acrylamide;
N-(5-(4-chloro-2-(3-(2-(pyrrolidine-1-yl)éthoxy)phényl)-1H-pyrrolo[2,3-b]pyridine-3-yl)-2-méthylphényl)acrylamide;
N-(5-(4-chloro-2-(4-(2-(pyrrolidine-1-yl)éthoxy)phényl)-1H-pyrrolo[2,3-b]pyridine-3-yl)-2-méthylphényl)acrylamide;
1-(7-(4-chloro-2-(4-(1-méthylpipéridine-4-yl)phényl)-1H-pyrrolo[2,3-b]pyridine-3-yl)-3,4-dihydroquinoline-1(2H)-yl)prop-2-ène-1-one ;
1-(7-(4-chloro-2-(3-(2-(diméthylamino)éthoxy)phényl)-1H-pyrrolo[2,3-b]pyridine-3-yl)-3,4-dihydroquinoline-1(2H)-yl)prop-2-ène-1-one ;
1-(7-(4-chloro-2-(6-((4-méthylpipérazine-1-yl)méthyl)pyridine-3-yl)-1H-pyrrolo[2,3-b]pyridine-3-yl)-3,4-dihydroquinoline-1(2H)-yl)prop-2-ène-1-one ;
N-(5-(4-chloro-2-(2-(4-méthylpipérazine-1-yl)pyrimidine-5-yl)-1H-pyrrolo[2,3-b]pyridine-3-yl)-2-méthylphényl)acrylamide ;
N-(5-(4-chloro-2-(4-(4-éthylpipérazine-1-yl)phényl)-1H-pyrrolo[2,3-b]pyridine-3-yl)-2-méthylphényl)acrylamide;
N-(5-(4-chloro-2-(4-(4-isopropylpipérazine-1-yl)phényl)-1H-pyrrolo[2,3-b]pyridine-3-yl)-2-méthylphényl)acrylamide ;
1 -(7-(4-chloro-2-(4-(2-(pyrrolidine-1-yl)éthoxy)phényl)-1H-pyrrolo[2,3-b]pyridine-3-yl)-3,4-dihydroquinoline-1(2H)-yl)prop-2-ène-1-one ;
1-(7-(4-chloro-2-(4-(4-isopropylpipérazine-1-yl)phényl)-1H-pyrrolo[2,3-b]pyridine-3-yl)-3,4-dihydroquinoline-1(2H)-yl)prop-2-ène-1-one ;
1-(7-(4-chloro-2-(4-(2-(diméthylamino)éthoxy)phényl)-1H-pyrrolo[2,3-b]pyridine-3-yl)-3,4-dihydroquinoline-1(2H)-yl)prop-2-ène-1-one ;
1-(7-(4-chloro-2-(6-(4-méthylpipérazine-1-yl)pyridine-3-yl)-1H-pyrrolo[2,3-b]pyridine-3-yl)-3,4-dihydroquinoline-1(2H)-yl)prop-2-ène-1-one ;
1 -(7-(4-chloro-2-(3-(2-(pyrrolidine-1-yl)éthoxy)phényl)-1H-pyrrolo[2,3-b]pyridine-3-yl)-3,4-dihydroquinoline-1(2H)-yl)prop-2-ène-1-one ;
1-(7-(4-chloro-2-(3-((4-méthylpipérazine-1-yl)méthyl)phényl)-1H-pyrrolo[2,3-b]pyridine-3-yl)-3,4-dihydroquinoline-1(2H)-yl)prop-2-ène-1-one ;
N-(5-(4-chloro-2-(4-(pipérazine-1-yl)phényl)-1H-pyrrolo[2,3-b]pyridine-3-yl)-2-méthylphényl)acrylamide;
1-(6-(4-chloro-2-(4-(4-méthylpipérazine-1-yl)phényl)-1H-pyrrolo[2,3-b]pyridine-3-yl)indoline-1-yl)prop-2-ène-1-one;
1-(6-(4-chloro-2-(3-fluoro-4-(4-méthylpipérazine-1-yl)phényl)-1H-pyrrolo[2,3-b]pyridine-3-yl)indoline-1-yl)prop-2-ène-1-one ;
1-(6-(2-(4-(4-acétylpipérazine-1-yl)phényl)-4-chloro-1H-pyrrolo[2,3-b]pyridine-3-yl)indoline-1-yl)prop-2-ène-1-one;
1-(6-(4-chloro-2-(2-(4-méthylpipérazine-1-yl)pyridine-4-yl)-1H-pyrrolo[2,3-b]pyridine-3-yl)indoline-1-yl)prop-2-ène-1-one;
1-(6-(4-chloro-2-(4-((4-méthylpipérazine-1-yl)méthyl)phényl)-1H-pyrrolo[2,3-b]pyridine-3-yl)indoline-1-yl)prop-2-ène-1-one ;
1-(6-(4-chloro-2-(4-((diméthylamino)méthyl)phényl)-1H-pyrrolo[2,3-b]pyridine-3-yl)indoline-1-yl)prop-2-ène-1-one;
1-(6-(4-chloro-2-(4-(4-isopropylpipérazine-1-yl)phényl)-1H-pyrrolo[2,3-b]pyridine-3-yl)indoline-1-yl)prop-2-ène-1-one;
1-(6-(4-chloro-2-(4-(4-éthylpipérazine-1-yl)phényl)-1H-pyrrolo[2,3-b]pyridine-3-yl)indoline-1-yl)prop-2-ène-1-one;
1-(6-(4-chloro-2-(3-(2-(pyrrolidine-1-yl)éthoxy)phényl)-1H-pyrrolo[2,3-b]pyridine-3-yl)indoline-1-yl)prop-2-ène-1-one;
1-(6-(4-chloro-2-(4-((2-(diméthylamino)éthyl)(méthyl)amino)phényl)-1H-pyrrolo[2,3-b]pyridine-3-yl)indoline-1-yl)prop-2-ène-1-one ;
1-(6-(4-chloro-2-(4-(4-méthylpipérazine-1-carbonyl)phényl)-1H-pyrrolo[2,3-b]pyridine-3-yl)indoline-1-yl)prop-2-ène-1-one;
1-(6-(4-chloro-2-(4-(2-(diméthylamino)éthoxy)phényl)-1H-pyrrolo[2,3-b]pyridine-3-yl)indoline-1-yl)prop-2-ène-1-one;
1-(6-(4-chloro-2-(4-(morpholinométhyl)phényl)-1H-pyrrolo[2,3-b]pyridine-3-yl)indoline-1-yl)prop-2-ène-1-one ;
1-(6-(4-chloro-2-(3-((4-méthylpipérazine-1-yl)méthyl)phényl)-1H-pyrrolo[2,3-b]pyridine-3-yl)indoline-1-yl)prop-2-ène-1-one ;
1-(6-(4-chloro-2-(6-(4-méthylpipérazine-1-yl)pyridine-3-yl)-1H-pyrrolo[2,3-b]pyridine-3-yl)indoline-1-yl)prop-2-ène-1-one;
1-(6-(4-chloro-2-(4-(3-(diméthylamino)propoxy)phényl)-1H-pyrrolo[2,3-b]pyridine-3-yl)indoline-1-yl)prop-2-ène-1-one;
N-(5-(4-chloro-2-(3-fluoro-4-(4-méthylpipérazine-1-yl)phényl)-1H-pyrrolo[2,3-b]pyridine-3-yl)-2-méthylphényl)acrylamide ;
1-(6-(4-chloro-2-(4-((2-(diméthylamino)éthyl)(méthyl)amino)phényl)-1H-pyrrolo[2,3-b]pyridine-3-yl)-2H-benzo[b][1,4]oxazine-4(3H)-yl)prop-2-ène-1-one;
1-(6-(4-chloro-2-(4-(4-isopropylpipérazine-1-yl)phényl)-1H-pyrrolo[2,3-b]pyridine-3-yl)-2H-benzo[b][1,4]oxazine-4(3H)-yl)prop-2-ène-1-one ;
N-(5-(4-chloro-2-(4-((2-(diméthylamino)éthyl)(méthyl)amino)phényl)-1H-pyrrolo[2,3-b]pyridine-3-yl)-2-méthylphényl)acrylamide ;
N-(5-(5-cyano-2-(4-(4-méthylpipérazine-1-yl)phényl)-1H-pyrrolo[2,3-b]pyridine-3-yl)-2-méthylphényl)acrylamide;
3-(3-acrylamido-4-méthylphényl)-N-isopropyl-2-(4-(4-méthylpipérazine-1-yl)phényl)-1H-pyrrolo[2,3-b]pyridine-5-carboxamide ;
1-(6-(4-chloro-2-(2-(4-méthylpipérazine-1-yl)pyridine-4-yl)-1H-pyrrolo[2,3-b]pyridine-3-yl)-2H-benzo[b][1,4]oxazin-4(3H)-yl)prop-2-ène-1-one ;
1-(6-(4-chloro-2-(4-(4-éthylpipérazine-1-yl)phényl)-1H-pyrrolo[2,3-b]pyridine-3-yl)-2H-benzo[b][1,4]oxazine-4(3H)-yl)prop-2-ène-1-one ;
1-(6-(4-chloro-2-(4-((4-méthylpipérazine-1-yl)méthyl)phényl)-1H-pyrrolo[2,3-b]pyridine-3-yl)-2H-benzo[b][1,4]oxazine-4(3H)-yl)prop-2-ène-1-one;
1-(6-(4-chloro-2-(4-(2-(pyrrolidine-1-yl)éthoxy)phényl)-1H-pyrrolo[2,3-b]pyridine-3-yl)-2H-benzo[b][1,4]oxazine-4(3H)-yl)prop-2-ène-1-one ;
1-(6-(4-chloro-2-(4-((diméthylamino)méthyl)phényl)-1H-pyrrolo[2,3-b]pyridine-3-yl)-2H-benzo[b][1,4]oxazine-4(3H)-yl)prop-2-ène-1-one ;
1-(6-(4-chloro-2-(3-(2-(pyrrolidine-1-yl)éthoxy)phényl)-1H-pyrrolo[2,3-b]pyridine-3-yl)-2H-benzo[b][1,4]oxazine-4(3H)-yl)prop-2-ène-1-one ;
3-(3-acrylamido-4-méthylphényl)-2-(4-(4-méthylpipérazine-1-yl)phényl)-1H-pyrrolo[2,3-b]pyridine-5-carboxylate d'isopropyle ;
1-(6-(4-éthoxy-2-(2-(4-méthylpipérazine-1-yl)pyridine-4-yl)-1H-pyrrolo[2,3-b]pyridine-3-yl)indoline-1-yl)prop-2-ène-1-one;
1-(6-(2-(4-((diméthylamino)méthyl)phényl)-4-éthoxy-1H-pyrrolo[2,3-b]pyridine-3-yl)indoline-1-yl)prop-2-ène-1-one;
1-(6-(4-éthoxy-2-(4-(2-(pyrrolidine-1-yl)éthoxy)phényl)-1H-pyrrolo[2,3-b]pyridine-3-yl)indoline-1-yl)prop-2-ène-1-one;
1-(6-(4-éthoxy-2-(4-((4-méthylpipérazine-1-yl)méthyl)phényl)-1H-pyrrolo[2,3-b]pyridine-3-yl)indoline-1-yl)prop-2-ène-1-one ;
1-(6-(4-éthoxy-2-(4-(4-méthylpipérazine-1-yl)phényl)-1H-pyrrolo[2,3-b]pyridine-3-yl)indoline-1-yl)prop-2-ène-1-one;
1-(6-(4-éthoxy-2-(3-(2-(pyrrolidine-1-yl)éthoxy)phényl)-1H-pyrrolo[2,3-b]pyridine-3-yl)indoline-1-yl)prop-2-ène-1-one;
3-(3-acrylamido-4-méthylphényl)-N-cyclohexyl-2-(4-(4-méthylpipérazine-1-yl)phényl)-1H-pyrrolo[2,3-b]pyridine-5-carboxamide ;
1-(6-(4-méthoxy-2-(4-(4-méthylpipérazine-1-yl)phényl)-1H-pyrrolo[2,3-b]pyridine-3-yl)indoline-1-yl)prop-2-ène-1-one;
1-(6-(4-méthoxy-2-(2-(4-méthylpipérazine-1-yl)pyridine-4-yl)-1H-pyrrolo[2,3-b]pyridine-3-yl)indoline-1-yl)prop-2-ène-1-one ;
1-(6-(4-méthoxy-2-(4-(2-(pyrrolidine-1-yl)éthoxy)phényl)-1H-pyrrolo[2,3-b]pyridine-3-yl)indoline-1-yl)prop-2-ène-1-one;
3-(3-acrylamido-4-méthylphényl)-N-benzyl-2-(4-(4-méthylpipérazine-1-yl)phényl)-1H-pyrrolo[2,3-b]pyridine-5-carboxamide;
N-(3-acétamidobenzyl)-3-(3-acrylamido-4-méthylphényl)-2-(4-(4-méthylpipérazine-1-yl)phényl)-1H-pyrrolo[2,3-b]pyridine-5-carboxamide;
1 -(6-(4-isopropoxy-2-(4-(4-méthylpipérazine-1-yl)phényl)-1H-pyrrolo[2,3-b]pyridine-3-yl)indoline-1-yl)prop-2-ène-1-one;
1-(6-(4-méthoxy-2-(4-((4-méthylpipérazine-1-yl)méthyl)phényl)-1H-pyrrolo[2,3-b]pyridine-3-yl)indoline-1-yl)prop-2-ène-1-one ;
1-(6-(4-méthoxy-2-(3-(2-(pyrrolidine-1-yl)éthoxy)phényl)-1H-pyrrolo[2,3-b]pyridine-3-yl)indoline-1-yl)prop-2-ène-1-one;
3-(3-acrylamido-4-méthylphényl)-N-(4-((4-méthylpipérazine-1-yl)méthyl)benzyl)-2-(4-(4-méthylpipérazine-1-yl)phényl)-1H-pyrrolo[2,3-b]pyridine-5-carboxamide;
3-(3-acrylamido-4-méthylphényl)-2-(4-(4-méthylpipérazine-1-yl)phényl)-N-(2-(pyridine-4-yl)éthyl)-1H-pyrrolo[2,3-b]pyridine-5-carboxamide ;
3-(3-acrylamido-4-méthylphényl)-N-(4-fluorophenéthyl)-2-(4-(4-méthylpipérazine-1-yl)phényl)-1H-pyrrolo[2,3-b]pyridine-5-carboxamide;
N-((1H-benzo[d]imidazole-2-yl)méthyl)-3-(3-acrylamido-4-méthylphényl)-2-(4-(4-méthylpipérazine-1-yl)phényl)-1H-pyrrolo[2,3-b]pyridine-5-carboxamide;
N-(2-méthyl-5-(2-(2-morpholinopyridine-4-yl)-1H-pyrrolo[2,3-b]pyridine-3-yl)phényl)acrylamide;
N-(2-méthyl-5-(2-(6-(4-méthylpipérazine-1-yl)pyridine-3-yl)-1H-pyrrolo[2,3-b]pyridine-3-yl)phényl)acrylamide;
3-(3-acrylamido-4-méthylphényl)-2-(4-(4-méthylpipérazine-1-yl)phényl)-N-(3-sulfamoylbenzyl)-1H-pyrrolo[2,3-b]pyridine-5-carboxamide;
3-(3-acrylamido-4-méthylphényl)-2-(4-(4-méthylpipérazine-1-yl)phényl)-N-(pyridine-4-ylméthyl)-1H-pyrrolo[2,3-b]pyridine-5-carboxamide ;
3-(3-acrylamido-4-méthylphényl)-2-(4-(4-méthylpipérazine-1-yl)phényl)-N-(pyridine-3-ylméthyl)-1H-pyrrolo[2,3-b]pyridine-5-carboxamide ;
3-(3-acrylamido-4-méthylphényl)-2-(4-(4-méthylpipérazine-1-yl)phényl)-N-((1-méthylpipéridine-4-yl)méthyl)-1H-pyrrolo[2,3-b]pyridine-5-carboxamide;
3-(3-acrylamido-4-méthylphényl)-N-((4-méthyl-4H-1,2,4-triazole-3-yl)méthyl)-2-(4-(4-méthylpipérazine-1-yl)phényl)-1H-pyrrolo[2,3-b]pyridine-5-carboxamide;
3-(3-acrylamido-4-méthylphényl)-N-(2,4-difluorobenzyl)-2-(4-(4-méthylpipérazine-1-yl)phényl)-1H-pyrrolo[2,3-b]pyridine-5-carboxamide;
N-(4-acétamidobenzyl)-3-(3-acrylamido-4-méthylphényl)-2-(4-(4-méthylpipérazine-1-yl)phényl)-1H-pyrrolo[2,3-b]pyridine-5-carboxamide;
3-(3-acrylamido-4-méthylphényl)-N-(3-carbamoylbenzyl)-2-(4-(4-méthylpipérazine-1-yl)phényl)-1H-pyrrolo[2,3-b]pyridine-5-carboxamide;
Acide 3-(3-acrylamido-4-méthylphényl)-2-(4-(4-méthylpipérazine-1-yl)phényl)-1H-pyrrolo[2,3-b]pyridine-5-carboxylique ;
2-chloro-N-(5-(4-chloro-2-(4-(4-méthylpipérazine-1-yl)phényl)-1H-pyrrolo[2,3-b]pyridine-3-yl)-2-méthylphényl)acétamide ;
(E)-N-(5-(4-chloro-2-(4-(4-méthylpipérazine-1-yl)phényl)-1H-pyrrolo[2,3-b]pyridine-3-yl)-2-méthylphényl)but-2-ènamide ;
N-(5-(4-chloro-2-(4-(4-méthylpipérazine-1-yl)phényl)-1H-pyrrolo[2,3-b]pyridine-3-yl)-2-méthylphényl)éthènesulfonamide ;
(E)-N-(5-(4-chloro-2-(4-(4-méthylpipérazine-1-yl)phényl)-1H-pyrrolo[2,3-b]pyridine-3-yl)-2-méthylphényl)-4-(diméthylamino)but-2-ènamide ;
3-(3-acrylamido-4-méthylphényl)-2-(4-(4-méthylpipérazine-1-yl)phényl)-1H-pyrrolo[2,3-b]pyridine-5-carboxylate de méthyle ;
3-(3-acrylamido-4-méthylphényl)-2-(4-(4-méthylpipérazine-1-yl)phényl)-1H-pyrrolo[2,3-b]pyridine-5-carboxylate d'éthyle ; cyclobutyl 3-(3-acrylamido-4-méthylphényl)-2-(4-(4-méthylpipérazine-1-yl)phényl)-1H-pyrrolo[2,3-b]pyridine-5-carboxylate ;
3-(3-acrylamido-4-méthylphényl)-2-(4-(4-méthylpipérazine-1-yl)phényl)-1H-pyrrolo[2,3-b]pyridine-5-carboxylate de cyclohexyle ;
3-(3-acrylamido-4-méthylphényl)-2-(4-(4-méthylpipérazine-1-yl)phényl)-1H-pyrrolo[2,3-b]pyridine-5-carboxylate de cyclopropyle ;
3-(3-acrylamido-4-méthylphényl)-2-(4-(4-méthylpipérazine-1-yl)phényl)-1H-pyrrolo[2,3-b]pyridine-5-carboxylate de cyclopentyle ;
2-méthoxyéthyl 3-(3-acrylamido-4-méthylphényl)-2-(4-(4-méthylpipérazine-1-yl)phényl)-1H-pyrrolo[2,3-b]pyridine-5-carboxylate ; N-(5-(4-chloro-2-(4-(4-méthylpipérazine-1-yl)phényl)-1H-pyrrolo[2,3-b]pyridine-3-yl)-2-méthylphényl)propiolamide; oxétane-3-yl 3-(3-acrylamido-4-méthylphényl)-2-(4-(4-méthylpipérazine-1-yl)phényl)-1H-pyrrolo[2,3-b]pyridine-5-carboxylate ;
(E)-N-(5-(4-chloro-2-(4-(4-méthylpipérazine-1-yl)phényl)-1H-pyrrolo[2,3-b]pyridine-3-yl)-2-méthylphényl)but-2-ènamide ;
(E)-N-(5-(4-chloro-2-(4-(4-méthylpipérazine-1-yl)phényl)-1H-pyrrolo[2,3-b]pyridine-3-yl)-2-méthylphényl)-4-(diméthylamino)but-2-ènamide ;
N-(5-(4-chloro-2-(4-(4-méthylpipérazine-1-yl)phényl)-1H-pyrrolo[2,3-b]pyridine-3-yl)-2-méthylphényl)-éthènesulfonamide ;
2-chloro-N-(5-(4-chloro-2-(4-(4-méthylpipérazine-1-yl)phényl)-1H-pyrrolo[2,3-b]pyridine-3-yl)-2-méthylphényl)acétamide ;
N-(5-(4-éthoxy-2-(4-(1-méthylpipéridine-4-yl)phényl)-1H-pyrrolo[2,3-b]pyridine-3-yl)-2-méthylphényl)acrylamide;
(E)-3-(3-(4-(diméthylamino)but-2-ènamido)-4-méthylphényl)-2-(4-(4-méthylpipérazine-1-yl)phényl)-1H-pyrrolo[2,3-b]pyridine-5-carboxylate d'isopropyle ;
3-(3-acrylamido-4-méthylphényl)-2-(4-(1-méthylpipéridine-4-yl)phényl)-1H-pyrrolo[2,3-b]pyridine-5-carboxylate d'isopropyle ;
N-(5-(4-chloro-2-(4-(4-méthylpipérazine-1-yl)phényl)-1H-pyrrolo[2,3-b]pyridine-3-yl)-2,3-diméthylphényl)acrylamide; 3-(3-acrylamido-4-méthylphényl)-2-(4-((diméthylamino)méthyl)phényl)-1H-pyrrolo[2,3-b]pyridine-5-carboxylate d'isopropyle ;
N-(5-(4-chloro-2-(4-(1-méthylpipéridine-4-yl)phényl)-1H-pyrrolo[2,3-b]pyridine-3-yl)-2,3-diméthylphényl)acrylamide;
Acide 3-(3-acrylamido-4-méthylphényl)-2-(4-((2-(diméthylamino)éthyl)(méthyl)amino)phényl)-1H-pyrrolo[2,3-b]pyridine-5-carboxylique;
3-(3-acrylamido-4-méthylphényl)-2-(4-((2-(diméthylamino)éthyl)(méthyl)amino)phényl)-1H-pyrrolo[2,3-b]pyridine-5-carboxylate d'isopropyle ;
N-(5-(5-chloro-4-méthyl-2-(4-(4-méthylpipérazine-1-yl)phényl)-1H-pyrrolo[2,3-b]pyridine-3-yl)-2-méthylphényl)acrylamide ;
3-(3-acrylamido-4-méthylphényl)-2-(2-(4-méthylpipérazine-1-yl)pyridine-4-yl)-1H-pyrrolo[2,3-b]pyridine-5-carboxylate d'isopropyle ;
3-(3-acrylamido-4-méthylphényl)-2-(4-(4-éthylpipérazine-1-yl)phényl)-1H-pyrrolo[2,3-b]pyridine-5-carboxylate d'isopropyle ;
3-(3-acrylamido-4-méthylphényl)-2-(4-(2-(pyrrolidine-1-yl)éthoxy)phényl)-1H-pyrrolo[2,3-b]pyridine-5-carboxylate d'isopropyle ;
3-(3-acrylamido-4-méthylphényl)-2-(4-(2-(diméthylamino)éthoxy)phényl)-1H-pyrrolo[2,3-b]pyridine-5-carboxylate d'isopropyle ; 3-(3-acrylamido-4-méthylphényl)-2-(4-((4-méthylpipérazine-1-yl)méthyl)phényl)-1H-pyrrolo[2,3-b]pyridine-5-carboxylate d'isopropyle ;
3-(3-acrylamido-4-méthylphényl)-2-(4-(2-(diméthylamino)éthyl)phényl)-1H-pyrrolo[2,3-b]pyridine-5-carboxylate d'isopropyle ; 2-(4-(4-acétylpipérazine-1-yl)phényl)-3-(3-acrylamido-4-méthylphényl)-1H-pyrrolo[2,3-b]pyridine-5-carboxylate d'isopropyle ;
3-(3-acrylamido-4-méthylphényl)-2-(6-(4-méthylpipérazine-1-yl)pyridine-3-yl)-1H-pyrrolo[2,3-b]pyridine-5-carboxylate d'isopropyle ;
3-(3-acrylamido-4-méthylphényl)-2-(3-fluoro-4-(4-méthylpipérazine-1-yl)phényl)-1H-pyrrolo[2,3-b]pyridine-5-carboxylate d'isopropyle ; 3-(3-acrylamido-5-fluoro-4-méthylphényl)-2-(4-(4-méthylpipérazine-1-yl)phényl)-1H-pyrrolo[2,3-b]pyridine-5-carboxylate d'isopropyle; 3-(3-acrylamido-4-méthylphényl)-2-(2-morpholinopyridine-4-yl)-1H-pyrrolo[2,3-b]pyridine-5-carboxylate d'isopropyle ;
3-(3-acrylamido-4-méthylphényl)-2-(4-((4-méthylpipérazine-1-yl)sulfonyl)phényl)-1H-pyrrolo[2,3-b]pyridine-5-carboxylate d'isopropyle ;
3-(3-acrylamido-4-méthylphényl)-2-(4-(2-hydroxyéthyl)phényl)-1H-pyrrolo[2,3-b]pyridine-5-carboxylate d'isopropyle ;
3-(3-acrylamido-4-méthylphényl)-2-(2-(4-méthylpipérazine-1-yl)thiazole-5-yl)-1H-pyrrolo[2,3-b]pyridine-5-carboxylate d'isopropyle ;
3-(3-acrylamido-4-méthylphényl)-2-(3-(2-(pyrrolidine-1-yl)éthoxy)phényl)-1H-pyrrolo[2,3-b]pyridine-5-carboxylate d'isopropyle ;
3-(3-acrylamido-4-méthylphényl)-2-(6-morpholinopyridine-3-yl)-1H-pyrrolo[2,3-b]pyridine-5-carboxylate d'isopropyle ;
3-(3-acrylamido-5-fluoro-4-méthylphényl)-2-(4-((2-(diméthylamino)éthyl)(méthyl)amino)phényl)-1H-pyrrolo[2,3-b]pyridine-5-carboxylate d'isopropyle ;
3-(3-acrylamido-4-méthylphényl)-2-(2-morpholinopyrimidine-5-yl)-1H-pyrrolo[2,3-b]pyridine-5-carboxylate d'isopropyle ;
3-(3-acrylamido-5-fluoro-4-méthylphényl)-2-(6-(4-méthylpipérazine-1-yl)pyridine-3-yl)-1H-pyrrolo[2,3-b]pyridine-5-carboxylate d'isopropyle ;
3-(3-acrylamido-4-méthylphényl)-2-(2-(4-méthylpipérazine-1-yl)pyrimidine-5-yl)-1H-pyrrolo[2,3-b]pyridine-5-carboxylate d'isopropyle ;
3-(3-acrylamido-4-méthylphényl)-2-(4-(4-isopropylpipérazine-1-yl)phényl)-1H-pyrrolo[2,3-b]pyridine-5-carboxylate d'isopropyle ;
3-(3-acrylamido-5-fluoro-4-méthylphényl)-2-(4-((diméthylamino)méthyl)phényl)-1H-pyrrolo[2,3-b]pyridine-5-carboxylate d'isopropyle ; 3-(3-acrylamido-4-méthylphényl)-2-(4-(2-hydroxyéthoxy)phényl)-1H-pyrrolo[2,3-b]pyridine-5-carboxylate d'isopropyle ;
3-(3-acrylamido-5-fluoro-4-méthylphényl)-2-(4-(1-méthylpipéridine-4-yl)phényl)-1H-pyrrolo[2,3-b]pyridine-5-carboxylate d'isopropyle ; 3-(3-acrylamido-5-fluoro-4-méthylphényl)-2-(2-(4-méthylpipérazine-1-yl)pyridine-4-yl)-1H-pyrrolo[2,3-b]pyridine-5-carboxylate d'isopropyle; 3-(3-acrylamido-4-méthylphényl)-2-(3-((4-méthylpipérazine-1-yl)méthyl)phényl)-1H-pyrrolo[2,3-b]pyridine-5-carboxylate d'isopropyle ;
3-(3-acrylamido-5-fluoro-4-méthylphényl)-2-(4-(2-(diméthylamino)éthoxy)phényl)-1H-pyrrolo[2,3-b]pyridine-5-carboxylate d'isopropyle ;
3-(3-acrylamido-4-méthylphényl)-2-(3-(3-(diméthylamino)propoxy)phényl)-1H-pyrrolo[2,3-b]pyridine-5-carboxylate d'isopropyle ; 3-(3-acrylamido-4-méthylphényl)-2-(4-(4-(éthylsulfonyl)pipérazine-1-yl)phényl)-1H-pyrrolo[2,3-b]pyridine-5-carboxylate d'isopropyle ;
2-(3-(4-acétylpipérazine-1-yl)phényl)-3-(3-acrylamido-4-méthylphényl)-1H-pyrrolo[2,3-b]pyridine-5-carboxylate d'isopropyle ;
2-(3-((4-acétylpipérazine-1-yl)méthyl)phényl)-3-(3-acrylamido-4-méthylphényl)-1H-pyrrolo[2,3-b]pyridine-5-carboxylate d'isopropyle ;
3-(3-acrylamido-4-méthylphényl)-2-(3-((diméthylamino)méthyl)phényl)-1H-pyrrolo[2,3-b]pyridine-5-carboxylate d'isopropyle ; N-(2-méthyl-5-(2-(4-(4-méthylpipérazine-1-yl)phényl)-5-(trifluorométhoxy)-1H-pyrrolo[2,3-b]pyridine-3-yl)phényl)acrylamide;
Acide 2-(3-(3-acrylamido-4-méthylphényl)-2-(4-(4-méthylpipérazine-1-yl)phényl)-1H-pyrrolo[2,3-b]pyridine-5-yl)acétique ;
2-(3-(3-acrylamido-4-méthylphényl)-2-(4-(4-méthylpipérazine-1-yl)phényl)-1H-pyrrolo[2,3-b]pyridine-5-yl)acétate d'isopropyle ; N-(5-(5-éthyl-2-(4-(4-méthylpipérazine-1-yl)phényl)-1H-pyrrolo[2,3-b]pyridine-3-yl)-2-méthylphényl)acrylamide;
3-(1-acryloylindoline-6-yl)-2-(4-(4-méthylpipérazine-1-yl)phényl)-1H-pyrrolo[2,3-b]pyridine-5-carboxylate d'isopropyle ;
N-(5-(5-éthoxy-2-(4-(4-méthylpipérazine-1-yl)phényl)-1H-pyrrolo[2,3-b]pyridine-3-yl)-2-méthylphényl)acrylamide;
N-(5-(5-(2-(diméthylamino)-2-oxoéthyl)-2-(4-(4-méthylpipérazine-1-yl)phényl)-1H-pyrrolo[2,3-b]pyridine-3-yl)-2-méthylphényl)acrylamide; méthyl 2-(3-(3-acrylamido-4-méthylphényl)-2-(4-(4-méthylpipérazine-1-yl)phényl)-1H-pyrrolo[2,3-b]pyridine-5-yl)acétate ;
N-(2-méthyl-5-(5-(2-(méthylamino)-2-oxoéthyl)-2-(4-(4-méthylpipérazine-1-yl)phényl)-1H-pyrrolo[2,3-b]pyridine-3-yl)phényl)acrylamide ;
Oxétane-3-yl 2-(3-(3-acrylamido-4-méthylphényl)-2-(4-(4-méthylpipérazine-1-yl)phényl)-1H-pyrrolo[2,3-b]pyridine-5-yl)acétate ;
3-(3-acrylamido-4-méthylphényl)-4-méthyl-2-(4-(4-méthylpipérazine-1-yl)phényl)-1H-pyrrolo[2,3-b]pyridine-5-carboxylate d'isopropyle ;
N-(5-(5-(hydroxyméthyl)-2-(4-(4-méthylpipérazine-1-yl)phényl)-1H-pyrrolo[2,3-b]pyridine-3-yl)-2-méthylphényl)acrylamide ;
N-(5-(5-isobutyramido-2-(4-(4-méthylpipérazine-1-yl)phényl)-1H-pyrrolo[2,3-b]pyridine-3-yl)-2-méthylphényl)acrylamide ;
N-(5-(5-acétamido-2-(4-(4-méthylpipérazine-1-yl)phényl)-1H-pyrrolo[2,3-b]pyridine-3-yl)-2-méthylphényl)acrylamide ;
3-(5-acrylamido-2,4-diméthylphényl)-2-(4-(4-méthylpipérazine-1-yl)phényl)-1H-pyrrolo[2,3-b]pyridine-5-carboxylate d'isopropyle ;
3-(3-(acrylamidométhyl)phényl)-2-(4-(4-méthylpipérazine-1-yl)phényl)-1H-pyrrolo[2,3-b]pyridine-5-carboxylate d'isopropyle ;
3-(3-acrylamido-4-méthylphényl)-2-(4-((2-(diméthylamino)éthyl)(méthyl)amino)phényl)-4-méthyl-1H-pyrrolo[2,3-b]pyridine-5-carboxylate d'isopropyle ;
3-(3-acrylamido-4-méthylphényl)-2-(4-(2-(diméthylamino)éthoxy)phényl)-4-méthyl-1H-pyrrolo[2,3-b]pyridine-5-carboxylate d'isopropyle ;
3-(3-acrylamido-4-éthylphényl)-2-(4-(4-méthylpipérazine-1-yl)phényl)-1H-pyrrolo[2,3-b]pyridine-5-carboxylate d'isopropyle ;
3-(3-acrylamido-2-méthylphényl)-2-(4-(4-méthylpipérazine-1-yl)phényl)-1H-pyrrolo[2,3-b]pyridine-5-carboxylate d'isopropyle ;
3-(3-acrylamido-2-méthylphényl)-2-(4-((2-(diméthylamino)éthyl)(méthyl)amino)phényl)-1H-pyrrolo[2,3-b]pyridine-5-carboxylate d'isopropyle et
3-(3-acrylamido-2-méthylphényl)-2-(4-(2-(diméthylamino)éthoxy)phényl)-1H-pyrrolo[2,3-b]pyridine-5-carboxylate d'isopropyle
ou un énantiomère, un mélange d'énantiomères, un diastéréomère, un mélange de diastéréomères, un tautomère, un hydrate, un solvate des composés susmentionnés, ou leurs sels pharmaceutiquement acceptables.

7. Un composé conforme à l'une des revendications 1 à 6 en tant qu'inhibiteur sélectif des mutations EGFR ou Her2 exon 20.

8. Un composé conforme à l'une des revendications 1 à 6, destiné à un usage en tant que médicament.

9. Un composé conforme à l'une des revendications 1 à 6 destiné à être utilisé dans le traitement du cancer ou dans le traitement d'un cancer ayant une mutation activatrice d'un récepteur appartenant à la famille de récepteurs ErbB.

10. Le composé destiné à une utilisation conformément à la revendication 9, où la mutation activatrice du récepteur est une insertion dans l'exon 20 du récepteur de facteur de croissance épidermique (EGFR) ou dans l'exon 20 du récepteur de facteur de croissance humain (HER), ou bien où la mutation activatrice du récepteur est sélectionnée parmi le groupe constitué de Her2 A775_G776insYVMA, EGFR D770_N771 insSVD, EGFR H773_V774insNPH, EGFR V769_D770insASV, EGFR P772_H773insPR, EGFR T790M et EGFR T790ML858R.

11. Le composé destiné à une utilisation conformément à la revendication 9, où le cancer est sélectionné parmi le groupe constitué du cancer du sein, du cancer du côlon, du cancer de la prostate, du cancer du poumon, du cancer de l'estomac, du cancer de l'ovaire, du cancer du rein, du cancer hépatocellulaire, du cancer de la thyroïde, du cancer de l'utérus, du cancer de l'œsophage, du cancer à cellule squameuse, de la leucémie, du lymphome, de l'ostéosarcome, du mélanome, du glioblastome et du neuroblastome.

12. Le composé destiné à une utilisation conformément à l'une des revendications 9 à 11, où le cancer est un cancer du poumon non à petite cellule ou un carcinome mammaire.

13. Un composé conforme aux revendications 1 à 12 en combinaison avec au moins un médicament contre le cancer destiné à une utilisation dans un traitement du cancer.

14. Une méthode permettant de produire un composé de la formule (**Ia-1**), comprenant : **Étape A1** : une réaction de couplage croisé d'un composé de pyridine 1* avec un composé d'alcyne **2a*** 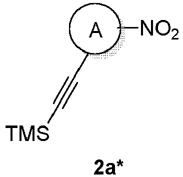 pour obtenir un composé **3*** 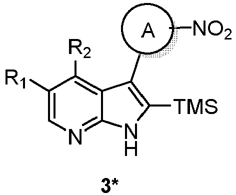 en présence d'un premier catalyseur au palladium et d'une première base :
**Étape B1** : la conversion d'un groupe triméthylsilyle du composé **3*** en halogénure comme un iodure pour obtenir un composé **4*** 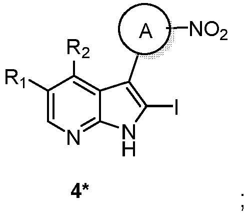
**Étape C1** : une deuxième réaction de couplage croisé de **4*** avec un composé **5*** 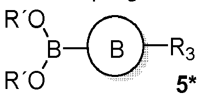 en présente d'un deuxième catalyseur au palladium et d'une deuxième base pour obtenir un composé **6*** 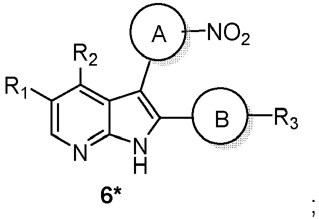
**Étape D1** : la réduction par un groupe nitro (NO₂) du composé **6*** en groupe amine primaire (NH₂) pour obtenir un composé **7*** ; et
**Étape E1** : une réaction de couplage du composé **7*** 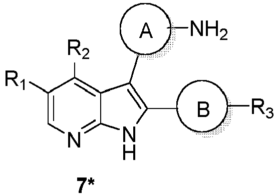 avec un composé HO-**R₅** ou AG-**R₅** pour obtenir un composé produit de la formule (**Ia-1**) 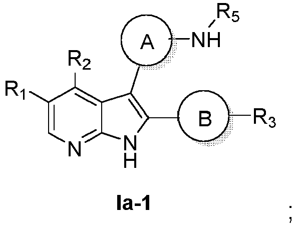 une méthode permettant de produire un composé de la formule (la-1), comprenant :
**Étape A1** : une première réaction de couplage croisé d'un composé de pyridine 1* avec un composé alcyne **2a*** 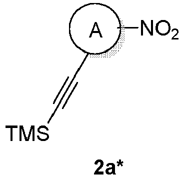 pour obtenir un composé **3*** 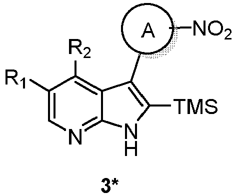 en présence d'un premier catalyseur au palladium et d'une première base :
**Étape D2** : la réduction par un groupe nitro (NO₂) du composé **3*** en un groupe amine primaire (NH₂) pour obtenir un composé **10*** 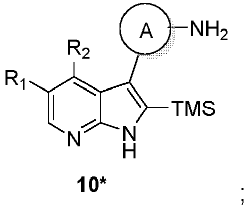
**Étape E2** : une réaction croisée du composé **10*** avec un composé HO-**R₅** ou AG-**R₅** pour obtenir un composé **11*** 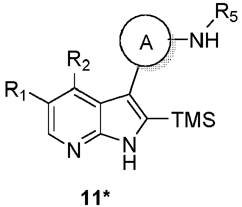
**Étape B2 :** la conversion d'un groupe triméthylsilyle du composé **11*** en halogénure comme un iodure pour obtenir un composé **12*** 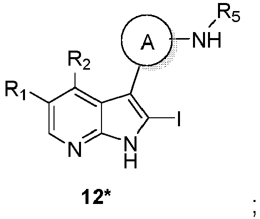 et
**Étape C2** : une deuxième réaction de couplage croisé du composé **12*** avec un composé **5*** en présence d'un deuxième catalyseur au palladium et d'une deuxième base pour obtenir un composé produit de la formule (**Ia-1**) 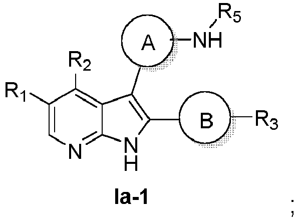 ou
une méthode permettant de produire un composé de la formule **(Ib**), comprenant :
**Étape A3** :
i) une première réaction de couplage croisé d'un composé de pyridine **1*** 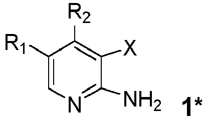 avec un composé alcyne **2b*** 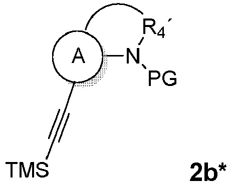 en présence d'un premier catalyseur au palladium et d'une première base ; et
ii) le retrait d'un groupe protecteur PG d'un composé obtenu après l'étape i) pour obtenir un composé **3b*** 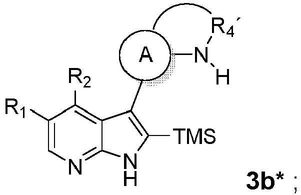
**Étape E3** : une réaction de couplage du composé **3b*** avec un composé HO-**R₅** ou AG-**R₅** pour obtenir un composé **11b*** 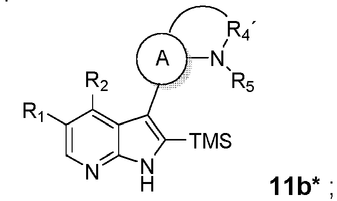
**Étape B3** : la conversion d'un groupe triméthylsilyle du composé **11b*** en un halogénure comme un iodure pour obtenir un composé **12b*** 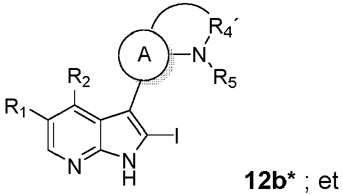
**Étape C3** : une deuxième réaction de couplage croisé du composé **12b*** avec un composé **5*** 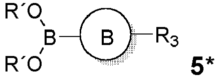 en présence d'un deuxième catalyseur au palladium et d'une deuxième base pour obtenir un composé de la formule (**Ib)** 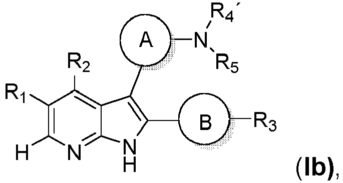
où A, B, R₁, R₂, R₃, R₄, R₄', R₅ et 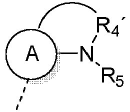 ont la signification définie dans la revendication 1.
X est un groupe partant et représente Cl, Br, I ou OTf ;
AG est un groupe activateur d'acide carboxylique ;
PG est un groupe protecteur d'amine ;
TMS est un groupe triméthylsilyle ;
R' est H ou une chaîne alkyle avec 1 à 10 atomes de carbone, une chaîne cycloalkyle avec 3 à 12 atomes de carbone ou les deux résidus R' représentent ensemble un résidu dérivé du pinacol.

15. Un composé intermédiaire sélectionné parmi le groupe constitué des composés **3***, **3b***, **4***, **7***, **10***, **11***, **11b***, **12*** et **12b*** : 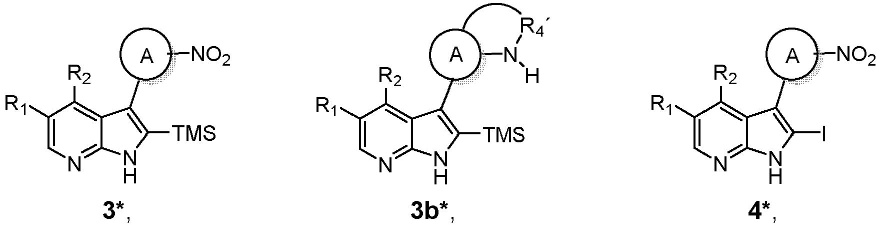 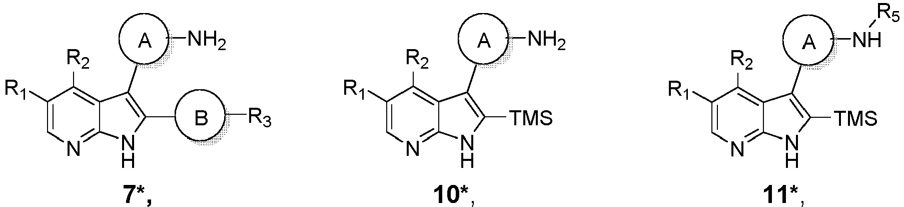 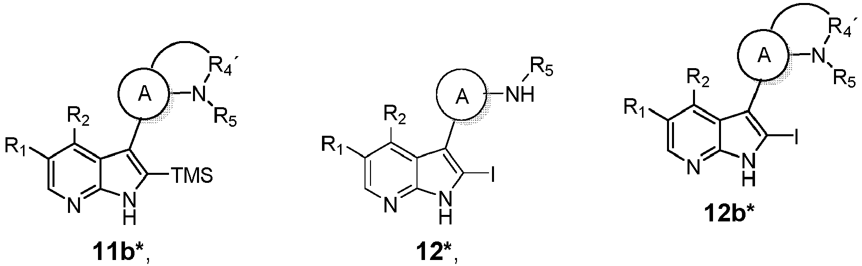 où A, B, R₁, R₂, R₃, R₄, R₄', R₅ et 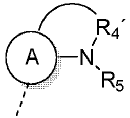 ont la même signification définie à la revendication 1 et TMS est un groupe triméthylsilyle.
